(19) **Europäisches Patentamt / European Patent Office / Office européen des brevets**

(11) **EP 4 722 230 A1**

(12) **EUROPEAN PATENT APPLICATION**
published in accordance with Art. 153(4) EPC

(43) Date of publication:
08.04.2026 Bulletin 2026/15

(21) Application number: 24814554.2

(22) Date of filing: 30.05.2024

(51) International Patent Classification (IPC):
$C07K\ 14/00^{(2006.01)}$    $C07K\ 19/00^{(2006.01)}$
$A61P\ 31/00^{(2006.01)}$    $A61K\ 38/00^{(2006.01)}$
$A61P\ 31/14^{(2006.01)}$    $A61K\ 39/39^{(2006.01)}$
$A61K\ 39/215^{(2006.01)}$

(52) Cooperative Patent Classification (CPC):
A61K 38/00; A61K 39/215; A61K 39/39;
A61P 31/00; A61P 31/14; C07K 14/00; C07K 19/00

(86) International application number:
PCT/CN2024/096427

(87) International publication number:
WO 2024/245358 (05.12.2024 Gazette 2024/49)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 30.05.2023 CN 202310663932
10.09.2023 CN 202311196430

(71) Applicant: SICHUAN CLOVER BIOPHARMACEUTICALS, INC.
Chengdu, Sichuan 610041 (CN)

(72) Inventors:
• LIANG, Peng
  Chengdu, Sichuan 610041 (CN)
• LIANG, Joshua
  Chengdu, Sichuan 610041 (CN)
• SU, Danmei
  Chengdu, Sichuan 610041 (CN)

(74) Representative: Kuttenkeuler, David
SKM-IP PartGmbB
Oberanger 45
80331 München (DE)

Remarks:
The complete document including Reference Table(s) and the Sequence Listing(s) can be downloaded from the EPO website

(54) **CORONAVIRUS VACCINE COMPOSITION, METHOD THEREFOR AND USE THEREOF**

(57) The present invention relates to an immunogenic composition comprising a recombinant peptide and protein, wherein the recombinant peptide and protein comprise a coronavirus antigen and immunogen, for example, a chimeric antigen and immunogen of an S protein peptide or a fragment, variant or mutant sequence thereof of SARS-CoV-2 Hu-1, SARS-CoV-2 Omicron (BA.5 and/or XBB.1.5) variant, and/or other variants. The immunogenic composition comprises a secreted fusion protein, which comprises a soluble coronavirus antigen, wherein the soluble coronavirus antigen protein is linked, by means of in-frame fusion, to a C-terminal moiety of a collagen capable of self-trimerization to form a disulfide bond-linked trimeric fusion protein. The immunogenic composition can be used for generating an immune response, and can be used in a vaccine composition. Further provided are methods for producing a recombinant peptide and protein, methods for prevention, treatment and/or diagnosis, and a related kit.

EP 4 722 230 A1

**Description**

The present application claims priority to the China Invention Patent Application No. 202310663932.3 filed on May 30, 2023, entitled "CORONAVIRUS VACCINE COMPOSITION, METHOD THEREFOR AND USE THEREOF" and the China Invention Patent Application No. 202311196430.0, entitled "CORONAVIRUS VACCINE COMPOSITION, METHOD THEREFOR AND USE THEREOF" filed on September 10, 2023, which are incorporated herein by reference in their entireties.

**TECHNICAL FIELD**

The present disclosure, in some aspects, relates to an immunogenic composition for treating and/or preventing coronavirus infection. The immunogenic composition includes recombinant peptides and proteins, the recombinant peptides and proteins including coronavirus antigens and immunogens, such as coronavirus S protein peptides, including subunit vaccines formed by disulfide bonds between trimerized™ tag polypeptides based on SARS-CoV-2 Hu-1, Omicron strain subtype BA.5 and XBB.1.5 S protein peptides and/or combinations thereof.

**BACKGROUND**

A coronavirus infects a wide range of birds and mammals, including humans. It may circulate annually in the human body, usually causing mild respiratory illness, although it is more severe in infants, the elderly, and the immunocompromised population. However, some coronaviruses, including Middle East respiratory syndrome coronavirus (MERS-CoV), severe acute respiratory syndrome coronavirus (SARS-CoV-1), and severe acute respiratory syndrome coronavirus 2 (SARS-CoV-2), are highly pathogenic. High pathogenicity, airborne transmission, high mortality, and ambiguous epidemiology of a coronavirus make an urgent need for an effective vaccine and a related therapeutic agent. In particular, there is an urgent need for a vaccine that can rapidly induce an effective immune response against SARS-CoV-2. The present disclosure provides a method, use, and article of manufacture that meet the above and other needs.

**SUMMARY**

Starting in late 2020, the emergence and spread of multiple variant SARS-CoV-2 strains carrying mutations that may lead to immune escape necessitate a rapid evaluation of a second-generation vaccine, with the goal of inducing an optimized immune response with broad protection.

Despite the tremendous progress and unprecedented speed of COVID-19 vaccine development, in April 2021, 16 months after the first outbreak of the SARS-CoV-2 virus, COVID-19 daily cases set a new record.

Most worryingly, while COVID-19 cases are surging worldwide, a plurality of new SARS-CoV-2 variants of concern (VOCs) have emerged since the end of 2020. These VOCs appear to be associated with mutations in the spike (S) protein, which may increase viral transmission rates and/or result in immune escape caused by the first wave of COVID-19 vaccination based on a SARS-CoV-2 Hu-1 strain. The emergence and spread of a B.1.1.7 variant in the United Kingdom (UK), a B.1.351 variant in South Africa, and a P.1 variant in Brazil lead to their classification as VOCs. These VOCs all include the N501Y mutation in the receptor binding domain (RBD) of the S protein, which is reported to increase transmission by 40% to 70%. The B.1.351 variant and P.1 variant have two additional RBD mutations, E484K and K417, which may allow immune escape from an antibody induced by a Hu-1 vaccine and an antibody induced by natural infection.

A randomized controlled clinical trial of a COVID-19 vaccine shows that the vaccine is less effective against a VOC compared to the SARS-CoV-2 Hu-1 strain. An adjuvant protein-based COVID-19 vaccine NVX-CoV2373 shows an efficacy of 89% in the UK (B.1.1.7 predominates), but only an efficacy of 49% in South Africa (B.1.351 predominates). An adenovirus vector COVID-19 vaccine ChAdOx1 only has an efficacy of 10% against the B.1.351 variant. A Pfizer vaccine recipient shows an efficacy of 75% against the B.1.351 variant and 95% against Hu-1. Coronavac is an inactivated vaccine based on the Hu-1 strain, and no neutralizing antibody titer against P.1 is detected in a subject vaccinated with the vaccine.

Though there is some encouraging evidence that the Hu-1 COVID-19 vaccine may prevent serious illness and death caused by a VOC, the vaccine is less effective against any COVID-19 disease with increased transmission rates. The rapid global spread of a SARS-CoV-2 VOC may lead to the continued emergence of a new target variant or VOC that may contain a new escape mutation, such as the Indian variant (B.1.617), which coincided with a massive surge in COVID-19 cases in the spring of 2021 and has been declared a new VOC by the World Health Organization. The B.1.617.2 variant of this lineage is named Delta, and the B.1.1.529 variant, first discovered in South Africa, is renamed Omicron, and Omicron subtypes BA.1, BA.2, BA.2.75, BA.5, BQ.1.1, BF.7, XBB, and XBB.1.5.

In these cases, re-booster dose strategies must be rapidly evaluated to enhance a neutralizing antibody response to a VOC and develop a new generation of monovalent and multivalent COVID-19 vaccines that may provide optimized broad protection and cross-neutralizing properties.

In some embodiments, the present disclosure discloses a protein including a plurality of recombinant polypeptides, each of which includes a coronavirus surface antigen linked to a C-terminal propeptide of collagen, in which the C-terminal propeptide of the recombinant polypeptide forms an inter-polypeptide disulfide bond. The recombinant polypeptide or protein can be used as an immunogen, such as a vaccine.

In some embodiments, the present disclosure discloses a recombinant subunit vaccine including an extracellular domain (for example, without a transmembrane domain and a cytoplasmic domain) of a subtype XBB.1.5 S protein or fragment thereof from a coronavirus such as SARS-CoV-2 Omicron (B.1.1.529). The extracellular domain is fused in-frame to a C-propeptide of collagen capable of forming a disulfide bond-connected homotrimer. The resulting recombinant subunit vaccine (for example, S-trimer) can be expressed and purified from transfected cells and is expected to be a native conformation of the trimer form. This solves the problem of viral antigen misfolding that is often encountered when a viral antigen is expressed in a soluble form as a recombinant peptide or protein without a transmembrane domain and/or a cytoplasmic domain. Such a misfolded viral antigen cannot faithfully maintain the native viral antigen conformation and often fails to produce a neutralizing antibody.

In some embodiments, the coronavirus is a severe acute respiratory syndrome (SARS) coronavirus (SARS-CoV-1), SARS coronavirus 2 (SARS-CoV-2), SARS-like coronavirus, Middle East respiratory syndrome (MERS) coronavirus (MERS-CoV), MERS-like coronavirus, NL63-CoV, 229E-CoV, OC43-CoV, HKU1-CoV, WIV1-CoV, MHV, HKU9-CoV, PEDV-CoV, or SDCV.

In some embodiments, the present disclosure discloses a multivalent vaccine including a coronavirus (S) protein surface antigen or a fragment, variant or mutant thereof, in which the coronavirus (S) protein surface antigen or the fragment, variant or mutant thereof is selected from soluble SARS-CoV-2 Hu-1, SARS-CoV-2 Omicron (B.1.1.529) and subtypes thereof, Alpha, Beta, Gamma, Delta, and Mu spike (S) protein surface antigens or fragments, variants or mutants thereof. The coronavirus (S) protein surface antigen or the fragment, variant or mutant is connected to a C-terminal propeptide portion of collagen to form a disulfide bond-connected trimeric fusion protein.

The multivalent vaccine described in any of the above embodiments, in which the SARS-CoV-2 Omicron subtype spike (S) protein surface antigen or the fragment, variant or mutant thereof includes BA.1, BA.2, BA.2.75, BA.5, BQ.1.1, BF.7, XBB, XBB.1.5, BQ.1.1 spike (S) protein surface antigens or fragments, variants or mutants thereof.

The multivalent vaccine described in any of the above embodiments includes the soluble SARS-CoV-2 Hu-1 (S) protein surface antigen or the fragment, variant or mutant thereof.

The multivalent vaccine described in any of the above embodiments includes the soluble SARS-CoV-2 Omicron BA.5 (S) protein surface antigen or the fragment, variant or mutant thereof.

The multivalent vaccine described in any of the above embodiments includes the soluble SARS-CoV-2 Omicron XBB.1.5 (S) protein surface antigen or the fragment, variant or mutant thereof.

The multivalent vaccine described in any of the above embodiments is a trivalent vaccine and includes the soluble SARS-CoV-2 Hu-1, the Omicron strains BA.5 and XBB.1.5 spike (S) protein surface antigens or the fragments, variants or mutants thereof in a weight ratio of 0.5-4:0.5-4:0.5-4.

The multivalent vaccine described in any of the above embodiments is a trivalent vaccine and includes the soluble SARS-CoV-2 Hu-1, the Omicron strains BA.5 and XBB.1.5 spike (S) protein surface antigen or fragments, variants or mutants thereof in a weight ratio of 1:1:1 or 1:2:2.

The multivalent vaccine described in any of the above embodiments, in which the coronavirus (S) protein surface antigen or the fragment, variant or mutant thereof includes a sequence set forth in any one of SEQ ID NOs: 27-66, or an amino acid sequence having at least or about 80%, 85%, 90%, 92%, 95%, 97%, or 99% sequence identity with the sequence set forth in any one of SEQ ID NOs: 27-66.

The multivalent vaccine described in any of the above embodiments, in which the coronavirus (S) protein surface antigen or the fragment, variant or mutant thereof includes a sequence set forth in any one of SEQ ID NOs: 81-84, or an amino acid sequence having at least or about 80%, 85%, 90%, 92%, 95%, 97%, or 99% sequence identity with the sequence set forth in any one of SEQ ID NOs: 81-84.

The multivalent vaccine described in any of the above embodiments, in which the coronavirus (S) protein surface antigen or the fragment, variant or mutant thereof includes a sequence set forth in SEQ ID NO: 126, or an amino acid sequence having at least or about 80%, 85%, 90%, 92%, 95%, 97%, or 99% sequence identity with the sequence set forth in SEQ ID NO: 126.

The multivalent vaccine described in any of the above embodiments, in which the coronavirus (S) protein surface antigen or the fragment, variant or mutant thereof includes a sequence set forth in SEQ ID NO: 122, or an amino acid sequence having at least or about 80%, 85%, 90%, 92%, 95%, 97%, or 99% sequence identity with the sequence set forth in SEQ ID NO: 122.

The multivalent vaccine in some embodiments is a bivalent vaccine and may be selected from SARS-CoV-2 Hu-1, Alpha, Beta, Gamma, Delta, Mu spike, Omicron (B.1.1.529), BA.1, BA.2, BA.2.75, BA.5, BQ.1.1, BF.7, XBB, XBB.1.5, BQ.1.1 spike (S) protein surface antigens or fragments, variants or mutants thereof, in which the bivalent vaccine includes two coronavirus (S) protein surface antigens or the fragments, variants or mutants thereof in a weight ratio of 0.5-4:0.5-4.

The bivalent vaccine described in the above embodiment includes the BA.5 and the XBB.1.5 in a weight ratio of 1:1 or 1:2; or the bivalent vaccine includes the Hu-1 and the BA.5 in a weight ratio of 1:1 or 1:2; or the bivalent vaccine includes the Hu-1 and the XBB.1.5 in a weight ratio of 1:1 or 1:2.

The multivalent vaccine described in any of the above embodiments, in which the multivalent vaccine is administered without an adjuvant and optionally is used as a primary series, an additional dose, and/or a homologous or heterologous booster dose, such as a first dose, a second dose, a third dose, a fourth dose, and/or more doses, and optionally the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines.

In some embodiments, the multivalent vaccine is administered together with one or more adjuvants, and optionally is used as a primary series, an additional dose and/or a homologous or heterologous booster dose, such as a first dose, a second dose, a third dose, a fourth dose and/or more doses, and optionally the primary series, the additional dose or the heterologous booster dose is used in combination with any one or more of other recombinant subunit vaccines, nanoparticle vaccines, mRNA vaccines, DNA vaccines, adenovirus vector vaccines, and inactivated virus vaccines.

The multivalent vaccine described in any of the above embodiments, in which the adjuvant in the primary series, the additional dose, and/or the homologous or heterologous booster dose may independently include: an aluminum-containing adjuvant, such as the adjuvant containing alum and/or aluminum hydroxide; an oligonucleotide-containing adjuvant, such as the adjuvant containing CpG oligodeoxynucleotide (CpG-ODN); a TLR9 agonist-containing adjuvant; an adjuvant containing metabolizable oil, α-tocopherol, and/or polyoxyethylene sorbitan monooleate (Tween-80), such as the adjuvant in the form of an oil-in-water emulsion containing squalene, α-tocopherol, and Tween-80 and/or Span 85; or any combination of the adjuvants.

The multivalent vaccine described in any of the above embodiments, in which the adjuvant in the primary series, additional dose, and/or homologous or heterologous booster dose may include the adjuvant containing CpG oligodeoxynucleotide (CpG-ODN) and/or the adjuvant containing alum and/or aluminum hydroxide.

The multivalent vaccine described in any of the above embodiments is administered by intramuscular injection.

The multivalent vaccine described in any of the above embodiments is administered by intranasal spray.

The multivalent vaccine described in any of the above embodiments is administered in a single dose or in a series of doses spaced weekly or monthly.

The multivalent vaccine described in any of the above embodiments can prevent or treat coronavirus infection, such as severe acute respiratory syndrome (SARS)-coronavirus 2 (SARS-CoV-2) infection.

The multivalent vaccine described in any of the above embodiments, in which the C-terminal propeptide includes any one of SEQ ID NOs: 67-80 or an amino acid sequence having at least 80%, 85%, 90%, 92%, 95%, 97%, or 99% identity thereto.

The present disclosure further provides an immunogen that includes the protein provided herein. The present disclosure provides a protein nanoparticle that includes the protein provided herein that is directly or indirectly linked to the nanoparticle. The present disclosure provides a virus-like particle (VLP) that includes the protein provided herein.

The present disclosure further provides an isolated nucleic acid encoding one, two, three, or more recombinant polypeptides of the protein provided herein. In some embodiments, the polypeptide encoding the S protein peptide is fused in-frame to the polypeptide encoding the C-terminal propeptide of collagen. In some embodiments, the isolated nucleic acid provided herein is operably linked to a promoter.

In some embodiments, the isolated nucleic acid provided herein is a DNA molecule. In some embodiments, the isolated nucleic acid provided herein is an RNA molecule, optionally an mRNA molecule, such as a nucleoside-modified mRNA, a non-amplifying mRNA, a self-amplifying mRNA, or a trans-amplifying mRNA.

The present disclosure further provides a vector that includes the isolated nucleic acid provided herein. In some embodiments, the vector is a viral vector.

In some aspects, the present disclosure provides a virus, pseudovirus, or cell that includes the vector provided herein. Optionally, the virus or cell has a recombinant genome. In some aspects, the present disclosure provides an immunogenic composition that includes the protein, immunogen, protein nanoparticle, VLP, isolated nucleic acid, vector, virus, pseudovirus, or cell provided herein and a pharmaceutically acceptable carrier.

The present disclosure further provides a vaccine that includes the immunogenic composition and the optional adjuvant provided herein, in which the vaccine is optionally a subunit vaccine. In some embodiments, the vaccine is a prophylactic and/or therapeutic vaccine. The optional adjuvant can be used in the primary series and/or the booster dose. Independently, the adjuvant of the primary series and/or any one or more booster doses may include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant;

an adjuvant containing metabolizable oil, α-tocopherol, and/or polyoxyethylene sorbitan monooleate (Tween-80), such as the adjuvant in the form of an oil-in-water emulsion containing squalene, α-tocopherol, and Tween-80 and/or Span 85; or any combination of the adjuvants.

In some aspects, the present disclosure provides a method for producing a protein, including: expressing the isolated nucleic acid or vector provided herein in a host cell to produce the protein provided herein; and purifying the protein. The present disclosure provides a protein produced by the method provided herein.

The present disclosure provides a method for generating an immune response to a coronavirus S protein peptide or a fragment or epitope thereof in a subject, including administering to the subject an effective amount of the protein, immunogen, protein nanoparticle, VLP, isolated nucleic acid, vector, virus, pseudovirus, cell, immunogenic composition or vaccine as provided herein to generate an immune response. In some embodiments, the method provided herein is used to treat or prevent coronavirus infection. In some embodiments, generating the immune response inhibits or reduces coronavirus replication in the subject. In some embodiments, the immune response includes a cell-mediated response and/or a humoral response, optionally including the production of one or more neutralizing antibodies, such as polyclonal antibodies or monoclonal antibodies. In some embodiments, the immune response targets the coronavirus S protein peptide or the fragment or epitope thereof, but not the C-terminal propeptide. In some embodiments, administration to a subject does not result in antibody-dependent enhancement (ADE) in a subject due to prior exposure to one or more coronaviruses. In some embodiments, administration does not result in antibody-dependent enhancement (ADE) in a subject upon subsequent exposure to one or more coronaviruses. In some embodiments, the method further includes a priming step and/or a boosting step. In some embodiments, a dosing step is performed by local, percutaneous, subcutaneous, intradermal, oral, intranasal (such as intranasal spray), intratracheal, sublingual, buccal, rectal, vaginal, inhalation, intravenous (such as intravenous injection), intraarterial, intramuscular (such as intramuscular injection), intracardiac, intraosseous, intraperitoneal, transmucosal, intravitreal, subretinal, intra-articular, periarticular, topical, or epicutaneous administration. In some embodiments, an effective amount is administered in a single dose or a series of doses with one or more intervals. In some embodiments, the effective amount is administered without the use of the adjuvant. In some embodiments, the effective amount is administered together with the adjuvant.

The present disclosure provides a method that includes administering an effective amount of the protein provided herein to a subject to produce a neutralizing antibody or neutralizing antiserum against a coronavirus in the subject. In some embodiments, the subject is a mammal, optionally a human or non-human primate. In some embodiments, the method further includes separating the neutralizing antibody or neutralizing antiserum from the subject. In some embodiments, the method further includes administering an effective amount of the separated neutralizing antibodies or neutralizing antiserum to a human subject through passive immunization to prevent or treat coronavirus infection. In some embodiments, the neutralizing antibody or neutralizing antiserum against a coronavirus includes a polyclonal antibody against the coronavirus S protein peptide or the fragment or epitope thereof. Optionally, the neutralizing antibody or neutralizing antiserum does not contain or is basically free of an antibody against the C-terminal propeptide of collagen. In some embodiments, the neutralizing antibody includes a monoclonal antibody against the coronavirus S protein peptide or the fragment or epitope thereof. Optionally, the neutralizing antibody does not contain or is basically free of an antibody against the C-terminal propeptide of collagen.

In some aspects, the protein, immunogen, protein nanoparticle, VLP, isolated nucleic acid, vector, virus, pseudovirus, cell, immunogenic composition, or vaccine provided herein is used to induce an immune response to a coronavirus in a subject, and/or to treat or prevent coronavirus infection.

In some aspects, the present disclosure provides the use of the protein, immunogen, protein nanoparticle, VLP, isolated nucleic acid, vector, virus, pseudovirus, cell, immunogenic composition, or vaccine provided herein for inducing an immune response to a coronavirus in a subject and/or for treating or preventing coronavirus infection. In some aspects, the present disclosure provides the use of the protein, immunogen, protein nanoparticle, VLP, isolated nucleic acid, vector, virus, pseudovirus, cell, immunogenic composition, or vaccine provided herein for manufacturing a drug or preventive agent to induce an immune response to a coronavirus in a subject, and/or to treat or prevent coronavirus infection.

The present disclosure also provides a method for analyzing a sample, including: contacting the sample with the protein provided herein and detecting the binding between the protein and an analyte that can specifically bind to the coronavirus S protein peptide or the fragment or epitope thereof. In some embodiments, the analyte is an antibody, receptor, or cell that recognizes the S protein peptide or the fragment or epitope thereof. In some embodiments, the binding indicates the presence of the analyte in the sample and/or the presence of coronavirus infection in the subject from which the sample originated.

The present disclosure provides a kit, which includes the protein provided herein and a matrix, pad, or vial containing or fixing the protein. Optionally, the kit is an ELISA or a lateral flow assay kit.

## BRIEF DESCRIPTION OF DRAWINGS

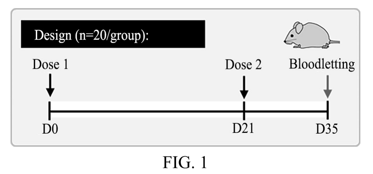

FIG. 1 is a schematic diagram of the study design of a cross-neutralizing immunogenicity based on pseudovirus neutralizing titers in mice using two doses of monovalent, bivalent, or trivalent S-trimeric antigens (a first dose primary immunization and a second dose homologous booster immunization). Mice were immunized twice with S-trimer (n = 20/group) formulated with CpG 1018 plus Alum as an adjuvant on day 0 (primary series) and day 21 (booster dose). In the Hu-1 homologous booster group, both the primary series and the booster dose are Hu-1 S-trimer; in the Omicron Ba.5 or XBB.1.5 homologous booster groups, both the primary series and the booster dose are Omicron S-trimer Ba.5 or XBB.1.5, respectively. In the adjuvant, CpG 1018 is 150 $\mu$g, and Alum is 75 $\mu$g. The total amount of S-trimeric antigen per dose is 3 $\mu$g. Intramuscular injection (single point), 0.05 mL/animal/time. After the injection, the animal was observed for conditions and placed back in the cage to move freely. On D0 (before immunization), blood was collected from the orbital venous plexus using a capillary glass blood collection tube, with a volume of approximately 200 $\mu$L. Mice were euthanized on D35, and all their blood was collected. All blood samples obtained were allowed to stand at room temperature for 0.5-1 h and then centrifuged ($\times$ 2500 g) for 10 min to separate the serum and package the serum in aliquots, with the storage condition of cryopreservation at $\leq$ -20°C. The neutralizing antibody titers against SARS-CoV-2 pseudoviruses are tested in D0 serum and D35 serum.

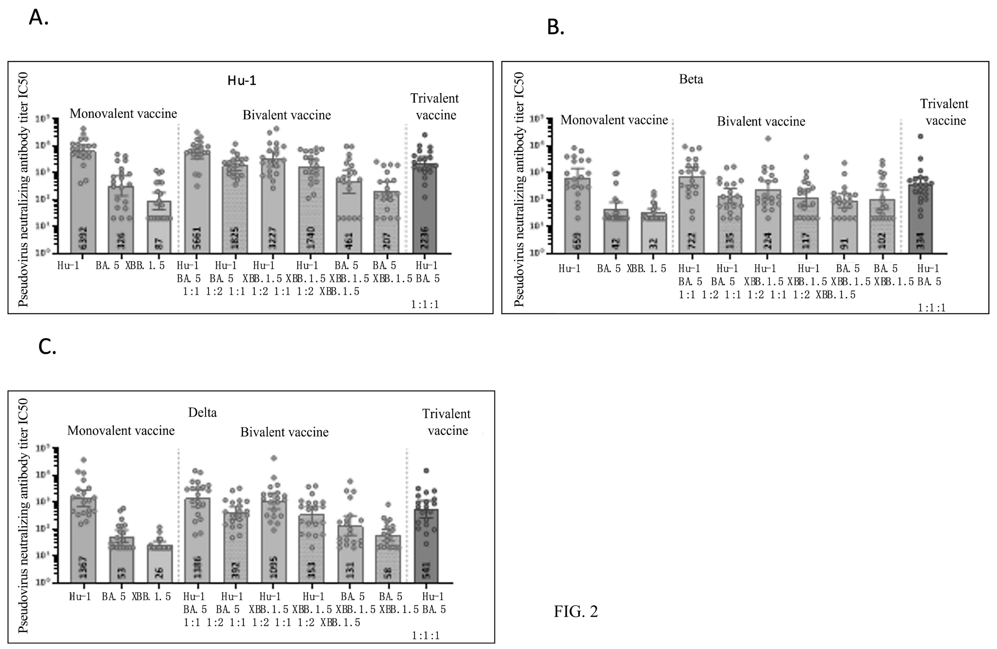

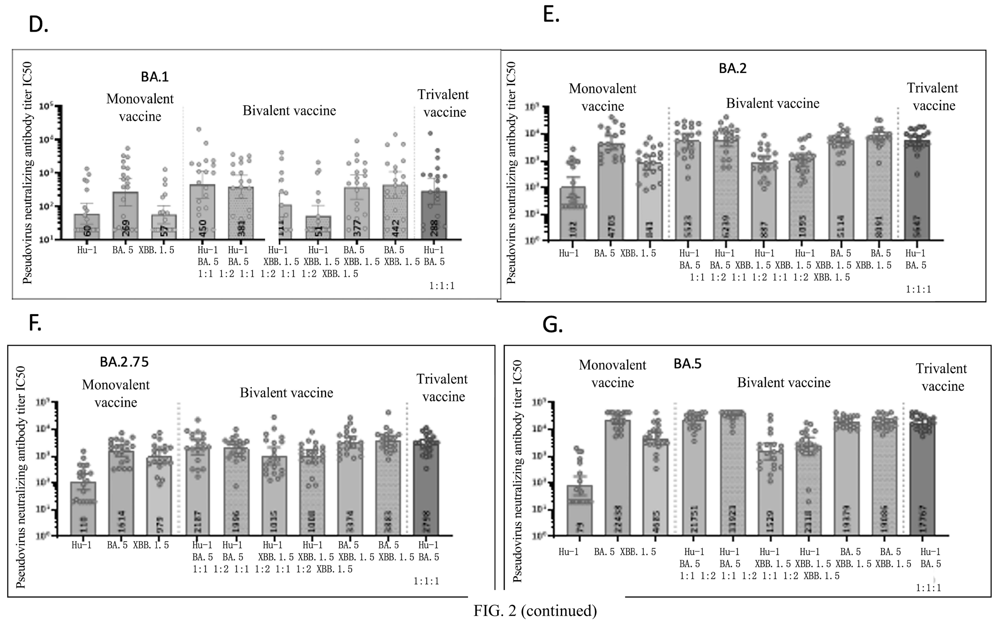

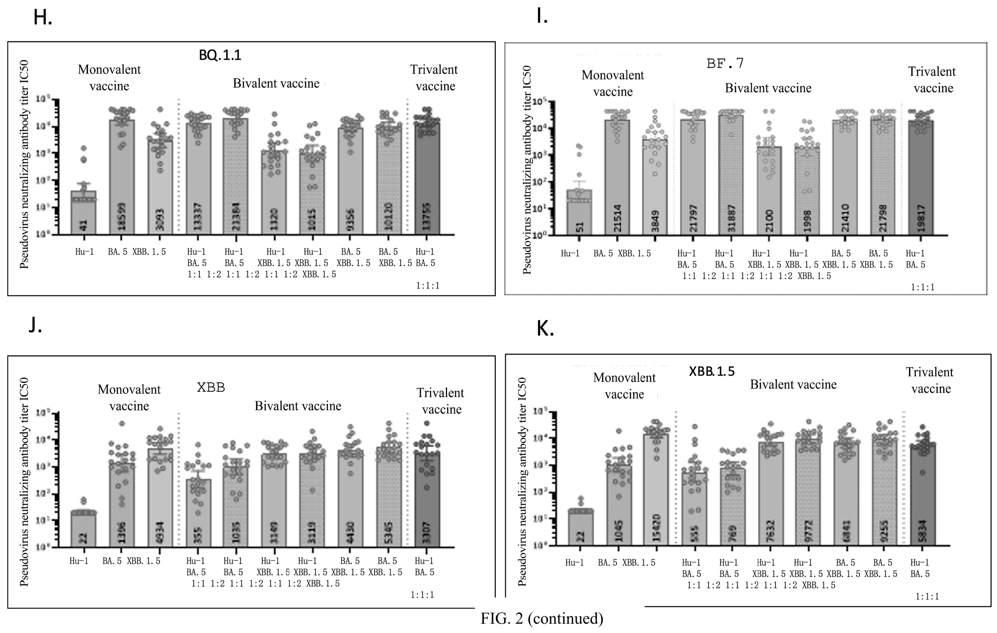

FIG. 2 shows the $IC_{50}$ data of neutralizing antibodies against pseudoviruses of strains SARS-CoV-2 Hu-1, Beta (beta, $\beta$, B.1.351), Delta (delta, $\delta$, B.1.617.2), and Omicron (Omicron, o, B.1.1.529) subtypes BA.1, BA.2, BA.2.75, BA.5, BQ.1.1, BF.7, XBB, XBB.1.5 in mice after the mice were immunized with two doses of S-trimeric antigen (a first dose primary immunization and a second dose homologous booster immunization) according to the study design in FIG. 1 and the vaccine dosage in Table 2. After two immunizations with 150 $\mu$g CpG 1018 plus 75 $\mu$g Alum as an adjuvant, blood was collected from mice on day 35 (D4 PD2) for a pseudovirus neutralizing antibody assay. A. shows the $IC_{50}$ data of the neutralizing antibodies against pseudoviruses of the Hu-1 strain, and the GMTs of neutralizing antibody titers ($IC_{50}$) against Hu-1 ((Prototype)) SARS-CoV-2 pseudoviruses in D35 serum from the immunized mice in groups 1-10 (n=20) were 6392, 326, 87, 5661, 1825, 3227, 1740, 461, 207, and 2236, respectively; B. shows the $IC_{50}$ data of the neutralizing antibodies against pseudoviruses of the Beta strain, and the GMTs of neutralizing antibody titers ($IC_{50}$) against SARS-CoV-2 pseudoviruses (Beta) in D35 serum from the immunized mice in groups 1-10 (n=20) were 659, 42, 32, 722, 135, 224, 117, 91, 102, and 334, respectively; C. shows the $IC_{50}$ data of the neutralizing antibodies against pseudoviruses of the Delta strain, and the GMTs of neutralizing antibody titers ($IC_{50}$) against SARS-CoV-2 pseudoviruses (Delta) in D35 serum from the immunized mice in groups 1-10 (n=20) were 1367, 53, 26, 1386, 392, 1095, 353, 131, 58, and 541, respectively; D. shows the $IC_{50}$ data of the neutralizing antibodies against pseudoviruses of the BA.1 strain, and the GMTs of neutralizing antibody titers ($IC_{50}$) against SARS-CoV-2 pseudoviruses (BA.1) in D35 serum from the immunized mice in groups 1-10 (n=20) were 60, 269, 57, 450, 381, 111, 51, 377, 442, and 288, respectively; E. shows the $IC_{50}$ data of the neutralizing antibodies against pseudoviruses of the BA.2 strain, and the GMTs of neutralizing antibody titers ($IC_{50}$) against SARS-CoV-2 pseudoviruses (BA.2) in D35 serum from the immunized mice in groups 1-10 (n=20) were 102, 4703, 843, 5523, 6239, 887, 1093, 5114, 8091, and 5647, respectively; F. shows the $IC_{50}$ data of the neutralizing antibodies against pseudoviruses of the BA.2.75 strain, and the DUGMTs of neutralizing antibody titers ($IC_{50}$) against SARS-CoV-2 pseudoviruses (BA.2.75) in D35 serum from the immunized mice in groups 1-10 (n=20) were 110, 1614, 979, 2187, 1996, 1035, 1008, 3374, 3883, and 2798, respectively; G. shows the $IC_{50}$ data of the neutralizing antibodies against pseudoviruses of the BA.5 strain, and the GMTs of neutralizing antibody titers ($IC_{50}$) against SARS-CoV-2 pseudoviruses (BA.5) in D35 serum from the immunized mice in groups 1-10 (n=20) were 79, 22438, 4685, 27151, 33923, 1529, 2318, 19379, 19086, and 17767, respectively; H. shows the $IC_{50}$ data of the neutralizing antibodies against pseudoviruses of the BQ.1.1 strain, and the GMTs of neutralizing antibody titers ($IC_{50}$) against SARS-CoV-2 pseudoviruses (BQ.1.1) in D35 serum from the immunized mice in groups 1-10 (n=20) were 41, 18599, 3093, 13337, 21384, 1320, 1015, 9356, and 10120, respectively; I. shows the $IC_{50}$ data of the neutralizing antibodies against pseudoviruses of the BF.7 strain, and the GMTs of neutralizing antibody titers ($IC_{50}$) against SARS-CoV-2 pseudoviruses (BF.7) in D35 serum from the immunized mice in groups 1-10 (n=20) were 51, 21514, 3849, 21797, 31887, 2100, 1998, 21410, 21798, and 19817, respectively; J. shows the $IC_{50}$ data of the neutralizing antibodies against pseudoviruses of the XBB strain, and the GMTs of neutralizing antibody titers ($IC_{50}$) against SARS-CoV-2 pseudoviruses (XBB) in D35 serum from the immunized mice in groups 1-10 (n=20) were 22, 1396, 4934, 355, 1035, 3149, 3119, 4430, 5345, and 3307, respectively; and K. shows the $IC_{50}$ data of the neutralizing antibodies against pseudoviruses of the XBB.1.5 strain, and the GMTs of neutralizing antibody titers ($IC_{50}$) against SARS-CoV-2 pseudoviruses (XBB.1.5) in D35 serum from the immunized mice in groups 1-10 (n=20) were 22, 1045, 15420, 555, 769, 7632, 9772, 6841, 9255, and 5834, respectively. GMT stands for geometric mean titer.

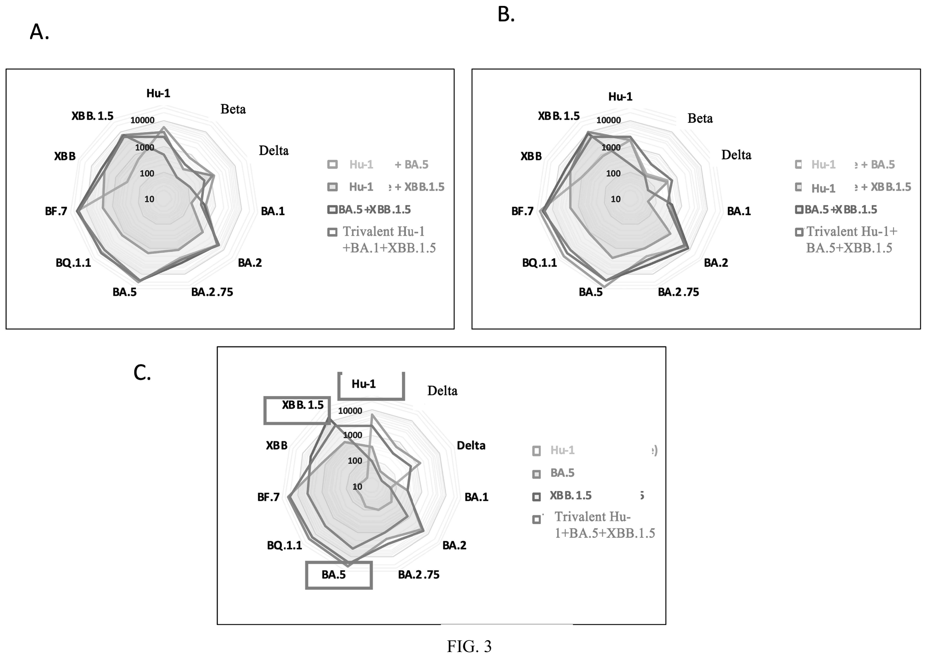

FIG. 3 shows a radar chart of the statistical analysis of the neutralizing antibody titers against SARS-CoV-2 pseudoviruses in the serum, comparing a trivalent S-trimer vaccine with a monovalent or bivalent S-trimer vaccine. A. is the radar chart of the "trivalent" vaccine compared with the "bivalent" vaccine with an antigen ratio of 1:1; B. is the radar chart of the "trivalent" vaccine compared with the "bivalent" vaccine with an antigen ratio of 1:2; and C. is the radar chart of the "trivalent" vaccine compared with the "monovalent" vaccine.

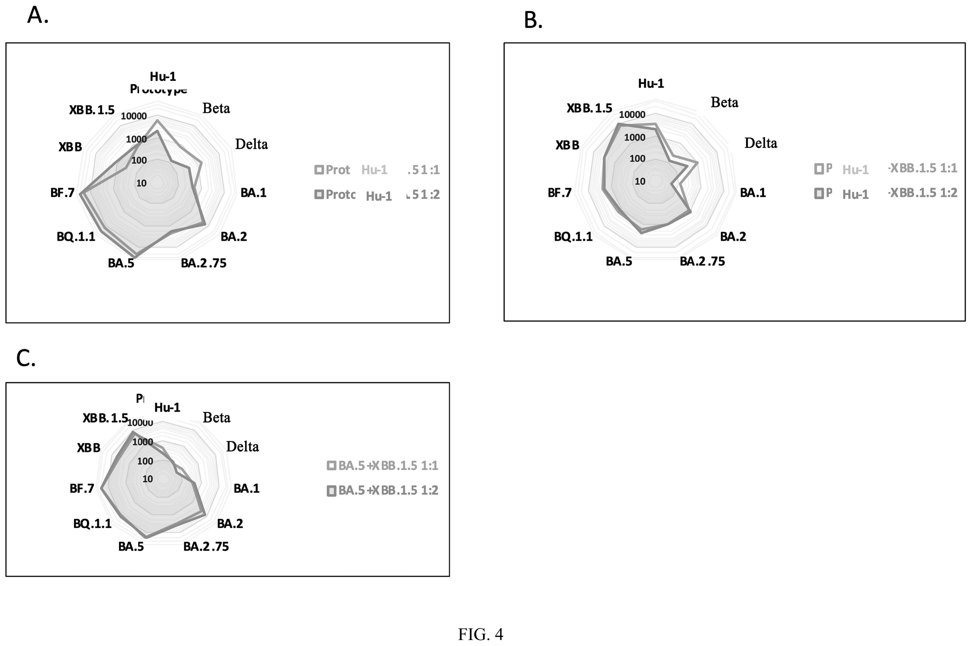

FIG. 4 shows a radar chart of the statistical analysis of the antigen ratio in a multivalent vaccine in relation to pseudovirus neutralizing titers. A. shows the bivalent vaccine Hu-1+BA.5 (1:1 VS. 1:2); B. shows the bivalent vaccine Hu-1+XBB.1.5 (1:1 VS. 1:2); and C. shows the bivalent vaccine BA.5+XBB.1.5 (1:1 VS. 1:2). Overall, there is not much difference between the 1:1 and 1:2 formulations of the three vaccines. However, reducing the proportion of Hu-1 may decrease the immunogenicity against earlier viruses such as Hu-1, Beta, and Delta variants. Adding BA.5 and XBB.1.5 antigens to the three vaccine preparations does not show any benefit.

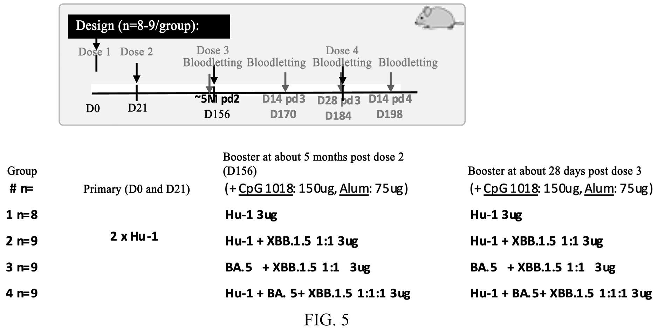

FIG. 5 shows the design of a heterologous booster group test of a Hu-1 S-trimer as a primary series and a bivalent or trivalent vaccine as a booster dose. Naive BALB/c mice were immunized twice on day 0 (a first dose) and day 21 (a second dose) with 150 $\mu$g CpG 1018 plus 3 $\mu$g Hu-1 S-trimer with 75 $\mu$g Alum as an adjuvant. On day 156 of the study, blood was collected to obtain serum, and the mice received a third dose of vaccination: immunization was performed with a 3 $\mu$g Hu-1 S-trimer vaccine (control) formulated with 150 $\mu$g CpG 1018 plus 75 $\mu$g Alum as an adjuvant or with a bivalent or trivalent S-trimer vaccine; and on day 170 (D14 PD3) of the study, blood was collected again to obtain serum, on day 184 (D28 PD3) of the study, blood was collected to obtain serum and mice were immunized again, and finally on day 198 (D14 PD4) and day 212 (D28 PD4), blood was collected to obtain serum.

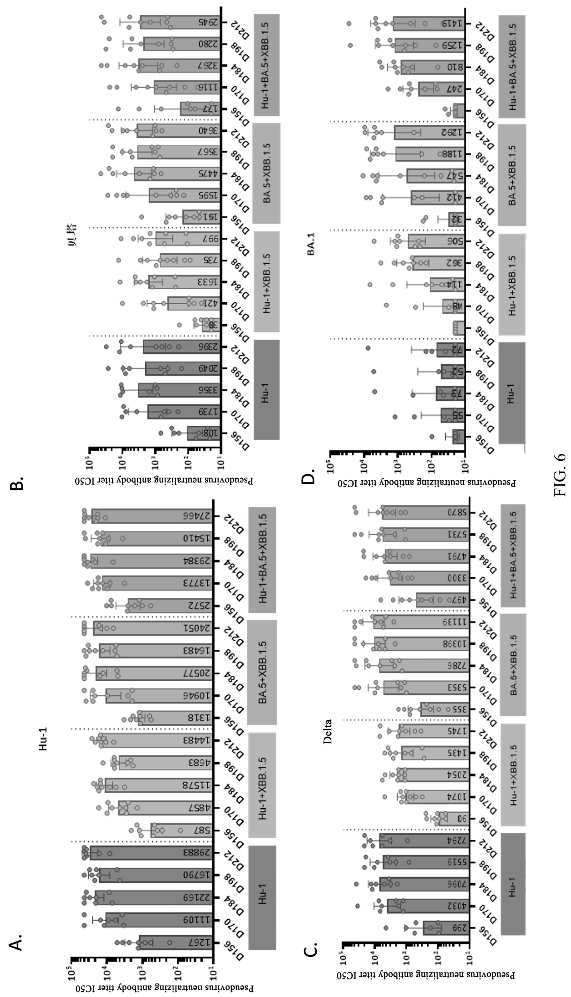

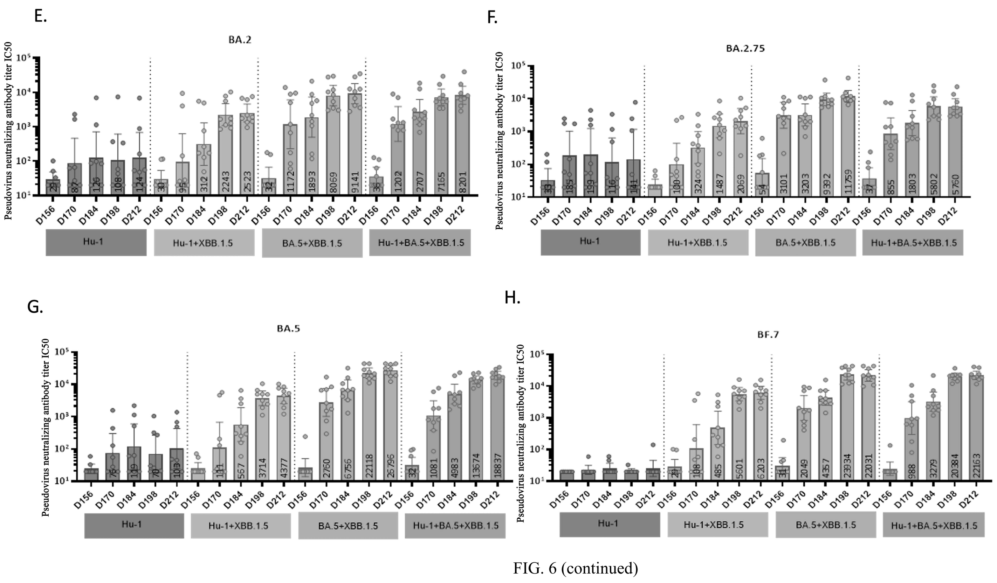

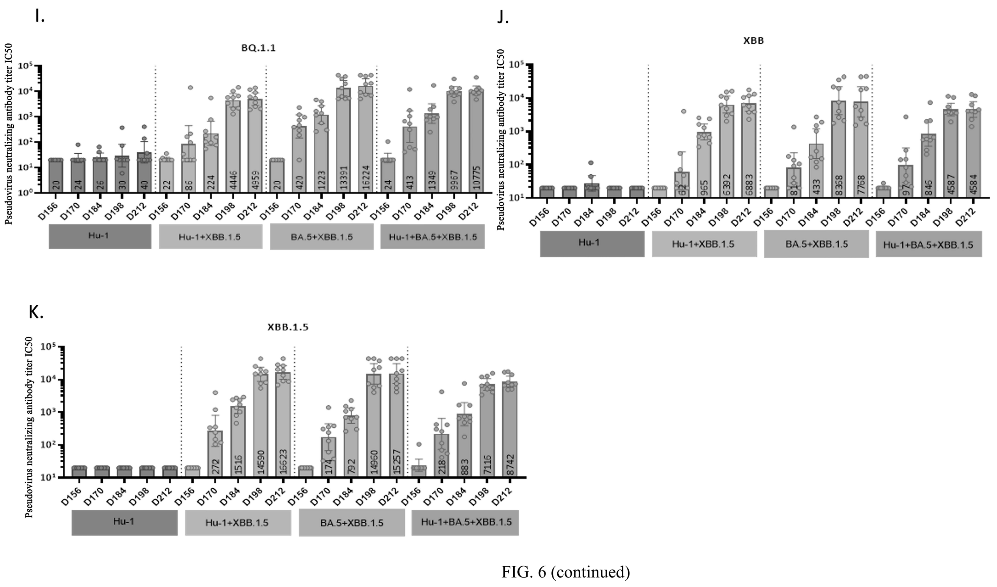

FIG. 6 shows the $IC_{30}$ data of neutralizing antibodies against pseudoviruses of strains SARS-CoV-2 Hu-1, Beta (beta, $\beta$, B.1.351), Delta (delta, $\delta$, B.1.617.2), and Omicron (Omicron, o, B.1.1.529) subtypes BA.1, BA.2, BA.2.75, BA.5, BQ.1.1, BF.7, XBB, and XBB.1.5 after mice were subjected to heterologous boost immunization with four doses of S-trimeric antigen, according to the vaccine doses in FIG. 5. A. shows the neutralizing antibody titers against SARS-CoV-2 pseudovirus Hu-1 in the serum of the immunized mice; B. shows the neutralizing antibody titers against SARS-CoV-2 pseudovirus Beta in the serum of the immunized mice; C. shows the neutralizing antibody titers against SARS-CoV-2 pseudovirus Delta in the serum of the immunized mice; D. shows the neutralizing antibody titers against SARS-CoV-2 pseudovirus BA.1 in the serum of the immunized mice; E. shows the neutralizing antibody titers against SARS-CoV-2 pseudovirus BA.2 in the serum of the immunized mice; F. shows the neutralizing antibody titers against SARS-CoV-2 pseudovirus BA.2.75 in the serum of the immunized mice; G. shows the neutralizing antibody titers against SARS-CoV-2 pseudovirus BA.5 in the serum of the immunized mice; H. shows the neutralizing antibody titers against SARS-CoV-2 pseudovirus BF.7 in the serum of the immunized mice; I. shows the neutralizing antibody titers against SARS-CoV-2 pseudovirus BQ.1.1 in the serum of the immunized mice; J. shows the neutralizing antibody titers against SARS-CoV-2 pseudovirus XBB in the serum of the immunized mice; and K. shows the neutralizing antibody titers against SARS-CoV-2 pseudovirus XBB.1.5 in the serum of the immunized mice. GMT stands for geometric mean titer.

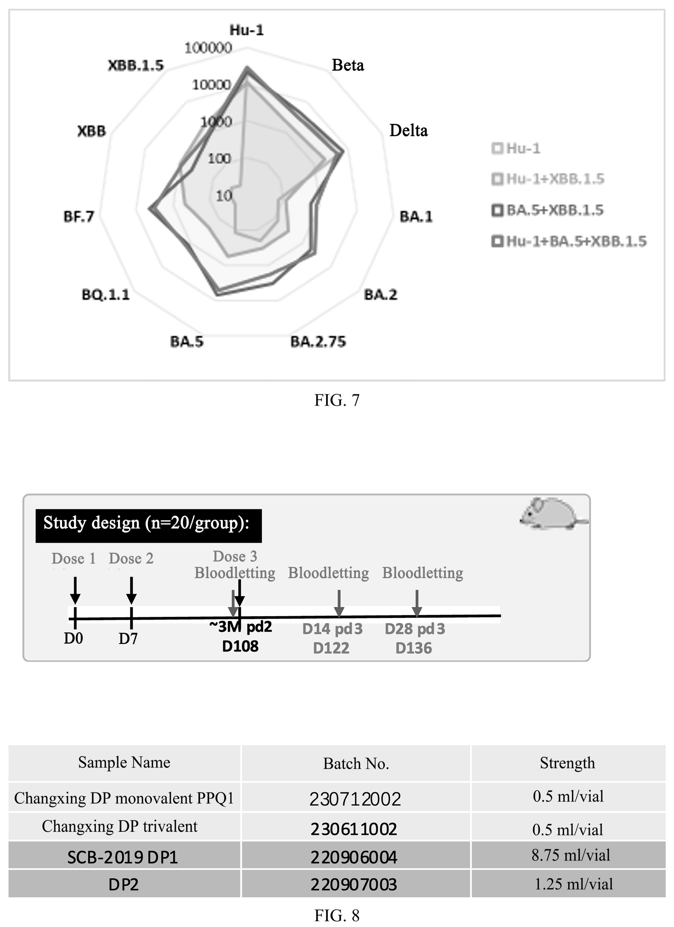

FIG. 7 shows a radar chart of neutralizing antibody titers on D184 (D28 after the first booster immunization).

FIG. 8 is a schematic diagram of the study design of a cross-neutralizing immunogenicity based on pseudovirus neutralizing titers in the immunized BALB/c mice using Hu-1 S-trimer as the primary series and a second dose, and monovalent or multivalent S-trimeric antigens as a third dose for heterologous booster immunization. After vaccination with Hu-1 as the basic immunization schedule, S-trimer recombinant subunit vaccines (multivalent or monovalent SARS-CoV-2 vaccines) under different combination conditions were selected for a third booster dose. Pre-existing immunization was performed on D0 and D7 using Hu-1 S-trimer antigens, and the pre-existing immunization ended after two doses. Booster immunization was performed on D108 by intramuscular injection (single point) at 0.05 mL/animal/dose. After the injection, the animal was observed for conditions and placed back in the cage to move freely. A total of three doses were administered during the whole process. The day of the first dose of pre-existing immunization was defined as D0. On D107 (before immunization), D122, and D136, blood was collected from the orbital venous plexus using a capillary glass blood collection tube, with a volume of approximately 400 $\mu$L. All blood samples obtained were allowed to stand at room temperature for 0.5-1 h and then centrifuged ($\times$ 2500 g) for 10 min to separate the serum and package it in aliquots, with the storage condition of cryopreservation at $\leq$ -20°C. Pseudoviruses of strains SARS-CoV-2 Hu-1, Beta, Delta, and Omicron (Omicron, o, B.1.1.529) subtypes BA.1, BA.2, BA.2.75, BA.5, BF.7, XBB, XBB.1.5, BQ.1.1, XBB.1.16.1, and EG.5.1 are used for a pseudovirus neutralizing antibody titer test. SARS-CoV-2 Hu-1, Beta, and Delta are pre-Omicron strains.

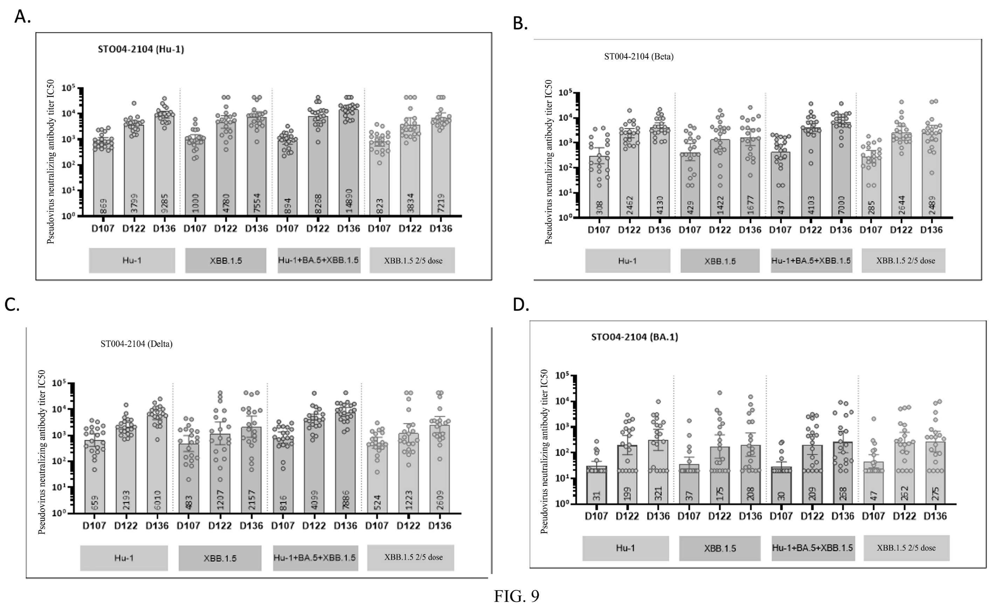

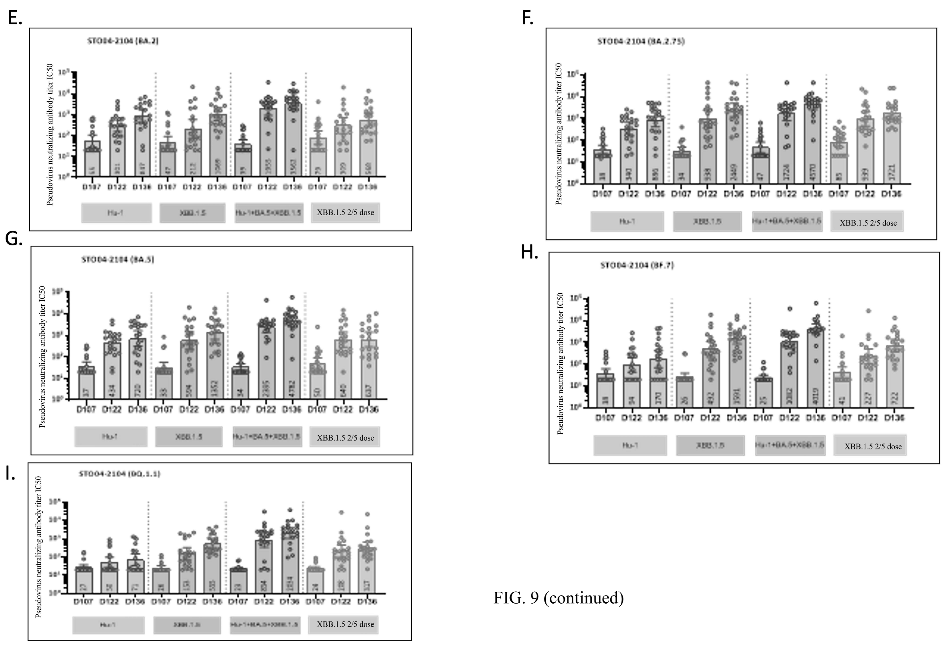

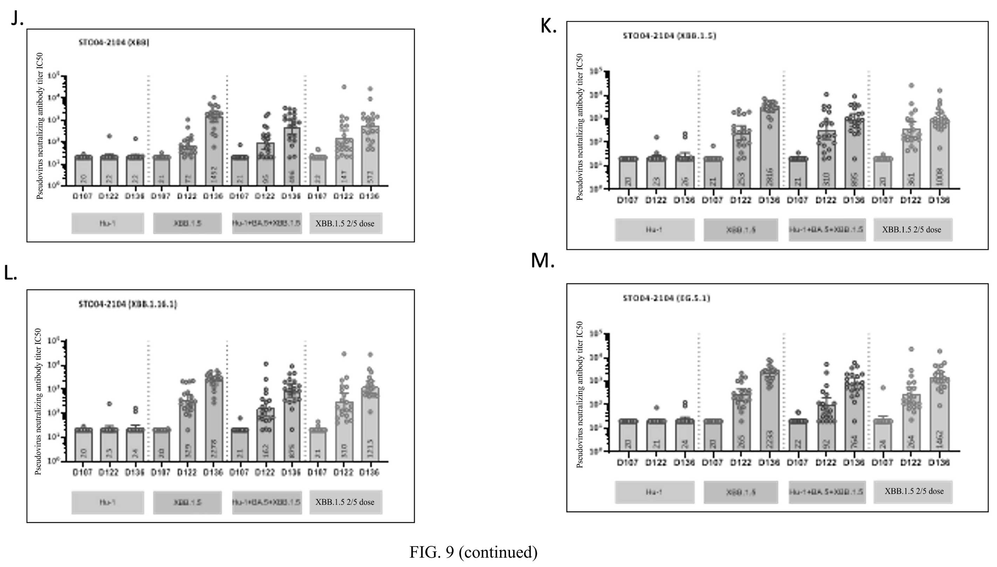

FIG. 9 shows the neutralizing antibody titers against SARS-CoV-2 pseudovirus (Hu-1/Beta/Delta/BA.1/BA.2/BA.2.75/BA.5/BF.7/BQ.1.1/XBB/XBB.1.5/XBB.1.16.1/EG.5.1) measured after immunization according to the study design in FIG. 8. A. shows the neutralizing antibody titers against SARS-CoV-2 pseudovirus Hu-1 in the serum of the immunized mice ($IC_{50}$); B. shows the neutralizing antibody titers against SARS-CoV-2 pseudovirus Beta in the serum of the immunized mice ($IC_{50}$); C. shows the neutralizing antibody titers against SARS-CoV-2 pseudovirus Delta in the serum of the immunized mice ($IC_{50}$); D. shows the neutralizing antibody titers against SARS-CoV-2 pseudovirus BA.1 in the serum of the immunized mice ($IC_{50}$); E. shows the neutralizing antibody titers against SARS-CoV-2 pseudovirus BA.2 in the serum of the immunized mice ($IC_{50}$); F. shows the neutralizing antibody titers against SARS-CoV-2 pseudovirus BA.2.75 in the serum of the immunized mice ($IC_{50}$); G. shows the neutralizing antibody titers against SARS-CoV-2 pseudovirus BA.5 in the serum of the immunized mice ($IC_{50}$); H. shows the neutralizing antibody titers against SARS-CoV-2 pseudovirus BF.7 in the serum of the immunized mice ($IC_{50}$); I. shows the

neutralizing antibody titers against SARS-CoV-2 pseudovirus BQ.1.1 in the serum of the immunized mice ($IC_{50}$); J. shows the neutralizing antibody titers against SARS-CoV-2 pseudovirus XBB in the serum of the immunized mice ($IC_{50}$); K. shows the neutralizing antibody titers against SARS-CoV-2 pseudovirus XBB.1.5 in the serum of the immunized mice ($IC_{50}$); L. shows the neutralizing antibody titers against SARS-CoV-2 pseudovirus XBB.1.16.1 in the serum of the immunized mice ($IC_{50}$); and M. shows the neutralizing antibody titers against SARS-CoV-2 pseudovirus EG.5.1 in the serum of the immunized mice ($IC_{50}$). GMT stands for geometric mean titer.

FIG. 10 shows a radar chart of neutralizing antibody titers on D122 (D14 after booster immunization).

FIG. 11 shows a radar chart of neutralizing antibody titers on D135 (D28 after booster immunization).

## DESCRIPTION OF EMBODIMENTS

The present disclosure provides an immunogenic composition, method, and use including a fusion peptide and protein of a coronavirus antigen or immunogen for the treatment of coronavirus infection (for example, preventive and therapeutic). In some embodiments, a composition and method of use of a recombinant soluble surface antigen from an RNA virus in the form of a covalently linked trimer are disclosed. In some embodiments, the resulting fusion protein is secreted as a disulfide bond-connected homotrimer, which has a more stable structure while retaining the native trimeric viral antigen conformation, and can therefore be used as a more effective vaccine against these dangerous pathogens.

In some embodiments, the present disclosure discloses a method for preventing viral infection using a viral antigen trimer as a vaccine or as part of a multivalent vaccine, without or with an adjuvant, or with more than one adjuvant, optionally administered through intramuscular injection or intranasal administration. The viral antigen trimer can be used in a primary series, an additional dose, and/or a booster dose. Independently, the primary series, the additional dose, and/or any one or more booster doses may not use or use the adjuvant. If the adjuvant is used, the optional adjuvant may include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

In some embodiments, the present disclosure discloses a method of using a viral antigen trimer as an antigen for diagnosing viral infection by detecting an antibody (such as IgM or IgG) that recognizes a viral antigen (such as a neutralizing antibody).

In some embodiments, the present disclosure discloses a method for using a viral antigen trimer as an antigen to produce a polyclonal or monoclonal antibody (for example, a neutralizing mAb for treating coronavirus infection) that can be used for passive immunization.

In some embodiments, the present disclosure discloses a viral antigen trimer as a vaccine or as part of a multivalent vaccine, in which the vaccine includes a plurality of trimer subunit vaccines, and the trimer subunit vaccine includes a viral antigen of the same protein of a virus, or a viral antigen including two or more different proteins of one or more viruses or a viral antigen of one or more strains of the same virus.

In some embodiments, the present disclosure discloses a monovalent vaccine that includes the viral antigen trimer disclosed herein. In some embodiments, the present disclosure discloses a bivalent vaccine that includes the viral antigen trimer disclosed herein. In some embodiments, the present disclosure discloses a trivalent vaccine that includes the viral antigen trimer disclosed herein. In some embodiments, the present disclosure discloses a quadrivalent vaccine that includes the viral antigen trimer disclosed herein.

In some embodiments, the present disclosure discloses a monovalent vaccine that includes the S-trimer disclosed herein. In some embodiments, the present disclosure discloses a bivalent vaccine that includes the S-trimer disclosed herein. In some embodiments, the present disclosure discloses a bivalent vaccine that includes at least one S-trimer including a first S protein antigen and at least one S-trimer including a second S protein antigen. In some embodiments, a first and a second S protein antigens are derived from the same S protein of one or more virus species or strains/subtypes, or two or more different S proteins from one or more virus species or one or more strains/subtypes of the same virus species. In some embodiments, the present disclosure discloses a trivalent vaccine that includes the S-trimer disclosed herein. In some embodiments, the present disclosure discloses a trivalent vaccine that includes at least one S-trimer including a first S protein antigen, at least one S-trimer including a second S protein antigen, and at least one S-trimer including a third S protein antigen. In some embodiments, a first, second, and third S protein antigens are derived from the same S protein of one or more virus species or strains/subtypes, or two, three, or more different S proteins from one or more virus species or one or more strains/subtypes of the same virus species. In some embodiments, the present disclosure discloses a quadrivalent vaccine that includes the S-trimer disclosed herein. In some embodiments, the present disclosure discloses a quadrivalent vaccine that includes at least one S-trimer including a first S protein antigen, at least one S-trimer including a second S protein antigen, at least one S-trimer including a third S protein antigen, and at least one S-trimer including a fourth S protein antigen. In some embodiments, a first, second, third, and fourth S protein antigens are derived from the same S protein of one or more virus species or strains/subtypes, or two, three, four, or more different S proteins from one or more virus species or one or more strains/subtypes of the same virus species.

In some embodiments, the present disclosure discloses a multivalent vaccine, such as a bivalent vaccine, including: a first trimeric fusion protein, which includes a soluble coronavirus SARS-CoV-2 Omicron (B.1.1.529) spike (S) subtype protein surface antigen or a fragment, variant or mutant, in which the soluble coronavirus surface antigen or the fragment, variant or mutant thereof is linked to a C-terminal portion of collagen, and the C-terminal portion of the collagen forms a disulfide bond-connected trimer, thereby forming the first trimeric fusion protein; a second and/or more trimeric fusion proteins, which include soluble coronavirus SARS-CoV-2 Hu-1, Alpha, Beta, Gamma, Delta, Omicron (B.1.1.529) and subtypes thereof or Mu spike (S) protein surface antigens or fragments, variants or mutants thereof, and the soluble coronavirus surface antigens or the fragments, variants or mutants thereof are linked to a C-terminal portion of collagen, and the C-terminal portion of the collagen forms a disulfide-linked trimer, thereby forming the second and/or more trimeric fusion proteins. In some embodiments, the Omicron subtype spike (S) protein surface antigen or the fragment, variant or mutant thereof includes BA.1, BA.2, BA.2.75, BA.5, BQ.1.1, BF.7, XBB, XBB.1.5, BQ.1.1 spike (S) protein surface antigens or fragments, variants or mutants thereof. In some embodiments, the first trimeric fusion protein includes a soluble coronavirus SARS-CoV-2 BA.2 or XBB.1.5 spike (S) protein surface antigen or a fragment, variant or mutant thereof. In some embodiments, the second trimeric fusion protein includes a soluble coronavirus SARS-CoV-2 Hu-1 or Omicron subtype BA.2 or XBB.1.5 spike (S) protein surface antigen or a fragment, variant or mutant thereof. In some embodiments, the present disclosure discloses a multivalent vaccine, such as a bivalent vaccine, including: soluble coronavirus SARS-CoV-2 Hu-1 and Omicron subtype BA.2 spike (S) protein surface antigens or fragments thereof, the weight ratio of which is 0.5-4:0.5-4, such as 4:1, 3:1, 2:1, 1:1, 1:2, 1:3, or 1:4. In some embodiments, the present disclosure discloses a multivalent vaccine, such as a bivalent vaccine, including: soluble coronavirus SARS-CoV-2 Omicron subtypes BA.5 and XBB.1.5 spike (S) protein surface antigens or fragments thereof, the weight ratio of which is 0.5-4:0.5-4, such as 4:1, 3:1, 2:1, 1:1, 1:2, 1:3, or 1:4. In some embodiments, the present disclosure discloses a multivalent vaccine, such as a bivalent vaccine, including: soluble coronavirus SARS-CoV-2 Hu-1 and Omicron subtype XBB.1.5 spike (S) protein surface antigens or fragments thereof, the weight ratio of which is 0.5-4:0.5-4, such as 4:1, 3:1, 2:1, 1:1, 1:2, 1:3, or 1:4. In some embodiments, the multivalent vaccine is administered without an adjuvant and optionally is used as a primary series, an additional dose, and/or a homologous or heterologous booster dose, such as a first dose, a second dose, a third dose, a fourth dose, and/or more doses, and optionally the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines. In some embodiments, the multivalent vaccine is administered with more than one adjuvant and optionally is used as a primary series, an additional dose, and/or a homologous or heterologous booster dose, such as a first dose, a second dose, a third dose, a fourth dose, and/or more doses. Optionally, the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines. Optionally, the adjuvant in the primary series, the additional dose, and/or the homologous or heterologous booster dose may independently include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethy-lene sorbitan monooleate (Tween-80), such as the adjuvant in the form of an oil-in-water emulsion containing squalene, $\alpha$-tocopherol, and Tween-80 and/or Span 85; or any combination of the adjuvants. In some embodiments, the first trimeric fusion protein in the multivalent vaccine includes a sequence set forth in any one of SEQ ID NOs: 81-84, 122, and 126, or an amino acid sequence having at least or about 80%, 85%, 90%, 92%, 95%, 97%, or 99% sequence identity with the sequence set forth in any one of SEQ ID NOs: 81-84, 122, and 126. In some embodiments, the second and/or more trimeric fusion proteins in the multivalent vaccine include a sequence set forth in any one of SEQ ID NOs: 27-66, or an amino acid sequence having at least or about 80%, 85%, 90%, 92%, 95%, 97%, or 99% sequence identity with the sequence set forth in any one of SEQ ID NOs: 27-66.

In some embodiments, the present disclosure discloses a multivalent vaccine, such as a trivalent vaccine, including: a first trimeric fusion protein, which includes a soluble coronavirus SARS-CoV-2 Omicron (B.1.1.529) spike (S) subtype protein surface antigen or a fragment, variant or mutant, in which the soluble coronavirus surface antigen or the fragment, variant or mutant thereof is linked to a C-terminal portion of collagen, and the C-terminal portion of the collagen forms a disulfide bond-connected trimer, thereby forming the first trimeric fusion protein; a second and third trimeric fusion proteins and/or more trimeric fusion proteins, which include soluble coronavirus SARS-CoV-2 Hu-1, Alpha, Beta, Gamma, Delta, and Omicron (B.1.1.529) spike (S) subtype different from the first trimeric fusion protein, or Mu spike (S) protein surface antigens or fragments, variants or mutants thereof, and the soluble coronavirus surface antigens or the fragments, variants or mutants thereof are linked to a C-terminal portion of collagen, and the C-terminal portion of the collagen forms a disulfide-connected trimer, thereby forming the second, third and/or more trimeric fusion proteins. In some embodiments, the Omicron subtype spike (S) protein surface antigen or the fragment, variant or mutant thereof includes BA.1, BA.2,

BA.2.75, BA.5, BQ.1.1, BF.7, XBB, XBB.1.5, BQ.1.1 spike (S) protein surface antigens or fragments, variants or mutants thereof. In some embodiments, the first and the third trimeric fusion proteins include soluble coronavirus SARS-CoV-2 Omicron subtypes BA.5 and XBB.1.5 spike (S) protein surface antigens or fragments, variants or mutants thereof. In some embodiments, the second trimeric fusion protein includes a soluble coronavirus SARS-CoV-2 Hu-1 spike (S) protein surface antigen or a fragment, variant or mutant thereof. In some embodiments, the multivalent vaccine is a trivalent vaccine, including: soluble SARS-CoV-2 Hu-1, Omicron strains BA.5 and XBB.1.5 spike (S) protein surface antigens or fragments, variants or mutants thereof in a weight ratio of 0.5-4:0.5-4:0.5-4, such as 1:1:1 or 1:2:2, 1:3:3, 1:4:4, 2:1:1, 3:1:1, or 4:1:1. In some embodiments, the multivalent vaccine is administered without an adjuvant and optionally is used as a primary series, an additional dose, and/or a homologous or heterologous booster dose, such as a first dose, a second dose, a third dose, a fourth dose, and/or more doses, and optionally the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines. In some embodiments, the multivalent vaccine is administered with more than one adjuvant and optionally is used as a primary series, an additional dose, and/or a homologous or heterologous booster dose, such as a first dose, a second dose, a third dose, a fourth dose, and/or more doses. Optionally, the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines. Optionally, the adjuvant in the primary series, the additional dose, and/or the homologous or heterologous booster dose may independently include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethy-lene sorbitan monooleate (Tween-80), such as the adjuvant in the form of an oil-in-water emulsion containing squalene, $\alpha$-tocopherol, and Tween-80 and/or Span 85; or any combination of the adjuvants. In some embodiments, the first and the third trimeric fusion proteins in the multivalent vaccine includes a sequence set forth in any one of SEQ ID NOs: 81-84, 122, and 126, or an amino acid sequence having at least or about 80%, 85%, 90%, 92%, 95%, 97%, or 99% sequence identity with the sequence set forth in any one of SEQ ID NOs: 81-84, 122, and 126. In some embodiments, the second and/or more trimeric fusion proteins in the multivalent vaccine include a sequence set forth in any one of SEQ ID NOs: 27-66, or an amino acid sequence having at least or about 80%, 85%, 90%, 92%, 95%, 97%, or 99% sequence identity with the sequence set forth in any one of SEQ ID NOs: 27-66.

The protein containing a coronavirus antigen and an immunogen provided herein, including a recombinant polypeptide and fusion protein, can be used to effectively and safely treat (for example, therapeutic and prophylactic) coronavirus infection. For example, the protein containing a coronavirus antigen and an immunogen provided herein treats coronavirus infection without mediated vaccine-enhanced disease (VED) and/or antibody-dependent enhancement (ADE). In addition, the protein containing a coronavirus antigen and an immunogen provided herein is easy to produce and exhibits stability under high stress conditions such as high temperature, extreme pH, high osmolality, and low osmolality. Therefore, the protein and immunogenic composition provided herein avoids and satisfies the production, stability, safety, and effectiveness issues that hinder the development of coronavirus vaccines.

In some aspects, the coronavirus antigen and the immunogen provided herein include a coronavirus spike (S) protein or peptide, especially an SARS-CoV or SARS-CoV-2 S protein. The spikes of SARS-CoV and SARS-CoV-2 are composed of S protein trimers, which belong to the class I virus fusion glycoprotein group, which also includes HIV glycoprotein 160 (Env), influenza hemagglutinin (HA), paramyxovirus F, and Ebola virus glycoprotein. The SARS-CoV and SARS-CoV-2 S proteins each encode a surface glycoprotein precursor, and the amino terminus and most of the protein are predicted to be located on the cell surface or the exterior of the virus particle. The S protein includes a signal peptide located at the N-terminus, an extracellular domain, a transmembrane domain, and an intracellular domain. Similar to other coronaviruses, the SARS-CoV and SARS-CoV-2 S proteins can be cleaved into S1 and S2 subunits by protease. In particular, SARS-CoV-2 contains a furin-like cleavage site that is lacking in other SARS-like CoVs.

In some embodiments, the present disclosure provides a recombinant S extracellular domain trimer. In some embodiments, the recombinant S extracellular domain trimer includes a recombinant S extracellular domain protomer from an $\alpha$-coronavirus (such as NL63-CoV or 229E-CoV). In some embodiments, the recombinant S extracellular domain trimer includes an S extracellular domain protomer from a $\beta$-coronavirus (such as OC43-CoV, SARS-CoV, SARS-CoV-2, MERS-CoV, HKU1-CoV, WIV1-CoV, mouse hepatitis virus (MHV), or HKU9-CoV).

Similar to other enveloped RNA viruses (such as HIV, RSV, and influenza), coronaviruses, including SARS-CoV-2, have a trimeric surface antigen on the viral envelopes thereof so that the coronaviruses can enter different host cells through specific cell surface receptors during infection. Similar to SARS-CoV-1, SARS-CoV-2 uses the trimeric virus surface antigen S protein to bind to the specific cell surface receptor ACE2 thereof and enter the host cell of the mammalian respiratory system. The prerequisite for producing an effective recombinant subunit vaccine is to be able to produce a native-like viral S antigen, especially to maintain the trimeric conformation thereof so that a sufficient number of antibodies can bind to the receptor binding domain (RBD) of the viral S protein, thereby preventing the virus from binding to the ACE2 receptor and eliminating the viral infection.

In some embodiments, a protein containing a coronavirus antigen or immunogen (such as an SARS-CoV or SARS-CoV-2 S protein peptide) is capable of generating an immune response, such as an immune response against the SARS-CoV or SARS-CoV-2 S protein peptide. In some embodiments, the immune response inhibits or reduces coronavirus replication in a subject (such as a patient). In some embodiments, the immune response includes producing one or more neutralizing antibodies, such as polyclonal and/or monoclonal antibodies. In some embodiments, the neutralizing antibody inhibits or reduces coronavirus replication in a subject (such as a patient). In some embodiments, administration of a protein to a subject, for example as an immunogenic composition, does not result in antibody-dependent enhancement (ADE) in the subject due to prior exposure to coronavirus. In some aspects, a protein containing a coronavirus antigen and an immunogen is used as a vaccine.

In some embodiments, a coronavirus antigen and an immunogen (such as an SARS-CoV or SARS-CoV-2 S protein peptide) are linked to a protein or peptide to form a fusion protein or recombinant polypeptide. In some embodiments, the protein or peptide linked to the coronavirus antigen or immunogen can bind to the protein or peptide (such as a protein or peptide of a fusion protein or recombinant polypeptide), for example, through covalent or non-covalent linkage. Therefore, in some cases, the protein or peptide linked to the coronavirus antigen or immunogen is a multimerization domain.

In some embodiments, a coronavirus antigen and an immunogen (such as a coronavirus S protein peptide) are linked to a propeptide of collagen (for example, at a C-terminus of a propeptide of collagen) to form a fusion peptide or recombinant polypeptide. Therefore, in some embodiments, the protein provided herein includes a recombinant polypeptide containing a coronavirus antigen and an immunogen (such as a coronavirus S protein peptide or a fragment or epitope thereof), linked to a C-terminal propeptide of collagen. In some embodiments, a propeptide of collagen is derived from a human C-propeptide of $\alpha$1 collagen and is capable of self-trimerization after expression.

In some embodiments, linking a coronavirus antigen and an immunogen (such as a coronavirus S protein peptide) to a propeptide of collagen (for example, at a C-terminus of a propeptide of collagen) contributes to the ability of a protein to produce an immune response. For example, the production of a recombinant protein can preserve the tertiary and quaternary structures of a coronavirus S protein peptide, which may be important for the stability of the native conformation of the coronavirus S protein peptide, while the availability of an antigenic site on a protein surface can in turn trigger an immune response, such as a neutralizing antibody. In addition, linking the coronavirus S protein peptide to a protein or peptide capable of self-trimerization causes the recombinant protein to aggregate, thereby mimicking the natural homotrimeric structure of the coronavirus S protein peptide on the viral envelope.

In some embodiments, linking the coronavirus S protein peptide to a C-terminal propeptide of collagen results in a self-trimerized recombinant polypeptide. In some embodiments, the protein provided herein includes a plurality of self-trimerized coronavirus S protein peptides and propeptides of collagen recombinant polypeptides. In some embodiments, the trimeric nature of a recombinant protein contributes to the stability of the protein. In some embodiments, the trimeric nature of the recombinant protein contributes to the ability of the protein to produce an immune response. In some embodiments, the trimeric nature of the recombinant protein and/or the macroscopic structure of a plurality of self-trimerized recombinant proteins contribute to the ability of the protein to produce an immune response.

The present disclosure further provides an immunogenic composition including the protein provided herein, a method for producing the protein provided herein, a method for treating a subject with the protein and the composition provided herein, and a kit.

All publications mentioned in the present application, including patent documents, scientific articles, and databases, are incorporated herein by reference in their entireties for all purposes to the same extent as individual publications are incorporated herein by reference separately. If the definition described herein is contrary to or inconsistent with the definition described in the patents, applications, published applications, and other publications incorporated herein by reference, the definition described herein shall take precedence over the definition incorporated herein by reference. The section headings used herein are for organizational purposes only and should not be construed as limiting the subject matter described.

## I. Viral antigen and immunogen

The protein provided herein includes a coronavirus antigen and an immunogen. The coronavirus antigen and the immunogen contemplated herein can promote or stimulate a cell-mediated response and/or humoral response. In some embodiments, the response (such as a cell-mediated or humoral response) includes the production of an antibody (such as a neutralizing antibody). In some embodiments, the coronavirus antigen or immunogen is a coronavirus spike protein peptide.

Coronaviruses are a family of positive-sense single-stranded RNA viruses, which are known to cause severe respiratory diseases. They have the largest genome (26-32 kb) of any known RNA virus and are phylogenetically divided into four genera ($\alpha$, $\beta$, $\gamma$, $\delta$), with $\beta$-coronaviruses further subdivided into four lineages (A, B, C, D). Currently, the known viruses from the coronavirus family that infect humans are from the α- and β-coronavirus genera. In addition, it is believed that γ- and δ-coronavirus genera may infect humans in the future. Non-limiting examples of β-coronaviruses include Middle East respiratory syndrome coronavirus (MERS-CoV), severe acute respiratory syndrome coronavirus (SARS-CoV), human coronavirus HKU1 (HKU1-CoV), human coronavirus OC43 (OC43-CoV), mouse hepatitis virus (MHV-CoV), bat SARS-like coronavirus WIV1 (WIV1-CoV), and human coronavirus HKU9 (HKU9-CoV). Non-limiting examples of α-coronaviruses include human coronavirus 229E (229E-CoV), human coronavirus NL63 (NL63-CoV), porcine epidemic diarrhea virus (PEDV), and transmissible gastroenteritis coronavirus (TGEV). A non-limiting example of a δ-coronavirus is swine δ-coronavirus (SDCV).

The present disclosure discloses a list of severe acute respiratory syndrome-related coronaviruses:

bat coronavirus Cp/Yunnan 2011; bat coronavirus RaTG13; bat coronavirus Rp/Shaanxi2011; bat SARS coronavirus HKU3, bat SARS coronavirus HKU3-1, bat SARS coronavirus HKU3-10, bat SARS coronavirus HKU3-11, bat SARS coronavirus HKU3-12, bat SARS coronavirus HKU3-13, bat SARS coronavirus HKU3-2, bat SARS coronavirus HKU3-3, bat SARS coronavirus HKU3-4, bat SARS coronavirus HKU3-5, bat SARS coronavirus HKU3-6, bat SARS coronavirus HKU3-7, bat SARS coronavirus HKU3-8, bat SARS coronavirus HKU3-9; bat SARS coronavirus Rp1; bat SARS coronavirus Rp2; bat SARS CoV Rf1/2004, bat CoV 273/2005; bat SARS CoV Rm1/2004, bat CoV 279/2005; bat SARS CoV Rp3/2004; bat SARS-like coronavirus; bat SARS-like coronavirus Rs3367; bat SARS-like coronavirus RsSHC014; bat SARS-like coronavirus WIV1; bat SARS-like coronavirus YNLF 31C; bat SARS-like coronavirus YNLF_34C; BtRf-BetaCoV/HeB2013; BtRf-BetaCoV/JL2012; BtRf-BetaCoV/SX2013; BtRs-BetaCoV/GX2013; BtRs-BetaCoV/HuB2013; BtRs-BetaCoV/YN2013; Civet SARS CoV 007/2004; Civet SARS CoV SZ16/2003; Civet SARS CoV SZ3/2003; Recombinant SARSr-CoV, SARS coronavirus ExoN1, SARS coronavirus MA15, SARS coronavirus MA15 ExoN1, SARS coronavirus wtic-MB; Rhinolophus affinis coronavirus; SARS bat coronavirus; SARS coronavirus A001; SARS coronavirus A013; SARS coronavirus A021; SARS coronavirus A022; SARS coronavirus A030; SARS coronavirus A031; SARS coronavirus AS; SARS coronavirus B012; SARS coronavirus B024; SARS coronavirus B029; SARS coronavirus B033; SARS coronavirus B039; SARS coronavirus B040; SARS coronavirus BJ01; SARS coronavirus BJ02; SARS coronavirus BJ03; SARS coronavirus BJ04; SARS coronavirus BJ162; SARS coronavirus BJ182-12; SARS coronavirus BJ182-4; SARS coronavirus BJ182-8; SARS coronavirus BJ182a; SARS coronavirus BJ182b; SARS coronavirus BJ202; SARS coronavirus BJ2232; SARS coronavirus BJ302; SARS coronavirus C013; SARS coronavirus C014; SARS coronavirus C017; SARS coronavirus C018; SARS coronavirus C019; SARS coronavirus C025; SARS coronavirus C028; SARS coronavirus C029; SARS coronavirus CDC#200301157; SARS coronavirus civet010; SARS coronavirus civet014; SARS coronavirus civet019; SARS coronavirus civet020; SARS coronavirus CS21; SARS coronavirus CS24; SARS coronavirus CUHK-AG01; SARS coronavirus CUHK-AG02; SARS coronavirus CUHK-AG03; SARS coronavirus CUHK-L2; SARS coronavirus CUHK-Su10; SARS coronavirus CUHK-W1; SARS coronavirus cw037; SARS coronavirus cw049; SARS coronavirus ES191; SARS coronavirus ES260; SARS coronavirus FRA; SARS coronavirus Frankfurt 1; SARS coronavirus Frankfurt1-v01; SARS coronavirus GD01; SARS coronavirus GD03T0013; SARS coronavirus GD322; SARS coronavirus GD69; SARS coronavirus GDH-BJH01; SARS coronavirus GZ-A; SARS coronavirus GZ-B; SARS coronavirus GZ-C; SARS coronavirus GZ-D; SARS coronavirus GZ02; SARS coronavirus GZ0401; SARS coronavirus GZ0402; SARS coronavirus GZ0403; SARS coronavirus GZ43; SARS coronavirus GZ50; SARS coronavirus GZ60; SARS coronavirus HB; SARS coronavirus HC/SZ/61/03; SARS coronavirus HGZ8L1-A; SARS coronavirus HGZ8L1-B; SARS coronavirus HGZ8L2; SARS coronavirus HHS-2004; SARS coronavirus HKU-36871; SARS coronavirus HKU-39849; SARS coronavirus HKU-65806; SARS coronavirus HKU-66078; SARS coronavirus Hong Kong/03/2003; SARS coronavirus HPZ-2003; SARS coronavirus HSR 1; SARS coronavirus HSZ-A; SARS coronavirus HSZ-Bb; SARS coronavirus HSZ-Bc; SARS coronavirus HSZ-Cb; SARS coronavirus HSZ-Cc; SARS coronavirus HSZ2-A; SARS coronavirus HZS2-Bb; SARS coronavirus HZS2-C; SARS coronavirus HZS2-D; SARS coronavirus HZS2-E; SARS coronavirus HZS2-Fb; SARS coronavirus HZS2-Fc; SARS coronavirus JMD; SARS coronavirus LC1; SARS coronavirus LC2; SARS coronavirus LC3; SARS coronavirus LC4; SARS coronavirus LC5; SARS coronavirus LLJ-2004; SARS coronavirus NS-1; SARS coronavirus P2; SARS coronavirus PC4-115; SARS coronavirus PC4-127; SARS coronavirus PC4-13; SARS coronavirus PC4-136; SARS coronavirus PC4-137; SARS coronavirus PC4-145; SARS coronavirus PC4-199; SARS coronavirus PC4-205; SARS coronavirus PC4-227; SARS coronavirus PC4-241; SARS coronavirus PUMC01; SARS coronavirus PUMC02; SARS coronavirus PUMC03; SARS coronavirus Rs_672/2006; SARS coronavirus sf098; SARS coronavirus sf099; SARS coronavirus ShanghaiQXC1; SARS coronavirus ShanghaiQXC2; SARS coronavirus Shanhgai LY; SARS coronavirus Sin0409; SARS coronavirus Sin2500; SARS coronavirus Sin2677; SARS coronavirus Sin2679; SARS coronavirus Sin2748; SARS coronavirus Sin2774; SARS coronavirus Sin3408; SARS coronavirus Sin3408L; SARS coronavirus Sin3725V; SARS coronavirus Sin3765V; SARS coronavirus Sin842; SARS coronavirus Sin845; SARS coronavirus Sin846; SARS coronavirus Sin847; SARS coronavirus Sin848; SARS coronavirus Sin849; SARS coronavirus Sin850; SARS coronavirus Sin852; SARS coronavirus Sin WNV; SARS coronavirus Sino1-11; SARS coronavirus Sino3-11; SARS coronavirus SinP1; SARS coronavirus SinP2; SARS coronavirus SinP3; SARS coronavirus SinP4; SARS coronavirus SinP5; SARS coronavirus SoD; SARS coronavirus SZ1; SARS coronavirus SZ13; SARS coronavirus Taiwan; SARS coronavirus Taiwan JC-2003; SARS coronavirus Taiwan TC1; SARS coronavirus Taiwan TC2; SARS coronavirus Taiwan TC3; SARS coronavirus TJ01; SARS coronavirus TJF; SARS coronavirus Tor2; SARS coronavirus TW, SARS coronavirus TW-GD1, SARS coronavirus TW-GD2, SARS coronavirus TW-GD3, SARS coronavirus TW-GD4, SARS coronavirus TW-GD5, SARS coronavirus TW-HP1, SARS coronavirus TW-HP2, SARS coronavirus TW-HP3, SARS coronavirus TW-HP4, SARS coronavirus TW-JC2, SARS coronavirus TW-KC1, SARS coronavirus TW-KC3, SARS coronavirus TW-PH1, SARS coronavirus TW-PH2, SARS coronavirus TW-YM1, SARS coronavirus TW-YM2, SARS coronavirus TW-YM3, SARS coronavirus TW-YM4; SARS coronavirus TW1; SARS coronavirus TW10; SARS coronavirus TW11; SARS coronavirus TW2; SARS coronavirus TW3; SARS coronavirus TW4; SARS coronavirus TW5; SARS coronavirus TW6; SARS coronavirus TW7; SARS coronavirus TW8; SARS coronavirus TW9; SARS coronavirus TWC; SARS coronavirus TWC2; SARS coronavirus TWC3; SARS coronavirus TWH; SARS coronavirus TWJ; SARS coronavirus TWK; SARS coronavirus TWS; SARS coronavirus TWY; SARS coronavirus Urbani; SARS coronavirus Vietnam; SARS coronavirus WF188; SARS coronavirus WH20; SARS coronavirus WHU; SARS coronavirus xw002; SARS coronavirus ZJ01; SARS coronavirus ZJ02; SARS coronavirus ZJ0301; SARS coronavirus ZMY 1; SARS coronavirus ZS-A; SARS coronavirus ZS-B; SARS coronavirus ZS-C; SARS-related bat coronavirus RsSHC014; SARS-related β-coronavirus Rp3/2004; severe acute respiratory syndrome coronavirus 2.

The following table shows exemplary SARS CoV-2 strains.

| Name/Designation | | Distribution | Significant Mutation | Impact | Sequence |
|---|---|---|---|---|---|
| D614G | | Worldwide | D614G | Increased infectivity, mainly spreading since June 2020 | P0DTC2 |
| B.1.1.7 | 501Y.V1 | The UK/Worldwide (primarily in the United States) | D614G, N501Y, P681H | Increased infectivity | B.1.1.7 lineage |
| B.1.351 | 501.V2 or N501Y.V2 | South Africa | N501Y, **E484K\***, K417N | Increased infectivity **\*Escape mutation\*** | B.1.351 lineage |
| B.1.1.248 | P1 | Brazil | N501Y, **E484K\***, K417T | Increased infectivity **\*Escape mutation\*** | P1 lineage |

A coronavirus genome is capped, polyadenylated, and covered by a nucleocapsid protein. A coronavirus particle includes a viral envelope containing a type I fusion glycoprotein called spike (S) protein. Most coronaviruses have a common genomic structure, in which a replicase gene is contained in the 5' part of the genome and a structural gene is contained in the 3' part of the genome.

The coronavirus spike (S) protein is a class I fusion glycoprotein originally synthesized as a precursor protein. A single precursor S polypeptide forms a homotrimer and is glycosylated and processed in the Golgi body to remove a signal peptide, and is cleaved by a cellular protease to produce separate S1 and S2 polypeptide chains, which are still bound as an S1/S2 protomer in the homotrimer and are therefore heterodimeric trimers. An S1 subunit is located at a far end of a viral membrane and contains a receptor binding domain (RBD) that mediates attachment of a virus to a host receptor thereof. An S2 subunit contains fusion protein machinery, such as two heptapeptide repeats (HR1 and HR2) unique to a fusion peptide and a fusion glycoprotein, a central helix, a transmembrane domain, and a cytosolic tail domain.

In some cases, the coronavirus antigen or immunogen is a coronavirus S protein peptide in a pre-fusion conformation. The coronavirus S protein peptide is a structural conformation adopted by the extracellular domain of the coronavirus spike (S) protein after being processed into the mature coronavirus S protein in the secretory system and before a fusion event that triggers a transition of the coronavirus S protein to a post-fusion conformation. Kirchdoerfer et al., "Pre-fusion structure of a human coronavirus spike protein," Nature, 531: 118-121, 2016 provides a three-dimensional structure of an exemplary coronavirus S protein (HKU1-CoV) in the pre-fusion conformation, which is incorporated by reference in its entirety for all purposes.

In some cases, the coronavirus antigen or immunogen includes one or more amino acid substitutions, deletions, or insertions compared to the natural coronavirus S sequence, which provides increased pre-fusion conformation retention compared to the coronavirus S extracellular domain trimer formed by the corresponding natural coronavirus S sequence. The "stabilization" of the pre-fusion conformation by the one or more amino acid substitutions, deletions, or insertions can be, for example, energy stabilization (such as reducing the energy of the pre-fusion conformation relative to the post-fusion open conformation) and/or kinetic stabilization (such as reducing the conversion rate from the pre-fusion conformation to the post-fusion conformation). In addition, compared to the corresponding natural coronavirus S sequence, the stabilization of the coronavirus S extracellular domain trimer in the pre-fusion conformation may include increased resistance to denaturation. This present disclosure provides a method for determining whether the coronavirus S extracellular domain trimer is in the pre-fusion conformation, including (but not limited to) negative staining electron microscopy and antibody binding analysis that use a pre-fusion conformation-specific antibody.

In some cases, the coronavirus antigen or immunogen is a fragment of the S protein peptide. In some embodiments, the antigen or immunogen is an epitope of the S protein peptide. Epitopes include antigenic determinant chemical groups or peptide sequences on molecules. The epitopes are antigenic, thereby triggering a specific immune response. For example, epitopes are antigenic regions to which B cells and/or T cells respond. Antibodies can bind to specific antigenic epitopes, such as the epitopes on the coronavirus S extracellular domain. Epitopes can be formed by either continuous amino acids or non-continuous amino acids juxtaposed by tertiary folding of proteins. In some embodiments, a coronavirus epitope is a linear epitope. In some embodiments, the coronavirus epitope is a conformational epitope. In some embodiments, the coronavirus epitope is a neutralizing epitope site. In some embodiments, all neutralizing epitopes of the coronavirus S protein peptide or the fragments thereof are present as antigens or immunogens.

In some cases, for example, when a viral antigen or immunogen is a fragment of an S protein peptide, only a single subunit of the S protein peptide exists, and the single subunit of the S protein peptide is trimerized. In some embodiments, a viral antigen or immunogen includes a signal peptide, an S1 subunit peptide, an S2 subunit peptide, or any combination thereof. In some embodiments, the viral antigen or immunogen includes a signal peptide, a receptor binding domain (RBD) peptide, a receptor binding motif (RBM) peptide, a fusion peptide (FP), a heptapeptide repeat 1 (HR1) peptide or a heptapeptide repeat 2 (HR2) peptide or any combination thereof. In some embodiments, the viral antigen or immunogen includes a receptor binding domain (RBD) of an S protein. In some embodiments, the viral antigen or immunogen includes an S1 subunit and an S2 subunit of an S protein. In some embodiments, the viral antigen or immunogen includes an S1 subunit of an S protein, but does not include an S2 subunit. In some embodiments, the viral antigen or immunogen includes an S2 subunit of an S protein, but does not include an S1 subunit. In some embodiments, the viral antigen or immunogen does not contain a transmembrane (TM) domain peptide and/or a cytoplasmic (CP) domain peptide.

In some embodiments, the viral antigen or immunogen includes a protease cleavage site, in which the protease is optionally furin, trypsin, factor Xa, or cathepsin L.

In some embodiments, the viral antigen or immunogen does not contain a protease cleavage site, in which the protease is optionally furin, trypsin, factor Xa or cathepsin L, or a protease cleavage site containing mutations that cannot be cleaved by a protease.

In some embodiments, the viral antigen or immunogen is a SARS-CoV-2 antigen including at least one SARS-CoV-2 protein or a fragment thereof. In some embodiments, the SARS-CoV-2 antigen is recognized by a SARS-CoV-2 reactive antibody and/or a T cell. In some embodiments, the SARS-CoV-2 antigen is an inactivated whole virus. In some embodiments, the SARS-CoV-2 antigen includes a subunit of a virus. In some embodiments, the SARS-CoV-2 antigen includes a SARS-CoV-2 structural protein or a fragment thereof. In some embodiments, the SARS-CoV-2 structural protein includes one or more of a group consisting of a spike (S) protein, membrane (M) protein, nucleocapsid (N) protein, and envelope (E) protein. In some embodiments, the SARS-CoV-2 antigen includes or further includes a SARS-CoV-2 nonstructural protein or a fragment thereof. A nucleotide sequence of a representative SARS-CoV-2 isolate (Hu-1) is recorded as GenBank No. MN908947.3 (Wu et al., Nature, 579:265-269, 2020), which is incorporated by reference in its entirety for all purposes.

In some embodiments, the viral antigen or immunogen includes a sequence set forth in any one of SEQ ID NOs: 81-84. In some embodiments, the viral antigen or immunogen includes an amino acid sequence having at least or about 80%, 85%, 90%, 92%, 95%, 97%, or 99% sequence identity with SEQ ID NO: 82 as shown below. In some embodiments, the viral antigen or immunogen includes an RBD trimer, for example, a SARS-CoV-2 RBD sequence linked to any one of SEQ ID NOs: 67-80.

In some embodiments, the viral antigen or immunogen includes a sequence set forth in SEQ ID NO: 55. In some embodiments, the viral antigen or immunogen includes an amino acid sequence having at least or about 80%, 85%, 90%, 92%, 95%, or 97% sequence identity with SEQ ID NO: 55 (the underlined sequence represents a receptor-binding motif (RBM) within receptor binding domain (RBD) of Thr333-Gly526 (in bold)) as shown below. In some embodiments, the viral antigen or immunogen includes an RBD trimer, for example, a SARS-CoV-2 RBD sequence linked to any one of SEQ ID NOs: 67-80.

In some embodiments, the viral antigen or immunogen includes a spike glycoprotein sequence of a Hu-1 coronavirus (such as NC_045512). In some embodiments, the viral antigen or immunogen includes a spike glycoprotein sequence of a virus in a B.1.526 lineage. In some embodiments, the viral antigen or immunogen includes a spike glycoprotein sequence of a Cluster 5 (ΔFVI-spike) virus. In some embodiments, the viral antigen or immunogen includes a spike glycoprotein sequence of a virus in a B.1.1.529 lineage. In some embodiments, the viral antigen or immunogen includes a spike glycoprotein sequence of a virus in a B.1.1.7 lineage. In some embodiments, the viral antigen or immunogen includes a spike glycoprotein sequence of a virus in a B.1.1.207 lineage. In some embodiments, the viral antigen or immunogen includes a spike glycoprotein sequence of a virus in a B.1.1.317 lineage. In some embodiments, the viral antigen or immunogen includes a spike glycoprotein sequence of a virus in a B.1.1.318 lineage. In some embodiments, the viral antigen or immunogen includes a spike glycoprotein sequence of a virus in a P.1 lineage. In some embodiments, the viral antigen or immunogen includes a spike glycoprotein sequence of a virus in a B.1.351 lineage. In some embodiments, the viral antigen or immunogen includes a spike glycoprotein sequence of a virus in a B.1.429/CAL.20C lineage. In some embodiments, the viral antigen or immunogen includes a spike glycoprotein sequence of a virus in a B.1.525 lineage. In some embodiments, the viral antigen or immunogen includes a spike glycoprotein sequence of a virus in a B.1.526 lineage. In some embodiments, the viral antigen or immunogen includes a spike glycoprotein sequence of a virus in a B.1.617 lineage. In some embodiments, the viral antigen or immunogen includes a spike glycoprotein sequence of a virus in a B.1.617.2 lineage. In some embodiments, the viral antigen or immunogen includes a spike glycoprotein sequence of a virus in a B.1.618 lineage. In some embodiments, the viral antigen or immunogen includes a spike glycoprotein sequence of a virus in a B.1.620 lineage. In some embodiments, the viral antigen or immunogen includes a spike glycoprotein sequence of a virus in a P.2 lineage. In some embodiments, the viral antigen or immunogen includes a spike glycoprotein sequence of a virus in a P.3 lineage. In some embodiments, the viral antigen or immunogen includes a spike glycoprotein sequence of a virus in a B.1.1.143 lineage. In some embodiments, the viral antigen or immunogen includes a spike glycoprotein sequence of a virus in an A.23.1 lineage. In some embodiments, the viral antigen or immunogen includes a spike glycoprotein sequence of a virus in a B.1.617 lineage. In some embodiments, the viral antigen or immunogen includes a sequence of a spike glycoprotein derived from any two or more viruses (in any suitable combination) selected from a group consisting of Hu-1, a virus in the B.1.526 lineage, a virus in the B.1.1.7 lineage, a virus in the P.1 lineage, a virus in the B.1.351 lineage, a virus in the P.2 lineage, a virus in the B.1.1.143 lineage, a virus in the A.23.1 lineage, and a virus in the B.1.617 lineage.

In some embodiments, the viral antigen or immunogen includes T95I, G142D, Δ143-145, and/or T478K, for example, as in a B.1.1.529 Omicron variant.

In some embodiments, the viral antigen or immunogen includes E484K and/or S477N, for example, as in a B.1.526 variant. In some embodiments, the viral antigen or immunogen includes Δ400-402 (ΔFVI), for example, as in a Cluster 5 (ΔFVI-spike) variant. In some embodiments, the viral antigen or immunogen includes Δ69-70 (ΔHV), Δ144 (ΔY), N501Y, A570D, D614G, P681H, T716I, S982A, and/or D1118H, for example, as in a B.1.1.7 variant. In some embodiments, the viral antigen or immunogen includes P681H, for example, as in a B.1.1.207 variant. In some embodiments, the viral antigen or immunogen includes L18F, T20N, P26S, D138Y, R190S, K417T, E484K, N501Y, D614G, H655Y, T1027I, and/or V1176F, for example, as in a P.1 variant. In some embodiments, the viral antigen or immunogen includes E484K, for example, as in a P.2 variant. In some embodiments, the viral antigen or immunogen includes E484K and/or N501Y, for example, as in a P.3 variant. In some embodiments, the viral antigen or immunogen includes L18F, D80A, D215G, Δ242-244 (ΔLAL), R246I, K417N, E484K, N501Y, D614G, and/or A701V, for example, as in a B.1.351 variant. In some embodiments, the viral antigen or immunogen includes S131, W152C, and/or L452R, for example, as in a B.1.429/CAL.20C variant. In some embodiments, the viral antigen or immunogen includes Δ69-70 (ΔHV), E484K, and/or F888L, for example, as in a B.1.525 variant. In some embodiments, the viral antigen or immunogen includes G142D, L452R, E484Q, and/or P681R, for example, as in a B.1.617 variant. In some embodiments, the viral antigen or immunogen includes G142D, L452R, and/or P681R, for example, as in a B.1.617.2 variant. In some embodiments, the viral antigen or immunogen includes E484K, for example, as in a B.1.618 variant. In some embodiments, the viral antigen or immunogen may include a fusion polypeptide (protomer), which includes any one or more of the above mutations in any appropriate combination. In some embodiments, the viral antigen or immunogen may include trimers of three fusion polypeptides, and any one of the three protomeric fusion polypeptides may include any one or more of the above mutations in any appropriate combination. In some embodiments, two or all three of the three protomer fusion polypeptides that form the trimers can include different mutations and/or different combinations of mutations in each protomer. In some embodiments, the viral antigen or immunogen may include a mixture of the trimers, and each trimer may include different mutations and/or different combinations of mutations.

In some embodiments, the viral antigen or immunogen includes any one, two, three, four, five, or more mutations selected from a group consisting of mutations (such as substitutions, deletions, and/or insertions) at amino acid positions 13, 18, 20, 26, 69, 70, 80, 138, 142, 144, 152, 190, 215, 242, 243, 244, 246, 400, 401, 402, 417, 440, 452, 477, 484, 501, 570, 614, 655, 681, 682, 683, 684, 685, 701, 716, 888, 982, 1027, 1118 and 1176 of SEQ ID NO: 55. In some embodiments, the viral antigen or immunogen includes any one, two, three, four, five, six, seven, eight, or all mutations selected from a group consisting of mutations (such as substitutions, deletions. and/or insertions) at amino acid positions 440, 452, 477, 484, 501, 614, 655, 681 and 701. In some embodiments, the viral antigen or immunogen includes a chimeric polypeptide containing sequences from different viruses, such as one or more mutations from a first variant of coronavirus and one or more mutations from a second variant of coronavirus different from the first variant. In some embodiments, such a chimeric viral antigen or immunogen (or a combination of chimeric viral antigens or immunogens) can be used to trigger a broad immune response against the first and the second variants of coronavirus.

In some embodiments, the viral antigen or immunogen includes any one, two, three, four, five, or more mutations selected from a group consisting of T95I, G142D, Δ143-145, T478K, S13I, L18F, T20N, P26S, Δ69-70 (ΔHV), D80A, D138Y, G142D, Δ144 (ΔY), W152C, R190S, D215G, Δ242-244 (ΔLAL), R246I, Δ400-402 (ΔFVI), K417T, K417N, N440K, L452R, S477N, S477G, E484K, E484Q, N501Y, A570D, D614G, H655Y, P681H, P681R, R682G, R683S, R685G, A701V, T716I, F888L, S982A, T1027I, D1118H, and V1176F. In some embodiments, the viral antigen or immunogen includes any one, two, three, four, five, or more mutations selected from a group consisting of N440K, L452R, S477G, S477N, E484K, E484Q, N501Y, D614G, H655Y, P681H, P681R, and A701V.

In some embodiments, a SARS-CoV-2 antigen includes a truncated S protein lacking a signal peptide, and a transmembrane domain and a cytoplasmic domain of a full-length S protein. In some embodiments, the SARS-CoV-2 antigen is a recombinant protein, while in other embodiments, the SARS-CoV-2 antigen is purified from a viral particle. In some preferred embodiments, the SARS-CoV-2 antigen is an isolated antigen.

In some embodiments, the viral antigen or immunogen includes a sequence set forth in any one of SEQ ID NOs: 27-51. In some embodiments, the viral antigen or immunogen includes an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity with a sequence set forth in any one of SEQ ID NOs: 27-51. The amino acid sequence includes a sequence containing a substitution, a deletion, and/or an insertion at one or more amino acid positions selected from a group consisting of 13, 18, 20, 26, 69, 70, 80, 138, 142, 144, 152, 190, 215, 242, 243, 244, 246, 400, 401, 402, 417, 440, 452, 477, 484, 501, 570, 614, 655, 681, 682, 683, 684, 685, 701, 716, 888, 982, 1027, 1118, and 1176 (with respect to SEQ ID NO: 55 amino acid positions). In some embodiments, the viral antigen or immunogen includes a variant of a sequence set forth in any one of SEQ ID NOs: 27-51, and the variant include any one, two, three, four, five or more mutations selected from a group consisting of S13I, L18F, T20N, P26S, Δ69-70 (ΔHV), D80A, D138Y, G142D, Δ144 (ΔY), W152C, R190S, D215G, Δ242-244 (ΔLAL), R246I, Δ400-402 (ΔFVI), K417T, K417N, N440K, L452R, S477N, S477G, E484K, E484Q, N501Y, A570D, D614G, H655Y, P681H, P681R, R682G, R683S, R685G, A701V, T716I, F888L, S982A, T1027I, D1118H, and V1176F.

In some embodiments, the viral antigen or immunogen includes a sequence set forth in SEQ ID NO: 52. In some embodiments, the viral antigen or immunogen includes an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity with SEQ ID NO: 52.

In some embodiments, the viral antigen or immunogen includes a signal peptide. In some embodiments, the viral antigen or immunogen includes a sequence set forth in SEQ ID NO: 53. In some embodiments, the viral antigen or immunogen includes an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity with SEQ ID NO: 53.

In some embodiments, the viral antigen or immunogen includes a sequence set forth in SEQ ID NO: 54. In some embodiments, the viral antigen or immunogen includes an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity with SEQ ID NO: 54.

In some embodiments, the viral antigen or immunogen includes a sequence set forth in SEQ ID NO: 55. In some embodiments, the viral antigen or immunogen includes an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity with SEQ ID NO: 55. The amino acid sequence includes a sequence containing a substitution, a deletion, and/or an insertion at one or more amino acid positions selected from a group consisting of 13, 18, 20, 26, 69, 70, 80, 138, 142, 144, 152, 190, 215, 242, 243, 244, 246, 400, 401, 402, 417, 440, 452, 477, 484, 501, 570, 614, 655, 681, 682, 683, 684, 685, 701, 716, 888, 982, 1027, 1118, and 1176. In some embodiments, the viral antigen or immunogen includes a variant of SEQ ID NO: 55, and the variant include any one, two, three, four, five or more mutations selected from a group consisting of S13I, L18F, T20N, P26S, Δ69-70 (ΔHV), D80A, D138Y, G142D, Δ144 (ΔY), W152C, R190S, D215G, Δ242-244 (ΔLAL), R246I, Δ400-402 (ΔFVI), K417T, K417N, N440K, L452R, S477N, S477G, E484K, E484Q, N501Y, A570D, D614G, H655Y, P681H, P681R, R682G, R683S, R685G, A701V, T716I, F888L, S982A, T1027I, D1118H, and V1176F.

In some embodiments, the viral antigen or immunogen includes a sequence set forth in SEQ ID NO: 56. In some embodiments, the viral antigen or immunogen includes an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity with a sequence set forth in any one of SEQ ID NO: 56. The amino acid sequence includes a sequence containing a substitution, a deletion, and/or an insertion at one or more amino acid positions selected from a group consisting of 13, 18, 20, 26, 69, 70, 80, 138, 142, 144, 152, 190, 215, 242, 243, 244, 246, 400, 401, 402, 417, 440, 452, 477, 484, 501, 570, 614, 655, 681, 682, 683, 684, 685, 701, 716, 888, 982, 1027, 1118, and 1176 (with respect to SEQ ID NO: 55 amino acid positions). In some embodiments, the viral antigen or immunogen includes a variant of SEQ ID NO: 56, and the variant include any one, two, three, four, five or more mutations selected from a group consisting of S13I, L18F, T20N, P26S, Δ69-70 (ΔHV), D80A, D138Y, G142D, Δ144 (ΔY), W152C, R190S, D215G, Δ242-244 (ΔLAL), R246I, Δ400-402 (ΔFVI), K417T, K417N, N440K, L452R, S477N, S477G, E484K, E484Q, N501Y, A570D, D614G, H655Y, P681H, P681R, R682G, R683S, R685G, A701V, T716I, F888L, S982A, T1027I, D1118H, and V1176F.

In some embodiments, the viral antigen or immunogen includes a sequence set forth in any one of SEQ ID NOs: 57-58. In some embodiments, the viral antigen or immunogen includes an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity with a sequence set forth in any one of SEQ ID NOs: 57-58. The amino acid sequence includes a sequence containing a substitution, a deletion, and/or an insertion at one or more amino acid positions of a sequence set forth in any one of SEQ ID NOs: 57-58.

In some embodiments, the viral antigen or immunogen includes a sequence set forth in SEQ ID NO: 59. In some embodiments, the viral antigen or immunogen includes a sequence containing a substitution, a deletion, and/or an insertion at one or more amino acid positions of SEQ ID NO: 59. In some embodiments, the viral antigen or immunogen includes a sequence set forth in SEQ ID NO: 60. In some embodiments, the viral antigen or immunogen includes a sequence containing a substitution, a deletion, and/or an insertion at one or more amino acid positions of SEQ ID NO: 60.

In some embodiments, the viral antigen or immunogen includes a sequence set forth in any one of SEQ ID NOs: 61-65 and 81-84. In some embodiments, the viral antigen or immunogen includes an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity with a sequence set forth in any one of SEQ ID NOs: 61-65 and 81-84. The amino acid sequence includes a sequence containing a substitution, a deletion, and/or an insertion at one or more amino acid positions of a sequence set forth in any one of SEQ ID NOs: 61-65 and 81-84.

In some embodiments, the viral antigen or immunogen includes a sequence set forth in any one of SEQ ID NOs: 122-123 and 126-127. In some embodiments, the viral antigen or immunogen includes an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity with SEQ ID NO: 122. The amino acid sequence includes a sequence containing a substitution, a deletion, and/or an insertion at one or more amino acid positions of a sequence set forth in any one of SEQ ID NOs: 122-123 and 126-127. In some embodiments, the viral antigen or immunogen includes an RBD trimer, for example, a SARS-CoV-2 RBD sequence linked to any one of SEQ ID NOs: 67-80.

In some embodiments, the viral antigen or immunogen does not include a transmembrane domain such as SEQ ID NO: 66 or a portion thereof. In some embodiments, the coronavirus antigen or immunogen includes a soluble S protein peptide. In some embodiments, the soluble S protein peptide lacks a TM domain peptide and a CP domain peptide. In some embodiments, the soluble S protein peptide is not bound to a lipid bilayer, such as a membrane or a viral envelope.

In some embodiments, an S protein peptide is generated from a codon-optimized nucleic acid sequence. In some embodiments, the S protein peptide is generated from a non-codon-optimized nucleic acid sequence.

In some embodiments, the viral antigen or immunogen mentioned herein may include a recombinant polypeptide or fusion peptide containing the viral antigen or immunogen. The term viral antigen or immunogen can be used to refer to a protein containing a coronavirus antigen or immunogen. In some cases, the coronavirus antigen or immunogen is a coronavirus protein peptide provided herein. The viral antigen or immunogen mentioned herein can be used in a primary series, an additional dose, and/or a booster dose. Independently, the primary series, the additional dose, and/or any one or more booster doses may not use or use the adjuvant. If the adjuvant is used, the optional adjuvant may include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

## II. Recombinant peptide and protein

It is expected that the coronavirus antigen and the immunogen provided herein, such as the S protein peptide (see Section I), can bind with another protein or peptide, such as via linkage, to form a recombinant polypeptide, including a fusion peptide. In some embodiments, the single recombinant polypeptide (such as a monomer) provided herein binds to form a multimer of the recombinant polypeptide, such as a trimer. In some embodiments, the binding of a single recombinant polypeptide monomer occurs through a covalent interaction. In some embodiments, the binding of a single recombinant polypeptide monomer occurs through a non-covalent interaction. In some embodiments, the interaction (such as covalent or non-covalent) is affected by a protein or peptide linked to a coronavirus antigen or immunogen (such as an S protein peptide). In some embodiments, for example, when the coronavirus antigen or immunogen is the S protein peptide described herein, the protein or peptide to which the coronavirus antigen or immunogen is linked can be selected so as to retain the natural homotrimeric structure of a glycoprotein. This can help trigger a strong and effective immunogenicity response to the S protein peptide. For example, the retention and/or maintenance of the native conformation of the coronavirus antigen or immunogen (such as the S protein peptide) can improve or allow access to an antigenic site that can produce an immune response. In some cases, the recombinant polypeptide containing the S protein peptide described herein (for example, see Section I) is optionally referred to as a recombinant S antigen, a recombinant S immunogen, or a recombinant S protein in the present disclosure.

It is further expected that in some cases, the recombinant polypeptide or a multimeric recombinant polypeptide thereof aggregates or can aggregate to form a protein or complex containing a plurality of coronavirus antigens and/or immunogenic recombinant polypeptides. The formation of this protein can be conducive to the production of a strong and effective immunogenicity response to the coronavirus antigen and/or immunogen. For example, a protein containing a plurality of recombinant polypeptides is formed, thereby forming a plurality of coronavirus antigens, such as the coronavirus S protein peptide, which can retain the tertiary and/or quaternary structure of the viral antigen and allow a mounted immune response against the natural structure. In some cases, aggregation can provide structural stability to the coronavirus antigen or immunogen, which in turn can provide access to a potential antigenic site that can promote an immune response.

*1. Fusion peptide and recombinant polypeptide*

In some embodiments, a coronavirus antigen or immunogen can be linked to a trimerization domain at a C-terminus thereof (C-terminal linkage) to promote trimerization of a monomer. In some embodiments, trimerization stabilizes a membrane-proximal aspect of the coronavirus antigen or immunogen (such as a coronavirus S protein peptide) in a trimeric configuration.

Non-limiting examples of an exogenous multimerization domain that promote the stabilization of a soluble recombinant protein trimer include: a GCN4 leucine zipper (Harbury et al. 1993 Science 262:1401-1407), a trimerization motif of pulmonary surfactant protein (Hoppe et al. 1994 FEBS Lett 344:191-195), collagen (McAlinden et al. 2003 J Biol Chem 278:42200-42207) and a bacteriophage T4 fibritin Foldon (Miroshnikov et al. 1998 Protein Eng 11:329-414), any of which can be linked to the coronavirus antigen or immunogen described herein (for example, by linking to a C-terminus of an S peptide) to promote the trimerization of a recombinant viral antigen or immunogen. The above documents are incorporated by reference in their entireties for all purposes. See also U.S. Patent Nos. 7,268,116, 7,666,837, 7,691,815, 10,618,949, 10,906,944, and 10,960,070, and US 2020/0009244, which are incorporated herein by reference in their entireties for all purposes.

In some embodiments, one or more peptide linkers (such as a glycine-serine linker, such as 10 amino acid glycine-serine peptide linkers) can be used to link the recombinant viral antigen or immunogen to a multimerization domain. As long as the recombinant viral antigen or immunogen trimer maintains the desired properties (such as a pre-fusion conformation), the trimer may include any stable mutations provided herein (or a combination thereof). In some embodiments, a recombinant polypeptide or fusion protein includes a first sequence set forth in any one of SEQ ID NOs: 27-66 and 81-84, the first sequence is linked to a second sequence set forth in any one of SEQ ID NOs: 67-80, in which a C-terminus of the first sequence is directly linked to an N-terminus of the second sequence. In some embodiments, a recombinant polypeptide or fusion protein includes a first sequence set forth in any one of SEQ ID NOs: 27-66 and 81-84, the first sequence is linked to a second sequence set forth in any one of SEQ ID NOs: 67-80, in which a C-terminus of the first sequence is indirectly linked to an N-terminus of the second sequence, for example, through a linker. In some embodiments, the linker includes a sequence containing a glycine-X-Y repeat.

To be therapeutically feasible, the trimerized protein portion required for biopharmaceutical design should meet the following criteria. Ideally, the trimerized protein portion should be part of a naturally secreted protein, such as immunoglobulin Fc, which is also abundant in circulation (non-toxic), derived from humans (lacking immunogenicity), relatively stable (long half-life), and capable of effective self-trimerization. The self-trimerization is enhanced by an interchain covalent disulfide bond so that a trimerized coronavirus antigen or immunogen is structurally stable.

Collagen belongs to the fibrin family, which is the main component of an extracellular matrix. Collagen is the most abundant protein in mammals, accounting for nearly 25% of total body protein. Collagen plays a crucial structural role in the formation of bones, tendons, skin, cornea, cartilage, blood vessels, and teeth. The fibrous types of collagen I, II, III, IV, V, and XI are all synthesized into larger trimeric precursors called procollagens, in which the central uninterrupted triple-helical domain composed of hundreds of "G-X-Y" repeats (or glycine repeats) is flanked by a non-collagen domain (NC), an N-propeptide, and a C-propeptide. Both C-terminal and N-terminal extensions undergo proteolysis after procollagen secretion, an event that triggers the assembly of mature proteins into collagen fibrils to form an insoluble cell matrix. BMP-1 is a protease that recognizes a specific peptide sequence of procollagen near the junction between a glycine repeat and a collagen C-prodomain and is responsible for removing a propeptide. The concentration of an exfoliated trimeric C-propeptide of type I collagen is in the range of 50-300 ng/mL in human serum from a normal adult, with a higher level found in a child, indicating active bone formation. In a population with familial high serum concentration of the C-propeptide of type I collagen, the level can be as high as 1-6 μg/mL without obvious abnormalities, indicating that the C-propeptide is non-toxic. Structural studies of the trimeric C-propeptide of collagen show that the trimeric C-propeptide of collagen is a trilobate structure with all three subunits clustering together near an N-terminal junction region thereof, linking to the rest of a procollagen molecule. The geometric shape of this protruding protein fuses in one direction, similar to an Fc dimer.

Types I, IV, V, and XI collagen are mainly assembled into heterotrimeric forms consisting of two $\alpha$-1 chains and one $\alpha$-2 chain (for types I, IV, V) or three different chains (for type XI), which are highly homologous in sequence. Both type II and type III collagen are homotrimers of the $\alpha$-1 chain. For type I collagen (the most abundant form of collagen), a stable $\alpha$ (I) homotrimer is also formed and exists at variable levels in different tissues. When overexpressed alone in cells, most of these collagen C-propeptide chains can self-assemble into homotrimers. Although the N-propeptide domain is synthesized first, molecular assembly of trimeric collagen begins with the registration binding of the C-propeptide. It is believed that a C-propeptide complex is stabilized by the formation of an interchain disulfide bond, but the necessity of disulfide bond formation for proper chain registration is unclear. The triple helix of glycine repeats and then spreads from an associated C-terminus to an N-terminus in a zipper-like manner. By using recombinant DNA technology to exchange C-propeptides of different collagen chains, this knowledge creates a non-natural type of collagen matrix. A non-collagen protein (such as cytokine and growth factor) has also been fused to the N-terminus of procollagen or mature collagen to form a new collagen matrix, the purpose of which is to slowly release the non-collagen protein from the cell matrix. However, in the two cases, the C-propeptide needs to be cleaved before recombinant collagen fibers are assembled into an insoluble cell matrix.

Although other protein trimerization domains (such as GCN4 of fibritin from bacteriophage T4 and those of aspartate transcarbamoylase from Escherichia coli) have been previously described to allow trimerization of heterologous proteins, these trimeric proteins are not of human origin and are not naturally secreted proteins. Therefore, any trimeric fusion protein must be synthesized within a cell, which may not only lead to misfolding of naturally secreted proteins (such as soluble receptors) but also make it difficult to purify such fusion proteins from thousands of other intracellular proteins. In addition, the fatal disadvantage of using such non-human protein trimerization domains (such as those from yeast, bacteriophages, and bacteria) in trimeric biopharmaceutical design is their presumed immunogenicity in humans, making such fusion proteins ineffective soon after injection into the human body.

Therefore, the use of collagen in the recombinant polypeptide described herein has many advantages, including: (1) Collagen is the most abundant protein in mammals, accounting for nearly 25% of total body protein; (2) The main form of collagen naturally appears in the form of a trimeric helix and the globular C-propeptide thereof is responsible for the initiation of trimerization; (3) The trimeric C-propeptide of collagen released from the mature collagen by proteolysis naturally occurs in mammalian blood at submicrogram/mL levels and is not known to be toxic to the body; (4) The linear triple-helical region of collagen can be included as a linker, with each residue predicted to have a spacing of 2.9 Å, or can be excluded as part of the fusion protein so that the distance between the protein to be trimerized and the C-propeptide of collagen can be precisely adjusted to achieve optimal biological activity; (5) The recognition site of BMP1, which cleaves the C-propeptide from procollagen, can be mutated or deleted to prevent damage to the trimeric fusion protein; (6) The C-propeptide domain self-trimerizes through the disulfide bond, and the C-propeptide domain provides a universal affinity tag that can be used to purify any secretory fusion protein produced. In some embodiments, the C-propeptide of collagen that binds to the coronavirus antigen and the immunogen (such as the S protein peptide) can be recombined to produce a soluble, covalently linked homotrimeric fusion protein.

In some embodiments, the coronavirus antigen or immunogen is linked to the C-terminal propeptide of collagen to form a recombinant polypeptide. In some embodiments, the C-terminal propeptide of the recombinant polypeptide forms an inter-polypeptide disulfide bond. In some embodiments, the recombinant protein forms a trimer. In some embodiments, the coronavirus antigen or immunogen is the S protein peptide as described in Section I.

For example, a fusion polypeptide can be generated and trimerized by an inter-polypeptide disulfide bond (Cys residues that can form inter-polypeptide disulfide bonds are shown in bold), and the fusion polypeptide includes a signal peptide MFVFLVLLPLVSS (SEQ ID NO: 54) on the N-terminus of the fusion polypeptide in SEQ ID NO: 1.

```
          10        20        30        40        50        60
MFVFLVLLPLVSSQCVNLTTRTQLPPAYTNSFTRGVYYPDKVFRSSVLHSTQDLFLPFFS
          70        80        90       100       110       120
NVTWFHAIHVSGTNGTKRFDNPVLPFNDGVYFASTEKSNIIRGWIFGTTLDSKTQSLLIV
         130       140       150       160       170       180
NNATNVVIKVCEFQFCNDPFLGVYYHKNNKSWMESEFRVYSSANNCTFEYVSQPFLMDLE
         190       200       210       220       230       240
GKQGNFKNLREFVFKNIDGYFKIYSKHTPINLVRDLPQGFSALEPLVDLPIGINITRFQT
         250       260       270       280       290       300
LLALHRSYLTPGDSSSGWTAGAAAYYVGYLQPRTFLLKYNENGTITDAVDCALDPLSETK
         310       320       330       340       350       360
CTLKSFTVEKGIYQTSNFRVQPTESIVRFPNITNLCPFGEVFNATRFASVYAWNRKRISN
         370       380       390       400       410       420
CVADYSVLYNSASFSTFKCYGVSPTKLNDLCFTNVYADSFVIRGDEVRQIAPGQTGKIAD
         430       440       450       460       470       480
YNYKLPDDFTGCVIAWNSNNLDSKVGGNYNYLYRLFRKSNLKPFERDISTEIYQAGSTPC
         490       500       510       520       530       540
NGVEGFNCYFPLQSYGFQPTNGVGYQPYRVVVLSFELLHAPATVCGPKKSTNLVKNKCVN
         550       560       570       580       590       600
FNFNGLTGTGVLTESNKKFLPFQQFGRDIADTTDAVRDPQTLEILDITPCSFGGVSVITP
         610       620       630       640       650       660
GTNTSNQVAVLYQDVNCTEVPVAIHADQLTPTWRVYSTGSNVFQTRAGCLIGAEHVNNSY
         670       680       690       700       710       720
ECDIPIGAGICASYQTQTNSPRRARSVASQSIIAYTMSLGAENSVAYSNNSIAIPTNFTI
         730       740       750       760       770       780
SVTTEILPVSMTKTSVDCTMYICGDSTECSNLLLQYGSFCTQLNRALTGIAVEQDKNTQE
         790       800       810       820       830       840
VFAQVKQIYKTPPIKDFGGFNFSQILPDPSKPSKRSFIEDLLFNKVTLADAGFIKQYGDC
         850       860       870       880       890       900
LGDIAARDLICAQKFNGLTVLPPLLTDEMIAQYTSALLAGTITSGWTFGAGAALQIPFAM
         910       920       930       940       950       960
QMAYRFNGIGVTQNVLYENQKLIANQFNSAIGKIQDSLSSTASALGKLQDVVNQNAQALN
         970       980       990      1000      1010      1020
TLVKQLSSNFGAISSVLNDILSRLDKVEAEVQIDRLITGRLQSLQTYVTQQLIRAAEIRA
        1030      1040      1050      1060      1070      1080
SANLAATKMSECVLGQSKRVDFCGKGYHLMSFPQSAPHGVVFLHVTYVPAQEKNFTTAPA
        1090      1100      1110      1120      1130      1140
ICHDGKAHFPREGVFVSNGTHWFVTQRNFYEPQIITTDNTFVSGNCDVVIGIVNNTVYDP

        1150      1160      1170      1180      1190      1200
LQPELDSFKEELDKYFKNHTSPDVDLGDISGINASVVNIQKEIDRLNEVAKNLNESLIDL
        1210      1220      1230      1240      1250      1260
QELGKYEQYIKRSNGLPGPIGPPGPRGRTGDAGPVGPPGPPGPPGPPGPPSAGFDFSFLP
        1270      1280      1290      1300      1310      1320
QPPQEKAHDGGRYYRANDANVVRDRDLEVDTTLKSLSQQIENIRSPEGSRKNPARTCRDL
        1330      1340      1350      1360      1370      1380
KMCHSDWKSGEYWIDPNQGCNLDAIKVFCNMETGETCVYPTQPSVAQKNWYISKNPKDKR
        1390      1400      1410      1420      1430      1440
HVWFGESMTDGFQFEYGGQGSDPADVAIQLTFLRLMSTEASQNITYHCKNSVAYMDQQTG
        1450      1460      1470      1480      1490      1500
NLKKALLLQGSNEIEIRAEGNSRFTYSVTVDGCTSHTGAWGKTVIEYKTTKTSRLPIIDV
        1510      1520
APLDVGAPDQEFGFDVGPVCFL
```

In some embodiments, the inter-peptide disulfide bond may include one or more or all of Cys15-136, Cys131-166, Cys291-301, Cys379-432, Cys336-361, Cys391-525, Cys480-488, Cys538-590, Cys617-649, Cys662-671, Cys743-749, Cys738-760, Cys840-851, Cys1032-1043, and Cys1082-1126, in any suitable combination. In some embodiments, the fusion polypeptide in the trimer may include one or more glycosylation sites (for example, Asn-linked), for example, at one or more or all of Asn residues at 17, 61, 122, 149, 165, 234, 282, 331, 343, 603, 616, 657, 709, 717, 801, 1074, 1098, and 1134, in any suitable combination.

In some embodiments, the C-terminal propeptide is human collagen. In some embodiments, the C-terminal propeptide includes a C-terminal polypeptide or a fragment thereof of proα1(I), proα1(II), proα1(III), proα1(V), proα1(XI), proα2(I), proα2(V), proα2(XI) or proα3(XI). In some embodiments, the C-terminal propeptide is or includes a C-terminal polypeptide of proα1 (I).

In some embodiments, the C-terminal propeptide is or includes an amino acid sequence set forth in any one of SEQ ID NOs: 67-80. In some embodiments, the C-terminal propeptide is an amino acid sequence having at least or about 85%, 90%, 92%, 95%, or 97% sequence identity with the amino acid sequence set forth in any one of SEQ ID NOs: 67-80.

In some embodiments, the C-terminal propeptide is or includes an amino acid sequence of a collagen trimerization domain (such as a C-propeptide of human α1(I) collagen) with a substitution from aspartic acid (D) to asparagine (N) at the BMP-1 site, for example, as shown in SEQ ID NO: 68, in which RAD is mutated to RAN. In some embodiments, the C-terminal propeptide is or includes an amino acid sequence of a collagen trimerization domain (such as a C-propeptide of human α1(1) collagen) with a substitution from alanine (A) to asparagine (N) at the BMP-1 site, for example, as shown in SEQ ID NO: 69, in which RAD is mutated to RND. In some embodiments, the C-terminal propeptide herein may include a mutated BMP-1 site, for example, RSAN instead of DDAN. In some embodiments, the C-terminal propeptide herein may include a BMP-1 site, for example, a sequence (such as SEQ ID NO: 68 or 69) containing a RAD (e.g., RADDAN) sequence instead of RAN (such as RANDAN) or RND (such as RNDDAN) can be used in the fusion polypeptide disclosed herein. For example, SEQ ID NO: 27 (underlined) or a fragment, variant or mutant thereof can be directly or indirectly linked to SEQ ID NO: 67 (italics) or a fragment, variant or mutant thereof, for example, to form the following fusion protein:

QCVNLTTRTQLPPAYTNSFTRGVYYPDKVFRSSVLHSTQDLFLPFFSNVTWFHAIHVSGTNGTKRFDNPVLPFND
GVYFASTEKSNIIRGWIFGTTLDSKTQSLLIVNNATNVVIKVCEFQFCNDPFLGVYYHKNNKSWMESEFRVYSSAN
NCTFEYVSQPFLMDLEGKQGNFKNLREFVFKNIDGYFKIYSKHTPINLVRDLPQGFSALEPLVDLPIGINITRFQTLL
ALHRSYLTPGDSSSGWTAGAAAYYVGYLQPRTFLLKYNENGTITDAVDCALDPLSETKCTLKSFTVEKGIYQTSN
FRVQPTESIVRFPNITNLCPFGEVFNATRFASVYAWNRKRISNCVADYSVLYNSASFSTFKCYGVSPTKLNDLCFT
NVYADSFVIRGDEVRQIAPGQTGKIADYNYKLPDDFTGCVIAWNSNNLDSKVGGNYNYLYRLFRKSNLKPFERDI
STEIYQAGSTPCNGVEGFNCYFPLQSYGFQPTNGVGYQPYRVVVLSFELLHAPATVCGPKKSTNLVKNKCVNFNF
NGLTGTGVLTESNKKFLPFQQFGRDIADTTDAVRDPQTLEILDITPCSFGGVSVITPGTNTSNQVAVLYQDVNCTE

*VPVAIHADQLTPTWRVYSTGSNVFQTRAGCLIGAEHVNNSYECDIPIGAGICASYQTQTNSPRRARSVASQSIIAYT*
*MSLGAENSVAYSNNSIAIPTNFTISVTTEILPVSMTKTSVDCTMYICGDSTECSNLLLQYGSFCTQLNRALTGIAVE*
*QDKNTQEVFAQVKQIYKTPPIKDFGGFNFSQILPDPSKPSKRSFIEDLLFNKVTLADAGFIKQYGDCLGDIAARDLI*
*CAQKFNGLTVLPPLLTDEMIAQYTSALLAGTITSGWTFGAGAALQIPFAMQMAYRFNGIGVTQNVLYENQKLIAN*
*QFNSAIGKIQDSLSSTASALGKLQDVVNQNAQALNTLVKQLSSNFGAISSVLNDILSRLDKVEAEVQIDRLITGRLQ*
*SLQTYVTQQLIRAAEIRASANLAATKMSECVLGQSKRVDFCGKGYHLMSFPQSAPHGVVFLHVTYVPAQEKNFT*
*TAPAICHDGKAHFPREGVFVSNGTHWFVTQRNFYEPQIITTDNTFVSGNCDVVIGIVNNTVYDPLQPELDSFKEEL*
*DKYFKNHTSPDVDLGDISGINASVVNIQKEIDRLNEVAKNLNESLIDLQELGKYEQYIKRSANVVRDRDLEVDTTLK*
*SLSQQIENIRSPEGSRKNPARTCRDLKMCHSDWKSGEYWIDPNQGCNLDAIKVFCNMETGETCVYPTQPSVAQKNWYIS*
*KNPKDKRHVWFGESMTDGFQFEYGGQGSDPADVAIQLTFLRLMSTEASQNITYHCKNSVAYMDQQTGNLKKALLLQG*
*SNEIEIRAEGNSRFTYSVTVDGCTSHTGAWGKTVIEYKTTKTSRLPIIDVAPLDVGAPDQEFGFDVGPVCFL*

In some embodiments, the C-terminal propeptide is or includes an amino acid sequence, which is a fragment of any one of SEQ ID NOs: 67-80.

In some embodiments, the C-terminal propeptide may include a sequence containing a glycine-X-Y repeat, where X and Y are independently any amino acids, or an amino acid sequence at least 85%, 90%, 92%, 95%, or 97% identical thereto, which is capable of forming inter-polypeptide disulfide bonds and trimerizing the recombinant polypeptide. In some embodiments, X and Y are independently proline or hydroxyproline.

In some cases, where the S protein peptide is linked to the C-terminal propeptide to form a recombinant polypeptide, the recombinant polypeptide forms a trimer, thereby forming a homotrimer of the S protein peptide. In some embodiments, the S protein peptide of the trimerized recombinant polypeptide is in a pre-fusion conformation. In some embodiments, the S protein peptide of the trimerized recombinant polypeptide is in a post-fusion conformation. In some embodiments, the confirmed conformation allows access to different antigenic sites on the S protein peptide. In some embodiments, the antigenic site is an epitope, such as a linear epitope or a conformational epitope. The advantage of having the trimerized recombinant polypeptide is that an immune response can be performed against a plurality of potential and diverse antigenic sites.

In some embodiments, the trimerized recombinant polypeptide includes a single recombinant polypeptide containing the same viral antigen or immunogen. In some embodiments, the trimerized recombinant polypeptide includes a single recombinant polypeptide, each of which contains a viral antigen or immunogen different from other recombinant polypeptides. In some embodiments, the trimerized recombinant polypeptide includes a single recombinant polypeptide, in which one of the single recombinant polypeptides includes a viral antigen or immunogen different from other recombinant polypeptides. In some embodiments, the trimerized recombinant polypeptide includes a single recombinant polypeptide, in which two of the single recombinant polypeptides include the same viral antigen or immunogen, and the viral antigen or immunogen is different from the viral antigen or immunogen contained in the remaining recombinant polypeptide.

In some embodiments, the recombinant polypeptide includes any coronavirus antigen or immunogen described in Section I. In some embodiments, the recombinant polypeptide includes any coronavirus antigen or immunogen described in Section I linked to the C-terminal propeptide of collagen as described herein.

In some embodiments, the immunogen includes a recombinant SARS-CoV or SARS-CoV-2 S extracellular domain trimer, such as a SARS-CoV-2 Omicron (B.1.1.529) coronavirus S extracellular domain trimer, which includes a protomer containing one or more proline substitutions (for example, two, for example, two consecutive) at or near the boundary between the HR1 domain and the central helical domain. The proline substitutions stabilize the S extracellular domain trimer in a pre-fusion conformation. In some such embodiments, the one or more (for example, two, for example, two consecutive) proline substitutions that stabilize the S extracellular domain trimer in a pre-fusion conformation are between the 15 N-terminal amino acids of the C-terminal residues of HR1 and the 5 C-terminal amino acids of the N-terminal residues of the central helix.

In some embodiments, the one or more (for example, two, for example, two consecutive) proline substitutions stabilize the S extracellular domain trimer of a coronavirus (such as SARS-CoV or SARS-CoV-2) in a pre-fusion conformation, such as a SARS-CoV-2 Omicron (B.1.1.529) coronavirus S extracellular domain trimer. In some embodiments, the SARS-CoV-2 S protein peptide includes a mutation from 986K/987V to 986P/987P.

In some embodiments, the stabilized S extracellular domain trimer of a recombinant coronavirus (such as SARS-CoV or SARS-CoV-2) in a pre-fusion conformation includes a single-stranded S extracellular domain protomer, which contains mutations at S1/S2 and/or S2' protease cleavage sites to prevent protease cleavage at these sites. In some embodiments, the SARS-CoV-2 S protein peptide includes a mutation from 685R to 685A. Exemplary protease cleavage sites for various viruses are shown below:

| Coronavirus | S1/S2, site 1 | S1/S2, site 2 | S2' |
|---|---|---|---|
| 2019-nCoV | SPRRAR↓SVAS | IAY↓TMS | SKPSKR↓SF |
| CoV-ZX21 | TASILR↓STGQ | IAY↓TMS | SKPSKR↓SF |
| Bat-AC45 | TASILR↓STGQ | IAY↓TMS | SKPSKR↓SF |
| SARS-CoV | TVSLLR↓STGQ | IAY↓TMS | SKPSKR↓SF |
| BM48-31 | SSTLVR↓SGGH | LAY↓TMS | SKPTKR↓SF |
| HKU9-1 | ADSLPR↓LQLV | VNY↓DPL | GATTYR↓SA |
| MERS-CoV | TPR3CR↓SVPG | | GSRSAR↓SA |
| HKU1 | SRRKRR↓SISA | | CGSSSR↓SF |
| HCoV-OC43 | KNRRSR↓GAITT | | SKASSR↓SA |
| HCoV-229E | IAVQPR↓NVSYD | | SRVAGR↓SA |
| HCoV-NL63 | IVRPR↓HSSDN | | SRIAGR↓SA |

In some embodiments, the protomer of the S extracellular domain trimer of the recombinant coronavirus (such as SARS-CoV or SARS-CoV-2) stabilized in a pre-fusion conformation by one or more proline substitutions (such as 986P/987P substitutions) includes additional modifications for stabilization in a pre-fusion conformation, such as mutations at protease cleavage sites to prevent protease cleavage.

With reference to the SARS-CoV-2 S protein sequence provided as SEQ ID NO: 55, the extracellular domain includes a signal peptide (SP), which is removed during cell processing; an N-terminal domain (NTD); a receptor binding domain (RBD); one or more S1/S2 cleavage sites; a fusion peptide (FP); an internal fusion peptide (IFP); and a heptapeptide repeat 1/2 (HR1/2) and a transmembrane domain (TM). Exemplary sources of the sequences can be found at ncbi.nlm.nih.gov/nuccore/MN908947.3, ncbi.nlm.nih.gov/nuccore/MN908947, and ncbi.nlm.nih.gov/nuccore/MN908947.2. Additional sequences can be found at ncbi.nlm.nih.gov/genbank/sars-cov-2-seqs/, including the complete genome of the pneumonia virus isolate Hu-1.

In some embodiments, a protomer of the SARS-CoV-2 S extracellular domain trimer that is stable before fusion, such as the protomer of the SARS-CoV-2 Omicron (B.1.1.529) coronavirus S extracellular domain trimer, may have an NTD, a RBD, an S1 (at S1/S2 site 1 or S1/S2 site 2), an FP, an IFP, an HR1, an HR2, or a C-terminal residue of the C-terminal residue of the extracellular domain (the C-terminal residue of the C-terminal residue of the extracellular domain can be linked to, for example, a trimerization domain or a transmembrane domain). The position numbering of the S protein may vary between SARS-CoV strains, but sequences can be aligned to determine associated domains and cleavage sites. It should be understood that some residues (for example, up to 10) on the N-terminus and C-terminus of any extracellular domain fragment can be removed or modified in the disclosed immunogen without reducing the effectiveness of the S extracellular domain trimer as an immunogen.

In some embodiments, the recombinant polypeptide is or includes an NTD peptide of a SARS-CoV or SARS-CoV-2 S protein. In some embodiments, the recombinant polypeptide is or includes an RBD peptide of a SARS-CoV or SARS-CoV-2 S protein. In some embodiments, the recombinant polypeptide is or includes an NTD peptide and an RBD peptide of a SARS-CoV or SARS-CoV-2 S protein. In some embodiments, the recombinant polypeptide is or includes an S1 domain peptide of a SARS-CoV or SARS-CoV-2 S protein. In some embodiments, the recombinant polypeptide is or includes an S2 domain peptide of a SARS-CoV or SARS-CoV-2 S protein.

An exemplary SARS-CoV-1 S recombinant polypeptide without a signal peptide is provided in SEQ ID NO: 26 (1491 aa).

The above SARS-CoV-1 S recombinant polypeptide may include an N-terminal signal peptide provided in SEQ ID NO: 53.

An exemplary SARS-CoV-2 S recombinant polypeptide without a signal peptide is provided in SEQ ID NO: 1.

The above SARS-CoV-2 S recombinant polypeptide may include an N-terminal signal peptide provided in SEQ ID NO: 54.

An exemplary SARS-CoV-2 S recombinant polypeptide without a signal peptide is provided in SEQ ID NO: 81 (1506 aa).

```
                    10         20         30         40         50         60
          QCVNLTTRTQ LPPAYTNSFT RGVYYPDKVF RSSVLHSTQD LFLPFFSNVT WFHVISGTNG
                    70         80         90        100        110        120
          TKRFDNPVLP FNDGVYFASI EKSNIIRGWI FGTTLDSKTQ SLLIVNNATN VVIKVCEFQF
                   130        140        150        160        170        180
          CNDPFLDHKN NKSWMESEFR VYSSANNCTF EYVSQPFLMD LEGKQGNFKN LREFVFKNID
                   190        200        210        220        230        240
          GYFKIYSKHT PIIVREPEDL PQGFSALEPL VDLPIGINIT RFQTLLALHR SYLTPGDSSS
                   250        260        270        280        290        300
          GWTAGAAAYY VGYLQPRTFL LKYNENGTIT DAVDCALDPL SETKCTLKSF TVEKGIYQTS
                   310        320        330        340        350        360
          NFRVQPTESI VRFPNITNLC PFDEVFNATK FASVYAWNRK RISNCVADYS VLYNLAPFFT
                   370        380        390        400        410        420
          FKCYGVSPTK LNDLCFTNVY ADSFVIRGDE VRQIAPGQTG NIADYNYKLP DDFTGCVIAW
                   430        440        450        460        470        480
          NSNKLDSKVS GNYNYLYRLF RKSNLKPFER DISTEIYQAG NKPCNGVAGF NCYFPLRSYS
                   490        500        510        520        530        540
          FRPTYGVGHQ PYRVVVLSFE LLHAPATVCG PKKSTNLVKN KCVNFNFNGL KGTGVLTESN
                   550        560        570        580        590        600
          KKFLPFQQFG RDIADTTDAV RDPQTLEILD ITPCSFGGVS VITPGTNTSN QVAVLYQGVN
                   610        620        630        640        650        660
          CTEVPVAIHA DQLTPTWRVY STGSNVFQTR AGCLIGAEYV NNSYECDIPI GAGICASYQT
                   670        680        690        700        710        720
          QTKSHRRARS VASQSIIAYT MSLGAENSVA YSNNSIAIPT NFTISVTTEI LPVSMTKTSV
                   730        740        750        760        770        780
          DCTMYICGDS TECSNLLLQY GSFCTQLKRA LTGIAVEQDK NTQEVFAQVK QIYKTPPIKY
                   790        800        810        820        830        840
          FGGFNFSQIL PDPSKPSKRS FIEDLLFNKV TLADAGFIKQ YGDCLGDIAA RDLICAQKFK
                   850        860        870        880        890        900
          GLTVLPPLLT DEMIAQYTSA LLAGTITSGW TFGAGAALQI PFAMQMAYRF NGIGVTQNVL
                   910        920        930        940        950        960
          YENQKLIANQ FNSAIGKIQD SLSSTASALG KLQDVVNHNA QALNTLVKQL SSKFGAISSV
                   970        980        990       1000       1010       1020
          LNDIFSRLDK VEAEVQIDRL ITGRLQSLQT YVTQQLIRAA EIRASANLAA TKMSECVLGQ
                  1030       1040       1050       1060       1070       1080


          SKRVDFCGKG YHLMSFPQSA PHGVVFLHVT YVPAQEKNFT TAPAICHDGK AHFPREGVFV
                  1090       1100       1110       1120       1130       1140
          SNGTHWFVTQ RNFYEPQIIT TDNTFVSGNC DVVIGIVNNT VYDPLQPELD SFKEELDKYF
                  1150       1160       1170       1180       1190   1195
          KNHTSPDVDL GDISGINASV VNIQKEIDRL NEVAKNLNES LIDLQELGKY EQYIK
```

The above SARS-CoV-2 S recombinant polypeptide may include an N-terminal signal peptide provided in SEQ ID NO: 54 (MFVFLVLLPLVSS).

An exemplary SARS-CoV-2 S recombinant polypeptide without a signal peptide is provided in SEQ ID NO: 82 (1506 aa).

```
         10         20         30         40         50         60
QCVNLTTRTQ LPPAYTNSFT RGVYYPDKVF RSSVLHSTQD LFLPFFSNVT WFHVISGTNG
         70         80         90        100        110        120
TKRFDNPVLP FNDGVYFASI EKSNIIRGWI FGTTLDSKTQ SLLIVNNATN VVIKVCEFQF
        130        140        150        160        170        180
CNDPFLDHKN NKSWMESEFR VYSSANNCTF EYVSQPFLMD LEGKQGNFKN LREFVFKNID
        190        200        210        220        230        240
GYFKIYSKHT PIIVREPEDL PQGFSALEPL VDLPIGINIT RFQTLLALHR SYLTPGDSSS
        250        260        270        280        290        300
GWTAGAAAYY VGYLQPRTFL LKYNENGTIT DAVDCALDPL SETKCTLKSF TVEKGIYQTS
        310        320        330        340        350        360
NFRVQPTESI VRFPNITNLC PFDEVFNATK FASVYAWNRK RISNCVADYS VLYNLAPFFT
        370        380        390        400        410        420
FKCYGVSPTK LNDLCFTNVY ADSFVIRGDE VRQIAPGQTG NIADYNYKLP DDFTGCVIAW
        430        440        450        460        470        480
NSNKLDSKVS GNYNYLYRLF RKSNLKPFER DISTEIYQAG NKPCNGVAGF NCYFPLRSYS
        490        500        510        520        530        540
FRPTYGVGHQ PYRVVVLSFE LLHAPATVCG PKKSTNLVKN KCVNFNFNGL KGTGVLTESN
        550        560        570        580        590        600
KKFLPFQQFG RDIADTTDAV RDPQTLEILD ITPCSFGGVS VITPGTNTSN QVAVLYQGVN
        610        620        630        640        650        660
CTEVPVAIHA DQLTPTWRVY STGSNVFQTR AGCLIGAEYV NNSYECDIPI GAGICASYQT
        670        680        690        700        710        720
QTKSHRRAAS VASQSIIAYT MSLGAENSVA YSNNSIAIPT NFTISVTTEI LPVSMTKTSV
        730        740        750        760        770        780
DCTMYICGDS TECSNLLLQY GSFCTQLKRA LTGIAVEQDK NTQEVFAQVK QIYKTPPIKY
        790        800        810        820        830        840
FGGFNFSQIL PDPSKPSKRS FIEDLLFNKV TLADAGFIKQ YGDCLGDIAA RDLICAQKFK
        850        860        870        880        890        900
GLTVLPPLLT DEMIAQYTSA LLAGTITSGW TFGAGAALQI PFAMQMAYRF NGIGVTQNVL
        910        920        930        940        950        960
YENQKLIANQ FNSAIGKIQD SLSSTASALG KLQDVVNHNA QALNTLVKQL SSKFGAISSV
        970        980        990       1000       1010       1020
LNDIFSRLDK VEAEVQIDRL ITGRLQSLQT YVTQQLIRAA EIRASANLAA TKMSECVLGQ
       1030       1040       1050       1060       1070       1080
SKRVDFCGKG YHLMSFPQSA PHGVVFLHVT YVPAQEKNFT TAPAICHDGK AHFPREGVFV
       1090       1100       1110       1120       1130       1140
SNGTHWFVTQ RNFYEPQIIT TDNTFVSGNC DVVIGIVNNT VYDPLQPELD SFKEELDKYF
       1150       1160       1170       1180       1190  1195
KNHTSPDVDL GDISGINASV VNIQKEIDRL NEVAKNLNES LIDLQELGKY EQYIK
```

The above SARS-CoV-2 S recombinant polypeptide may include an N-terminal signal peptide provided in SEQ ID NO: 54 (MFVFLVLLPLVSS).

An exemplary SARS-CoV-2 S recombinant polypeptide without a signal peptide is provided in SEQ ID NO: 83 (1506 aa).

```
         10         20         30         40         50         60
QCVNLTTRTQ LPPAYTNSFT RGVYYPDKVF RSSVLHSTQD LFLPFFSNVT WFHVISGTNG
         70         80         90        100        110        120
TKRFDNPVLP FNDGVYFASI EKSNIIRGWI FGTTLDSKTQ SLLIVNNATN VVIKVCEFQF
        130        140        150        160        170        180
CNDPFLDHKN NKSWMESEFR VYSSANNCTF EYVSQPFLMD LEGKQGNFKN LREFVFKNID
        190        200        210        220        230        240
GYFKIYSKHT PIIVREPEDL PQGFSALEPL VDLPIGINIT RFQTLLALHR SYLTPGDSSS
        250        260        270        280        290        300
GWTAGAAAYY VGYLQPRTFL LKYNENGTIT DAVDCALDPL SETKCTLKSF TVEKGIYQTS
```

```
        310        320        330        340        350        360
NFRVQPTESI VRFPNITNLC PFDEVFNATK FASVYAWNRK RISNCVADYS VLYNLAPFFT
        370        380        390        400        410        420
FKCYGVSPTK LNDLCFTNVY ADSFVIRGDE VRQIAPGQTG NIADYNYKLP DDFTGCVIAW
        430        440        450        460        470        480
NSNKLDSKVS GNYNYLYRLF RKSNLKPFER DISTEIYQAG NKPCNGVAGF NCYFPLRSYS
        490        500        510        520        530        540
FRPTYGVGHQ PYRVVVLSFE LLHAPATVCG PKKSTNLVKN KCVNFNFNGL KGTGVLTESN
        550        560        570        580        590        600
KKFLPFQQFG RDIADTTDAV RDPQTLEILD ITPCSFGGVS VITPGTNTSN QVAVLYQGVN
        610        620        630        640        650        660
CTEVPVAIHA DQLTPTWRVY STGSNVFQTR AGCLIGAEYV NNSYECDIPI GAGICASYQT
        670        680        690        700        710        720
QTKSHRRAAS VASQSIIAYT MSLGAENSVA YSNNSIAIPT NFTISVTTEI LPVSMTKTSV
        730        740        750        760        770        780
DCTMYICGDS TECSNLLLQY GSFCTQLKRA LTGIAVEQDK NTQEVFAQVK QIYKTPPIKY
        790        800        810        820        830        840
FGGFNFSQIL PDPSKPSKRS FIEDLLFNKV TLADAGFIKQ YGDCLGDIAA RDLICAQKFK
        850        860        870        880        890        900
GLTVLPPLLT DEMIAQYTSA LLAGTITSGW TFGAGAALQI PFAMQMAYRF NGIGVTQNVL
        910        920        930        940        950        960
YENQKLIANQ FNSAIGKIQD SLSSTASALG KLQDVVNHNA QALNTLVKQL SSKFGAISSV
        970        980        990       1000       1010       1020
LNDIFSRLDP PEAEVQIDRL ITGRLQSLQT YVTQQLIRAA EIRASANLAA TKMSECVLGQ
       1030       1040       1050       1060       1070       1080
SKRVDFCGKG YHLMSFPQSA PHGVVFLHVT YVPAQEKNFT TAPAICHDGK AHFPREGVFV
       1090       1100       1110       1120       1130       1140
SNGTHWFVTQ RNFYEPQIIT TDNTFVSGNC DVVIGIVNNT VYDPLQPELD SFKEELDKYF
       1150       1160       1170       1180       1190       1195
KNHTSPDVDL GDISGINASV VNIQKEIDRL NEVAKNLNES LIDLQELGKY EQYIK
```

The above SARS-CoV-2 S recombinant polypeptide may include an N-terminal signal peptide provided in SEQ ID NO: 54 (MFVFLVLLPLVSS).

An exemplary SARS-CoV-2 S recombinant polypeptide without a signal peptide is provided in SEQ ID NO: 84 (1506 aa).

```
         10         20         30         40         50         60
QCVNLTTRTQ LPPAYTNSFT RGVYYPDKVF RSSVLHSTQD LFLPFFSNVT WFHVISGTNG
         70         80         90        100        110        120
TKRFDNPVLP FNDGVYFASI EKSNIIRGWI FGTTLDSKTQ SLLIVNNATN VVIKVCEFQF
        130        140        150        160        170        180
CNDPFLDHKN NKSWMESEFR VYSSANNCTF EYVSQPFLMD LEGKQGNFKN LREFVFKNID
        190        200        210        220        230        240
GYFKIYSKHT PIIVREPEDL PQGFSALEPL VDLPIGINIT RFQTLLALHR SYLTPGDSSS
        250        260        270        280        290        300
GWTAGAAAYY VGYLQPRTFL LKYNENGTIT DAVDCALDPL SETKCTLKSF TVEKGIYQTS
        310        320        330        340        350        360
NFRVQPTESI VRFPNITNLC PFDEVFNATK FASVYAWNRK RISNCVADYS VLYNLAPFFT
        370        380        390        400        410        420
FKCYGVSPTK LNDLCFTNVY ADSFVIRGDE VRQIAPGQTG NIADYNYKLP DDFTGCVIAW
        430        440        450        460        470        480
NSNKLDSKVS GNYNYLYRLF RKSNLKPFER DISTEIYQAG NKPCNGVAGF NCYFPLRSYS
        490        500        510        520        530        540
FRPTYGVGHQ PYRVVVLSFE LLHAPATVCG PKKSTNLVKN KCVNFNFNGL KGTGVLTESN
        550        560        570        580        590        600
KKFLPFQQFG RDIADTTDAV RDPQTLEILD ITPCSFGGVS VITPGTNTSN QVAVLYQGVN
        610        620        630        640        650        660
CTEVPVAIHA DQLTPTWRVY STGSNVFQTR AGCLIGAEYV NNSYECDIPI GAGICASYQT
        670        680        690        700        710        720
QTKSHRRARS VASQSIIAYT MSLGAENSVA YSNNSIAIPT NFTISVTTEI LPVSMTKTSV
        730        740        750        760        770        780
DCTMYICGDS TECSNLLLQY GSFCTQLKRA LTGIAVEQDK NTQEVFAQVK QIYKTPPIKY
        790        800        810        820        830        840
FGGFNFSQIL PDPSKPSKRS FIEDLLFNKV TLADAGFIKQ YGDCLGDIAA RDLICAQKFK
        850        860        870        880        890        900
GLTVLPPLLT DEMIAQYTSA LLAGTITSGW TFGAGAALQI PFAMQMAYRF NGIGVTQNVL
        910        920        930        940        950        960
YENQKLIANQ FNSAIGKIQD SLSSTASALG KLQDVVNHNA QALNTLVKQL SSKFGAISSV

        970        980        990       1000       1010       1020
LNDIFSRLDP PEAEVQIDRL ITGRLQSLQT YVTQQLIRAA EIRASANLAA TKMSECVLGQ
       1030       1040       1050       1060       1070       1080
SKRVDFCGKG YHLMSFPQSA PHGVVFLHVT YVPAQEKNFT TAPAICHDGK AHFPREGVFV
       1090       1100       1110       1120       1130       1140
SNGTHWFVTQ RNFYEPQIIT TDNTFVSGNC DVVIGIVNNT VYDPLQPELD SFKEELDKYF
       1150       1160       1170       1180       1190       1195
KNHTSPDVDL GDISGINASV VNIQKEIDRL NEVAKNLNES LIDLQELGKY EQYIK
```

The above SARS-CoV-2 S recombinant polypeptide may include an N-terminal signal peptide provided in SEQ ID NO: 54 (MFVFLVLLPLVSS).

In some embodiments, the recombinant polypeptide is or includes a sequence set forth in any one of SEQ ID NOs: 1-25. In some embodiments, the recombinant polypeptide is or includes an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity with a sequence set forth in any one of SEQ ID NOs: 1-25. The amino acid sequence includes a sequence containing a substitution, a deletion, and/or an insertion at one or more amino acid positions (such as 13, 18, 20, 26, 69, 70, 80, 138, 142, 144, 152, 190, 215, 242, 243, 244, 246, 400, 401, 402, 417, 440, 452, 477, 484, 501, 570, 614, 655, 681, 682, 683, 684, 685, 701, 716, 888, 982, 1027, 1118 and/or 1176 (with respect to SEQ ID NO: 55 amino acid positions) or any combination thereof). In some embodiments, the recombinant polypeptide is or includes a variant of a sequence set forth in any one of SEQ ID NO: 1-25, and the variant include any one, two, three, four, five or more mutations selected from a group consisting of S13I, L18F, T20N, P26S, Δ69-70 (ΔHV), D80A, D138Y, G142D, Δ144 (ΔY), W152C, R190S, D215G, Δ242-244 (ΔLAL), R246I, Δ400-402 (ΔFVI), K417T, K417N, N440K, L452R, S477N, S477G, E484K, E484Q, N501Y, A570D, D614G, H655Y, P681H, P681R, R682G, R683S, R685G, A701V, T716I, F888L, S982A, T1027I, D1118H, and V1176F or any combination thereof.

In some embodiments, the recombinant polypeptide is or includes a sequence set forth in any one of SEQ ID NOs: 26, 85-92, 120-121, and 124-125. In some embodiments, the recombinant polypeptide is or includes an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity with a sequence set forth in any one of SEQ ID NOs: 26, 85-92, 120-121, and 124-125. The amino acid sequence includes a sequence containing a substitution, a deletion, and/or an insertion at one or more amino acid positions of a sequence set forth in any one of SEQ ID NO: 26, 85-92, 120-121, and 124-125.

As described above, in some embodiments, the recombinant polypeptide provided herein not only binds to form the trimer, but can also aggregate or be aggregated to produce the protein containing a plurality of recombinant polypeptides. In some embodiments, the formed protein has a macroscopic structure. In some cases, the macroscopic structure can provide structural stability to recombinant polypeptides of a coronavirus antigen or an immunogen, which in turn can provide access to a potential antigenic site that can promote an immune response.

In some embodiments, the trimerized recombinant polypeptide aggregates to form a protein containing a plurality of trimerized recombinant polypeptides. In some embodiments, a plurality of trimerized recombinant polypeptides form proteins having macroscopic structures.

In some embodiments, the protein containing a plurality of recombinant polypeptides described herein is an immunogen. In some embodiments, the protein containing a plurality of recombinant polypeptides described herein is contained in a nanoparticle. For example, in some embodiments, the protein is directly linked to a nanoparticle, such as a protein nanoparticle. In some embodiments, the protein is indirectly linked to the nanoparticle. In some embodiments, the protein containing a plurality of recombinant polypeptides described herein is contained in a virus-like particle (VLP).

In some embodiments, the present disclosure provides a complex, which includes a recombinant polypeptide or fragments, variants or mutants thereof selected from a group consisting of SEQ ID NOs: 1-26 and 85-92, in any suitable combination. In some embodiments, the present disclosure provides a complex, which includes a member selected from a trimer of a recombinant polypeptide or a fragment, a variant or a mutant thereof of a group consisting of SEQ ID NOs: 1-26 and 85-92, where the recombinant polypeptide forms a trimer by trimerization of inter-polypeptide disulfide bonds

In some embodiments, the present disclosure provides a fusion protein including a plurality of recombinant polypeptides, and each of the recombinant polypeptides includes, from an amino group to a carboxyl terminus: a) a first region, which includes a portion of an extracellular domain of a coronavirus spike protein located front of a receptor binding domain (RBD) of the coronavirus spike protein in a non-chimeric coronavirus spike protein of a first coronavirus; b) a second region, which includes a receptor binding domain (RBD) of a coronavirus spike protein of a second coronavirus different from the first coronavirus; and c) a C-terminal propeptide of collagen, where the C-terminal propeptide of the recombinant polypeptide forms an inter-polypeptide disulfide bond. In some embodiments, the fusion protein further includes a third region between the second region and the C-terminal propeptide of collagen. In some embodiments, the third region includes an S1 domain of a third coronavirus, where the third coronavirus is the same as or different from the first or second coronavirus. In some embodiments, the third region includes an S2 domain of a fourth coronavirus, where the fourth coronavirus is the same as or different from the first, second or fourth coronavirus. In some embodiments, the first region includes an N-terminal domain (NTD) of the first coronavirus. In some embodiments, the first region includes one or more amino acid residues different from the corresponding amino acid residues in the second coronavirus. In some embodiments, the second region includes one or more amino acid residues different from the corresponding amino acid residues in the first coronavirus. In some embodiments, the first and the second coronaviruses are different variants or strains of a same coronavirus. In some embodiments, the first region includes the NTD of the first coronavirus, the second region includes the RBD of the second coronavirus, and the first and the second coronaviruses are different variants of SARS-CoV-2. In some embodiments, the first coronavirus and the second coronavirus are independently selected from a group consisting of SARS-CoV-2 viruses in the B.1.1.529, B.1.617.2, B.1.526, B.1.1.143, P.2, B.1.351, P.1, B.1.1.7, B.1.617, and A.23.1 lineages.

In some embodiments, the present disclosure provides a trimeric fusion protein including three recombinant polypeptides, and each of the recombinant polypeptides includes, from an amino group to a carboxyl terminus: a) a first region, which includes a spike protein N-terminal domain (NTD) of SARS-CoV-2 in the B.1.526 lineage; b) a second region, which includes a spike protein receptor binding domain (RBD) of SARS-CoV-2 in the B.1.351 lineage; and c) a C-terminal propeptide of collagen, where the C-terminal propeptide of the recombinant polypeptide forms an inter-polypeptide disulfide bond. In some embodiments, the present disclosure provides a trimeric fusion protein including three recombinant polypeptides, and each of the recombinant polypeptides includes, from an amino group to a carboxyl terminus: a) a first region, which includes a spike protein N-terminal domain (NTD) of SARS-CoV-2 in the B.1.1.529 lineage; b) a second region, which includes a spike protein receptor binding domain (RBD) of SARS-CoV-2 in the B.1.1.529 lineage or the non-B.1.1.529 lineage; and c) a C-terminal propeptide of collagen, where the C-terminal propeptide of the recombinant polypeptide forms an inter-polypeptide disulfide bond. In some embodiments, the present disclosure provides a trimeric fusion protein including three recombinant polypeptides, and each of the recombinant polypeptides includes, from an amino group to a carboxyl terminus: a) a first region, which includes a spike protein N-terminal domain (NTD) of SARS-CoV-2 in the B.1.1.529 lineage or the non-B.1.1.529 lineage; b) a second region, which includes a spike protein receptor binding domain (RBD) of SARS-CoV-2 in the B.1.1.529 lineage; and c) a C-terminal propeptide of collagen, where the C-terminal propeptide of the recombinant polypeptide forms an inter-polypeptide disulfide bond.

In some embodiments, the present disclosure provides a method for preventing coronavirus infection in a mammal, including immunizing the mammal with an effective amount of a fusion protein disclosed herein. In some embodiments, neutralizing antibodies against the first and the second coronaviruses are produced in the mammal. In some embodiments, the first and the second coronaviruses are different variants of SARS-CoV-2, and neutralizing antibodies produced in the mammal neutralize two or more SARS-CoV-2 viruses in the B.1.1.529, B.1.617.2, B.1.526, B.1.1.143, P.2, B.1.351, P.1, B.1.1.7, B.1.617, and A.23.1 lineages. In some embodiments, the neutralizing antibody produced in a mammal neutralizes three or more SARS-CoV-2 viruses in the B.1.1.529, B.1.617.2, B.1.526, B.1.1.143, P.2, B.1.351, P.1, B.1.1.7, B.1.617, and A.23.1 lineages. In some embodiments, the method includes immunizing a mammal with two or more doses of a fusion protein. In some embodiments, the fusion protein is administered with a booster dose after one or more doses of an immunogen (including a spike protein peptide containing NTD and RBD from the same SARS-CoV-2 variants).

In some embodiments, the present disclosure provides an engineered fusion polypeptide derived or modified from a spike (S) glycoprotein of coronaviruses, including SARS-CoV-1 and SARS-CoV-2. In some embodiments, the fusion polypeptide disclosed herein can be stabilized in a pre-fusion conformation compared to a wild-type S protein sequence of a coronavirus. In some embodiments, fusion with a trimerization domain can prevent the S protein peptide in the fusion protein from forming a straight helix (for example, similar to what happens during membrane fusion). For example, the frozen EM structure of an S-trimer subunit candidate vaccine shows that the structure mainly adopts a tightly closed pre-fusion state, which is different from a full-length wild-type spike protein, which forms pre-fusion and post-fusion states in the presence of detergent. Ma et al., *J Virol* (2021) doi:10.1128/JVI.00194-21 is incorporated by reference in its entirety for all purposes. In some embodiments, the fusion protein may include an altered soluble S sequence with a modification that inactivates the S1/S2 cleavage site; a mutation in the corner region between a heptapeptide repeat 1 (HR1) region and a central helix (CH) region that prevents HR1 and CH from forming a straight helix; and/or truncation of a heptapeptide repeat 2 (HR2) region in addition to a stable mutation. In some embodiments, the fusion protein herein may but need not include one or more mutations, such as K986G/V987G, K986P/V987P, K986G/V987P, or K986P/V987G, which are considered to stabilize a spike protein in a pre-fusion state. In some embodiments, mutations such as K986G/V987G, K986P/V987P, K986G/V987P, or K986P/V987G are not necessary to stabilize the fusion polypeptide containing the protein trimerization™ trimerization domain disclosed herein.

In some of these embodiments, a mutation that inactivates the S1/S2 cleavage site may include using GSAG (SEQ ID NO: 60) replace RRAR (682-685 in SEQ ID NO: 55), and the mutation in the corner region may include a double mutation K986G/V987G, K986P/V987P, K986G/V987P, or K986P/V987G. In some embodiments, the truncation of HR2 must delete one or more residues shown in SEQ ID NO: 65 at a C-terminus of a wild-type soluble S sequence. In some embodiments, the immunogenic polypeptide may further include (a) one or more proline or glycine substitutions in the HR1 region interacting with the HR2, and/or (b) insertion of one or more amino acid residues. In some of these embodiments, the immunogenic polypeptide may have one or more substitutions selected from A942P, S943P, A944P, A942G, S943G, and A944G. In some of these embodiments, the insertion can be a G or GS inserted between any residues in A942-A944.

The recombinant polypeptide or the fragment, variant or mutant thereof mentioned herein, in any suitable combination, includes a member selected from a trimer of a recombinant polypeptide or a fragment, a variant or a mutant thereof of a group consisting of SEQ ID NOs: 1-26 and 85-92, where the recombinant polypeptide forms a trimer by trimerization of inter-polypeptide disulfide bonds, and can be used in a primary series and/or a booster dose. Independently, the primary series and/or any one or more booster doses may not use or use an adjuvant. If the adjuvant is used, the optional adjuvant may include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

### 2. Polynucleotide and vector

A polynucleotide (nucleic acid molecule) encoding the coronavirus antigen or immunogen and recombinant polypeptide provided herein is further provided, as well as a vector for genetically engineering a cell to express such coronavirus antigen or immunogen and recombinant polypeptide.

In some embodiments, the polynucleotide encoding the recombinant polypeptide provided herein is provided. In some aspects, the polynucleotide includes a single nucleic acid sequence, such as a nucleic acid sequence encoding a recombinant polypeptide. In other examples, the polynucleotide includes a first nucleic acid sequence encoding a recombinant polypeptide, particularly a coronavirus antigen or immunogen, and a second nucleic acid sequence encoding a recombinant polypeptide containing a different coronavirus antigen or immunogen.

In some embodiments, the polynucleotide encoding the recombinant polypeptide includes at least one promoter, which is operably linked to control the expression of the recombinant polypeptide. In some embodiments, the polynucleotide includes two, three, or more promoters, which are operably linked to control the expression of the recombinant polypeptide.

In some embodiments, for example, when the polynucleotide includes two or more nucleic acid coding sequences, such as a sequence encoding the recombinant polypeptide containing a different coronavirus antigen or immunogen, at least one promoter is operably linked to control the expression of the two or more nucleic acid sequences. In some embodiments, the polynucleotide includes two, three, or more promoters, which are operably linked to control the expression of the recombinant polypeptide.

In some embodiments, the expression of the recombinant polypeptide is inducible or conditional. Therefore, in some aspects, the polynucleotide encoding the recombinant polypeptide includes a conditional promoter, an enhancer, or a transactivator. In some such aspects, the conditional promoter, enhancer, or transactivator is an inducible promoter, enhancer, or transactivator or an inhibitory promoter, enhancer, or transactivator. For example, in some embodiments, the inducible or conditional promoter can be used to limit the expression of the recombinant polypeptide to a specific microenvironment. In some embodiments, the expression driven by the inducible or conditional promoter is regulated through exposure to an exogenous agent (such as heat, radiation, or a drug).

In the case where the polynucleotide includes more than one nucleic acid sequence encoding the recombinant polypeptide, the polynucleotide may further include a nucleic acid sequence encoding a peptide between one or more nucleic acid sequences. In some cases, a nucleic acid encodes a peptide located between nucleic acid sequences. The peptide separates a translation product of the nucleic acid sequences during or after translation. In some embodiments, the peptide includes an internal ribosome entry site (IRES), a self-cleaving peptide, or a peptide that causes ribosomal skipping, such as a T2A peptide.

In some embodiments, the polynucleotide encoding the recombinant polypeptide is introduced into a composition containing cultured cells (such as host cells), such as by retroviral transduction, infection, or transformation. In some embodiments, this may allow the expression (such as production) of the recombinant polypeptide. In some embodiments, the expressed recombinant polypeptide is purified.

In some embodiments, the polynucleotide (nucleic acid molecule) provided herein encodes the coronavirus antigen or immunogen as described herein. In some embodiments, the polynucleotide (nucleic acid molecule) provided herein encodes the recombinant polypeptide containing the coronavirus antigen or immunogen (such as a coronavirus S protein peptide) described herein.

A vector or construct containing the nucleic acid molecule described herein is further provided. In some embodiments, the vector or construct includes one or more promoters, which are operably linked to a nucleic acid molecule encoding a recombinant polypeptide to drive the expression of the recombinant polypeptide. In some embodiments, the promoter is operably linked to one or more nucleic acid molecules, such as a nucleic acid molecule encoding a recombinant polypeptide containing a different coronavirus antigen or immunogen.

In some embodiments, the vector is a viral vector. In some embodiments, the viral vector is a retroviral vector. In some embodiments, the retroviral vector is a lentiviral vector. In some embodiments, the retroviral vector is a γ-retroviral vector.

In some embodiments, the vector or construct includes a single promoter that drives the expression of one or more nucleic acid molecules of the polynucleotide. In some embodiments, such a promoter may be polycistronic (bicistronic or tricistronic, see, for example, U.S. Patent No. 6,060,273). For example, in some embodiments, a transcription unit can be engineered as a bicistronic unit containing IRES (internal ribosome entry site), which allows co-expression of gene products (for example, encoding different recombinant polypeptides) through a message from a single promoter. In some embodiments, the vector provided herein is bicistronic, allowing the vector to contain and express two nucleic acid sequences. In some embodiments, the vector provided herein is tricistronic, allowing the vector to contain and express three nucleic acid sequences.

In some embodiments, a single promoter directs the expression of an RNA containing two or three genes (for example, encoding a chimeric signaling receptor and a recombinant receptor) in a single open reading frame (ORF), in which these genes are separated from each other by a sequence encoding a self-cleaving peptide (such as 2A sequence) or a protease recognition site (such as furin). Therefore, ORF encodes a single polypeptide, which is processed into a single protein during translation (for 2A) or after translation. In some cases, a peptide (such as T2A) can cause ribosomal skipping synthesis of a peptide bond at the C-terminus of a 2A element, resulting in separation between an end of a 2A sequence and the next downstream peptide (see, for example, de Felipe. Genetic Vaccines and Ther. 2:13 (2004) and deFelipe et al. Traffic 5:616-626 (2004), which are incorporated by reference in their entireties for all purposes). Many 2A elements are known in the art. Examples of 2A sequences that can be used in the method and the nucleic acid disclosed herein include, but are not limited to, 2A sequences from foot-and-mouth disease virus (F2A), equine rhinitis A virus (E2A), Thosea asigna virus (T2A), and porcine teschovirus-1 (P2A) as described in U.S. Patent Publication No. 20070116690.

In some embodiments, the vector is contained in a virus. In some embodiments, the virus is a pseudovirus. In some embodiments, the virus is a virus-like particle. In some embodiments, the carrier is contained in a cell. In some embodiments, the virus or cell containing the vector contains a recombinant genome.

**III. Immunogenic composition and preparation**

In some embodiments, the present disclosure provides an immunogenic composition including a trimer of a recombinant polypeptide, in which the recombinant polypeptide includes a sequence selected from the group consisting of SEQ ID NOs: 1-26, 85-92, 120, and 124, or a combination of any two or more trimers. In some embodiments, the present disclosure provides an immunogenic composition including a trimer of a recombinant polypeptide having a sequence set forth in SEQ ID NO: 1. The immunogenic composition mentioned herein includes a member selected from a trimer of a recombinant polypeptide or a fragment, a variant or a mutant thereof of a group consisting of SEQ ID NOs: 85-92, where the recombinant polypeptide forms a trimer by trimerization of inter-polypeptide disulfide bonds, and can be used in a primary series and/or a booster dose. The immunogenic composition mentioned herein includes a member selected from a trimer of a recombinant polypeptide or a fragment, a variant or a mutant thereof of SEQ ID NO: 120, where the recombinant polypeptide forms a trimer by trimerization of inter-polypeptide disulfide bonds, and can be used in a primary series and/or a booster dose. The immunogenic composition mentioned herein includes a member selected from a trimer of a recombinant polypeptide or a fragment, a variant or a mutant thereof of a group consisting of SEQ ID NO: 124, where the recombinant polypeptide forms a trimer by trimerization of inter-polypeptide disulfide bonds, and can be used in a primary series and/or a booster dose. Independently, the primary series and/or any one or more booster doses may not use or use an adjuvant. If the adjuvant is used, the optional adjuvant may include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing meta-bolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

In some embodiments, a unit dose of the immunogenic composition may include about 10 $\mu$g to about 100 $\mu$g of SARS-CoV-2 antigens, preferably about 25 $\mu$g to about 75 $\mu$g of SARS-CoV-2 antigens, preferably about 40 $\mu$g to about 60 $\mu$g of SARS-CoV-2 antigens or about 50 $\mu$g of SARS-CoV-2 antigens. In some embodiments, a dose includes 3 $\mu$g SARS-CoV-2 antigens. In other embodiments, a dose includes 9 $\mu$g of SARS-CoV-2 antigens. In a further embodiment, a dose includes 30 $\mu$g of SARS-CoV-2 antigen.

In some cases, it may be necessary to combine the disclosed immunogen with other pharmaceutical products (such as vaccines) that induce a protective response to other agents. For example, a composition including the recombinant coronavirus S antigen (such as a trimer or protein) described herein may be administered simultaneously (usually alone) or sequentially with other vaccines (such as influenza or varicella-zoster vaccines) recommended by the Advisory Committee on Immunization Practices (ACIP; cdc.gov/vaccines/acip/index.html) for the target age group (for example, infants about one to six months old). Therefore, the disclosed immunogen including the recombinant coronavirus S antigen described herein can be administered simultaneously or sequentially with vaccines against, for example, hepatitis B (HepB), diphtheria, tetanus and pertussis (DTaP), pneumococcus (PCV), Haemophilus influenzae type b (Hib), polio, influenza, and rotavirus.

A multivalent or combined vaccine provides protection against a plurality of pathogens. In some aspects, the multivalent vaccine can protect against a plurality of strains of the same pathogen. In some aspects, the multivalent vaccine protects against a plurality of pathogens, such as the combined vaccine Tdap, which protects against strains of tetanus, pertussis, and diphtheria. The multivalent vaccine is necessary to minimize the number of immunizations required to provide protection against a plurality of pathogens or viral strains, so as to reduce administrative costs and increase coverage. This can be particularly useful, for example, when vaccinating infants or children.

In some embodiments, for example, a vaccine comprising the immunogenic composition described herein is a multivalent vaccine. In some embodiments, antigenic materials used for incorporation into the multivalent vaccine composition are derived from a coronavirus strain or type, such as described herein (see, for example, Section I). An antigen used for incorporation into the multivalent vaccine composition can be derived from one strain or a plurality of strains of a coronavirus (for example, from two to five strains) to provide a wider range of protection. In one embodiment, the antigen used for incorporation into the multivalent vaccine composition is derived from a plurality of strains of a coronavirus. Other useful antigens include live, attenuated, and inactivated viruses, such as an inactivated poliovirus (Jiang et al., J. Biol. Stand., (1986) 14:103-9), an attenuated strain of hepatitis A virus (Bradley et al., J. Med. Virol., (1984) 14:373-86), an attenuated measles virus (James et al., N. Engl. J. Med., (1995) 332:1262-6), and an epitope of pertussis virus (such as ACEL-IMUNE acellular DTP, Wyeth-Lederle vaccine, and Pediatrics). The above documents are incorporated by reference in their entireties for all purposes.

In some aspects, the vaccine provided herein is a universal vaccine. In some embodiments, the universal vaccine is a vaccine that protects against a plurality of strains of the same virus (such as multiple strains of a coronavirus). The development of an effective universal coronavirus vaccine will reduce costs and labor, such as using seasonal vaccine formulations, and will allow for more robust pandemic prevention.

In some embodiments, the immunogen described herein can be used as a primary series, an additional dose, and/or a homologous or heterologous booster dose, such as a first dose, a second dose, a third dose, a fourth dose, and/or more doses. In some embodiments, the immunogen described herein can be used as a seasonal vaccine. In some embodiments, the immunogen described herein can be used as a first dose, a second dose, a third dose, a fourth dose,

and/or more doses within five years, four years, three years, two years, one year, and/or six months.

In some aspects, the universal vaccine is a vaccine composed of a plurality of epitopes derived from different virus strains. In some aspects, the universal vaccine is composed of a single epitope that is conserved in different virus strains. For example, the universal vaccine can be based on a relatively conserved domain of an S protein.

An immunogenic composition including the disclosed immunogen (for example, the disclosed recombinant coronavirus S antigen or a nucleic acid molecule encoding a protomer of the disclosed recombinant coronavirus S antigen) and a pharmaceutically acceptable carrier. In some embodiments, the immunogenic composition includes the trimerized recombinant polypeptide provided herein and an optional pharmaceutically acceptable carrier. In some embodiments, the immunogenic composition includes the trimerized recombinant polypeptide provided herein and disodium hydrogen phosphate, such as disodium hydrogen phosphate dihydrate, sodium dihydrogen phosphate, such as disodium hydrogen phosphate monohydrate, sodium chloride, and Tween 80. In some embodiments, a 1.0 mL aqueous solution of the immunogenic composition includes 720 $\mu$g of the trimerized recombinant polypeptide provided herein and 0.62 mg of disodium hydrogen phosphate dihydrate, 0.62 mg of disodium hydrogen phosphate monohydrate, 9.0 mg of sodium chloride, and 0.2 mg of Tween 80. In some embodiments, the immunogenic composition includes the protein including a plurality of trimerized recombinant polypeptides provided herein and an optional pharmaceutically acceptable carrier. In some embodiments, the immunogenic composition includes the protein nanoparticle provided herein and an optional pharmaceutically acceptable carrier. In some embodiments, the immunogenic composition includes the VLP provided herein and an optional pharmaceutically acceptable carrier. In some embodiments, the immunogenic composition includes the isolated nucleic acid provided herein and an optional pharmaceutically acceptable carrier. In some embodiments, the immunogenic composition includes the carrier provided herein and an optional pharmaceutically acceptable carrier. In some embodiments, the immunogenic composition includes the virus provided herein and an optional pharmaceutically acceptable carrier. In some embodiments, the immunogenic composition includes the pseudovirus provided herein and an optional pharmaceutically acceptable carrier. In some embodiments, the immunogenic composition includes the cell provided herein and an optional pharmaceutically acceptable carrier. In some embodiments, the immunogenic composition described herein is a vaccine. In some embodiments, the vaccine is a prophylactic vaccine. In some embodiments, the vaccine is a therapeutic vaccine. In some embodiments, the vaccine is a preventive vaccine and a therapeutic vaccine. Such a pharmaceutical composition can be administered to a subject through a variety of administration modes known to those of ordinary skill in the art, such as intramuscular, intradermal, subcutaneous, intravenous, intraarterial, intra-articular, intraperitoneal, intranasal, sublingual, tonsillar, oropharyngeal, or other parenteral and mucosal routes. In several embodiments, the pharmaceutical composition including one or more disclosed immunogens is an immunogenic composition. The actual method for preparing an administrable composition is known or obvious to those skilled in the art and is described in more detail in publications such as Remingtons Pharmaceutical Sciences, 19th Ed., Mack Publishing Company, Easton, Pa., 1995.

Therefore, the immunogen described herein, such as the recombinant coronavirus S antigen, such as the trimer and the protein, can be formulated with a pharmaceutically acceptable carrier to help maintain biological activity while also promoting increased stability during storage within an acceptable temperature range. A potential carrier includes, but is not limited to, a physiologically balanced medium, a phosphate buffered saline solution, water, an emulsion (such as an oil/water or water/oil emulsion), various types of wetting agents, an antifreeze additive or stabilizer such as a protein, peptide or hydrolyzate (such as albumin and gelatin), a sugar (such as sucrose, lactose, and sorbitol), an amino acid (such as sodium glutamate) or other protective agents. The resulting aqueous solution can be used in the original packaging or freeze-dried. The freeze-dried preparation is mixed with a sterile solution before a single dose or a plurality of doses.

The formulation composition, especially the liquid preparation, may contain an antibacterial agent to prevent or minimize degradation during storage, including but not limited to an effective concentration (usually 1% w/v) of benzyl alcohol, phenol, m-cresol, chlorobutanol, methylparaben, and/or propylparaben. The antibacterial agent may be contraindicated in some patients; therefore, the freeze-dried preparation can be reconstituted in a solution with or without such components.

The immunogenic composition of the present disclosure may contain a pharmaceutically acceptable carrier substance required for near-physiological conditions, such as a pH regulator and buffer, a tension regulator, a wetting agent, and so on, such as sodium acetate, sodium lactate, sodium chloride, potassium chloride, calcium chloride, sorbitan monolaurate and triethanolamine oleate, and polysorbate 80. The immunogenic composition may optionally include an adjuvant to enhance an immune response of a host. A suitable adjuvant is, for example, a toll-like receptor (TLR) agonist, alum, $AlPO_4$, alhydrogel, lipid-A and a derivative or variant thereof, an oil emulsion, a saponin, a neutral liposome, a liposome containing a vaccine and a cytokine, a nonionic block copolymer, and a chemokine. A nonionic block polymer containing polyoxyethylene (POE) and polyxylpropylene (POP) can be used as an adjuvant among many other suitable adjuvants known in the art, such as a POE-POP-POE block copolymer, MPL™ (3-O-deacyl monophosphate A; Corixa, Hamilton, Ind.) and IL-12 (Genetics Institute, Cambridge, Mass.) (Newman et al., 1998, Critical Reviews in Therapeutic Drug Carrier Systems 15:89-142, incorporated by reference in its entirety for all purposes). The advantage of these adjuvants is that they help stimulate the immune system in a non-specific manner, thereby enhancing the immune response to drug products. In some embodiments, the immunogenic composition of the present disclosure may include or be administered together with more than one adjuvant. In some embodiments, the immunogenic composition of the present disclosure may include or be administered together with two adjuvants. In some embodiments, the immunogenic composition of the present disclosure may include or be administered together with a plurality of adjuvants. For example, in some cases, such as a vaccine including the immunogenic composition provided herein may include or be administered in combination with a plurality of adjuvants.

For a vaccine composition, examples of a suitable adjuvant include, for example, aluminum hydroxide, lecithin, Freund's adjuvant, MPL™, and IL-1, one or any combination thereof can be used in combination with a member selected from a trimer of a recombinant polypeptide or a fragment, a variant or a mutant thereof of a group consisting of SEQ ID NOs: 85-92. In some embodiments, the vaccine composition or nanoparticle immunogen disclosed herein (such as a SARS-CoV-2 vaccine composition) can be formulated as a controlled-release or sustained-release preparation. This can be achieved through a composition containing a sustained-release polymer, or via a microencapsulation delivery system or a bioadhesive gel. Various pharmaceutical compositions can be prepared according to standard procedures well known in the art.

In some embodiments, the immunogenic composition of the present disclosure includes a member selected from a trimer of a recombinant polypeptide or a fragment, a variant or a mutant thereof of a group consisting of SEQ ID NOs: 85-92, where the recombinant polypeptide forms a trimer by trimerization of inter-polypeptide disulfide bonds, and may contain an adjuvant preparation, where the adjuvant preparation includes metabolizable oil (such as squalene) and $\alpha$-tocopherol (such as DL-$\alpha$-tocopherol) in the form of an oil-in-water emulsion, and polyoxyethylene sorbitan monooleate (Tween-80). In some embodiments, the adjuvant preparation may include about 2% to about 10% squalene, about 2% to about 10% $\alpha$-tocopherol (such as D-$\alpha$-tocopherol), and about 0.3% to about 3% polyoxyethylene sorbitan monooleate. In some embodiments, the adjuvant preparation may include about 5% squalene, about 5% tocopherol, and about 0.4% polyoxyethylene sorbitan monooleate. In some embodiments, the immunogenic composition of the present disclosure may include 3-O-deacyl-monophosphoryl lipid A (3D-MPL) and an adjuvant in the form of an oil-in-water emulsion including metabolizable oil, $\alpha$-tocopherol, and polyoxyethylene sorbitan monooleate. In some embodiments, the immunogenic composition of the present disclosure may include QS21 (an extract of *Quillaja saponaria Molina:* component 21), 3D-MPL and an oil-in-water emulsion, where the oil-in-water emulsion includes metabolizable oil, $\alpha$-tocopherol, and polyoxyethylene sorbitan monooleate. In some embodiments, the immunogenic composition of the present disclosure may include QS21, 3D-MPL and an oil-in-water emulsion, where the oil-in-water emulsion has the following composition: metabolizable oil such as squalene, $\alpha$-tocopherol, and Tween-80 and/or Span 85. In some embodiments, the immunogenic composition of the present disclosure may include an adjuvant in the form of a liposome composition.

In some embodiments, the immunogenic composition of the present disclosure includes a member selected from a trimer of a recombinant polypeptide or a fragment, a variant or a mutant thereof of a group consisting of SEQ ID NOs: 120 and 124, where the recombinant polypeptide forms a trimer by trimerization of inter-polypeptide disulfide bonds, and may contain an adjuvant preparation, where the adjuvant preparation includes metabolizable oil (such as squalene), $\alpha$-tocopherol, polyoxyethylene sorbitan monooleate (Tween-80), and/or Span 85. In some embodiments, the adjuvant preparation may include about 5% (w/v) squalene, about 5% (w/v) $\alpha$-tocopherol, about 0.5% (w/v) polyoxyethylene sorbitan monooleate, and/or about 0.5% (w/v) Span 85.

In some embodiments, the immunogenic composition of the present disclosure includes a member selected from a trimer of a recombinant polypeptide or a fragment, a variant or a mutant thereof of a group consisting of SEQ ID NOs: 85-92, where the recombinant polypeptide forms a trimer by trimerization of inter-polypeptide disulfide bonds, and may contain an adjuvant preparation, where the adjuvant preparation includes saponin *(Quillaja),* cholesterol, and phospholipid, for example, in the form of a nanoparticle composition. In some embodiments, the immunogenic composition of the present disclosure may include a mixture of individually purified *Quillaja saponaria Molina* parts, which are subsequently formulated with cholesterol and phospholipid.

In some embodiments, the immunogenic composition of the present disclosure includes a member selected from a trimer of a recombinant polypeptide or a fragment, a variant or a mutant thereof of a group consisting of SEQ ID NOs: 85-92, 120, and 124, where the recombinant polypeptide forms a trimer by trimerization of inter-polypeptide disulfide bonds, and may contain an adjuvant selected from a group consisting of MF59™, Matrix-A™, Matrix-C™, Matrix-M™, AS01, AS02, AS03, and AS04. Optional adjuvants include any one or any combination of several adjuvants disclosed by O'Hagan et al, The history of MF59® adjuvant: a phoenix that arose from the ashes, Expert Review of Vaccines, DOI:10.1586/ERV.12.140 (2013); Garçon et al, Development and evaluation of AS03, an Adjuvant System containing $\alpha$-tocopherol and squalene in an oil-in-water emulsion, Expert Review of Vaccines, 11(3), 349-366

(2012); Morel et al., Adjuvant System AS03 containing $\alpha$-tocopherol modulates innate immune response and leads to improved adaptive immunity, Vaccine, doi:10.1016j.vaccine.2011.01.011 (2011). The foregoing documents are incorporated herein by reference in their entireties for all purposes.

In some embodiments, the immunogenic composition of the present disclosure includes a member selected from a trimer of a recombinant polypeptide or a fragment, a variant or a mutant thereof of a group consisting of SEQ ID NOs:

85-92, 120, and 124, where the recombinant polypeptide forms a trimer by trimerization of inter-polypeptide disulfide bonds, and may contain a toll-like receptor 9 (TLR9) agonist, where the TLR9 agonist is an oligonucleotide with a length of 8 to 35 nucleotides, containing an unmethylated cytidine-phosphate-guanine (also known as CpG or cytosine-phosphate-guanine) motif, and SARS-CoV-2 antigens and oligonucleotides are present in the immunogenic composition in an amount effective to stimulate immune responses against SARS-CoV-2 antigens in mammalian subjects (such as human subjects requiring SARS-CoV-2 antigens and oligonucleotides). TLR9 (CD289) recognizes the unmethylated cytidine-phosphate-guanine (CpG) motif found in microbial DNA and the motif can be mimicked using synthetic CpG-containing oligodeoxynucleotides (CpG-ODN). CpG-ODN is known to enhance antibody production and stimulate T helper 1 (Th1) cellular responses (Coffman et al., Immunity, 33:492-503, 2010, incorporated by reference in its entirety for all purposes).

The best oligonucleotide TLR9 agonist usually contains a palindrome sequence of the following general formula: 5'-purine-purine-CG-pyrimidine-pyrimidine-3' or 5'-purine-purine-CG-pyrimidine-pyrimidine-CG-3'. U.S. Patent No. 6,589,940 is incorporated herein by reference in its entirety. In some embodiments, the CpG oligonucleotide is linear. In other embodiments, the CpG oligonucleotide is cyclic or includes a hairpin ring. CpG oligonucleotides can be single-stranded or double-stranded. In some embodiments, the CpG oligonucleotide may include a modification. Modifications include but are not limited to modification of 3'OH or 5'OH groups, modification of nucleotide bases, modification of sugar components, and modification of phosphate groups. Modified bases can be included in the palindrome sequence of CpG oligonucleotides as long as the modified bases maintain a same specificity for their natural complement through Watson-Crick base pairing (for example, the palindrome part is still self-complementary). In some embodiments, the CpG oligonucleotide includes a non-classical base. In some embodiments, the CpG oligonucleotide includes a modified nucleoside. In some embodiments, the modified nucleoside is selected from 2'-deoxy-7-deazaguanosine, 2'-deoxy-6-thioguanosine, arabinoguanosine, 2'-deoxy-2' substituted-arabinoguanosine, and 2'-O-substituted-arabinoguanosine. The CpG oligonucleotide may contain modifications of a phosphate groups. For example, in addition to the phosphodiester bond, phosphoric acid modifications include but are not limited to methyl phosphonates, phosphorothioates, phosphoramides (bridged or unbridged), phosphotriesters, and phosphorodithioates, and can be used in any combination. Other non-phosphate bonds can also be used. In some embodiments, oligonucleotide includes only a phosphorothioate backbone. In some embodiments, oligonucleotide includes only a phosphodiester backbone. In some embodiments, oligonucleotide includes a combination of phosphate bonds in a phosphate backbone, such as a combination of a phosphodiester bond and a phosphorothioate bond. Oligonucleotides with a phosphorothioate backbone can have higher immunogenicity than oligonucleotides with a phosphodiester backbone and appear to be more resistant to degradation after injection into a host (Braun et al., J Immunol, 141:2084-2089, 1988; and Latimer et al., Mol Immunol, 32:1057-1064, 1995, incorporated by reference in their entireties for all purposes). The CpG oligonucleotide of the present disclosure includes at least one, two or three inter-nucleotide phosphorothioate bonds. In some embodiments, when a plurality of CpG oligonucleotide molecules are present in a pharmaceutical composition containing at least one excipient, two stereoisomers of the phosphorothioate bond are present in the plurality of CpG oligonucleotide molecules. In some embodiments, all inter-nucleotide bonds of the CpG oligonucleotide are phosphorothioate bonds, or in other words, the CpG oligonucleotide has a phosphorothioate backbone.

In the present disclosure, any suitable CpG oligodeoxynucleotide (ODN) or a combination thereof may be used as an adjuvant. For example, K-type ODN (also known as B-type) encodes a plurality of CpG motifs on the phosphorothioate backbone. K-type ODN can be based on the following sequence TCCATGGACGTTCCTGAGCGTT. Compared with natural phosphodiester nucleotides, the use of phosphorothioate nucleotides enhances resistance to nuclease digestion, resulting in a significant extension of the half-life in vivo. K-type ODN triggers pDC differentiation and production of TNF-$\alpha$, and triggers B cell proliferation and secretion of IgM. D-type ODN (also known as A-type) consists of a mixed phosphodiester/phosphorothioate backbone containing a single CpG motif flanked by palindromic sequences and having poly G tails at the 3' and 5' ends (structural motifs that promote concatemer formation). D-type ODN can be based on the following sequence GGTGCATCGATGCAGGGGGG. D-type ODN triggers pDC maturation and secretion of IFN-$\alpha$, but has no effect on B cells. C-type ODN is similar to K-type and consists entirely of phosphorothioate nucleotides, but is similar D-type ODN and contains a palindromic CpG motif. C-type ODN can be based on the following sequence <u>TCGTCGT TCGAACGACGTTGAT.</u> This type of ODN stimulates B cells to secrete IL-6 and pDC to produce IFN-$\alpha$. P-type ODN contains two palindrome sequences, allowing them to form a higher ordered structure. P-type ODN can be based on the following sequence **T**CG**TCG**A<u>C</u>G**AT**CG**G**CGCGCG**C**CG. Compared with C-type ODN, P-type ODN activates B cells and PDCs and induces greater production of IFN-$\alpha$. In this paragraph, boldface words in the ODN sequence indicate self-complementary palindromes and CpG motifs are underlined.

Exemplary CpG ODNs, such as CpG 7909 (5'-TCGTCGTTTTGTCGTTTGTCGTTGTCGTT-3') and CpG 1018 (5'-TGACTGTGAACGTTCGAGATGA-3'), are known and disclosed in U.S. Patent Nos. 7,255,868, 7,491,706, 7,479,285, 7,745,598, 7,785,610, 8,003,115, 8,133,874, 8,114,418, 8,222,398, 8,333,980, 8,597,665, 8,669,237, 9,028,845, and 10,052,378; and Bode et al., "CpG DNA as a vaccine adjuvant", Expert Rev Vaccines (2011), 10(4): 499-511, all of which are incorporated herein by reference in their entireties for all purposes.

One or more adjuvants may be used in combination, including but not limited to alum (aluminium salts), oil-in-water emulsions, water-in-oil emulsions, liposomes, and microparticles such as poly(lactide-co-glycolide) microparticles (Shah et al., Methods Mol Biol, 1494:1-14, 2017, incorporated by reference in its entirety for all purposes). In some embodiments, the immunogenic composition further includes an aluminum salt adjuvant that adsorbs SARS-CoV-2 antigens. In some embodiments, the aluminum salt adjuvant includes one or more of a group consisting of amorphous aluminum hydroxyphosphate sulfate, aluminum hydroxide, aluminum phosphate, and potassium aluminum sulfate. In some embodiments, the aluminum salt adjuvant includes one or both of aluminum hydroxide and aluminum phosphate. In some embodiments, the aluminum salt adjuvant includes aluminum hydroxide. In some embodiments, a unit dose of the immunogenic composition includes about 0.01 mg to about 0.8 mg $Al^{3+}$; or about 0.05 mg to about 0.7 mg $Al^{3+}$; or about 0.06 mg to about 0.6 mg $Al^{3+}$; or about 0.07 mg $Al^{3+}$, about 0.08 mg to about 0.45 mg $Al^{3+}$; or about 0.08 mg to about 0.30 mg, about 0.35 mg $Al^{3+}$, about 0.40 mg $Al^{3+}$; or about 0.25 mg to about 0.50 mg $Al^{3+}$; or about 0.068 mg $Al^{3+}$, about 0.078 mg $Al^{3+}$, about 0.13 mg $Al^{3+}$, about 0.19 mg $Al^{3+}$, about 0.22 mg $Al^{3+}$, about 0.26 mg, and about 0.35 mg $Al^{3+}$. In some embodiments, the immunogenic composition further includes additional adjuvants. Other suitable adjuvants include, but are not limited to, squalene-in-water emulsions (such as MF59 or AS03), TLR3 agonists (such as polyIC or polyICLC), TLR4 agonists (such as bacterial lipopolysaccharide derivatives such as monophosphate A (MPL), and/or saponins such as Quil A or QS-21 such as AS01 or AS02), TLR5 agonists (bacterial flagellin) and TLR7, TLR8 and/or TLR9 agonists (imidazoquinoline derivatives, such as imiquimod and requimod) (Coffman et al., Immunity, 33:492-503, 2010, incorporated by reference in its entirety for all purposes). In some embodiments, additional adjuvants include MPL and aluminum hydroxide/alum (such as AS04). For veterinary use and for the production of non-human animal antibodies, the mitogenic components (intact and incomplete) of Freund's adjuvant can be used.

In some embodiments, a unit dose of the immunogenic composition may include about 3 μg to about 1,000 μg of one or more adjuvants, about 8 μg to about 1,000 μg of one or more adjuvants, preferably about 25 μg to about 500 μg of one or more adjuvants, preferably about 9 μg to about 500 μg of one or more adjuvants, preferably about 50 μg to about 300 μg of one or more adjuvants, preferably about 100 μg to about 250 μg of one or more adjuvants, and preferably about 150 μg to about 225 μg of one or more adjuvants. In some embodiments, a unit dose of the immunogenic composition may include about 3 μg to about 700 μg, 750 μg, or 800 ptg of an aluminum-containing adjuvant, about 3 μg to about 500 μg of the aluminum-containing adjuvant, about 8 μg to about 250 μg of the aluminum-containing adjuvant, about 9 μg to about 240 μg of the aluminum-containing adjuvant, preferably about 25 μg to about 230 μg of the aluminum-containing adjuvant, preferably about 200 μg to about 800 μg of the aluminum-containing adjuvant, preferably about 700 μg to about 800 μg of the aluminum-containing adjuvant, preferably about 740 μg to about 780 μg of the aluminum-containing adjuvant, preferably about 750, 760, 770 μg to about 780 μg of the aluminum-containing adjuvant, preferably about 260 μg to about 280 μg of the aluminum-containing adjuvant, preferably about 200 μg to about 240 μg of the aluminum-containing adjuvant, preferably about 210 μg to about 230 μg of the aluminum-containing adjuvant, preferably about 220 μg to about 230 μg of the aluminum-containing adjuvant, Preferably about 224 μg to about 228 μg of the aluminum-containing adjuvant, preferably about 50 μg to about 125 μg of the aluminum-containing adjuvant, preferably about 7 50 μg of the aluminum-containing adjuvant, such as Alum, about 50 μg of the aluminum-containing adjuvant, about 45 μg of the aluminum-containing adjuvant, about 40 μg of the aluminum-containing adjuvant, about 35 μg of the aluminum-containing adjuvant, about 25 μg of the aluminum-containing adjuvant, about 24 μg, about 23 μg, about 22 μg, about 20 μg of the aluminum-containing adjuvant, about 15 μg of the aluminum-containing adjuvant, about 10 μg of the aluminum-containing adjuvant, about 9 μg of the aluminum-containing adjuvant, or about 8, about 7, about 6, about 5, about 4, and about 3 μg of the aluminum-containing adjuvant. In some embodiments, a unit dose of the immunogenic composition may include about 10 μg to about 2,000 μg of a CpG adjuvant, or about 20 μg to about 1,800, 1,700, 1,600 μg of the CpG adjuvant, or about 40 μg to about 1,500 μg of the CpG adjuvant, or about 1,000 μg to about 1,900 μg of the CpG adjuvant, or about 1,200 μg to about 1,800 μg of the CpG adjuvant, or about 1,300 μg to about 1,700 μg of the CpG adjuvant, or about 1,400 μg to about 1,600 ng of the CpG adjuvant, or about 10 μg to about 500 μg of the CpG adjuvant, or about 460 μg to about 470 μg of the CpG adjuvant, or about 410 μg, 420 μg, 430 μg, 440 μg to about 490 μg of the CpG adjuvant, or about 10 μg to about 500 μg of the CpG adjuvant, or preferably about 25 μg to about 300 μg of the CpG adjuvant, preferably about 50 μg to about 250 μg of the CpG adjuvant, preferably about 75 μg to about 200 μg of the CpG adjuvant, preferably about 100 gg to about 175 μg of the CpG adjuvant, or preferably about 1,500 μg of the CpG adjuvant, or preferably about 1,600 μg of the CpG adjuvant, or preferably about 1,400 μg, about 1,450 μg, about 1,455 μg of the CpG adjuvant, and preferably about 150 μg of the CpG adjuvant, such as CpG 1018A. In some embodiments, a unit dose of the immunogenic composition may include about 10 μg to about 500 μg of an aluminum-containing adjuvant and about 10 μg to about 500 μg of a CpG adjuvant. In some embodiments, a unit dose of the immunogenic composition may include about 10 μg to about 800 μg of an aluminum-containing adjuvant and about 10 μg to about 1800 μg of a CpG adjuvant. In some embodiments, a unit dose of the immunogenic composition may include about 25 μg to about 250 μg of an aluminum-containing adjuvant and about 25 μg to about 300 μg of a CpG adjuvant. In some embodiments, a unit dose of the immunogenic composition may include about 200 μg to about 800 μg of an aluminum-containing adjuvant and about 500 μg to about 1,800 μg of a CpG adjuvant. In some embodiments, a unit dose of the immunogenic composition may include about 200 μg to about 250 μg of an aluminum-containing adjuvant and about 400 μg to about 500 μg of a CpG adjuvant. In some embodiments, a unit dose of the immunogenic composition may include about 210 μg to about 240 μg of an aluminum-containing adjuvant and about 420 μg to about 490 μg of a CpG adjuvant. In some embodiments, a unit dose of the immunogenic composition may include about 220 μg to about 230 μg of an aluminum-containing adjuvant and about 430 μg to about 470 μg of a CpG adjuvant. In some embodiments, a unit dose of the immunogenic composition may include about 221 μg to about 227 μg of an aluminum-containing adjuvant and about 440 μg to about 460 μg of a CpG adjuvant. In some embodiments, a unit dose of the immunogenic composition may include about 600 μg to about 900 μg of an aluminum-containing adjuvant and about 1,000 μg to about 2,000 μg of a CpG adjuvant. In some embodiments, a unit dose of the immunogenic composition may include about 700 μg to about 800 μg of an aluminum-containing adjuvant and about 1,100 μg to about 1,900 or 1,800 μg of a CpG adjuvant. In some embodiments, a unit dose of the immunogenic composition may include about 720 μg to about 790 μg of an aluminum-containing adjuvant and about 1,200 or 1,300 μg to about 1,700 μg of a CpG adjuvant. In some embodiments, a unit dose of the immunogenic composition may include about 730 or 740 μg to about 760 μg of an aluminum-containing adjuvant and about 1,400 μg to about 1,600 μg of a CpG adjuvant. In some embodiments, a unit dose of the immunogenic composition may include about 50 μg to about 125 μg of an aluminum-containing adjuvant and about 50 μg to about 250 μg of a CpG adjuvant. In some embodiments, a unit dose of the immunogenic composition may include about 50 μg to about 100 μg of an aluminum-containing adjuvant and about 75 μg to about 200 μg of a CpG adjuvant. In some embodiments, a unit dose of the immunogenic composition may include about 50 μg to about 100 μg of an aluminum-containing adjuvant and about 100 μg to about 175 μg of a CpG adjuvant. In some embodiments, a unit dose of the immunogenic composition may include about 75 μg to about 100 μg of an aluminum-containing adjuvant, such as Alum, and about 150 μg of a CpG adjuvant, such as CpG 1018A. In some embodiments, a unit dose of the immunogenic composition may include about 200 μg to about 250 μg of an aluminum-containing adjuvant, such as Alum, and about 400 μg to about 500 μg of a CpG adjuvant, such as CpG 1018A. In some embodiments, a unit dose of the immunogenic composition may include about 210 μg to about 240 μg of an aluminum-containing adjuvant, such as Alum, and about 420 μg to about 490 μg of a CpG adjuvant, such as CpG 1018A. In some embodiments, a unit dose of the immunogenic composition may include about 220 μg to about 230 μg of an aluminum-containing adjuvant, such as Alum, and about 430 μg to about 470 μg of a CpG adjuvant, such as CpG 1018A. In some embodiments, a unit dose of the immunogenic composition may include about 221 μg to about 227 μg of an aluminum-containing adjuvant, such as Alum, and about 440 μg to about 460 μg of a CpG adjuvant, such as CpG 1018A. In some embodiments, a unit dose of the immunogenic composition may include about 600 μg to about 900 μg of an aluminum-containing adjuvant, such as Alum, and about 1,000 μg to about 2,000 μg of a CpG adjuvant, such as CpG 1018A. In some embodiments, a unit dose of the immunogenic composition may include about 700 μg to about 800 μg of an aluminum-containing adjuvant, such as Alum, and about 1,100 μg to about 1,900 or 1,800 μg of a CpG adjuvant, such as CpG 1018A. In some embodiments, a unit dose of the immunogenic composition may include about 720 μg to about 790 μg of an aluminum-containing adjuvant, such as Alum, and about 1,200 or 1,300 μg to about 1,700 μg of a CpG adjuvant, such as CpG 1018A. In some embodiments, a unit dose of the immunogenic composition may include about 730 or 740 μg to about 760 μg of an aluminum-containing adjuvant, such as Alum, and about 1,400 μg to about 1,600 μg of a CpG adjuvant, such as CpG 1018A. In some embodiments, a unit dose of the immunogenic composition may include about 10 μg to about 100 μg of SARS-CoV-2 antigens, such as any one or more SARS-CoV-2 S fusion proteins or S-trimers in the present disclosure, such as SARS-CoV-2 coronavirus Omicron (B.1.1.529) variant S protein peptide or a peptide fragment, variant or mutant fusion protein or S fusion protein trimer. In some embodiments, a unit dose of the immunogenic composition may include about 20 μg to about 75 μg of a SARS-CoV-2 S fusion protein or S-trimer, preferably about 25 μg to about 60 μg of the SARS-CoV-2 S fusion protein or S-trimer, or about 30 μg, about 40 μg, about 50 μg of the SARS-CoV-2 S fusion protein or S-trimer. In some embodiments, a unit dose of the immunogenic composition may include about 3 μg of a SARS-CoV-2 S fusion protein or S-trimer. In other embodiments, a dose includes 15 μg of a SARS-CoV-2 S fusion protein or S-trimer. In other embodiments, a dose includes 9 μg of the SARS-CoV-2 S fusion protein or S-trimer. In a further embodiment, a dose includes 30 μg of a SARS-CoV-2 S fusion protein or S-trimer. In some embodiments, a unit dose of the immunogenic composition may include about 5 μg to about 20 μg of SARS-CoV-2 antigens, such as any one or more SARS-CoV-2 S fusion proteins or S-trimers in the present disclosure, for example, including SARS-CoV-2 coronavirus Omicron (B.1.1.529) variant S protein peptide or a peptide fragment, variant or mutant fusion protein or S fusion protein trimer, may include about 200 μg to about 250 μg of an aluminum-containing adjuvant, such as Alum, and about 400 μg to about 500 μg of a CpG adjuvant, such as CpG 1018A. In some embodiments, a unit dose of the immunogenic composition may include about 6 μg to about 17, 18, 19 μg of SARS-CoV-2 antigens, such as any one or more SARS-CoV-2 S fusion proteins or S-trimers in the present disclosure, for example, including SARS-CoV-2 coronavirus Omicron (B.1.1.529) variant S protein peptide or a peptide fragment, variant or mutant fusion protein or S fusion protein trimer, may include about 210 μg to about 240 μg of an aluminum-containing adjuvant, such as Alum, and about 420 μg to about 490 μg of a CpG adjuvant, such as CpG 1018A. In some embodiments, a unit dose of the immunogenic composition may include about 8 μg to about 12, 13, 14, 15, 16 μg of SARS-CoV-2 antigens, such as any one or more SARS-CoV-2 S fusion proteins or S-trimers in the present disclosure, for example, including SARS-CoV-2 coronavirus Omicron (B.1.1.529) variant S protein peptide or a peptide fragment, variant or mutant fusion protein or S fusion protein trimer, may include about 220 μg to about 230 μg of an aluminum-containing adjuvant, such as Alum, and about 430 µg to about 470 µg of a CpG adjuvant, such as CpG 1018A. In some embodiments, a unit dose of the immunogenic composition may include about 8 µg to about 11 µg of SARS-CoV-2 antigens, such as any one or more SARS-CoV-2 S fusion proteins or S-trimers in the present disclosure, for example including SARS-CoV-2 coronavirus Omicron (B.1.1.529) variant S protein peptide or a peptide fragment, variant or mutant fusion protein or S fusion protein trimer, may include about 221 µg to about 227 µg of an aluminum-containing adjuvant, such as Alum, and about 440 µg to about 460 µg of a CpG adjuvant, such as CpG 1018A. In some embodiments, a unit dose of the immunogenic composition may include about 8 µg to about 12, 13, 14, 15, 16 µg of SARS-CoV-2 antigens, such as any one or more SARS-CoV-2 S fusion proteins or S-trimers in the present disclosure, for example, including SARS-CoV-2 coronavirus Omicron (B.1.1.529) variant S protein peptide or a peptide fragment, variant or mutant fusion protein or S fusion protein trimer, may include about 600 µg to about 900 µg of an aluminum-containing adjuvant, such as Alum, and about 1,000 µg to about 2,000 µg of a CpG adjuvant, such as CpG 1018A. In some embodiments, a unit dose of the immunogenic composition may include about 10 µg to about 80 µg of SARS-CoV-2 antigens, such as any one or more SARS-CoV-2 S fusion proteins or S-trimers in the present disclosure, for example including SARS-CoV-2 coronavirus Omicron (B.1.1.529) variant S protein peptide or a peptide fragment, variant or mutant fusion protein or S fusion protein trimer, may include about 700 µg to about 800 µg of an aluminum-containing adjuvant, such as Alum, and about 1,100 µg to about 1,900 or 1,800 µg of a CpG adjuvant, such as CpG 1018A. In some embodiments, a unit dose of the immunogenic composition may include about 15 µg to about 60 or 70 µg of SARS-CoV-2 antigens, such as any one or more SARS-CoV-2 S fusion proteins or S-trimers in the present disclosure, for example including SARS-CoV-2 coronavirus Omicron (B.1.1.529) variant S protein peptide or a peptide fragment, variant or mutant fusion protein or S fusion protein trimer, may include about 720 µg to about 790 µg of an aluminum-containing adjuvant, such as Alum, and about 1,200 or 1,300 µg to about 1,700 µg of a CpG adjuvant, such as CpG 1018A. In some embodiments, a unit dose of the immunogenic composition may include about 25 µg to about 40 or 50 µg of SARS-CoV-2 antigens, such as any one or more SARS-CoV-2 S fusion proteins or S-trimers in the present disclosure, for example including SARS-CoV-2 coronavirus Omicron (B.1.1.529) variant S protein peptide or a peptide fragment, variant or mutant fusion protein or S fusion protein trimer, may include about 730 or 740 µg to about 760 µg of an aluminum-containing adjuvant, such as Alum, and about 1,400 µg to about 1,600 µg of a CpG adjuvant, such as CpG 1018A. In some embodiments, a unit dose of the immunogenic composition may include about 30 ng of a SARS-CoV-2 S fusion protein or S-trimer, about 75 µg to about 100 µg of an aluminum-containing adjuvant, such as Alum, and about 150 µg of a CpG adjuvant, such as CpG 1018A. In some embodiments, a unit dose of the immunogenic composition may include about 9 µg of SARS-CoV-2 antigens, such as any one or more SARS-CoV-2 S fusion proteins or S-trimers in the present disclosure, for example including SARS-CoV-2 coronavirus Omicron (B.1.1.529) variant S protein peptide or a peptide fragment, variant or mutant fusion protein or S fusion protein trimer, may include about 225 µg of an aluminum-containing adjuvant, such as Alum, and about 450 µg of a CpG adjuvant, such as CpG 1018A. In some embodiments, a unit dose of the immunogenic composition may include about 30 µg of SARS-CoV-2 antigens, such as any one or more SARS-CoV-2 S fusion proteins or S-trimers in the present disclosure, for example including SARS-CoV-2 coronavirus Omicron (B.1.1.529) variant S protein peptide or a peptide fragment, variant or mutant fusion protein or S fusion protein trimer, may include about 750 µg of an aluminum-containing adjuvant, such as Alum, and about 1,500 µg of a CpG adjuvant, such as CpG 1018A.

In some embodiments, the immunogenic composition includes pharmaceutically acceptable excipients including, for example, solvents, fillers, buffers, tension regulators, and preservatives (Pramanick et al., Pharma Times, 45:65-77, 2013, incorporated by reference in its entirety for all purposes). In some embodiments, the immunogenic composition may include an excipient that acts as one or more of a solvent, filler, buffer, and tension regulator (for example, sodium chloride in saline can be used as both an aqueous carrier and a tension regulator).

In some embodiments, the immunogenic composition includes an aqueous carrier as a solvent. Suitable carriers include, for example, sterile water, saline, phosphate buffered saline, and Ringer's solution. In some embodiments, the composition is isotonic.

The immunogenic composition may include a buffer. The pH of the buffer is controlled to inhibit degradation of an active agent during handling, storage, and optional reconstitution. Suitable buffers include, for example, salts containing acetates, citrates, phosphates (such as sodium dihydrogen phosphate monohydrate, disodium hydrogen phosphate dihydrate) or sulfates. For example, other suitable buffers include, for example, amino acids such as arginine, glycine, histidine, and lysine. The buffer may further include hydrochloric acid or sodium hydroxide. In some embodiments, the buffer maintains the pH of the composition in a range of 6 to 9. In some embodiments, the pH is greater than (lower limit) 6, 7 or 8. In some embodiments, the pH is less than (upper limit) 9, 8 or 7. That is, the pH is in a range of about 6 to 9, where the lower limit is less than the upper limit. In some embodiments, the immunogenic composition may include 0.01-1 or 0.02-0.09 or 0.03-0.08 or 0.04-0.07 or 0.04-0.06 mg/ml sodium dihydrogen phosphate monohydrate, 0.01-1 or 0.02-0.09 or 0.03-0.08 or 0.04-0.07 or 0.04-0.06 mg/ml disodium hydrogen phosphate dihydrate. In some embodiments, the immunogenic composition may include 0.052 mg/ml sodium dihydrogen phosphate monohydrate and 0.052 mg/ml disodium hydrogen phosphate dihydrate. In some embodiments, the immunogenic composition may include 0.08-1 or 0.09-0.9 or 0.1-0.8 or 0.2-0.7 or 0.3-0.65 or 0.35-0.55 or 0.38-0.45 hydrochloric acid (37%). In some embodiments, the immunogenic composition may include 0.406 mg/ml hydrochloric acid (37%).

The immunogenic composition may include a tension regulator. Suitable tension regulators include, for example, glucose, glycerol, sodium chloride, glycerin, and mannitol. In some embodiments, the immunogenic composition may include 1.00-12.00 or 2.00-11.00 or 3.00-10.00 or 4.00-9.00 or 5.00-8.00 or 6.00-7.00 mg/ml sodium chloride. In some embodiments, the immunogenic composition may include 6.860 mg/ml sodium chloride.

The immunogenic composition may include a filler. The filler is particularly useful when the pharmaceutical composition is freeze-dried before administration. In some embodiments, the filler is a protective agent that helps stabilize and prevent degradation of the active agent during freezing or spray drying and/or during storage. Suitable fillers are sugars (mono, di and polysaccharides), such as sucrose, lactose, trehalose, mannitol, sorbitol, tromethamine, polysorbate 80, glucose, and raffinose. In some embodiments, the immunogenic composition may include 0.08-1 or 0.09-0.9 or 0.1-0.8 or 0.2-0.7 or 0.3-0.65 or 0.4-0.64 or 0.5-0.61 mg/ml tromethamine. In some embodiments, the immunogenic composition may include 0.605 mg/ml tromethamine. In some embodiments, the immunogenic composition may include 0.001-0.8 or 0.002-0.7 or 0.003-0.6 or 0.004-0.7 or 0.005-0.6 or 0.008-0.4 or 0.01-0.2 or 0.02-0.09 or 0.021-0.07 or 0.022-0.05 or 0.023-0.03 mg/ml polysorbate 80. In some embodiments, the immunogenic composition may include 0.025 mg/ml polysorbate 80.

The immunogenic composition may include a preservative. Suitable preservatives include, for example, antioxidants and antibacterial agents. However, in a preferred embodiment, the immunogenic composition is prepared under sterile conditions and in a disposable container, so it does not need to contain a preservative.

In some embodiments, the immunogenic composition may include 0.001-0.1 mg/ml SARS-CoV-2 Hu-1 spike (S) protein surface antigen or a fragment, variant or mutant thereof, 0.001-0.1 mg/m BA.5 spike (S) protein surface antigen or a fragment, variant or mutant thereof, 0.001-0.1 mg/ml XBB.1.5 spike (S) protein surface antigen or a fragment, variant or mutant thereof, 0.5-5 mg/ml of Alum, 0.5-6 mg/ml CpG1018, 0.02-0.09 mg/ml sodium dihydrogen phosphate monohydrate, 0.02-0.09 mg/ml disodium hydrogen phosphate dihydrate, 3.00-10.00 mg/ml sodium chloride, 0.003-0.6 mg/ml polysorbate 80, tromethamine, 0.09-0.9 mg/ml hydrochloric acid (37%), and an appropriate amount of water for injection (WFI). In some embodiments, the immunogenic composition may include 0.012 mg/ml SARS-CoV-2 Hu-1 spike (S) protein surface antigen or a fragment, variant or mutant thereof, 0.024 mg/m BA.5 spike (S) protein surface antigen or a fragment, variant or mutant thereof, 0.024 mg/ml XBB.1.5 spike (S) protein surface antigen or a fragment, variant or mutant thereof, 1.500 mg/ml Alum, 3.00 mg/ml CpG1018, 0.052 mg/ml sodium dihydrogen phosphate monohydrate, 0.052 mg/ml disodium hydrogen phosphate dihydrate, 6.86 mg/ml sodium chloride, 0.025 mg/ml polysorbate 80, 0.605 mg/ml tromethamine, 0.406 mg/ml hydrochloric acid (37%), and an appropriate amount of WFI.

In some embodiments, the composition can be provided as a sterile composition. A pharmaceutical composition generally contains an effective amount of the disclosed immunogen and can be prepared by conventional techniques. Typically, the amount of immunogen in each dose of the immunogenic composition is selected as the amount that induces an immune response without significant adverse side effects. In some embodiments, the composition can be provided in a unit dose form for inducing an immune response in a subject. The unit dose form contains a suitable single pre-selected dose for administration to the subject, or a suitable labeled or measured multiple of two or more pre-selected unit doses, and/or a metering mechanism for administering a unit dose or a multiple thereof. In other embodiments, the composition further includes one or more adjuvants.

## IV. Method for inducing immune response

In some embodiments, the present disclosure provides a method for generating an immune response to a coronavirus surface antigen in a subject, including administering to the subject an effective amount of a complex, where the complex includes a recombinant polypeptide selected from a group consisting of SEQ ID NOs: 1-26, 85-92, 120, and 124, and optionally the complex is used as a primary series, an additional dose, and/or a homologous or heterologous booster dose, such as a first dose, a second dose, a third dose, a fourth dose, and/or more doses. Optionally, the primary series, additional dose or heterologous booster dose is used in combination with any one or more of other recombinant subunit vaccines, nanoparticle vaccines, mRNA vaccines, DNA vaccines, adenovirus vector vaccines, and inactivated virus vaccines. In some embodiments, the present disclosure provides a method for generating an immune response to a coronavirus surface antigen in a subject, where the surface antigen includes an S protein or an antigenic fragment thereof, and the method includes administering to the subject an effective amount of a complex, where the complex includes a recombinant polypeptide selected from a group consisting of SEQ ID NOs: 1-26 and 85-92, and optionally the complex is used as a primary series, an additional dose, and/or a homologous or heterologous booster dose, such as a first dose, a second dose, a third dose, a fourth dose, and/or more doses. Optionally, the primary series, additional dose or heterologous booster dose is used in combination with any one or more of other recombinant subunit vaccines, nanoparticle vaccines, mRNA vaccines, DNA vaccines, adenovirus vector vaccines, and inactivated virus vaccines. In some embodiments, the present disclosure provides a method for generating an immune response to a coronavirus surface antigen in a subject, where the surface antigen includes a sequence selected from a group consisting of SEQ ID NOs: 27-66 and 81-84, and the method includes administering to the subject an effective amount of a complex, where the complex includes a recombinant polypeptide selected from a group consisting of SEQ ID NOs: 1-26 and 85-92, and optionally the complex is used as a primary series, an additional dose, and/or a homologous or heterologous booster dose, such as a first dose, a second dose, a third dose, a fourth dose, and/or more doses. Optionally, the primary series, additional dose or heterologous booster dose is used in combination with any one or more of other recombinant subunit vaccines, nanoparticle vaccines, mRNA vaccines, DNA vaccines, adenovirus vector vaccines, and inactivated virus vaccines. In some embodiments, the present disclosure provides a method for generating an immune response to a coronavirus surface antigen in a subject, where the surface antigen includes the S protein of a coronavirus or an antigenic fragment thereof, and optionally, the surface antigen includes a sequence selected from a group consisting of SEQ ID NO: 27-66 and 81-84 or an antigenic fragment thereof, and the method includes administering to the subject an effective amount of a complex, where the complex includes a recombinant polypeptide, the recombinant polypeptide includes a sequence described in any one of SEQ ID NOs: 85-92, and optionally the complex is used as a primary series, an additional dose, and/or a homologous or heterologous booster dose, such as a first dose, a second dose, a third dose, a fourth dose, and/or more doses. Optionally, the primary series, additional dose or heterologous booster dose is used in combination with any one or more of other recombinant subunit vaccines, nanoparticle vaccines, mRNA vaccines, DNA vaccines, adenovirus vector vaccines, and inactivated virus vaccines. Optionally, the adjuvant in any primary series, additional dose, and/or booster dose may independently include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

In some embodiments, the present disclosure provides a method for generating an immune response to a coronavirus surface antigen in a subject, where the surface antigen includes an S protein or an antigenic fragment thereof, and the method includes administering to the subject an effective amount of a complex or a combination of any two or more in the complex, the complex includes a recombinant polypeptide, the recombinant polypeptide includes a sequence selected from a group consisting of SEQ ID NOs: 1-26 and 85-92, and optionally the complex or combination is used as a primary series and/or as a booster dose, such as a booster dose of a second and/or third dose. In some embodiments, the method includes administering to a subject an effective amount of a complex, the complex includes a recombinant polypeptide, and the recombinant polypeptide includes sequences described in SEQ ID NO: 85, SEQ ID NO: 86, SEQ ID NO: 87, and/or SEQ ID NO: 88. Optionally, the adjuvant in any primary series, additional dose, and/or booster dose may independently include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

The disclosed immunogen (such as a recombinant coronavirus S antigen, such as the S-trimer or S protein described herein, a nucleic acid molecule (such as an RNA molecule) or vector encoding a protopolymer of the disclosed recombinant coronavirus S antigen, or a protein nanoparticle or virus-like particle containing the disclosed recombinant coronavirus S antigen) can be administered to a subject to induce an immune response to the corresponding coronavirus S antigen in the subject. In a specific example, the subject is a human. The immune response can be a protective immune response, such as a response that suppresses subsequent corresponding coronavirus infection. Stimulation of an immune response can further be used to treat or suppress infections and diseases associated with the corresponding coronavirus.

Subjects can be selected for treatment that has or is at risk of contracting the coronavirus, such as because of exposure or possible exposure to the coronavirus. After administering the disclosed immunogen, the subject can be monitored for infection or coronavirus-related symptoms, or both.

Typical subjects to be treated with the therapy and method of the present disclosure include humans as well as non-human primates and other animals. In order to identify subjects for prevention or treatment according to the method of the present disclosure, an acceptable screening method is used to determine risk factors associated with a target or suspected disease or condition, or to determine the status of an existing disease or condition in a subject. These screening methods include, for example, routine examinations to identify environmental, familial, occupational, and other such risk factors that may be associated with the target or suspected disease or condition, as well as diagnostic methods such as various ELISA and other immunoassay methods used to detect and/or characterize coronavirus infection. These and other conventional methods allow clinicians to select patients who need treatment using the method and pharmaceutical composition of the present disclosure. According to these methods and principles, the composition can be administered according to the teachings of this article or other conventional methods as an independent prevention or treatment plan, or as a follow-up, auxiliary or coordinated treatment plan for other treatments.

Administering the disclosed immunogen (such as coronavirus S antigen, such as trimer, protein) can be used for prophylactic or therapeutic purposes. When provided prophylactically, the disclosed therapeutic agent is provided before any symptoms, such as before infection. The prophylactic administration of the disclosed therapeutic agent is used to prevent or ameliorate any subsequent infection. When provided therapeutically, the disclosed therapeutic agent is provided at or after an onset of symptoms of a disease or infection, for example, after the development of symptoms of coronavirus infection corresponding to the coronavirus S antigen, or after the diagnosis of coronavirus infection. Therefore, therapeutic agents can be provided before an expected exposure to the coronavirus in order to reduce the expected severity, duration or degree of symptoms of infection and/or related diseases after exposure or suspected exposure to the virus, or after an actual onset of infection.

The immunogen and the immunogenic composition described herein are provided to the subject in an amount that effectively induces or enhances the immune response of the subject (preferably human) against the coronavirus S antigen. An actual dose of the disclosed immunogen may vary depending on factors such as disease signs and specific conditions of a subject (such as age, physique, health status, degree of symptoms, and susceptibility factors), administration time and route, other drugs or treatments administered simultaneously, and the specific pharmacology of the composition to stimulate a desired activity or biological response in the subject. Dosage regimens can be adjusted to provide a best prophylactic or therapeutic response.

Immunogenic compositions including one or more of the disclosed immunogens may be used in a coordinated (or priming-boosting) vaccination regimen or combined preparation. In certain embodiments, individual immunogens or preparations are used in the new combined immunogenic composition and coordinated immunization protocol, and each immunogen or preparation is intended to induce an antiviral immune response, such as an immune response to the coronavirus S antigen. Individual immunogenic compositions that elicit an antiviral immune response can be combined in a multivalent immunogenic composition administered to a subject in a single immunization step, or the individual immunogenic compositions can be administered alone (in a monovalent immunogenic composition) in a coordinated (or priming-boosting) immunization protocol.

There may be several booster doses, each of the booster doses may be a different disclosed immunogen. In some examples, the booster dose can be the same immunogen as another booster dose or priming dose. The priming dose and the booster dose can be administered as a single dose or multiple doses, for example, two, three, four, five, six or more doses can be administered to the subject over days, weeks or months. Multiple booster doses may also be applied, such as one to five times (such as 1, 2, 3, 4 or 5 booster doses) or more. Different doses can be used for a series of consecutive immunizations. For example, a relatively large dose is used in primary immunization and then boosted with a relatively small dose.

In some embodiments, the booster dose can be applied about two weeks, about three to eight weeks or about four weeks after a primary immunization or about a few months after the primary immunization. In some embodiments, the booster dose may be applied at about 5, about 6, about 7, about 8, about 10, about 12, about 18, about 24 months after the priming dose or a longer or shorter period after the priming dose. Additional booster doses can also be used regularly at appropriate time points to boost a subject's "immune memory". The suitability of selected vaccine parameters, such as preparation, dosage, and regimen, can be determined by taking aliquots of serum from a subject and measuring antibody titers during an immunization program. In addition, clinical conditions of the subject can be monitored to obtain the desired effect, such as preventing infection or improving health status (such as reducing viral load) against a disease. If such monitoring indicates that vaccination is suboptimal, additional doses of the immunogenic composition may be used to strengthen the subject and vaccination parameters may be improved in a manner expected to boost the immune response.

In some embodiments, the priming-boosting immunization method may include a DNA-primer and protein-booster vaccination regimen for the subject. The method may include applying nucleic acid molecules or proteins two or more times.

For a protein therapy, typically, each human dose will include 1-1,000 $\mu$g of protein, such as from about 1 $\mu$g to about 100 $\mu$g, such as from about 1 $\mu$g to about 50 $\mu$g, such as about 1 $\mu$g, about 2 $\mu$g, about 5 $\mu$g, about 10 $\mu$g, about 15 $\mu$g, about 20 $\mu$g, about 25 $\mu$g, about 30 $\mu$g, about 40 $\mu$g or about 50 $\mu$g.

The amount used in the immunogenic composition is selected based on the subject population (such as infants or the elderly). The optimal dosage of a specific component can be determined by standard studies involving observation of antibody titers and other reactions in subjects. It should be understood that therapeutically effective amounts of disclosed immunogens (such as disclosed recombinant coronavirus S antigens, such as trimers, proteins, viral vectors or nucleic acid molecules in immunogenic compositions) may include amounts that are ineffective when inducing immune responses by single-dose administration but effective when administered at multiple doses, such as in a priming-boosting dosing regimen.

After administering the immunogen disclosed in the present disclosure, the immune system of the subject generally responds to the immunogenic composition by producing antibodies specific for the coronavirus S protein peptide contained in the immunogen. This response means that an immune-effective dose is delivered to the subject.

In some embodiments, the antibody response of a subject will be determined in the context of evaluating an effective dose/immunization protocol. In most cases, it is sufficient to evaluate the antibody titer in serum or plasma obtained from a subject. The decision on whether to give a booster vaccination and/or change the amount of therapeutic dose given to an individual can be based at least in part on the antibody titer level. The antibody titer level can be based on, for example, an immunobinding assay that measures the concentration of antibodies binding to antigens (including, for example, recombinant coronavirus S antigen, such as S-trimer) in serum.

The method can be effective without completely eliminating, reducing or preventing coronavirus infection. For example, stimulating an immune response to a coronavirus with one or more disclosed immunogens can reduce or suppress a desired amount of coronavirus infection by, such as at least 10%, at least 20%, at least 50%, at least 60%, at least 70%, at least 80%, at least 90%, at least 95%, at least 98%, and even at least 100% (eliminating or preventing detectable infected cells) compared to coronavirus infection in the absence of an immunogen. In another example, coronavirus replication can be reduced or inhibited by the disclosed method. The method can be effective without completely eliminating coronavirus replication. For example, stimulating an immune response with one or more disclosed immunogens can reduce a desired amount of corresponding coronavirus replication by, such as at least 10%, at least 20%, at least 50%, at least 60%, at least 70%, at least 80%, at least 90%, at least 95%, at least 98%, and even at least 100% (eliminating or preventing detectable coronavirus replication) compared to coronavirus replication in the absence of an immune response.

In some embodiments, the disclosed immunogen is administered to a subject while administering an adjuvant. In other embodiments, the disclosed immunogen is administered to a subject after administration of the adjuvant and for a sufficient time to induce an immune response.

One method of nucleic acid administration is direct immunization with plasmid DNA, such as using a mammalian expression plasmid. Immunization by nucleic acid constructs is well known in the art and is disclosed, for example, in U.S. Patent No. 5,643,578 (which describes a method of immunizing vertebrates by introducing DNA encoding a desired antigen to elicit a cell-mediated or humoral response) and U.S. Patent Nos. 5,593,972 and 5,817,637 (which describe operably linking a nucleic acid sequence encoding an antigen to a regulatory sequence capable of expression). U.S. Patent No. 5,880,103 describes several methods for delivering nucleic acids encoding immunogenic peptides or other antigens to organisms. The method includes liposome delivery of nucleic acids (or their own synthetic peptides) and immunostimulatory constructs or ISCOMS™, which is a 30-40 nm negatively charged cage structure spontaneously formed by mixing cholesterol and Quil A™ (saponin). The use of ISCOMS™ as an antigen delivery vehicle has produced protective immunity in experimental models of various infections, including toxoplasmosis and Epstein-Barr virus-induced tumors (Mowat and Donachie, *Immunol*. Today 12:383, 1991). Antigen doses as low as 1 $\mu$g encapsulated in ISCOMS™ have been found to produce a class I mediated CTL response (Takahashi et al., *Nature* 344:873, 1990, incorporated by reference in its entirety for all purposes).

In some embodiments, the plasmid DNA vaccine is used to express the disclosed immunogen in a subject. For example, a nucleic acid molecule encoding the disclosed immunogen can be administered to a subject to induce an immune response to the coronavirus S antigen. In some embodiments, the nucleic acid molecule may be included on a plasmid vector for DNA immunization, such as a pVRC8400 vector (as described in Barouch et al., J. Virol, 79, 8828-8834, 2005, incorporated by reference in its entirety for all purposes).

In another method of immunization using nucleic acids, the disclosed recombinant coronavirus S antigen (such as trimer, protein) can be expressed by an attenuated viral host or vector or bacterial vector. Recombinant vaccinia virus, adenovirus-associated virus (AAV), herpes virus, retrovirus, cytogmeglo virus or other viral vectors can be used to express peptides or proteins to induce CTL response. For example, U.S. Patent No. 4,722,848 describes a cowpox vector and method useful in immunization programming, which is incorporated by reference in its entirety for all purposes. Bacillus Calmette Guerin Vaccine provides another vector for expressing peptides (see Stover, Nature 351: 456-460, 1991, incorporated by reference in its entirety for all purposes).

In one embodiment, the nucleic acid encoding the disclosed recombinant coronavirus S antigen is directly introduced into a cell. For example, nucleic acids can be loaded onto gold microcarriers by standard methods and introduced into skin through equipment such as Bio-Rad HELIOS™ gene gun. Nucleic acids can be "naked" and consist of plasmids under the control of strong promoters. Usually, DNA is injected into the muscle, but it can also be injected directly into other parts. The injection dose is generally about 0.5 $\mu$g/kg to about 50 mg/kg, and typically about 0.005 mg/kg to about 5 mg/kg (see, for example, U.S. Patent No. 5,589,466).

In another embodiment, an mRNA-based immunization protocol can be used to deliver a nucleic acid encoding the disclosed recombinant coronavirus S antigen directly into cells. In some embodiments, mRNA-based nucleic acid vaccines can provide an effective alternative to the foregoing methods. mRNA vaccines eliminate the safety issues of DNA integration into a host genome and can be translated directly in cytoplasm of the host cell. In addition, the simple cell-free in vitro synthesis of RNA avoids manufacturing complications associated with viral vectors. Exemplary forms of two RNA-based vaccines that can be used to deliver the nucleic acid encoding the disclosed recombinant coronavirus S antigen include conventional non-amplified mRNA immunization (see, for example, Petsch et al., "Protective efficacy of in vitro synthesized, specific mRNA vaccines against influenza A virus infection," Nature biotechnology, 30(12):1210-6, 2012) and self-amplified mRNA immunization (see, for example, Geall et al., "Nonviral delivery of self-amplifying RNA vaccines,"

**EP 4 722 230 A1**

PNAS, 109(36): 14604-14609, 2012; Magini et al., "Self-Amplifying mRNA Vaccines Expressing Multiple Conserved Influenza Antigens Confer Protection against Homologous and Heterosubtypic Viral Challenge," PLoS One, 11(8):e0161193, 2016;和 Brito et al., "Self-amplifying mRNA vaccines," Adv Genet., 89:179-233, 2015). All documents in this paragraph are incorporated by reference in their entireties for all purposes.

In some embodiments, administering a therapeutically effective amount of one or more disclosed immunogens to a subject can induce a neutralizing immune response in the subject. To assess neutralizing activity, serum may be collected from subjects at reasonable time points after immunization, frozen and stored for neutralization assay. Methods for determining neutralizing activity are known to ordinarily skilled persons in the art and are further described herein, including but not limited to plaque reduction neutralization test (PRNT) assays, microneutralization assays, flow cytometry-based assays, and single-cycle infection assays. In some embodiments, a group of coronavirus pseudoviruses can be used to determine neutralizing activity of serum.

In some embodiments, the neutralizing immune response induced by the immunogen disclosed herein produces neutralizing antibodies against coronaviruses (such as SARS-CoV-2). In some embodiments, the neutralizing antibody in this article binds to a cell receptor or co-receptor of a coronavirus (such as SARS-CoV-2) or its components. In some embodiments, the viral receptor or co-receptor is a coronavirus receptor or co-receptor, preferably a pneumonia virus receptor or co-receptor, and more preferably a human coronavirus receptor, such as a SARS-CoV-2 receptor or co-receptor. In some embodiments, the neutralizing antibodies herein regulate, reduce, antagonize, alleviate, block, inhibit, eliminate and/or interfere with at least one coronavirus (such as SARS-CoV-2) activity or binding in vitro, in situ and/or in vivo, or coronavirus (such as SARS-CoV-2) receptor activity or binding, such as SARS-CoV-2 release, SARS-CoV-2 receptor signaling, Membrane SARS-CoV-2 cleavage, SARS-CoV-2 activity, and SARS-CoV-2 production and/or synthesis. In some embodiments, the immunogen disclosed herein induces neutralizing antibodies against SARS-CoV-2 that regulate, reduce, antagonize, alleviate, block, inhibit, eliminate and/or interfere with the binding of SARS-CoV-2 to SARS-CoV-2 receptors or co-receptors, such as angiotensin converting enzyme 2 (ACE2), dipeptidyl peptidase 4 (DPP4), dendritic cell-specific intercellular adhesion molecule-3-grabbing nonintegrin (DC-SIGN) and/or liver/lymph node-SIGN (L-SIGN).

**V. Products or kits**

A product or kit containing the provided recombinant polypeptide, protein and immunogenic composition is further provided. The product may include a container and a label or packaging description on or associated with the container. Suitable containers include, for example, bottles, vials, syringes, tubes, and IV solution bags. Containers can be formed of various materials (such as glass or plastic). In some embodiments, the container has a sterile access port. Exemplary containers include intravenous solution bags, vials, and containers with stoppers that can be pierced by injection needles. The product or kit may further include a packaging description indicating that the composition can be used to treat a specific disease, such as the disease described herein (such as coronavirus infection). Alternatively, or in addition, the product or kit may further include another or a same container containing a pharmaceutically acceptable buffer. The product or kit may further include other materials, such as other buffers, diluents, filters, needles and/or syringes.

The label or packaging description may indicate that the composition is used to treat coronavirus infection in an individual. The label or package description on or associated with the container may indicate the reconstitution and/or instructions for use of a preparation. The label or packaging description may further indicate that the preparation is used or intended for subcutaneous, intravenous or other administration methods to treat or prevent coronavirus infection in an individual.

In some embodiments, the container contains a composition that is effective alone or in combination with another composition for treating, preventing and/or diagnosing a condition. The product or kit may include (a) a first container having a composition (namely, a first agent) contained therein, where the composition includes an immunogenic composition or its protein or recombinant polypeptide; and (b) a second container having a composition (namely, a second agent) contained therein, where the composition includes another agent, such as an adjuvant or other therapeutic agent, and the product or kit further includes instructions on the label or packaging description to treat a subject with the second agent in an effective amount.

**Terminology**

Unless otherwise defined, all special terms, symbols and other technical and scientific terms or terminology used in this article are intended to have a same meaning as those generally understood by ordinarily skilled persons in the art of the claimed subject matter. In some cases, for the sake of clarity and/or ease of reference, this article defines terms with commonly understood meanings, and such definitions contained in this article do not necessarily represent substantial differences from what is generally understood in the art.

41

The terms "polypeptide" and "protein" are used interchangeably to refer to polymers of amino acid residues and are not limited to the minimum length. The polypeptide (including the provided receptor and other polypeptides, such as connectors or peptides) may include amino acid residues, including natural and/or non-natural amino acid residues. Terms also include post-expression modifications of polypeptides, such as glycosylation, sialylation, acetylation, and phosphorylation. In some aspects, as long as a protein maintains a desired activity, a polypeptide may contain modifications to a native or natural sequence. These modifications can be intentional, such as through site-directed mutagenesis, or accidental, such as through mutations in a host that produces the protein or due to errors caused by PCR amplification.

As used herein, a "subject" is a mammal, such as a human or other animal, and usually a human. In some embodiments, the subject (such as patient) to whom one or more agents, cells, cell groups, or compositions are administered is a mammal, typically a primate, such as a human. In some embodiments, the primate is a monkey or an ape. The subjects can be male or female and can be of any appropriate age group, including infants, teenagers, adolescents, adults, and elderly subjects. In some embodiments, the subject is a non-primate mammal, such as a rodent.

As used herein, "treatment" (and its grammatical variants such as "treat" or "treating") means the complete or partial improvement or reduction of a disease, condition or disorder, or the symptoms, adverse reactions or outcomes associated therewith, or a phenotype. A desired effect of treatment includes but is not limited to preventing the occurrence or recurrence of a disease, alleviating symptoms, reducing any direct or indirect pathological consequences of the disease, preventing metastasis, reducing the rate of disease progression, improving health or alleviating the disease state, and alleviating or improving prognosis. The term does not mean a complete cure of the disease or a complete elimination of any symptoms or effects on all symptoms or outcomes.

As used herein, "slowing development of a disease" means delaying, hindering, slowing, slowing down, stabilizing, inhibiting and/or delaying the development of a disease (such as cancer). Depending on the medical history and/or an individual receiving treatment, the length of slowing may vary. In some embodiments, sufficient or significant slowing can actually include prevention because an individual may not develop the disease. For example, the development of advanced cancers, such as metastases, may be slowed.

As used herein, "prevention" includes providing prevention of disease occurrence or recurrence for subjects who may be susceptible to a disease but have not yet been diagnosed with the disease. In some embodiments, the provided cells and compositions are used to slow the development of a disease or slow down the progression of a disease.

As used herein, "inhibition" of function or activity means a reduction in function or activity when compared to the same condition other than the condition or parameter of interest or compared to another condition. For example, cells that inhibit tumor growth will reduce the growth rate of tumors compared to when there are no cells.

In the case of drug administration, the "effective amount" of an agent (such as a pharmaceutical preparation, cell or composition) refers to a dose/amount and effective amount for the time period required to achieve a desired effect (such as a therapeutic or prophylactic effect).

The "therapeutically effective amount" of an agent (such as a pharmaceutical preparation or cell) means an effective amount at the dose and time period required to achieve a desired therapeutic effect (such as for treating a disease, condition or disorder) and/or the pharmacokinetic or pharmacodynamic effects of the treatment. The therapeutically effective amount may vary depending on factors such as the disease state, age, sex, and weight of a subject and the cell population administered. In some embodiments, the method provided involves administering cells and/or compositions in an effective amount (such as a therapeutically effective amount).

"Prophylactically effective amount" refers to an effective amount of dose and time period required to achieve an expected prophylactic effect. Usually, but not necessarily, because a prophylactic dose is used for subjects before or in the early stages of a disease, the prophylactically effective amount will be less than the therapeutically effective amount. In cases where the tumor burden is low, some aspects of prevention will be more effective than treatment. An effective amount of a vaccine or other agent is sufficient to produce a desired response, such as reducing or eliminating signs or symptoms of an illness or disease, such as pneumonia. For example, this may be the amount necessary to inhibit viral replication or measurably change the external symptoms of a viral infection. Generally, this amount will be sufficient to measurably inhibit viral (such as SARS-CoV-2) replication or infectivity. When administered to a subject, the dose typically used will reach the target tissue concentration that has been shown to achieve inhibition of viral replication in vitro. In some embodiments, an "effective amount" is an amount that treats (including prevents) one or more symptoms and/or potential causes of any condition or disease, such as for treating coronavirus infection. In some embodiments, the effective amount is a therapeutically effective amount. In some embodiments, the effective amount is an amount that prevents the development of one or more signs or symptoms of a specific disease or condition, such as one or more signs or symptoms associated with coronavirus infection.

As used herein, the terms "antigen" or "immunogen" are interchangeably used to refer to a substance capable of inducing an immune response in a subject, typically a protein. The term also refers to an immunocompetent protein that, once administered to a subject (either directly or by administering a nucleotide sequence or vector encoding the protein) is capable of eliciting a humoral and/or cellular type immune response against the protein. Unless otherwise specified, the term "vaccine immunogen" is used interchangeably with "protein antigen" or "immunogenic polypeptide".

The term "conservatively modified variant" applies to amino acid and nucleic acid sequences. With respect to a specific nucleic acid sequence, conservatively modified variants refer to those nucleic acids encoding identical or substantially identical amino acid sequences, or in the case where the nucleic acid does not encode an amino acid sequence, refer to substantially same sequences. Due to the degeneracy of the genetic code, a large number of functionally identical nucleic acids encode any given protein. For a polypeptide sequence, a "conservatively modified variant" refers to a variant with a conservative amino acid substitution in which an amino acid residue is replaced by another amino acid residue having a side chain of similar charge. A family of amino acid residues having side chains with similar charges has been defined in the art. These families include those with basic side chains (such as lysine, arginine, histidine), acidic side chains (such as aspartic acid, glutamic acid), uncharged polar side chains (such as glycine, asparagine, glutamine, serine, threonine, tyrosine, cysteine), non-polar side chains (such as alanine, valine, leucine, isoleucine, proline, phenylalanine, methionine, tryptophan), β-branched chains (such as threonine, valine, isoleucine), and aromatic side chains (such as tyrosine, phenylalanine, tryptophan, histidine).

Epitopes refer to antigenic determinants. These are specific chemical groups or peptide sequences on antigen molecules, so they trigger specific immune responses. For example, epitopes are antigenic regions to which B cells and/or T cells respond. Epitopes can be formed by either continuous amino acids or non-continuous amino acids juxtaposed by tertiary folding of proteins.

Unless otherwise specified, fusion proteins are recombinant proteins containing amino acid sequences of at least two unrelated proteins that are connected together by peptide bonds to form a single protein. Therefore, it does not contain naturally occurring coronavirus surface antigens, namely the fusion (F) protein described herein. Unrelated amino acid sequences can be directly connected to each other, or they can be connected using a connector sequence. As used herein, a protein is unrelated if its amino acid sequences are not normally connected together by peptide bonds in their natural environment (such as within a cell). For example, the amino acid sequences of viral antigens and collagen or procollagen are not usually connected together by peptide bonds.

An immunogen is a protein or part thereof that can induce an immune response in mammals, such as those infected by a pathogen or at risk of pathogen infection. The administration of an immunogen can lead to protective immunity and/or active immunity against the target pathogen.

An immunogenic composition refers to a composition including an immunogenic polypeptide that induces a measurable CTL response against a virus expressing the immunogenic polypeptide, or induces a measurable B cell response (such as production of antibodies) against the immunogenic polypeptide.

The sequence identity or similarity between two or more nucleic acid sequences or two or more amino acid sequences is expressed as the identity or similarity between the sequences. Sequence consistency can be measured as percentage consistency; the higher the percentage, the more identical the sequence. When comparing and aligning the specified areas measured by a comparison window or using one of the following sequence comparison algorithms or by manual alignment and visual inspection to obtain maximum correspondence, if two sequences have a specified percentage of identical amino acid residues or nucleotides, then the two sequences are "substantially identical" (namely, 60% identity over the specified area, or in the unspecified case over the entire sequence, 65%, 70%, 75%, 80%, 85%, 90%, 95% or 99% identity is preferred). Optionally, the identity exists over a region of at least about 50 nucleotides (or 10 amino acids) in length, or more preferably over a region of 100 to 500 or 1,000 or more nucleotides (or 20, 50, 200 or more amino acids) in length.

A vaccine is a pharmaceutical composition that induces a prophylactic or therapeutic immune response in a subject. In some cases, the immune response is a protective immune response. Usually, vaccines induce antigen-specific immune responses against antigens of pathogens (such as viral pathogens) or cellular components associated with pathological conditions. Vaccines may include polynucleotides (such as nucleic acids encoding disclosed antigens), peptides or polypeptides (such as disclosed antigens), viruses, cells or one or more cellular components. In some embodiments, the vaccine or vaccine immunogen or vaccine composition is expressed from a fusion construct and self-assembled into nanoparticles that display an immunogenic polypeptide or protein on a surface.

A virus-like particle (VLP) is a non-replicable viral shell derived from any of several viruses. VLPs are generally composed of one or more viral proteins, such as but not limited to proteins called capsid, coating, shell, surface and/or envelope proteins, or particle-forming polypeptides derived from these proteins. After recombinantly expressing the protein in an appropriate expression system, VLPs can form spontaneously. The method for producing a specific VLP is known in the art. The presence of VLPs after recombinant expression of viral proteins can be detected using conventional techniques known in the art, such as by electron microscopy or biophysical characterization. See, for example, Baker et al. (1991) Biophys. J. 60:1445-1456; and Hagensee et al. (1994) J. Virol. 68:4503-4505, which are incorporated by reference in their entireties for all purposes. For example, VLPs can be separated by density gradient centrifugation and/or identified by characteristic density bands. Alternatively, a cryo-electron microscopy can be operated on the vitrified aqueous sample prepared by the VLP in question and the image recorded under appropriate exposure conditions.

The term "approximately/about" as used herein refers to the usual error range of various values that are easily known to those skilled in the art. References to "approximately/about" values or parameters in this article include (and describe) embodiments for the value or parameter itself.

In this article, various aspects of the claimed subject matter are presented in a range format. It should be understood that the description of the range format is only for convenience and brevity, and should not be interpreted as an inflexible limitation on the range of the claimed subject matter. Therefore, it should be considered that the description of the range has specifically disclosed all possible sub-ranges and individual values within the range. For example, in the case of providing a range of values, it should be understood that each intervention value between the upper and lower limits of a range and any other statement or intervention value within the range is included in the claimed subject matter. The upper and lower limits of these smaller ranges may be included independently within the smaller ranges and also within the claimed subject matter, subject to any expressly excluded limitations within the range. If the range includes one or two limitations, the range that does not include one or both of those limitations is also included in the claimed subject matter. This applies to a width of any range.

As used herein, composition means any mixture of two or more products, substances or compounds (including cells). It can be a solution, suspension, liquid, powder, paste, aqueous substance, non-aqueous substance or any combination thereof.

The term "vector" as used herein refers to a nucleic acid molecule capable of transmitting another nucleic acid attached thereto. The term includes vectors that are self-replicating nucleic acid structures and vectors incorporated into a host cell genome into which the vector has been introduced. Certain vectors can guide the expression of nucleic acids operably connected to them. Such vectors are referred to as "expression vectors" in this article.

**Exemplary embodiment I**

Embodiment 1. A protein including a plurality of recombinant polypeptides is provided. Each of the recombinant polypeptide includes an XBB.1.5 surface antigen of a coronavirus SARS-CoV-2 Omicron (B.1.1.529) connected to a C-terminal propeptide of collagen, where the C-terminal propeptide of the recombinant polypeptide forms an inter-polypeptide disulfide bond.

Embodiment 2. For the protein according to Embodiment 1, coronavirus infection is SARS-coronavirus 2 (SARS-CoV-2) infection.

Embodiment 3. For the protein according to Embodiment 1 or 2, the surface antigen includes a coronavirus spike (S) protein or a fragment or epitope thereof, where the epitope is optionally a linear epitope or a conformational epitope, and where the protein includes three recombinant polypeptides.

Embodiment 4. For the protein according to Embodiment 3, the surface antigen includes a signal peptide, an S1 subunit peptide, an S2 subunit peptide or any combination thereof.

Embodiment 5. For the protein according to Embodiment 3, the surface antigen includes a signal peptide, a receptor binding domain (RBD) peptide, a receptor binding motif (RBM) peptide, a fusion peptide (FP), a heptapeptide repeat 1 (HR1) peptide or a heptapeptide repeat 2 (HR2) peptide or any combination thereof.

Embodiment 6. For the protein according to any one of Embodiments 3-5, the surface antigen includes a receptor binding domain (RBD) of an S protein.

Embodiment 7. For the protein according to any one of Embodiments 3-6, the surface antigen includes an S1 subunit and an S2 subunit of the S protein.

Embodiment 8. For the protein according to any one of Embodiments 3-7, the surface antigen does not include a transmembrane (TM) domain peptide and/or a cytoplasmic (CP) domain peptide.

Embodiment 9. For the protein according to any one of Embodiments 3-8, the surface antigen includes a protease cleavage site, where the protease is optionally furin, trypsin, factor Xa, thrombin or cathepsin L.

Embodiment 10. For the protein according to any one of Embodiments 3-8, the surface antigen does not include a protease cleavage site, where the protease is optionally furin, trypsin, factor Xa, thrombin or cathepsin L.

Embodiment 11. For the protein according to any one of Embodiments 1-10, the surface antigen is soluble or not directly bound to a lipid bilayer, such as a membrane or a viral envelope.

Embodiment 12. For the protein according to any one of Embodiments 1-11, the surface antigen is the same or different in the recombinant polypeptide of the protein.

Embodiment 13. For the protein according to any one of Embodiments 1-12, the surface antigen is directly fused to the C-terminal propeptide or connected to the C-terminal propeptide via a connector (such as a connector including a glycine-X-Y repeat), where X and Y are independently any amino acids and are optionally proline or hydroxyproline.

Embodiment 14. The protein according to any one of Embodiments 1-13 is soluble or not directly bound to a lipid bilayer, such as a membrane or a viral envelope.

Embodiment 15. The protein according to any one of Embodiments 1-14 is capable of binding to a cell surface receptor of a subject, optionally where the subject is a mammal, such as a primate, such as a human.

Embodiment 16. For the protein according to Embodiment 15, the cell surface receptor is angiotensin converting enzyme 2 (ACE2), dipeptidyl peptidase 4 (DPP4), dendritic cell-specific intercellular adhesion molecule-3-grabbing nonintegrin (DC-SIGN) or liver/lymph node-SIGN (L-SIGN).

Embodiment 17. For the protein according to any one of Embodiments 1-16, the C-terminal propeptide is human collagen.

Embodiment 18. For the protein according to any one of Embodiments 1-17, the C-terminal propeptide includes a C-terminal polypeptide or a fragment thereof of proα1(I), proα1(II), proα1(III), proα1(V), proα1(XI), proα2(I), proα2(V), proα2(XI) or proα3(XI).

Embodiment 19. For the protein according to any one of Embodiments 1-18, the C-terminal propeptide is the same or different in the recombinant polypeptide.

Embodiment 20. For the protein according to any one of Embodiments 1-19, the C-terminal propeptide includes any one of SEQ ID NOs: 67-80 or an amino acid sequence at least 90% identical thereto, which is capable of forming inter-polypeptide disulfide bonds and trimerizing the recombinant polypeptide.

Embodiment 21. For the protein according to any one of Embodiments 1-20, the C-terminal propeptide includes SEQ ID NO: 67 or an amino acid sequence at least 90% identical thereto, which is capable of forming inter-polypeptide disulfide bonds and trimerizing the recombinant polypeptide.

Embodiment 22. For the protein according to any one of Embodiments 1-20, the C-terminal propeptide includes SEQ ID NO: 68 or an amino acid sequence at least 90% identical thereto, which is capable of forming inter-polypeptide disulfide bonds and trimerizing the recombinant polypeptide.

Embodiment 23. For the protein according to any one of Embodiments 1-20, the C-terminal propeptide includes SEQ ID NO: 69 or an amino acid sequence at least 90% identical thereto, which is capable of forming inter-polypeptide disulfide bonds and trimerizing the recombinant polypeptide.

Embodiment 24. For the protein according to any one of Embodiments 1-20, the C-terminal propeptide includes SEQ ID NO: 70 or an amino acid sequence at least 90% identical thereto, which is capable of forming inter-polypeptide disulfide bonds and trimerizing the recombinant polypeptide.

Embodiment 25. For the protein according to any one of Embodiments 1-20, the C-terminal propeptide includes SEQ ID NO: 71 or an amino acid sequence at least 90% identical thereto, which is capable of forming inter-polypeptide disulfide bonds and trimerizing the recombinant polypeptide.

Embodiment 26. For the protein according to any one of Embodiments 1-20, the C-terminal propeptide includes SEQ ID NO: 72 or an amino acid sequence at least 90% identical thereto, which is capable of forming inter-polypeptide disulfide bonds and trimerizing the recombinant polypeptide.

Embodiment 27. For the protein according to any one of Embodiments 1-20, the C-terminal propeptide includes SEQ ID NO: 73 or an amino acid sequence at least 90% identical thereto, which is capable of forming inter-polypeptide disulfide bonds and trimerizing the recombinant polypeptide.

Embodiment 28. For the protein according to any one of Embodiments 1-20, the C-terminal propeptide includes SEQ ID NO: 74 or an amino acid sequence at least 90% identical thereto, which is capable of forming inter-polypeptide disulfide bonds and trimerizing the recombinant polypeptide.

Embodiment 29. For the protein according to any one of Embodiments 1-20, the C-terminal propeptide includes SEQ ID NO: 75 or SEQ ID NO: 76 or an amino acid sequence at least 90% identical thereto, which is capable of forming inter-polypeptide disulfide bonds and trimerizing the recombinant polypeptide.

Embodiment 30. For the protein according to any one of Embodiments 1-29, the C-terminal propeptide includes a sequence containing a N-terminal glycine-X-Y repeat connected to any one of SEQ ID NOs: 67-80, where X and Y are independently any amino acids, and optionally proline or hydroxyproline, or an amino acid sequence at least 90% identical thereto, which is capable of forming inter-polypeptide disulfide bonds and trimerizing the recombinant polypeptide.

Embodiment 31. For the protein according to any one of Embodiments 1-30, the surface antigen in each recombinant polypeptide is in a pre-fusion conformation or a post-fusion conformation.

Embodiment 32. For the protein according to any one of Embodiments 1-31, the surface antigen in each recombinant polypeptide includes SEQ ID NO: 27-66 and any one of SEQ ID NOs: 81-84, 122, and 126 or an amino acid sequence at least 80% identical thereto.

Embodiment 33. For the protein according to any one of Embodiments 1-32, the recombinant polypeptide includes SEQ ID NO: 1-26 and any one of SEQ ID NOs: 85-92, 120, and 124 or an amino acid sequence at least 80% identical thereto.

Embodiment 34. An immunogen including a protein of any one of Embodiments 1-33 is provided. Optionally, the immunogen is used as a primary series, an additional dose, and/or a homologous or heterologous booster dose, such as a first dose, a second dose, a third dose, a fourth dose, and/or more doses. Optionally, the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines. Optionally, the adjuvant in the primary series, the additional dose, and/or the homologous or heterologous booster dose may independently include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

Embodiment 35. A protein nanoparticle including a protein of any one of Embodiments 1-33 is provided. The protein is directly or indirectly connected to the nanoparticle and optionally used as a primary series, an additional dose, and/or a homologous or heterologous booster dose, such as a first dose, a second dose, a third dose, a fourth dose, and/or more doses. Optionally, the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines. Optionally, the adjuvant in the primary series, the additional dose, and/or the homologous or heterologous booster dose may independently include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

Embodiment 36. A virus-like particle (VLP) including a protein of any one of Embodiments 1-33 is provided. Optionally, the VLP is used as a primary series, an additional dose, and/or a homologous or heterologous booster dose, such as a first dose, a second dose, a third dose, a fourth dose, and/or more doses. Optionally, the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines. Optionally, the adjuvant in the primary series, the additional dose, and/or the homologous or heterologous booster dose may independently include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

Embodiment 37. An isolated nucleic acid encoding one, two, three or more recombinant polypeptides of the protein according to any one of Embodiments 1-33.

Embodiment 38. For the isolated nucleic acid according to Embodiment 37, a polypeptide encoding a surface antigen is fused within a framework to a polypeptide encoding a C-terminal propeptide of collagen.

Embodiment 39. The isolated nucleic acid according to Embodiment 37 or 38 is operably connected to a promoter.

Embodiment 40. The isolated nucleic acid according to any one of Embodiments 37-39 is a DNA molecule.

Embodiment 41. The isolated nucleic acid according to any one of Embodiments 37-39 is an RNA molecule, optionally an mRNA molecule, such as a nucleoside-modified mRNA, a non-amplified mRNA, a self-amplified mRNA or a trans-amplified mRNA.

Embodiment 42. A vector including the isolated nucleic acid according to any one of Embodiments 37-41.

Embodiment 43. The vector according to Embodiment 42 is a viral vector.

Embodiment 44. A virus, pseudovirus or cell including the vector according to Embodiment 42 or 43 is provided and optionally the virus or cell has a recombinant genome.

Embodiment 45. An immunogenic composition is provided, including the protein, immunogen, protein nanoparticle, VLP, isolated nucleic acid, vector, virus, pseudovirus, or cell according to any one of Embodiments 1-44 and a pharmaceutically acceptable carrier.

Embodiment 46. A vaccine including an immunogenic composition according to Embodiment 45 and an optional adjuvant is provided, where the vaccine is optionally a subunit vaccine, and/or optionally where the vaccine is a prophylactic and/or therapeutic vaccine, optionally the vaccine is used as a primary series, an additional dose, and/or a homologous or heterologous booster dose, such as a first dose, a second dose, a third dose, a fourth dose, and/or more doses. Optionally, the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines. Optionally, the adjuvant in the primary series, the additional dose, and/or the homologous or heterologous booster dose may independently include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

Embodiment 47. The vaccine according to Embodiment 46 includes a plurality of different adjuvants.

Embodiment 48. A method for producing a protein, including: expressing an isolated nucleic acid or vector according to any one of Embodiments 37-43 in a host cell to produce the protein according to any one of Embodiments 1-33; and purifying the protein.

Embodiment 49. Protein produced by the method of Embodiment 48.

Embodiment 50. A method for generating an immune response to a coronavirus surface antigen in a subject is provided, including administering to the subject an effective amount of the protein, immunogen, protein nanoparticle, VLP, isolated nucleic acid, vector, virus, pseudovirus, cell, immunogenic composition or vaccine according to any one of Embodiments 1-47 and 49 to generate an immune response.

Embodiment 51. The method according to Embodiment 50 is used for treating or preventing coronavirus infection.

Embodiment 52. For the method according to Embodiment 50 or 51, an immune response is generated to suppress or reduce coronavirus replication in a subject.

Embodiment 53. For the method according to any one of Embodiments 50-52, the immune response includes a cell-mediated response and/or a humoral response, optionally including the production of one or more neutralizing antibodies, such as polyclonal antibodies or monoclonal antibodies.

Embodiment 54. For the method according to any one of Embodiments 50-53, the immune response is directed against the surface antigen of the coronavirus but not against the C-terminal propeptide.

Embodiment 55. For the method according to any one of Embodiments 50-54, the administration does not cause antibody-dependent enhancement (ADE) in a subject due to previous exposure to one or more coronaviruses.

Embodiment 56. For the method according to any one of Embodiments 50-55, the administration does not cause antibody-dependent enhancement (ADE) in a subject when subsequently exposed to one or more coronaviruses.

Embodiment 57. The method according to any one of Embodiments 50-56 further includes a priming immunization step and/or a boosting immunization step.

Embodiment 58. For the method according to any one of Embodiments 50-57, a dosing step is performed by local, percutaneous, subcutaneous, intradermal, oral, intranasal (such as intranasal spray), intratracheal, sublingual, buccal, rectal, vaginal, inhalation, intravenous (such as intravenous injection), intraarterial, intramuscular (such as intramuscular injection), intracardiac, intraosseous, intraperitoneal, transmucosal, intravitreal, subretinal, intra-articular, periarticular, topical, or epicutaneous administration.

Embodiment 59. For the method according to any one of Embodiments 50-58, an effective amount is administered in a single dose or a series of doses having one interval or more intervals.

Embodiment 60. For the method according to any one of Embodiments 50-59, an effective amount is administered without an adjuvant.

Embodiment 61. For the method according to any one of Embodiment 50-59, an effective amount is administered together with an adjuvant or multiple adjuvants.

Embodiment 62. A method is provided, including administering to a subject an effective amount of the protein according to any one of Embodiments 1-33 to produce neutralizing antibodies or neutralizing antiserum against a coronavirus in the subject.

Embodiment 63. For the method according to Embodiment 62, the subject is a mammal, optionally a human or a non-human primate.

Embodiment 64. The method according to Embodiment 62 or 63 further includes separating neutralizing antibodies or neutralizing antiserum from the subject.

Embodiment 65. The method according to Embodiment 64 further includes administering an effective amount of the isolated neutralizing antibodies or neutralizing antiserum to a human subject through passive immunization to prevent or treat coronavirus infection.

Embodiment 66. For the method according to any one of Embodiments 62-65, the neutralizing antibody or neutralizing antiserum includes a polyclonal antibody against a coronavirus surface antigen, optionally where the neutralizing antibody or neutralizing antiserum does not contain or substantially contains an antibody against the C-terminal propeptide of collagen.

Embodiment 67. For the method according to any one of embodiments 62-65, the neutralizing antibody includes a monoclonal antibody against a coronavirus surface antigen, optionally where the neutralizing antibody does not contain or substantially contains an antibody against the C-terminal propeptide of collagen.

Embodiment 68. The protein, immunogen, protein nanoparticle, VLP, isolated nucleic acid, vector, virus, pseudovirus, cell, immunogenic composition or vaccine according to any one of Embodiments 1-47 and 49 is used to induce an immune response to a coronavirus in a subject, and/or to treat or prevent coronavirus infection.

Embodiment 69. Use of the protein, immunogen, protein nanoparticle, VLP, isolated nucleic acid, vector, virus, pseudovirus, cell, immunogenic composition or vaccine according to any one of Embodiments 1-47 and 49 is provided to induce an immune response to a coronavirus in a subject, and/or to treat or prevent coronavirus infection.

Embodiment 70. Use of the protein, immunogen, protein nanoparticle, VLP, isolated nucleic acid, vector, virus, pseudovirus, cell, immunogenic composition or vaccine according to any one of Embodiments 1-47 and 49 is provided for manufacturing a drug or prophylactic agent to induce an immune response to a coronavirus in a subject, and/or to treat or prevent coronavirus infection.

Embodiment 71. A method for analyzing a sample is provided, including: contacting the sample with the protein according to any one of Embodiments 1-33, and detecting the binding between the protein and an analyte that can specifically bind to a coronavirus surface antigen.

Embodiment 72. For the method according to Embodiment 71, the analyte is an antibody, receptor or cell that recognizes a surface antigen.

Embodiment 73. For the method according to Embodiment 71 or 72, the binding indicates the presence of an analyte in a sample and/or the presence of coronavirus infection in a subject from which the sample originated.

Embodiment 74. A kit including the protein according to any one of Embodiments 1-33 and a substrate, plate or vial containing or fixing the protein is provided. Optionally, the kit is an ELISA or a lateral flow assay kit.

**Exemplary embodiment II**

Embodiment 1. A method for preventing coronavirus infection in a mammal is provided. The method includes immunizing the mammal with an effective amount of a recombinant subunit vaccine, the recombinant subunit vaccine includes a soluble surface antigen of XBB.1.5, a subtype of SARS-CoV-2 Omicron (B.1.1.529), or a fragment, variant or mutant of the soluble surface antigen, and the soluble surface antigen of the coronavirus or the fragment, variant or mutant thereof is connected to a C-terminal portion of collagen by in-frame fusion to form a trimeric fusion protein connected by disulfide bonds.

Embodiment 2. For the method according to Embodiment 1, the coronavirus infection is a severe acute respiratory syndrome (SARS)-coronavirus 2 (SARS-CoV-2) infection.

Embodiment 3. For the method according to Embodiment 1 or 2, the surface antigen of the coronavirus encapsulates a coronavirus spike (S) protein or a fragment or epitope thereof.

Embodiment 4. According to any one of Embodiments 1-3, the coronavirus surface antigen includes a SARS-CoV-2 spike (S) extracellular domain peptide or a fragment or epitope thereof, and optionally the S extracellular domain peptide or a fragment or epitope thereof includes a subtype XBB.1.5 variant S extracellular domain peptide of SARS-CoV-2 Omicron (B.1.1.529) or a fragment, variant or mutant thereof, for example, chimeric sequences containing the Omicron variant receptor binding domain (RBD) and Hu-1 or other variant S protein peptide sequences.

Embodiment 5. For the method according to any one of Embodiments 1-4, the coronavirus surface antigen includes a SARS-CoV-2 spike (S) N-terminal domain (NTD) peptide or a fragment or epitope thereof, and optionally the NTD peptide is a SARS-CoV-2 Hu-1, Alpha, Beta, Gamma, Delta, Mu, or Omicron and its subtype NTD peptide or fragments, variants or mutants thereof.

Embodiment 6. For the method according to any one of Embodiments 1-5, the coronavirus surface antigen includes a SARS-CoV-2 spike (S) receptor binding domain (RBD) peptide or a fragment or epitope thereof, optionally the RBD peptide is a subtype XBB.1.5 variant RBD peptide of SARS-CoV-2 Omicron (B.1.1.529) or a fragment, variant or mutant thereof.

Embodiment 7. For the method according to any one of Embodiments 1-6, the coronavirus surface antigen includes a SARS-CoV-2 spike (S) S1 peptide or a fragment or epitope thereof, and optionally the S1 peptide is a subtype XBB.1.5 variant S1 peptide of SARS-CoV-2 Omicron (B.1.1.529) or a fragment, variant or mutant thereof.

Embodiment 8. For the method according to any one of Embodiments 1-7, the coronavirus surface antigen includes a SARS-CoV-2 spike (S) S2 peptide or a fragment or epitope thereof, and optionally the S2 peptide is a SARS-CoV-2 Hu-1, Alpha, Beta, Gamma, Delta, Mu, or Omicron and its subtype S2 peptide or fragments, variants or mutants thereof.

Embodiment 9. For the method according to any one of Embodiments 1-8, the coronavirus surface antigen includes a SARS-CoV-2 spike (S) extracellular domain peptide having a mutation or a fragment or epitope thereof.

Embodiment 10. For the method according to Embodiment 9, the mutation includes a furin cleavage site mutation, optionally the mutation is caused by deletion, substitution or addition of one or more amino acids so that the furin cleavage site no longer has activity as a furin cleavage site, optionally the mutation is located at any one or several of Sites 682-685, and optionally the mutation includes 685R→685A.

Embodiment 11. For the method according to Embodiment 9 or 10, the mutation includes a mutation located at or adjacent to a junction of heptapeptide repeat HR1 and a central helix, and optionally the mutation includes proline substitution, such as 986K→986P and/or 987V→987P.

Embodiment 12. For the method according to any one of Embodiments 9-11, the mutation includes consecutive site amino acid substitution, such as 986K→986P and 987V→987P.

Embodiment 13. For the method according to any one of Embodiments 1-12, the recombinant subunit vaccine includes a sequence in any one of SEQ ID NOs: 81-92 and 124-127 or an amino acid sequence having at least or about 80%, 85%, 90%, 92%, 95%, 97%, 99% sequence identity with a sequence in any one of SEQ ID NOs: 81-92, 122, and 126.

Embodiment 14. For the method according to any one of Embodiments 1-13, the recombinant subunit vaccine includes SEQ ID NO: 124 or an amino acid sequence having at least or about 80%, 85%, 90%, 92%, 95%, 97%, 99% sequence identity with SEQ ID NO: 124.

Embodiment 15. For the method according to any one of Embodiments 1-14, the recombinant subunit vaccine includes SEQ ID NO: 125 or an amino acid sequence having at least or about 80%, 85%, 90%, 92%, 95%, 97%, 99% sequence identity with SEQ ID NO: 125.

Embodiment 16. For the method according to any one of Embodiments 1-15, the recombinant subunit vaccine includes SEQ ID NO: 126 or an amino acid sequence having at least or about 80%, 85%, 90%, 92%, 95%, 97%, 99% sequence identity with SEQ ID NO: 126.

Embodiment 17. For the method according to any one of Embodiments 1-16, the recombinant subunit vaccine includes SEQ ID NO: 127 or an amino acid sequence having at least or about 80%, 85%, 90%, 92%, 95%, 97%, 99% sequence identity with SEQ ID NO: 127.

Embodiment 18. For the method according to any one of Embodiments 1-17, the recombinant subunit vaccine includes connecting a first sequence according to any one of SEQ ID NO: 81-84 to a second sequence according to any one of SEQ ID NO: 67-80, and a C-terminus of the first sequence is directly or indirectly connected to a N-terminus of the second sequence.

Embodiment 19. For the method according to any one of Embodiments 1-18, the recombinant subunit vaccine is administered via intramuscular injection.

Embodiment 20. For the method according to any one of Embodiments 1-19, the recombinant subunit vaccine is administered via an intranasal spray.

Embodiment 21. For the method according to any one of Embodiments 1-20, the recombinant subunit vaccine is administered in a single dose or in a series of doses spaced weekly or monthly.

Embodiment 22. For the method according to any one of Embodiments 1-21, the recombinant subunit vaccine is administered without an adjuvant, and optionally the recombinant subunit vaccine is used as a primary series, an additional dose, and/or a homologous or heterologous booster dose, such as a first dose, a second dose, a third dose, a fourth dose, and/or more doses, and optionally the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines.

Embodiment 23. For the method according to any one of Embodiments 1-22, the recombinant subunit vaccine is administered together with an adjuvant, and optionally the recombinant subunit vaccine is used as a primary series, an additional dose, and/or a homologous or heterologous booster dose, such as a first dose, a second dose, a third dose, a fourth dose, and/or more doses. Optionally, the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines. Optionally, the adjuvant in the primary series, the additional dose, and/or the homologous or heterologous booster dose may independently include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

Embodiment 24. For the method according to any one of Embodiments 1-23, the recombinant subunit vaccine is administered together with more than one adjuvant, and optionally the recombinant subunit vaccine is used as a primary series, an additional dose, and/or a homologous or heterologous booster dose, such as a first dose, a second dose, a third dose, a fourth dose, and/or more doses. Optionally, the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines. Optionally, the adjuvant in the primary series, the additional dose, and/or the homologous or heterologous booster dose may independently include: an aluminum-containing adjuvant, such as the adjuvant containing alum and/or aluminum hydroxide; an oligonucleotide-containing adjuvant, such as the adjuvant containing CpG oligodeoxynucleotides (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

Embodiment 25. A method for detecting coronavirus antibodies from mammalian serum is provided. The method includes a step of contacting the serum with a surface antigen of XBB.1.5, a subtype of SARS-CoV-2 Omicron (B.1.1.529), and the soluble surface antigen of the coronavirus is connected to a C-terminal portion of collagen by in-frame fusion to form a trimeric fusion protein connected by disulfide bonds.

Embodiment 26. For the method according to Embodiment 25, the soluble surface antigen of the coronavirus is an S protein or a peptide.

Embodiment 27. A method for using a recombinant subunit vaccine containing a soluble surface antigen of XBB.1.5, a subtype from SARS-CoV-2 Omicron (B.1.1.529), is provided, and the soluble surface antigen is connected to a C-terminal portion of collagen by in-frame fusion to form a trimeric fusion protein connected by disulfide bonds. The method includes immunizing a mammal, purifying the resulting neutralizing antibodies, and using the neutralizing antibodies to passively immunotherapy patients infected with the coronavirus.

Embodiment 28. For the method according to Embodiment 27, the neutralizing antibody includes a polyclonal antibody.

Embodiment 29. For the method according to Embodiment 27, the neutralizing antibody is a monoclonal antibody.

Embodiment 30. For the method according to Embodiment 27, the neutralizing antibody is a monoclonal antibody of an S protein or peptide.

Embodiment 31. For the method according to Embodiment 27, the neutralizing antibody is a monoclonal antibody of an S protein of SARS-CoV-2.

Embodiment 32. For the method according to Embodiment 27, the neutralizing antibody is a monoclonal antibody of an S protein of SARS-CoV-2 Hu-1, Alpha, Beta, Gamma, Delta, Mu, Omicron and their subtypes and/or other strains.

Embodiment 33. For the method according to Embodiment 27, the neutralizing antibody is a monoclonal antibody of a subtype XBB.1.5 S protein of SARS-CoV-2 Omicron (B.1.1.529).

Embodiment 34. A complex including a recombinant polypeptide selected from a group consisting of SEQ ID NOs: 124-127.

Embodiment 35. A complex including a trimer of a recombinant polypeptide selected from a group consisting of SEQ ID NOs: 124-127 is provided, and the recombinant polypeptide is trimerized by inter-polypeptide disulfide bonds to form a trimer.

Embodiment 36. An immunogenic composition including a trimer of a recombinant polypeptide or a combination of any two or more trimers is provided, and the recombinant polypeptide includes a sequence selected from a group consisting of SEQ ID NOs: 124-127.

Embodiment 37. The immunogenic composition according to Embodiment 36 includes a trimer of a recombinant polypeptide having a sequence according to SEQ ID NO: 125.

Embodiment 38. A method for generating an immune response to a coronavirus surface antigen in a subject is provided. The method includes administering to the subject an effective amount of a complex, where the complex includes a recombinant polypeptide selected from a group consisting of SEQ ID NOs: 85-92, and optionally the complex is used as a primary series, an additional dose, and/or a homologous or heterologous booster dose, such as a first dose, a second dose, a third dose, a fourth dose, and/or more doses. Optionally, the primary series, additional dose or heterologous booster dose is used in combination with any one or more of other recombinant subunit vaccines, nanoparticle vaccines, mRNA vaccines, DNA vaccines, adenovirus vector vaccines, and inactivated virus vaccines.

Embodiment 39. A method for generating an immune response to a coronavirus surface antigen in a subject is provided.

The surface antigen includes an S protein or an antigenic fragment thereof, and the method includes administering to a subject an effective amount of a complex, where the complex includes a recombinant polypeptide selected from a group consisting of SEQ ID NOs: 124-127, and optionally the complex is used as a primary series, an additional dose, and/or a homologous or heterologous booster dose, such as a first dose, a second dose, a third dose, a fourth dose, and/or more doses. Optionally, the primary series, additional dose or heterologous booster dose is used in combination with any one or more of other recombinant subunit vaccines, nanoparticle vaccines, mRNA vaccines, DNA vaccines, adenovirus vector vaccines, and inactivated virus vaccines.

Embodiment 40. A method for generating an immune response to a coronavirus surface antigen in a subject is provided.

The surface antigen includes a sequence selected from a group consisting of SEQ ID NOs: 27-66, 81-84, 122, and 124, and the method includes administering to a subject an effective amount of a complex, where the complex includes a recombinant polypeptide selected from a group consisting of SEQ ID NOs: 85-92, and optionally the complex is used as a primary series, an additional dose, and/or a homologous or heterologous booster dose, such as a first dose, a second dose, a third dose, a fourth dose, and/or more doses. Optionally, the primary series, additional dose or heterologous booster dose is used in combination with any one or more of other recombinant subunit vaccines, nanoparticle vaccines, mRNA vaccines, DNA vaccines, adenovirus vector vaccines, and inactivated virus vaccines.

Embodiment 41. A method for generating an immune response to a coronavirus surface antigen in a subject is provided.

The surface antigen includes an S protein of the coronavirus or an antigenic fragment thereof, and optionally the surface antigen includes a sequence selected from a group consisting of SEQ ID NOs: 27-66, 81-84, 122, and 124 or an antigenic fragment thereof. The method includes administering to a subject an effective amount of a complex, where the complex includes a recombinant polypeptide selected from a group consisting of SEQ ID NOs: 85-92, and optionally the complex is used as a primary series, an additional dose, and/or a homologous or heterologous booster dose, such as a first dose, a second dose, a third dose, a fourth dose, and/or more doses. Optionally, the primary series, additional dose or heterologous booster dose is used in combination with any one or more of other recombinant subunit vaccines, nanoparticle vaccines, mRNA vaccines, DNA vaccines, adenovirus vector vaccines, and inactivated virus vaccines.

Embodiment 42. A method for generating an immune response to a coronavirus surface antigen in a subject is provided.

The surface antigen includes an S protein or an antigenic fragment thereof, and

the method includes administering to a subject an effective amount of a complex or a combination of any two or more complexes, where the complex includes a recombinant polypeptide of a sequence selected from a group consisting of SEQ ID NOs: 85-92, 120, and 125, and optionally the complex or the combination of the complexes is used as a primary series, an additional dose, and/or a homologous or heterologous booster dose, such as a first dose, a second dose, a third dose, a fourth dose, and/or more doses. Optionally, the primary series, additional dose or heterologous booster dose is used in combination with any one or more of other recombinant subunit vaccines, nanoparticle vaccines, mRNA vaccines, DNA vaccines, adenovirus vector vaccines, and inactivated virus vaccines.

Embodiment 43. The method according to Embodiment 42 includes administering to a subject an effective amount of a complex including a recombinant polypeptide, and the recombinant polypeptide includes the sequence according to SEQ ID NO: 125.

Embodiment 44. A fusion protein including a plurality of recombinant polypeptides is provided, and each of the recombinant polypeptides includes, from an amino group to a carboxyl terminus:

a) a first region, where the first region includes a portion of an extracellular domain of a coronavirus spike protein located front of a receptor binding domain (RBD) of the coronavirus spike protein in a non-chimeric coronavirus spike protein of a first coronavirus;

b) a second region, where the second region includes a receptor binding domain (RBD) of a coronavirus spike protein of a second coronavirus different from the first coronavirus; and

c) a C-terminal propeptide of collagen, where the C-terminal propeptide of the recombinant polypeptide forms an inter-polypeptide disulfide bond, where at least one of the first coronavirus and the second coronavirus is XBB.1.5, a subtype of SARS-CoV-2 Omicron (B.1.1.529), optionally the fusion protein can be used as a primary series, an additional dose, and/or a homologous or heterologous booster dose, such as a first dose, a second dose, a third dose, a fourth dose, and/or more doses, and optionally, the primary series, additional dose or heterologous booster dose is used in combination with any one or more of other recombinant subunit vaccines, nanoparticle vaccines, mRNA vaccines, DNA vaccines, adenovirus vector vaccines, and inactivated virus vaccines.

Embodiment 45. The fusion protein according to Embodiment 44 further includes a third region between the second region and the C-terminal propeptide of collagen.

Embodiment 46. For the fusion protein according to Embodiment 45, the third region includes an S1 domain of a third coronavirus, where the third coronavirus is the same as or different from the first or second coronavirus.

Embodiment 47. For the fusion protein according to Embodiment 45 or 46, the third region includes an S2 domain of a fourth coronavirus, where the fourth coronavirus is the same as or different from the first, second or fourth coronavirus.

Embodiment 48. For the fusion protein according to any one of Embodiments 44-47, the first region includes an N-terminal domain (NTD) of the first coronavirus.

Embodiment 49. For the fusion protein according to any one of Embodiments 44-48, the first region includes one or more amino acid residues different from the corresponding amino acid residues in the second coronavirus.

Embodiment 50. For the fusion protein according to any one of Embodiments 44-49, the second region includes one or more amino acid residues different from the corresponding amino acid residues in the first coronavirus.

Embodiment 51. For the fusion protein according to any one of Embodiments 44-50, the first and second coronaviruses are different variants or strains of a same coronavirus.

Embodiment 52. For the fusion protein according to Embodiment 51, the first region includes the NTD of the first coronavirus, the second region includes the RBD of the second coronavirus, and the first and second coronaviruses are different variants of SARS-CoV-2.

Embodiment 53. For the fusion protein according to any one of Embodiments 44-52, the first coronavirus and the second coronavirus are independently selected from a group consisting of SARS-CoV-2 viruses in the B.1.1.529, B.1.617.2, B.1.526, B.1.1.143, P.2, B.1.351, P.1, B.1.1.7, B.1.617, A.23.1, BA.1, BA.2, BA.2.75, BA.5, BQ.1.1, BF.7, XBB, XBB.1.5, and BQ.1.1 lineages.

Embodiment 54. A trimeric fusion protein including three recombinant polypeptides is provided, and each of the recombinant polypeptides includes, from an amino group to a carboxyl terminus:

a) a first region, where the first region includes a spike protein N-terminal domain (NTD) of SARS-CoV-2 in the XBB.1.5 lineage;

b) a second region, where the second region includes a spike protein receptor binding domain (RBD) of SARS-CoV-2 in the XBB.1.5 lineage; and

c) a C-terminal propeptide of collagen, where the C-terminal propeptide of the recombinant polypeptide forms an inter-polypeptide disulfide bond, and optionally the trimeric fusion protein is used as a primary series, an additional dose, and/or a homologous or heterologous booster dose, such as a first dose, a second dose, a third dose, a fourth dose, and/or more doses, and optionally, the primary series, additional dose or heterologous booster dose is used in combination with any one or more of other recombinant subunit vaccines, nanoparticle vaccines, mRNA vaccines, DNA vaccines, adenovirus vector vaccines, and inactivated virus vaccines.

Embodiment 55. A method for preventing coronavirus infection in a mammal is provided, and the method includes immunizing the mammal with an effective amount of a fusion protein according to any one of Embodiments 44-54.

Embodiment 56. For the method according to Embodiment 55, neutralizing antibodies against the first and second coronaviruses are produced in the mammal.

Embodiment 57. For the method according to Embodiment 56, the first and second coronaviruses are different variants of SARS-CoV-2 and neutralizing antibodies produced in the mammal neutralize two or more SARS-CoV-2 viruses in the B.1.1.529, B.1.617.2, B.1.526, B.1.1.143, P.2, B.1.351, P.1, B.1.1.7, B.1.617, A.23.1, BA.1, BA.2, BA.2.75, BA.5, BQ.1.1, BF.7, XBB, and BQ.1.1 lineages.

Embodiment 58. For the method according to Embodiment 57, the neutralizing antibody produced in a mammal neutralizes three or more SARS-CoV-2 viruses in the B.1.1.529, B.1.617.2, B.1.526, B.1.1.143, P.2, B.1.351, P.1, B.1.1.7, B.1.617, A.23.1, BA.1, BA.2, BA.2.75, BA.5, BQ.1.1, BF.7, XBB and BQ.1.1 lineages.

Embodiment 59. The method according to any one of Embodiments 55-58 includes immunizing a mammal with two or more doses of a fusion protein, where at least one of the two or more doses of the fusion protein includes an amino acid sequence of SARS-CoV-2 Omicron XBB.1.5 spike protein, and optionally the at least one dose of the fusion protein includes a sequence in any one of SEQ ID NOs: 24-27 or an amino acid sequence with at least or about 80%, 85%, 90%, 92%, 95%, 97%, and 99% sequence identity with the sequence in any one of SEQ ID NOs: 24-27.

Embodiment 60. For the method according to any one of Embodiments 55-59, the fusion protein is administered with a booster dose after one or more doses of an immunogen, the immunogen includes a spike protein peptide including NTD and RBD from the same or different SARS-CoV-2 variants, optionally the one or more doses of the immunogen include a sequence in any one of SEQ ID NOs: 27-66, 81-84, and 120-127 or an amino acid sequence with at least or about 80%, 85%, 90%, 92%, 95%, 97%, and 99% sequence identity with the sequence in any one of SEQ ID NOs: 27-66, 81-84, and 120-127, and optionally, the booster dose fusion protein includes a sequence in any one of SEQ ID NOs: 24-27 or an amino acid sequence with at least or about 80%, 85%, 90%, 92%, 95%, 97%, and 99% sequence identity with the sequence in any one of SEQ ID NOs: 24-27.

## Examples

The following examples are included for illustrative purposes only and are not intended to limit the scope of the present disclosure.

### Example 1: Generation of SARS-CoV-2 S- trimeric fusion protein connected by recombinant disulfide bonds

A polypeptide connected by secretory recombinant disulfide bonds is produced, and the polypeptide contains a SARS-CoV-2 protein peptide fused to a trimerization domain as a candidate protein subunit vaccine. In one example, an extracellular domain of a spike protein from SARS-CoV-2 including its signal peptide (SP), S1 and S2 domains is fused to a mammalian expression vector encoding human C-propeptide of $\alpha$1 collagen at an in-frame C-terminus so that the secreted trimer (S-trimer) fusion antigen can be expressed.

For rapid expression of the S-trimeric antigen, a protein trimerization™ technology was used (Liu et al., Scientific Reports, 7(1): 8953, 2017, incorporated by reference in its entirety for all purposes). The cDNA encoding an extracellular domain of the SARS-CoV-2 spike (S) protein was subcloned into a pTRIMER mammalian expression vector to allow in-frame fusion to a protein trimerization™ tag, and the protein trimerization™ tag can self-trimerize via disulfide bonds. After stable transfection into CHO cells, subsequent screening of high titer production clones and extensive process optimization, a fed-batch serum-free cell culture process in a bioreactor was developed, resulting in high levels of expression of S-trimer as a secretory protein. Liang et al., Nat. Comms., 12:1346, 2021; Richmond et al., Lancet, 397: 682-694, 2021, are incorporated by reference in their entireties for all purposes.

In order to obtain a high-purity form of S-trimer for vaccine research, an affinity purification protocol was developed taking advantage of the high binding affinity between the protein trimerization™ tag and Endo180, where Endo180 is a collagen receptor that can bind to the C-terminal region of type 1 procollagen and mature collagen. The Endo180-Fc fusion protein was loaded onto a protein A column and captured by the resin through high affinity binding between protein A and the IgG1 Fc domain of Endo180-Fc. Then, a serum-free cell culture medium containing S-trimer secreted by CHO cells was loaded onto the protein A column with pre-captured Endo180-Fc. After washing away any unbound contaminating host cell proteins (HCP) and other impurities, the bound S-trimer is purified to near homogeneity using a mild salt elution step without separating Endo180-Fc from the protein A column. The S-trimer was further purified, and the low pH value treatment was used as a virus inactivation (VI) step, and then the host cell DNA and residual endotoxin were removed by anion exchange chromatography. Nanofiltration is used as a process step for prophylactic virus removal (VR). Finally, the S-trimer was concentrated to a required concentration of the preparation buffer by UF/DF, and the active drug substance (DS) of the S-trimer subunit candidate vaccine was obtained by sterile filtration. Stability analysis of the purified S-trimer showed that the S-trimer is stable in a liquid solution preparation at 2-8°C.

SDS-PAGE analysis under non-reducing and reducing conditions confirmed that the purified S-trimer is a trimer connected by disulfide bonds and is partially cleaved by furin produced by CHO cells at the S1/S2 boundary. Under non-reducing conditions, S-trimers appear in a variety of high molecular weight forms, which may be a result of partial cleavage of antigens, releasing non-covalently connected and cleaved S1 during sample processing.

## Production and characterization of spike antigens based on Hu-1 strain and VOC strain

The protein trimerization™ technology (Liang et al., Nat. Comms., 12:1346, 2021) was used to produce covalent trimers of a spike antigen based on Hu-1 strains and VOC strains, such as BA.5 and XBB.1.5. An 8% SDS-PAGE analysis of S-trimer expression in fed-batch serum-free CHO cell cultures was performed in a 10L bioreactor. A cell-free conditioned medium under reducing conditions was analyzed and then a Coomassie brilliant blue staining was performed.

The binding affinity of the purified S-trimeric antigen to the human ACE2 receptor was evaluated by Fortebio BioLayer interferometry using a protein A sensor.

## Example 2: Immunogenicity of SARS-CoV-2 vaccine with an adjuvant

Immunogenicity of the S-trimer in BALB/c mice was evaluated. In a two-dose priming-boosting protocol (day 0 and day 21), mice were injected intramuscularly with the S-trimer twice. The effect of adjuvants on humoral immunogenicity is significant because the S-trimer binding antibody titer, ACE2 competitive titer, and neutralizing antibody titer of the adjuvanted group are significantly higher than those of the unadjuvanted vaccine at a corresponding antigen dose level. S-trimers with different adjuvants elicit ACE2-competitive antibody titers and pseudovirus neutralizing antibody titers at levels similar to or higher than those observed in human convalescent serum samples. Similar results were observed in rats immunized with S-trimers. PCT/CN2021/087066, PCT/CN2021/093895, and PCT/CN2021/099285 are incorporated by reference in their entireties for all purposes.

Cell-mediated immunity (CMI) specific to S-trimeric antigens was investigated by collecting spleen cells from sacrificed immunized mice, then stimulating them with S-trimeric antigens and detecting Th1 (IL-2 and IFN$\gamma$) and Th2 (IL-4 and IL-5) cytokines by ELISpot. The adjuvanted group appeared to induce a stronger overall CMI response than the unadjuvanted S-trimer. A Th1-preferred cell-mediated immune response was observed in the unadjuvanted group and some adjuvanted S-trimer groups, while a mixed Th1-Th2 distribution was observed in other adjuvanted groups. CMI does not seem to be dependent on the dose of antigen.

## Example 3: Immunogenicity of vaccine against Omicron strain-based SARS-CoV-2

### Method

### Construction and production of pseudoviruses

The spike (S) protein gene of SARS-CoV-2 original strain and variant of concern was optimized using mammalian codons, synthesized by Genscript, and then cloned into a pcDNA3.1(+) eukaryotic expression vector. Plasmids encoding the S-glycoprotein of Hu-1, Beta ($\beta$), delta ($\delta$), Omicron (o) BA.1, BA.2, BA.2.75, BA.5, BQ.1.1, BF.7, XBB, XBB.1.5, BQ.1.1, XBB.1.16.1, and EG.5.1 SARS-CoV-2 variants were constructed. Specifically, total 18 amino acids at the C-terminus were deleted from a full-length sequence of the S gene and the sequence was constructed on a pcDNA3.1(+) vector through Hind III and Xba I sites; the reporter gene is pLVX-AcGFP-N1-Fluc from the honorgene; the lentiviral packaging plasmid is psPAX2 from honorgene; Thermo Lipo3000 was used for lentivirus packaging, the above three plasmids were transfected into 293T cells at a mass ratio of 5:3:4 for virus packaging, and the virus supernatant was harvested at 46 h $\pm$ 2 h. After the virus titer was determined, a pseudovirus detection experiment was carried out.

The lentiviral packaging plasmid psPAX2 and the pLVX-AcGFP-N1-Fluc lentivirus reporter plasmid expressing GFP and luciferase were from HonorGene, China. Pseudoviruses were produced by co-transfecting HEK 293T cells with psPAX2, pLVX-AcGFP-N1-Fluc and plasmids encoding various S genes using Lipofectamine 3000 (Invitrogen, L3000-015). The supernatant was harvested at 24 h $\pm$ 2 h after transfection, centrifuged at 1,500 rpm for 5 min to remove cell debris, and then stored at -80°C. The pseudovirus reservoir was titrated by infecting 293T-ACE2 cells, and the luciferase activity was measured using a microplate reader (TECAN, Spark) after an incubation period of 44 h to 48 h at 37°C and 5% $CO_2$ by adding a Bright-Glo luciferase detection system (Promega, E2650). Then according to the Reed-Muench method (Quantification of SARS-CoV-2 neutralizing antibody by a pseudotyped virus based assay. Nie J. et al. DOI: 10.21203/rs.3.pex-941/v11), the $TCID_{50}$ of pseudo viruses was calculated.

**Neutralization assay**

Aliquots of serum samples for the assay were first heat inactivated at 56°C for 30 min and then centrifuged at 10,000 rcf for 5 min for clarification. The samples were serially diluted (3-fold) with a test medium (100 ml) and incubated with 650 $TCID_{50}$ pseudovirus (50 ml, 50 $\mu$L/well) at 37°C for 1 h while using virus-infected untreated controls (individual viruses) and separate cells (background control). Fresh trypsinized 293T-ACE2 cells were then added to each well at 100 mL of 20,000 cells/well (100 $\mu$L/well). After incubation at 37°C for 44 h to 48 h in a 5% $CO_2$ incubator, the cells were lysed and the luciferase activity was measured by the Bright-Glo Luciferase Assay System (Promega) according to the manufacturer's protocol. The $IC_{50}$ neutralizing antibody titer of a given serum sample was defined as the serum dilution, where the sample showed a 50% reduction in relative light units (RLU) compared to a virus-infected control well. The detailed method was reported based on Quantification of SARS-CoV-2 neutralizing antibody by a pseudotyped virus based assay. Nie J. et al. DOI: 10.21203/rs.3.pex-941/v11.

**Expression and purification of variants of concern (VOC) S- trimeric fusion protein**

Gene synthesis of cDNA encoding the extracellular domain of SARS-CoV-2 spike (S) protein from Beta, Delta, Omicron, BA.5, and XBB.1.5 by GenScript using codons preferred by *Cricetulus griseus*. The cDNA was subcloned into the pTRIMER expression vector (GenHunter) at *Hind III* and *Bgl II* sites to allow soluble S protein to be fused within the frame to a protein trimerization™ tag (amino acid residues 1156-1406 from human type I($\alpha$) collagen), as previously described. The expression vector was transiently transfected into the HEK-293F cell line (Clover Biopharma) using PEI (Polyscience) and grown in OPM-293 CD05 medium (OPM) and OPM-293 proFeed supplement (OPM). The S-trimeric protein was purified from a conditioned medium to homogeneity using a protein trimerization™ tag specific affinity column (Clover Biopharma).

**Pseudovirus neutralizing antibody $IC_{50}$ titer data processing**

Pseudovirus test data processing ($IC_{50}$ calculation): 50% inhibition dilution ($IC_{50}$) means: after deducting the blank control RLU value, the serum sample RLU value is the dilution factor corresponding to a 50% decrease in the RLU value of virus wells (cells infected with individual viruses). GraphPad Prism, software, was used for four-parameter fitting and $IC_{50}$ calculation.

$$\text{Inhibition ratio (\%)}=100 \times \frac{(\text{Mean RLU of viral wells} - \text{Mean RLU of blank wells}) - (\text{Mean RLU of sample wells} - \text{Mean RLU of blank wells})}{(\text{Mean RLU of viral wells} - \text{Mean RLU of blank wells})}$$

Interpretation of results: If the inhibition rate of the first well of the sample is $\leq$ 50%, the titer result is judged as negative, and the dilution factor of the first well is used as the titer value; if the inhibition rate of the first well of the assay samples is higher than 50%, the four-parameter fitting result is used as the $IC_{50}$ titer.

Acceptance criteria: The inhibition rate of the first well of negative control should be < 50%, and the inhibition rate of the first well of positive control SCB-719 should be $\geq$ 90%.

Statistical analysis and software: In this study, R language (version 4.2.1) and SAS (version 9) were used for statistical analysis. A non-inferiority test was carried out on the immunogenicity titers of trivalent vaccine with monovalent and bivalent vaccines respectively. The lower limit of one-sided 90% confidence interval of GMT ratio between groups > 0.5 was taken as the cutoff value of the non-inferiority test. The major computerized systems used in this study include (but are not limited to):

Table 1. Data processing software

| Software name | Version No. | Purpose |
| --- | --- | --- |
| R | 4.2.1 | Statistical analysis |

(continued)

| Software name | Version No. | Purpose |
|---|---|---|
| SAS | 9 | Statistical analysis |
| Microsoft Excel | O365 | Data entry |
| GraphPad Prism | 9.0 | Data processing |

**Information on test articles**

Table 2 lists the composition of the test samples and the corresponding animal groups.

Table 2 Information on test articles and corresponding animal groups (homologous booster)

| Animal group No. | Antigen composition of vaccines | | Weight ratio of antigens contained in a multivalent vaccine | Antigen dose contained in the vaccine (+ CpG 1018: 150 μg, Alum: 75 μg) |
|---|---|---|---|---|
| 1 | Monovalent vaccine | Hu-1 | / | 3.0 μg/antigen |
| 2 | | BA.5 | / | |
| 3 | | XBB.1.5 | / | |
| 4 | Bivalent vaccine | Hu-1 + BA.5 | 1:1 | 1.5 μg + 1.5 μg Or 1 μg + 2 μg/antigen |
| 5 | | Hu-1 + BA.5 | 1:2 | |
| 6 | | Hu-1 + XBB.1.5 | 1:1 | |
| 7 | | Hu-1 +XBB.1.5 | 1:2 | |
| 8 | | BA.5 + XBB.1.5 | 1:1 | |
| 9 | | BA.5 + XBB.1.5 | 1:2 | |
| 10 | Trivalent vaccine | Hu-1 + BA.5+ XBB.1.5 | 1:1:1 | 1.0 μg/antigen |

**Example 4: Preparation and formulation of vaccines**

The antigen bulk and CpG1018 adjuvant DS were thawed separately, then the antigen bulk, CpG1018 adjuvant intermediate and aluminum adjuvant were mixed properly to prepare a semi-finished product, which was then filled, stoppered, capped, visually inspected, and warehoused.

Table 3. Composition list of preparation prescription

| Component | Content | Manufacturer | Function |
|---|---|---|---|
| Hu-1 S-trimer (SCB-2019) | 0.012 mg/ml | In-house | Active drug substance (raw material) |
| BA.5 S-trimer | 0.024 mg/ml | In-house | Active drug substance (raw material) |
| XBB.1.5 S-trimer | 0.024 mg/ml | In-house | Active drug substance (raw material) |
| Alum | 1.500 mg/ml | CRODA | Adjuvant |
| CpG1018 | 3.000 mg/ml | Dynavax | Adjuvant |
| Sodium dihydrogen phosphate monohydrate | 0.052 mg/ml | Merck | Buffer |
| Disodium hydrogen phosphate dihydrate | 0.052 mg/ml | Merck | Buffer |
| Sodium chloride | 6.860 mg/ml | Merck | Stabilizer/Osmolarity regulator |
| Polysorbate 80 | 0.025 mg/ml | Nanjing Well | Stabilizer |
| Tromethamine | 0.605 mg/ml | Merck | Buffer |
| Hydrochloric acid (37%) | 0.406 mg/ml | Merck | Buffer |

(continued)

| Component | Content | Manufacturer | Function |
|---|---|---|---|
| WFI | Q.S. | In-house | Solvent |

## Example 5: Clinical trial

Investigational vaccine: trivalent vaccine: including 30 $\mu$g of antigen (including 12 $\mu$g of Omicron BA.5, 12 $\mu$g of Omicron XBB1.5, and 6 $\mu$g of Hu-1 wild strain Hu-1) and CpG 1018/alum adjuvant system (1.5 mg of CpG 1018 adjuvant and 0.75 mg of aluminum hydroxide) in a 0.5 mL dose.

Control vaccine: Hu-1 vaccine (monovalent): including 30 $\mu$g oh Hu-1 antigen (wild strain) and CpG 1018/alum adjuvant system (1.5 mg of CpG 1018 adjuvant and 0.75 mg of aluminum hydroxide) in a 0.5 mL dose.

Clinical trial design of trivalent vaccines: The test subjects had received three doses of Hu-1 S-trimer vaccine at an interval greater than or equal to 6 months (randomization 2:1, N=600). Screening and vaccination were performed on D1 for eligible test subjects. Blood tests were taken on D15, followed by follow-up calls on D29 and D90 respectively, blood tests were taken again on D180, and finally the last telephone follow-up was conducted on D180.

Route of administration: intramuscular (i.m.).

## Results

## Results of pseudovirus neutralizing antibody titers

A monovalent vaccine based on subtype BA.5 and XBB.1.5 protein trimerization™ tag subunits of Hu-1 and Omicron strains, and a bivalent or trivalent vaccine consisting of subtype BA.5 and XBB.1.5 antigens selected from Hu-1 and Omicron strains were constructed and produced, and given to mice as a primary series and/or booster vaccine for homologous boosting, or used in combination with Hu-1 S-trimer for heterologous boosting.

## Homologous boosting

D35 pseudovirus neutralizing antibody titer results in homologous boosting: FIG. 2 shows the $IC_{50}$ data of pseudovirus neutralizing antibodies against Hu-1, Beta, Delta, and Omicron subtypes BA.1, BA.2, BA.2.75, BA.5, BQ.1.1, BF.7, XBB, XBB.1.5 strains in Naive (naive) mice immunized with two doses of S-trimeric antigen (first dose primary vaccination-second dose booster) according to FIG. 1 and Table 2. FIG. 2 shows $IC_{50}$ data of pseudovirus neutralizing antibodies against SARS-CoV-2 Hu-1, Beta ($\beta$, B.1.351), Delta ($\delta$, B.1.617.2), and subtypes BA.1, BA.2, BA.2.75, BA.5, BQ.1.1, BF.7, XBB, and XBB.1.5 of Omicron (o, B.1.1.529) in mice immunized with two doses of S-trimeric antigen (first dose for priming-second dose for boosting) according to the test design of FIG. 1 and the vaccine doses in Table 2. After two immunizations with 150 $\mu$g of CpG 1018 plus 75 $\mu$g of Alum as an adjuvant, blood was collected from mice on D35 (D4 PD2) for pseudovirus neutralizing antibody testing. A. $IC_{50}$ data of pseudovirus neutralizing antibodies against Hu-1 strain; B. $IC_{50}$ data of pseudovirus neutralizing antibodies against Beta strain; C. $IC_{50}$ data of pseudovirus neutralizing antibodies against Delta strain; D. $IC_{50}$ data of pseudovirus neutralizing antibodies against BA.1 strain; E. $IC_{50}$ data of pseudovirus neutralizing antibodies against BA.2 strain; F. $IC_{50}$ data of pseudovirus neutralizing antibodies against BA.2.75 strain; G. $IC_{50}$ data of pseudovirus neutralizing antibodies against BA.5 strain; H. $IC_{50}$ data of pseudovirus neutralizing antibodies against BQ.1.1 strain; I. $IC_{50}$ data of pseudovirus neutralizing antibodies against BF.7 strain; J. $IC_{50}$ data of pseudovirus neutralizing antibodies against XBB strain; and K. $IC_{50}$ data of pseudovirus neutralizing antibodies against XBB.1.5 strain.

In order to further understand the advantages of trivalent vaccines over monovalent and bivalent vaccines, this article drew a spider web diagram based on the neutralizing antibody titer levels of 11 pseudoviruses corresponding to mice in each immunization group. From the data of "trivalent" vs. "monovalent" (C in FIG. 3), it can be observed that trivalent vaccines have the highest broad spectrum; while Hu-1 (Prototype) S-trimer and XBB.1.5 S-trimer monovalent vaccines have the highest neutralizing titer against their own strains of pseudoviruses, but are less prominent than trivalent vaccines for broad spectrum types other than their respective branches.

Statistically, the non-inferiority test for the geometric mean titer (GMT) ratio of neutralizing antibody titers corresponding to 11 pseudoviruses between trivalent vaccines and monovalent vaccines including Hu-1 S-trimer, BA.5 S-trimer and XBB.1.5 S-trimer was analyzed in this article, respectively (set threshold: the lower limit of 90% CI (LowCI) corresponding to the GMT ratio was >0.5, which is comparable). It was found that for the early Hu-1, Beta, and Delta strains of SARS-CoV-2, except for the Hu-1 S-trimer monovalent vaccine, the trivalent vaccine was not inferior to the BA.5 S-trimer and XBB.1.5 S-trimer monovalent vaccines; for eight Omicron series branch strains, although the monovalent vaccine XBB.1.5 S-trimer showed advantages against XBB and XBB.1.5, and BA.5 S-trimer showed advantages against BA.1 and BQ.1.1, compared with the monovalent vaccines, six of the eight Omicron series strains were not inferior to the monovalent vaccine, indicating that the trivalent vaccine has a better broad spectrum among the Omicron series strains. Specific non-inferiority test results are shown in Table 4 and C of FIG. 3.

Table 4. Non-inferiority test results of "trivalent vaccine" vs. "monovalent vaccine"

| Category | Early SARS-CoV-2 strains | | | Omicron series branch strains | |
|---|---|---|---|---|---|
| Strain | Hu-1 | Beta | Delta | BA.5 | XBB.1.5 |
| Trivalent: Hu-GMT ratio (90% CI) | 0.3 (0.19, 0.66) | 0.5 (0.22, 1.19) | 0.4 (0.18, 0.89) | 224.5 (111.92, 450.53)** | 267.4 (193.23, 369.98)** |
| Trivalent: BA.5 | 6.9 (3.01, 15.64)** | 7.9 (3.70, 16.76)** | 10.1 (4.93, 20.72)** | 0.8 (0.55, 1.13) * | 5.6 (3.13, 9.96)** |
| Trivalent: XBB.1.5 | 25.6 (12.07, 54.48) ** | 10.5 (5.59, 19.73)** | 20.5 (11.14, 37.87)** | 3.8 (2.31, 6.21) ** | 0.4 (0.25, 0.58) |

Table 4 (continued). Non-inferiority test results of "trivalent vaccine" vs. "monovalent vaccine"

| Category | Omicron series branch strains | | | | | |
|---|---|---|---|---|---|---|
| Strain | XBB | BA.1 | BA.2 | BA.2.75 | BF.7 | BQ.1.1 |
| Trivalent: Hu-GMT ratio (90% CI) | 149.3 (88.60, 251.51)** | 4.8 (1.89, 11.98)** | 55.2 (25.04, 121.52)** | 25.5 (13.40, 48.33)** | 387.1 (202.03, 741.80)** | 335.4 (188.78, 595.92)** |
| Trivalent: BA.5 | 2.4 (1.07, 5.27)** | 1.1 (0.37, 3.06) | 1.2 (0.66, 2.19)* | 1.7 (1.05, 2.86)** | 0.9 (0.63, 1.34)* | 0.7 (0.47, 1.16) |
| Trivalent: XBB.1.5 | 0.7 (0.35, 1.27) | 5.1 (2.10, 12.32)** | 6.7 (3.62, 12.41)** | 2.9 (1.63, 5.02)** | 5.1 (2.92, 9.06)** | 4.4 (2.63, 7.52)** |
| Note: **: superior; *: non-inferior; others: ratio > 0.5/L CI < 0.5. | | | | | | |

As shown in A and B in the spider web diagram FIG. 3 of "trivalent" vs. "bivalent", when the bivalent antigen ratio is 1:1 or 1:2, the comprehensive data of 11 pseudoviruses show that the trivalent vaccine has a higher broad spectrum.

Statistically, the GMT ratio of neutralizing antibody titers corresponding to 11 pseudoviruses in mice in each immunization group of "trivalent" and "bivalent" was tested for non-inferiority (set threshold: the lower limit of 90% CI (LowCI) corresponding to the GMT ratio was >0.5, which is comparable). In the exploration of bivalent vaccine antigen ratios, the three combinations of Hu-1+BA.5, Hu-1+XBB.1.5, and BA.5+XBB.1.5 showed advantages against the early SARS-CoV-2 Hu-1/Beta/Delta strains when the bivalent antigen ratio was 1:1 (A in FIG. 3); when the bivalent antigen ratio was 1:2, none of the three "bivalent" antigen combinations showed a significant advantage (B in FIG. 3). For specific data, see Table 5 for statistics on non-inferiority results of "trivalent" vs. "bivalent" against 11 pseudovirus strains.

Table 5. Non-inferiority test results of "trivalent vaccine" vs. "bivalent vaccine (1:1 or 1:2)"

| Category | Early SARS-CoV-2 strains | | | Omicron series branch strains | | | | | | | |
|---|---|---|---|---|---|---|---|---|---|---|---|
| Strain | Hu-1 | Beta | Delta | BA.5 | XBB.1.5 | XBB | BA.1 | BA.2 | BA.2.75 | BF.7 | BQ.1.1 |
| Geometric mean titer ratio (90% CI) trivalent/bivalent (1:1) | 4.8 (1.94, 12.12) ** | 3.7 (1.75, 7.75) ** | 4.1 (1.69, 10.03) ** | 0.9 (0.68, 1.23)* | 0.9 (0.55, 1.33)* | 0.7 (0.40, 1.38) | 0.8 (0.28, 2.05) | 1.1 (0.68, 1.80)* | 0.8 (0.50, 1.38)* | 0.9 (0.68, 1.26)* | 1.5 (1.00, 2.17)* |
| Geometric mean titer ratio (90% CI) Trivalent/bivalent (1:2) | 10.8 (4.98, 23.50)** | 3.3 (1.37, 7.86)** | 9.2 (4.53, 18.73)** | 0.9 (0.68, 1.27)* | 0.6 (0.40, 0.99) | 0.6 (0.34, 1.14) | 0.7 (0.23, 1.81) | 0.7 (0.43, 1.12) | 0.7 (0.45, 1.16) | 0.9 (0.66, 1.25)* | 1.4 (0. 93, 1.99)* |
| Note: **: superior; *: non-inferior; others: ratio > 0.5/L CI < 0.5. | | | | | | | | | | | |

In this test (FIGS. 5 and 6), serum samples were collected on D14 and D28 after the third and fourth immunizations (D156: D0 of the third immunization; D170: D14 after the third immunization; D184: D28 after the third immunization and D0 of the fourth immunization; D198: D14 after the fourth immunization; D212: D28 after the fourth immunization), and the neutralizing antibody titer of SARS-CoV-2 pseudoviruses (Hu-1, Beta, Delta, Omicron subtype BA.1, BA.2, BA.2.75, BA.5, BQ.1.1, BF.7, XBB, XBB.1.5) in mouse serum was detected by pseudovirus experiments.

In order to further compare the advantages and disadvantages of three heterologous boosters after the HU-1 S-trimer being taken as primary immunization, namely, one trivalent vaccine and two bivalent vaccines vs. monovalent vaccine as booster, we comprehensively drew a spider web diagram (FIG. 7) based on the neutralizing antibody titer levels of 11 pseudoviruses corresponding to mice in each immunization group. It can be observed from the figure that both trivalent and bivalent vaccines have higher broad spectrum than monovalent vaccines. Among them, the trivalent vaccine and the bivalent vaccine (BA.5+XBB.1.5) have similar and highest broad spectrum types, followed by the bivalent vaccine (Hu-1+XBB.1.5). When the monovalent vaccine is used as a booster, the neutralizing antibody titer level against the Omicron series is relatively weak. Statistically, a non-inferiority test was performed in terms of the GMT ratios of neutralizing antibody titers of 11 pseudoviruses corresponding to a broad-spectrum heterologous booster, namely, trivalent vaccine, vs. bivalent vaccine and monovalent vaccine (set threshold: the lower limit of 90% CI (LowCI) corresponding to the GMT ratio was >0.5, which is comparable) in this article. The results showed that compared with the neutralizing antibody levels of the monovalent vaccine and bivalent vaccine (Hu-1+XBB.1.5), the statistical analysis result of the trivalent vaccine was "non-inferior" in more than half of pseudovirus strains, indicating that the trivalent vaccine was superior to both monovalent vaccine and bivalent vaccine (Hu-1+XBB.1.5); compared with the neutralizing antibody level of the bivalent vaccine (BA.5+XBB.1.5), although the statistical analysis results of eight strains showed "inferior", it was observed that their GMT ratios were all greater than 0.5, and some GMT ratios were greater than 1, resulting in an "inferior" result, which may be caused by too few samples in each group.

**Pre-existing heterologous booster immunization**

Animal grouping and dose design: This study is divided into two parts, namely early pre-existing immunization and late booster immunization. The early pre-existing immunization protocol is shown in Table 6 below:

Table 6. Design of pre-existing immunization experiment & dosing regimen

| Sample information | 1st immunization (D0) & 2nd immunization (D7) | | | Dosing volume | Animal ID |
|---|---|---|---|---|---|
| | Antigen | Aluminum agent | CpG 1018 | | |
| Hu-1 S-trimer monovalent vaccine | 3 μg/dose | 75 μg/dose | 150 μg/dose | 0.05 mL/animal in total | All mice |

During the booster immunization, a total of 80 female BALB/c mice were selected and randomly divided into 4 groups according to body weight, with 20 mice in each group. The experiment design and dosing regimen are shown in Table 7.

Table 7. Design of booster immunization experiment & dosing regimen

| Group | 3rd immunization (D107) | | | Dosing volume | Animal ID |
|---|---|---|---|---|---|
| | Antigen | Aluminum agent | CpG 1018 | | |
| Group 1: SCB-2019 monovalent vaccines, 2 vial | 3 μg/dose | 75 μg/dose | 150 μg/dose | 0.05 mL/animal in total | 1F01-1F20 |
| Group 2: XBB.1.5 S-trimer monovalent vaccines, Changxing, 1 vial | 3 μg/dose | 75 μg/dose | 150 μg/dose | 0.05 mL/animal in total | 2F01-2F20 |
| Group 3: S-trimer trivalent vaccines (Hu-1+BA.5+XBB.1.5, 1:2:2), 1 vial | 3 μg/dose | 75 μg/dose | 150 μg/dose | 0.05 mL/animal in total | 3F01-3F20 |

(continued)

| Group | 3rd immunization (D107) | | | Dosing volume | Animal ID |
|---|---|---|---|---|---|
| | Antigen | Aluminum agent | CpG 1018 | | |
| Group 4: XBB.1.5 S-trimer monovalent vaccines, 2/5 doses, Changxing, 1 vial | 1.2 μg/dose | 30 μg/dose | 60 μg/dose | 0.05 mL/animal in total | 4F01-4F20 |
| Note: "1 vial" means that the antigen, aluminum agent and CpG1018 are in a same bottle; and "2 vials" means that the antigen and aluminum agent are in a same bottle. | | | | | |

In this experiment, mouse serum samples were collected 1 day before booster immunization (D107), 14 days after booster immunization (D122), and 28 days after booster immunization (D136). The neutralizing antibody titer of SARS-CoV-2 pseudoviruses (Hu-1/Beta/Delta/BA.1/BA.2/BA.2.75/BA.5/BF.7/BQ.1.1/XBB/XBB.1.5/XBB.1.16.1/EG.5.1) in mouse serum was detected by pseudovirus experiments (FIG. 9).

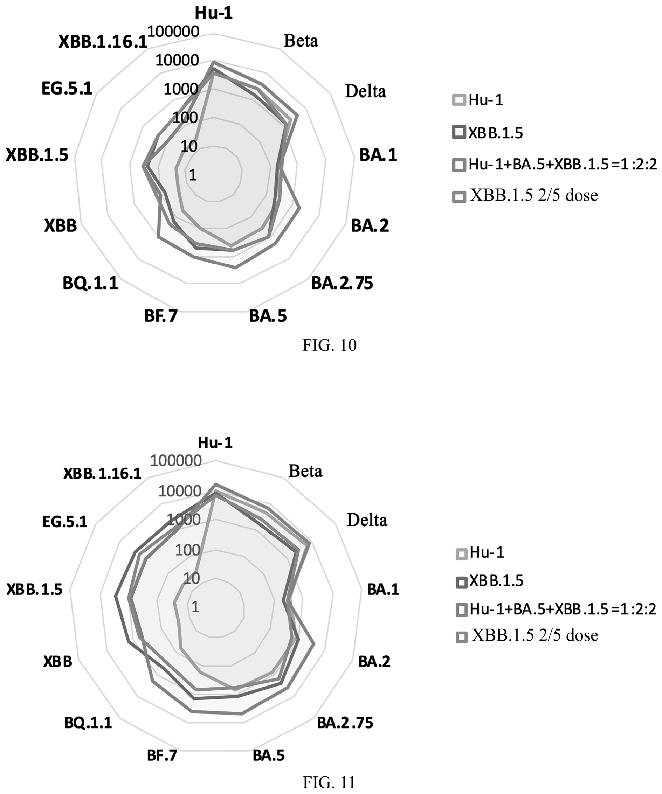

In order to further compare the advantages and disadvantages of two heterologous boosters after two doses of HU-1 S-trimer as primary immunization, namely, Group 2 monovalent vaccine XBB.1.5 S-trimer and Group 3 S-trimer trivalent vaccine (1:2:2) vs. Group 1 first-generation SARS-CoV-2 monovalent vaccine HU-1 S-trimer as booster. We combined the geometric mean titer (GMT) levels of neutralizing antibodies against 12 pseudoviruses in mice in each immunization group to draw a spider web diagram (FIG. 10 and FIG. 11). From the figure, we observed that both the s-trimer trivalent vaccine (1:2:2) and the XBB.1.5 S-trimer monovalent vaccine show a higher broad spectrum than the first generation of monovalent vaccine HU-1 S-trimer. Among them, the advantage of the S-trimer trivalent vaccine (1:2:2) is that it has the highest broad spectrum among the 13 pseudovirus neutralizing antibodies detected in the observed D122 and D136 serum. When the XBB.1.5 S-trimer monovalent vaccine was used as a booster, the neutralizing antibody titers against all pseudoviruses were significantly improved, especially showing higher protective efficacy against XBB-related branches.

In addition, similar titer levels were observed between the two groups on D14 of booster immunization in Group 2: XBB.1.5 S-trimer full dose group (3 μg XBB.1.5) and Group 4: XBB.1.5 S-trimer 2/5 dose group (equal dose to XBB.1.5 antigen in trivalent vaccine, namely, 1.2 μg XBB.1.5) (FIG. 10). However, on D28 of booster immunization, Group 2: XBB.1.5 S-trimer full dose group showed an advantage in neutralizing antibody levels against eight pseudoviruses from BA.2 to XBB.1.16.1, and we also observed that the antibody levels of neutralizing XBB series strains produced by Group 4 using the same dose as the XBB.1.5 antigen in the trivalent vaccine were very similar to those of the trivalent vaccine (FIG. 11). However, it did not show an advantage in strains other than the XBB series, indicating that the dose of the trivalent vaccine was positively correlated with the antibodies it produced. The additional two antigens, Hu-1 S-trimer and BA.5 S-trimer, played a beneficial role in neutralizing strains other than XBB.

Statistical analysis of antibody titers after a heterologous booster injection: Statistically, we conducted a non-inferiority test on a GMT ratio of the monovalent vaccine XBB.1.5 S-trimer to the first generation monovalent vaccine HU-1 S-trimer, S-trimer trivalent vaccine (1:2:2) and 2/5 doses of XBB.1.5 S-trimer corresponding to neutralizing antibody titers of 13 pseudoviruses (set non-inferiority standards: the lower limit of 90% CI (LowCI>0.5) corresponding to the GMT ratio). The results of the D136 non-inferiority test of pseudovirus neutralizing antibody titers (Table 8) showed that, compared with the neutralizing antibody level of the first generation monovalent vaccine HU-1 S-trimer, the monovalent vaccine XBB.1.5 S-trimer showed "non-inferior" results for all the other strains except the results of Pre-Omicron related strains Beta and Delta and BA.1 (inferior), and showed a very high advantage for the GMT ratio of XBB-related strains. In the comparison between monovalent vaccine XBB.1.5 S-trimer and trivalent vaccine XBB.1.5 S-trimer, neutralizing antibodies against XBB-related strains were shown to be "non-inferior", with GMT ratios greater than 2, indicating that XBB.1.5 S-trimer monovalent vaccine mainly showed superiority in neutralizing antibody levels against XBB-related evolutionary strains.

Table 8. Results of non-inferiority test of pseudovirus neutralizing antibody titers on D136

| Category | Pre-Omicron strains | | | Omicron series branch strains | | | | | | | | |
|---|---|---|---|---|---|---|---|---|---|---|---|---|
| Variant | Hu-1 | Beta | Delta | BA.1 | BA.2 | BA.2.75 | BA.5 | BF.7 | BQ.1.1 | XBB | XBB.1.5 | XBB.1.16.1 |
| XBB.1.5/Hu-1 | 0.81* (0.523,1.266) | 0.41 (0.201,0.821) | 0.36 0.157,0.819 | 0.65 (0.205,2.049) | 1.27* (0.555,2.865) | 2.78** 1.188,6.452) | 1.89* (0.777,4.545) | 9.09** (3.876,22.222 | 7.69** ) (3.717,16.393) | 100** (40,11.11) | 100** 76.92,16.67) | 100** (62.5, 142.86) |
| XBB.1.5/ Trivalent 1:2:2 | 0.51 (0.323,0.796) | 0.24 (0.119, 0.483) | 0.27 (0.119, 0.626) | 0.78 (0.245, 2.458) | 0.3 (0.134, 0.671) | 0.54 (0.253, 1.134) | 0.28 (0.132, 0.606) | 0.4 (0.202, 0.775) | 0.27 (0.134, 0.555) | 2.93** (1.366,6.293) | 3.15** (1.785,5.547) | 2.61** (1.433, 4.74) |
| XBB.1.5/ 2/5(XBB.1.5) | 1.05* (0.637,1.719) | 0.67 (0.291,1.558) | 0.83 (0.319,2.145) (0.246,2.327) | 0.76 | 1.88* (0.827,4.276) | 1.42* (0.67,3.024) | 2.12* (0.898,5.025) | 2,2** (1.062,4.573) | 1.75* (0.847,3.632) | 2.54** (1.162,5.551) | 2.79** (1.672,4,667) | 1.88** (1.092,3.226) |

Note: **: superior; *: non-inferior; others: ratio > 0.5/LCI < 0.5 or ratio < 0.5/LCI < 0.5.

In conclusion, XBB.1.5 S-trimer monovalent vaccine and trivalent vaccine as heterologous boosters showed high broad spectrum in the study of pre-existing immunization mouse models with both XBB.1.5 S-trimer monovalent vaccine and SCB-2023 trivalent vaccine. And XBB.1.5 S-trimer as a booster can effectively increase the neutralizing antibody level against Pre-omicron and BA.5-related strains, and has a high improvement in the neutralizing antibody level against XBB-related strains, especially for the currently prevalent XBB-related evolutionary strains.

The present disclosure is not intended to limit its scope to specific disclosed embodiments, which are provided, for example, to illustrate various aspects of the present disclosure. Various modifications to the composition and method will become apparent through the description and teaching of this article. These changes can be implemented without departing from the true scope and spirit of the present disclosure, and these changes are intended to fall within the scope of the present disclosure.

**Sequence**

| SEQ ID NO. | Sequence | Description |
|---|---|---|
| 1 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQDVNCTEVPVAIHADQLTPTWR VYSTGSNVFQTRAGCLIGAEHVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | Hu-1 SARS-CoV-2 S-trimer fusion polypeptide without signal peptide, 1509 aa |

| SEQ ID NO. | Sequence | Description |
|---|---|---|
| 2 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQDVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | Hu-1 SARS-CoV-2 S-trimer fusion polypeptide without signal peptide, 1509 aa, S1/S2 furin cleavage site 1 (685R→685 A) |

| SEQ ID NO. | Sequence | Description |
|---|---|---|
| 3 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQDVNCTEVPVAIHADQLTPTWR VYSTGSNVFQTRAGCLIGAEHVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | Hu-1 SARS-CoV-2 S-trimer fusion polypeptide without signal peptide, 1509 aa, proline mutant (986K/987V →986P/987 P) |

| SEQ ID NO. | Sequence | Description |
|---|---|---|
| 4 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQDVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | Hu-1 SARS-CoV-2 S-trimer fusion polypeptide without signal peptide, 1509 aa, S1/S2 furin cleavage site 1 and proline mutant (685R→685 A, 986K/987V →986P/987 P) |
| 5 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCRSNGLPGPIGPPGPRGRTGDAGPVGPP GPPGPPGPPGPPSAGFDFSFLPQPPQEKAHDGGRYYRANDANVVRDRDLEVDTTLKSLSQQIENIRSPE GSRKNPARTCRDLKMCHSDWKSGEYWIDPNQGCNLDAIKVFCNMETGETCVYPTQPSVAQKNWYISKNP KDKRHVWFGESMTDGFQFEYGGQGSDPADVAIQLTFLRLMSTEASQNITYHCKNSVAYMDQQTGNLKKA LLLQGSNEIEIRAEGNSRFTYSVTVDGCTSHTGAWGKTVIEYKTTKTSRLPIIDVAPLDVGAPDQEFGF DVGPVCFL | Hu-1 SARS-CoV-2 spike NTD/RBD-trimeric fusion protein without fusion peptide, 836 aa |

EP 4 722 230 A1

66

| SEQ ID NO. | Sequence | Description |
|---|---|---|
| 6 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQDVNCTEVPVAIHADQLTPTWR VYSTGSNVFQTRAGCLIGAEHVNNSYECDIPIGAGICASYQTQTNSPRSNGLPGPIGPPGPRGRTGDAG PVGPPGPPGPPGPPGPPSAGFDFSFLPQPPQEKAHDGGRYYRANDANVVRDRDLEVDTTLKSLSQQIEN IRSPEGSRKNPARTCRDLKMCHSDWKSGEYWIDPNQGCNLDAIKVFCNMETGETCVYPTQPSVAQKNWY ISKNPKDKRHVWFGESMTDGFQFEYGGQGSDPADVAIQLTFLRLMSTEASQNITYHCKNSVAYMDQQTG NLKKALLLQGSNEIEIRAEGNSRFTYSVTVDGCTSHTGAWGKTVIEYKTTKTSRLPIIDVAPLDVGAPD QEFGFDVGPVCFL | Hu-1 SARS-CoV-2 spike S I-trimer fusion polypeptide without signal peptide, 979 aa |
| 7 | SVASQSIIAYTMSLGAENSVAYSNNSIAIPTNFTISVTTEILPVSMTKTSVDCTMYICGDSTECSNLLL QYGSFCTQLNRALTGIAVEQDKNTQEVFAQVKQIYKTPPIKDFGGFNFSQILPDPSKPSKRSFIEDLLF NKVTLADAGFIKQYGDCLGDIAARDLICAQKFNGLTVLPPLLTDEMIAQYTSALLAGTITSGWTFGAGA ALQIPFAMQMAYRFNGIGVTQNVLYENQKLIANQFNSAIGKIQDSLSSTASALGKLQDVVNQNAQALNT LVKQLSSNFGAISSVLNDILSRLDKVEAEVQIDRLITGRLQSLQTYVTQQLIRAAEIRASANLAATKMS ECVLGQSKRVDFCGKGYHLMSFPQSAPHGVVFLHVTYVPAQEKNFTTAPAICHDGKAHFPREGVFVSNG THWFVTQRNFYEPQIITTDNTFVSGNCDVVIGIVNNTVYDPLQPELDSFKEELDKYFKNHTSPDVDLGD ISGINASVVNIQKEIDRLNEVAKNLNESLIDLQELGKYEQYIKRSNGLPGPIGPPGPRGRTGDAGPVGP PGPPGPPGPPGPPSAGFDFSFLPQPPQEKAHDGGRYYRANDANVVRDRDLEVDTTLKSLSQQIENIRSP EGSRKNPARTCRDLKMCHSDWKSGEYWIDPNQGCNLDAIKVFCNMETGETCVYPTQPSVAQKNWYISKN PKDKRHVWFGESMTDGFQFEYGGQGSDPADVAIQLTFLRLMSTEASQNITYHCKNSVAYMDQQTGNLKK ALLLQGSNEIEIRAEGNSRFTYSVTVDGCTSHTGAWGKTVIEYKTTKTSRLPIIDVAPLDVGAPDQEFG FDVGPVCFL | Hu-1 SARS-CoV-2 spike S2-trimer fusion polypeptide, 837 aa (cleaved at site 1, S1/S2) |
| 8 | TMSLGAENSVAYSNNSIAIPTNFTISVTTEILPVSMTKTSVDCTMYICGDSTECSNLLLQYGSFCTQLN RALTGIAVEQDKNTQEVFAQVKQIYKTPPIKDFGGFNFSQILPDPSKPSKRSFIEDLLFNKVTLADAGF IKQYGDCLGDIAARDLICAQKFNGLTVLPPLLTDEMIAQYTSALLAGTITSGWTFGAGAALQIPFAMQM AYRFNGIGVTQNVLYENQKLIANQFNSAIGKIQDSLSSTASALGKLQDVVNQNAQALNTLVKQLSSNFG | Hu-1 SARS-CoV-2 spike S2-trimer fusion |

EP 4 722 230 A1

(continued)

| SEQ ID NO. | Sequence | Description |
|---|---|---|
|  | AISSVLNDILSRLDKVEAEVQIDRLITGRLQSLQTYVTQQLIRAAEIRASANLAATKMSECVLGQSKRV DFCGKGYHLMSFPQSAPHGVVFLHVTYVPAQEKNFTTAPAICHDGKAHFPREGVFVSNGTHWFVTQRNF YEPQIITTDNTFVSGNCDVVIGIVNNTVYDPLQPELDSFKEELDKYFKNHTSPDVDLGDISGINASVVN IQKEIDRLNEVAKNLNESLIDLQELGKYEQYIKRSNGLPGPIGPPGPRGRTGDAGPVGPPGPPGPPGPP GPPSAGFDFSFLPQPPQEKAHDGGRYYRANDANVVRDRDLEVDTTLKSLSQQIENIRSPEGSRKNPART CRDLKMCHSDWKSGEYWIDPNQGCNLDAIKVFCNMETGETCVYPTQPSVAQKNWYISKNPKDKRHVWFG ESMTDGFQFEYGGQGSDPADVAIQLTFLRLMSTEASQNITYHCKNSVAYMDQQTGNLKKALLLQGSNEI EIRAEGNSRFTYSVTVDGCTSHTGAWGKTVIEYKTTKTSRLPIIDVAPLDVGAPDQEFGFDVGPVCFL | polypeptide, 827 aa (cleaved at site 2, S1/S2) |
| 9 | SFIEDLLFNKVTLADAGFIKQYGDCLGDIAARDLICAQKFNGLTVLPPLLTDEMIAQYTSALLAGTITS GWTFGAGAALQIPFAMQMAYRFNGIGVTQNVLYENQKLIANQFNSAIGKIQDSLSSTASALGKLQDVVN QNAQALNTLVKQLSSNFGAISSVLNDILSRLDKVEAEVQIDRLITGRLQSLQTYVTQQLIRAAEIRASA NLAATKMSECVLGQSKRVDFCGKGYHLMSFPQSAPHGVVFLHVTYVPAQEKNFTTAPAICHDGKAHFPR EGVFVSNGTHWFVTQRNFYEPQIITTDNTFVSGNCDVVIGIVNNTVYDPLQPELDSFKEELDKYFKNHT SPDVDLGDISGINASVVNIQKEIDRLNEVAKNLNESLIDLQELGKYEQYIKRSNGLPGPIGPPGPRGRT GDAGPVGPPGPPGPPGPPGPPSAGFDFSFLPQPPQEKAHDGGRYYRANDANVVRDRDLEVDTTLKSLSQ QIENIRSPEGSRKNPARTCRDLKMCHSDWKSGEYWIDPNQGCNLDAIKVFCNMETGETCVYPTQPSVAQ KNWYISKNPKDKRHVWFGESMTDGFQFEYGGQGSDPADVAIQLTFLRLMSTEASQNITYHCKNSVAYMD QQTGNLKKALLLQGSNEIEIRAEGNSRFTYSVTVDGCTSHTGAWGKTVIEYKTTKTSRLPIIDVAPLDV GAPDQEFGFDVGPVCFL | Hu-1 SARS-CoV-2 spike S2-trimer fusion polypeptide, 707 aa (cleaved at S2') |

| SEQ ID NO. | Sequence | Description |
|---|---|---|
| 10 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGNIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | SARS-CoV-2 South African variant B.1.351 S-trimer fusion polypeptide without signal peptide, 1509 aa |

EP 4 722 230 A1

69

(continued)

| SEQ ID NO. | Sequence | Description |
|---|---|---|
| 11 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGNIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | SARS-CoV-2 South African variant B.1.351 S-trimer fusion polypeptide without signal peptide, 1509 aa, S1/S2 furin cleavage site 1 mutant (685R→685 A) |
| 12 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV | SARS-CoV-2 South African variant B.1.351 S- |

70

| SEQ ID NO. | Sequence | Description |
|---|---|---|
|  | DCALDPLSETKCTLKSFTVEKGIYQTSNFRVQPTESIVRFPNITNLCPFGEVFNATRFASVYAWNRKRI<br>SNCVADYSVLYNSASFSTFKCYGVSPTKLNDLCFTNVYADSFVIRGDEVRQIAPGQTGNIADYNYKLPD<br>DFTGCVIAWNSNNLDSKVGGNYNYLYRLFRKSNLKPFERDISTEIYQAGSTPCNGVKGFNCYFPLQSYG<br>FQPTYGVGYQPYRVVVLSFELLHAPATVCGPKKSTNLVKNKCVNFNFNGLTGTGVLTESNKKFLPFQQF<br>GRDIADTTDAVRDPQTLEILDITPCSFGGVSVITPGTNTSNQVAVLYQGVNCTEVPVAIHADQLTPTWR<br>VYSTGSNVFQTRAGCLIGAEHVNNSYECDIPIGAGICASYQTQTNSPRRARSVASQSIIAYTMSLGAEN<br>SVAYSNNSIAIPTNFTISVTTEILPVSMTKTSVDCTMYICGDSTECSNLLLQYGSFCTQLNRALTGIAV<br>EQDKNTQEVFAQVKQIYKTPPIKDFGGFNFSQILPDPSKPSKRSFIEDLLFNKVTLADAGFIKQYGDCL<br>GDIAARDLICAQKFNGLTVLPPLLTDEMIAQYTSALLAGTITSGWTFGAGAALQIPFAMQMAYRFNGIG<br>VTQNVLYENQKLIANQFNSAIGKIQDSLSSTASALGKLQDVVNQNAQALNTLVKQLSSNFGAISSVLND<br>ILSRLDPPEAEVQIDRLITGRLQSLQTYVTQQLIRAAEIRASANLAATKMSECVLGQSKRVDFCGKGYH<br>LMSFPQSAPHGVVFLHVTYVPAQEKNFTTAPAICHDGKAHFPREGVFVSNGTHWFVTQRNFYEPQIITT<br>DNTFVSGNCDVVIGIVNNTVYDPLQPELDSFKEELDKYFKNHTSPDVDLGDISGINASVVNIQKEIDRL<br>NEVAKNLNESLIDLQELGKYEQYIKRSNGLPGPIGPPGPRGRTGDAGPVGPPGPPGPPGPPGPPSAGFD<br>FSFLPQPPQEKAHDGGRYYRANDANVVRDRDLEVDTTLKSLSQQIENIRSPEGSRKNPARTCRDLKMCH<br>SDWKSGEYWIDPNQGCNLDAIKVFCNMETGETCVYPTQPSVAQKNWYISKNPKDKRHVWFGESMTDGFQ<br>FEYGGQGSDPADVAIQLTFLRLMSTEASQNITYHCKNSVAYMDQQTGNLKKALLLQGSNEIEIRAEGNS<br>RFTYSVTVDGCTSHTGAWGKTVIEYKTTKTSRLPIIDVAPLDVGAPDQEFGFDVGPVCFL | trimer fusion polypeptide without signal peptide, 1509 aa, proline mutant (986K/987V →986P/987 P) |

EP 4 722 230 A1

| SEQ ID NO. | Sequence | Description |
|---|---|---|
| 13 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGNIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | SARS-CoV-2 South African variant B.1.351 S-trimer fusion polypeptide without signal peptide, 1509 aa, S1/S2 furin cleavage site 1 and proline mutant (685R→685 A, 986K/987V →986P/987 P) |

| SEQ ID NO. | Sequence | Description |
|---|---|---|
| 14 | QCVNFTNRTQLPSAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNYPFLGVYYHKNNKS WMESEFRVYSSANNCTFEYVSQPFLMDLEGKQGNFKNLSEFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGTIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEYVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAAIKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | SARS-CoV-2 Brazilian variant P.1 S-trimer fusion polypeptide without signal peptide, 1509 aa |
| 15 | QCVNFTNRTQLPSAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNYPFLGVYYHKNNKS WMESEFRVYSSANNCTFEYVSQPFLMDLEGKQGNFKNLSEFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI | SARS-CoV-2 Brazilian variant P.1 S-trimer fusion polypeptide |

| SEQ ID NO. | Sequence | Description |
|---|---|---|
| | SNCVADYSVLYNSASFSTFKCYGVSPTKLNDLCFTNVYADSFVIRGDEVRQIAPGQTGTIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEYVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAAIKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | without signal peptide, 1509 aa, S1/S2 furin cleavage site 1 mutant (685R→685 A) |
| 16 | QCVNFTNRTQLPSAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNYPFLGVYYHKNNKS WMESEFRVYSSANNCTFEYVSQPFLMDLEGKQGNFKNLSEFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGTIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEYVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAAIKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | SARS-CoV-2 Brazilian variant P.1 S-trimer fusion polypeptide without signal peptide, 1509 aa, proline mutant (986K/987V →986P/987 P) |

74

EP 4 722 230 A1

(continued)

| SEQ ID NO. | Sequence | Description |
|---|---|---|
| 17 | QCVNFTNRTQLPSAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNYPFLGVYYHKNNKS WMESEFRVYSSANNCTFEYVSQPFLMDLEGKQGNFKNLSEFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGTIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEYVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAAIKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | SARS-CoV-2 Brazilian variant P.1 S-trimer fusion polypeptide without signal peptide, 1509 aa, S1/S2 furin cleavage site 1 and proline mutant (685R→685 A, 986K/987V →986P/987 P) |
| 18 | QCVNLTTRTQLPPAYTNSFTRGVYYPDKVFRSSVLHSTQDLFLPFFSNVTWFHAISGTNGTKRFDNPVL PFNDGVYFASTEKSNIIRGWIFGTTLDSKTQSLLIVNNATNVVIKVCEFQFCNDPFLGVYYHKNNKSWM ESEFRVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF | SARS-CoV-2 British variant B.1.1.7 S-trimer fusion polypeptide |

| SEQ ID NO. | Sequence | Description |
|---|---|---|
|  | TGCVIAWNSNNLDSKVGGNYNYLYRLFRKSNLKPFERDISTEIYQAGSTPCNGVEGFNCYFPLQSYGFQ PTYGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSHRRARSVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQDVVNQNAQALNTLVKQLSSNFGAISSVLNDIL SRLDKVEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQYIKRSNGLPGPIGPPGPRGRTGDAGPVGPPGPPGPPGPPGPSAGFDFS FLPQPPQEKAHDGGRYYRANDANVVRDRDLEVDTTLKSLSQQIENIRSPEGSRKNPARTCRDLKMCHSD WKSGEYWIDPNQGCNLDAIKVFCNMETGETCVYPTQPSVAQKNWYISKNPKDKRHVWFGESMTDGFQFE YGGQGSDPADVAIQLTFLRLMSTEASQNITYHCKNSVAYMDQQTGNLKKALLLQGSNEIEIRAEGNSRF TYSVTVDGCTSHTGAWGKTVIEYKTTKTSRLPIIDVAPLDVGAPDQEFGFDVGPVCFL | without signal peptide, 1507 aa |
| 19 | QCVNLTTRTQLPPAYTNSFTRGVYYPDKVFRSSVLHSTQDLFLPFFSNVTWFHAISGTNGTKRFDNPVL PFNDGVYFASTEKSNIIRGWIFGTTLDSKTQSLLIVNNATNVVIKVCEFQFCNDPFLGVYYHKNNKSWM ESEFRVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYLYRLFRKSNLKPFERDISTEIYQAGSTPCNGVEGFNCYFPLQSYGFQ PTYGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSHRRAASVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQDVVNQNAQALNTLVKQLSSNFGAISSVLNDIL SRLDKVEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQYIKRSNGLPGPIGPPGPRGRTGDAGPVGPPGPPGPPGPPGPSAGFDFS FLPQPPQEKAHDGGRYYRANDANVVRDRDLEVDTTLKSLSQQIENIRSPEGSRKNPARTCRDLKMCHSD WKSGEYWIDPNQGCNLDAIKVFCNMETGETCVYPTQPSVAQKNWYISKNPKDKRHVWFGESMTDGFQFE YGGQGSDPADVAIQLTFLRLMSTEASQNITYHCKNSVAYMDQQTGNLKKALLLQGSNEIEIRAEGNSRF TYSVTVDGCTSHTGAWGKTVIEYKTTKTSRLPIIDVAPLDVGAPDQEFGFDVGPVCFL | SARS-CoV-2 British variant B.1.1.7 S-trimer fusion polypeptide without signal peptide, 1507 aa, S1/S2 furin cleavage site 1 mutant (685R→685 A) |

EP 4 722 230 A1

76

| SEQ ID NO. | Sequence | Description |
|---|---|---|
| 20 | QCVNLTTRTQLPPAYTNSFTRGVYYPDKVFRSSVLHSTQDLFLPFFSNVTWFHAISGTNGTKRFDNPVL PFNDGVYFASTEKSNIIRGWIFGTTLDSKTQSLLIVNNATNVVIKVCEFQFCNDPFLGVYYHKNNKSWM ESEFRVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYLYRLFRKSNLKPFERDISTEIYQAGSTPCNGVEGFNCYFPLQSYGFQ PTYGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSHRRARSVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQDVVNQNAQALNTLVKQLSSNFGAISSVLNDIL SRLDPPEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQYIKRSNGLPGPIGPPGPRGRTGDAGPVGPPGPPGPPGPPSAGFDFS FLPQPPQEKAHDGGRYYRANDANVVRDRDLEVDTTLKSLSQQIENIRSPEGSRKNPARTCRDLKMCHSD WKSGEYWIDPNQGCNLDAIKVFCNMETGETCVYPTQPSVAQKNWYISKNPKDKRHVWFGESMTDGFQFE YGGQGSDPADVAIQLTFLRLMSTEASQNITYHCKNSVAYMDQQTGNLKKALLLQGSNEIEIRAEGNSRF TYSVTVDGCTSHTGAWGKTVIEYKTTKTSRLPIIDVAPLDVGAPDQEFGFDVGPVCFL | SARS-CoV-2 British variant B.1.1.7 S-trimer fusion polypeptide without signal peptide, 1507 aa, proline mutant (986K/987V →986P/987 P) |
| 21 | QCVNLTTRTQLPPAYTNSFTRGVYYPDKVFRSSVLHSTQDLFLPFFSNVTWFHAISGTNGTKRFDNPVL PFNDGVYFASTEKSNIIRGWIFGTTLDSKTQSLLIVNNATNVVIKVCEFQFCNDPFLGVYYHKNNKSWM ESEFRVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYLYRLFRKSNLKPFERDISTEIYQAGSTPCNGVEGFNCYFPLQSYGFQ | SARS-CoV-2 British variant B.1.1.7 S-trimer fusion poly-peptide without |

EP 4 722 230 A1

| SEQ ID NO. | Sequence | Description |
|---|---|---|
|  | PTYGVGYQPYRVVVLSFELLHAPATVCGPKKSTNLVKNKCVNFNFNGLTGTGVLTESNKKFLPFQQFGR<br>DIADTTDAVRDPQTLEILDITPCSFGGVSVITPGTNTSNQVAVLYQGVNCTEVPVAIHADQLTPTWRVY<br>STGSNVFQTRAGCLIGAEHVNNSYECDIPIGAGICASYQTQTNSHRRAASVASQSIIAYTMSLGAENSV<br>AYSNNSIAIPTNFTISVTTEILPVSMTKTSVDCTMYICGDSTECSNLLLQYGSFCTQLNRALTGIAVEQ<br>DKNTQEVFAQVKQIYKTPPIKDFGGFNFSQILPDPSKPSKRSFIEDLLFNKVTLADAGFIKQYGDCLGD<br>IAARDLICAQKFNGLTVLPPLLTDEMIAQYTSALLAGTITSGWTFGAGAALQIPFAMQMAYRFNGIGVT<br>QNVLYENQKLIANQFNSAIGKIQDSLSSTASALGKLQDVVNQNAQALNTLVKQLSSNFGAISSVLNDIL<br>SRLDPPEAEVQIDRLITGRLQSLQTYVTQQLIRAAEIRASANLAATKMSECVLGQSKRVDFCGKGYHLM<br>SFPQSAPHGVVFLHVTYVPAQEKNFTTAPAICHDGKAHFPREGVFVSNGTHWFVTQRNFYEPQIITTDN<br>TFVSGNCDVVIGIVNNTVYDPLQPELDSFKEELDKYFKNHTSPDVDLGDISGINASVVNIQKEIDRLNE<br>VAKNLNESLIDLQELGKYEQYIKRSNGLPGPIGPPGPRGRTGDAGPVGPPGPPGPPGPPGPPSAGFDFS<br>FLPQPPQEKAHDGGRYYRANDANVVRDRDLEVDTTLKSLSQQIENIRSPEGSRKNPARTCRDLKMCHSD<br>WKSGEYWIDPNQGCNLDAIKVFCNMETGETCVYPTQPSVAQKNWYISKNPKDKRHVWFGESMTDGFQFE<br>YGGQGSDPADVAIQLTFLRLMSTEASQNITYHCKNSVAYMDQQTGNLKKALLLQGSNEIEIRAEGNSRF<br>TYSVTVDGCTSHTGAWGKTVIEYKTTKTSRLPIIDVAPLDVGAPDQEFGFDVGPVCFL | signal peptide, 1507 aa, S1/S2 furin cleavage site 1 and proline mutant (685R→685 A, 986K/987V →986P/987 P) |
| 22 | QCVNLTTRTQLPPAYTNSFTRGVYYPDKVFRSSVLHSTQDLFLPFFSNVTWFHAIHVSGTNGTKRFDNP<br>VLPFNDGVYFASTEKSNIIRGWIFGTTLDSKTQSLLIVNNATNVVIKVCEFQFCNDPFLGVYYHKNNKS<br>WMESEFRVYSSANNCTFEYVSQPFLMDLEGKQGNFKNLREFVFKNIDGYFKIYSKHTPINLVRDLPQGF<br>SALEPLVDLPIGINITRFQTLLALHRSYLTPGDSSSGWTAGAAAYYVGYLQPRTFLLKYNENGTITDAV<br>DCALDPLSETKCTLKSFTVEKGIYQTSNFRVQPTESIVRFPNITNLCPFGEVFNATRFASVYAWNRKRI<br>SNCVADYSVLYNSASFSTFKCYGVSPTKLNDLCFTNVYADSFVIRGDEVRQIAPGQTGKIADYNYKLPD<br>DFTGCVIAWNSNNLDSKVGGNYNYLYRLFRKSNLKPFERDISTEIYQAGSTPCNGVEGFNCYFPLQSYG<br>FQPTNGVGYQPYRVVVLSFELLHAPATVCGPKKSTNLVKNKCVNFNFNGLTGTGVLTESNKKFLPFQQF<br>GRDIADTTDAVRDPQTLEILDITPCSFGGVSVITPGTNTSNQVAVLYQGVNCTEVPVAIHADQLTPTWR<br>VYSTGSNVFQTRAGCLIGAEHVNNSYECDIPIGAGICASYQTQTNSPRRARSVASQSIIAYTMSLGAEN<br>SVAYSNNSIAIPTNFTISVTTEILPVSMTKTSVDCTMYICGDSTECSNLLLQYGSFCTQLNRALTGIAV<br>EQDKNTQEVFAQVKQIYKTPPIKDFGGFNFSQILPDPSKPSKRSFIEDLLFNKVTLADAGFIKQYGDCL<br>GDIAARDLICAQKFNGLTVLPPLLTDEMIAQYTSALLAGTITSGWTFGAGAALQIPFAMQMAYRFNGIG<br>VTQNVLYENQKLIANQFNSAIGKIQDSLSSTASALGKLQDVVNQNAQALNTLVKQLSSNFGAISSVLND<br>ILSRLDKVEAEVQIDRLITGRLQSLQTYVTQQLIRAAEIRASANLAATKMSECVLGQSKRVDFCGKGYH<br>LMSFPQSAPHGVVFLHVTYVPAQEKNFTTAPAICHDGKAHFPREGVFVSNGTHWFVTQRNFYEPQIITT<br>DNTFVSGNCDVVIGIVNNTVYDPLQPELDSFKEELDKYFKNHTSPDVDLGDISGINASVVNIQKEIDRL<br>NEVAKNLNESLIDLQELGKYEQYIKRSNGLPGPIGPPGPRGRTGDAGPVGPPGPPGPPGPPGPPSAGFD<br>FSFLPQPPQEKAHDGGRYYRANDANVVRDRDLEVDTTLKSLSQQIENIRSPEGSRKNPARTCRDLKMCH<br>SDWKSGEYWIDPNQGCNLDAIKVFCNMETGETCVYPTQPSVAQKNWYISKNPKDKRHVWFGESMTDGFQ<br>FEYGGQGSDPADVAIQLTFLRLMSTEASQNITYHCKNSVAYMDQQTGNLKKALLLQGSNEIEIRAEGNS<br>RFTYSVTVDGCTSHTGAWGKTVIEYKTTKTSRLPIIDVAPLDVGAPDQEFGFDVGPVCFL | SARS-CoV-2 variant D614G S-trimer fusion polypeptide without signal peptide, 1509 aa |

EP 4 722 230 A1

78

(continued)

| SEQ ID NO. | Sequence | Description |
|---|---|---|
| 23 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | SARS-CoV-2 variant D614G S-trimer fusion polypeptide without signal peptide, 1509 aa, S1/S2 furin cleavage site 1 mutant (685R→685 A) |
| 24 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF | SARS-CoV-2 variant D614G S-trimer fusion polypeptide without signal peptide, 1509 |

| SEQ ID NO. | Sequence | Description |
|---|---|---|
|  | GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | aa, proline mutant (986K/987V →986P/987 P) |
| 25 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | SARS-CoV-2 variant D614G S-trimer fusion polypeptide without signal peptide, 1509 aa, S1/S2 furin cleavage site 1 and proline mutant (685R→685 A, 986K/987V →986P/987 P) |

| SEQ ID NO. | Sequence | Description |
|---|---|---|
| 26 | SDLDRCTTFDDVQAPNYTQHTSSMRGVYYPDEIFRSDTLYLTQDLFLPFYSNVTGFHTINHTFDNPVIP<br>FKDGIYFAATEKSNVVRGWVFGSTMNNKSQSVIIINNSTNVVIRACNFELCDNPFFAVSKPMGTQTHTM<br>IFDNAFNCTFEYISDAFSLDVSEKSGNFKHLREFVFKNKDGFLYVYKGYQPIDVVRDLPSGFNTLKPIF<br>KLPLGINITNFRAILTAFLPAQDTWGTSAAAYFVGYLKPTTFMLKYDENGTITDAVDCSQNPLAELKCS<br>VKSFEIDKGIYQTSNFRVVPSRDVVRFPNITNLCPFGEVFNATKFPSVYAWERKRISNCVADYSVLYNS<br>TFFSTFKCYGVSATKLNDLCFSNVYADSFVVKGDDVRQIAPGQTGVIADYNYKLPDDFMGCVLAWNTRN<br>IDATSTGNYNYKYRYLRHGKLRPFERDISNVPFSPDGKPCTPPALNCYWPLNDYGFYTTTGIGYQPYRV<br>VVLSFELLNAPATVCGPKLSTDLIKNQCVNFNFNGLTGTGVLTPSSKRFQPFQQFGRDVSDFTDSVRDP<br>KTSEILDISPCSFGGVSVITPGTNASSEVAVLYQDVNCTDVSTAIHADQLTPAWRIYSTGNNVFQTQAG<br>CLIGAEHVDTSYECDIPIGAGICASYHTVSLLRSTSQKSIVAYTMSLGADSSIAYSNNTIAIPTNFSIS<br>ITTEVMPVSMAKTSVDCNMYICGDSTECANLLLQYGSFCTQLNRALSGIAAEQDRNTREVFAQVKQMYK<br>TPTLKDFGGFNFSQILPDPLKPTKRSFIEDLLFNKVTLADAGFMKQYGECLGDINARDLICAQKFNGLT<br>VLPPLLTDDMIAAYTAALVSGTATAGWTFGAGAALQIPFAMQMAYRFNGIGVTQNVLYENQKQIANQFN<br>KAISQIQESLTTTSTALGKLQDVVNQNAQALNTLVKQLSSNFGAISSVLNDILSRLDKVEAEVQIDRLI<br>TGRLQSLQTYVTQQLIRAAEIRASANLAATKMSECVLGQSKRVDFCGKGYHLMSFPQAAPHGVVFLHVT<br>YVPSQERNFTTAPAICHEGKAYFPREGVFVFNGTSWFITQRNFFSPQIITTDNTFVSGNCDVVIGIINN<br>TVYDPLQPELDSFKEELDKYFKNHTSPDVDLGDISGINASVVNIQEEIDRLNEVAKNLNESLIDLQELG<br>KYEQYIKRSNGLPGPIGPPGPRGRTGDAGPVGPPGPPGPPGPGPPSAGFDFSFLPQPPQEKAHDGGRY<br>YRANDANVVRDRDLEVDTTLKSLSQQIENIRSPEGSRKNPARTCRDLKMCHSDWKSGEYWIDPNQGCNL<br>DAIKVFCNMETGETCVYPTQPSVAQKNWYISKNPKDKRHVWFGESMTDGFQFEYGGQGSDPADVAIQLT<br>FLRLMSTEASQNITYHCKNSVAYMDQQTGNLKKALLLQGSNEIEIRAEGNSRFTYSVTVDGCTSHTGAW<br>GKTVIEYKTTKTSRLPIIDVAPLDVGAPDQEFGFDVGPVCFL | SARS-CoV-1 S-trimer fusion polypeptide without signal peptide, 1491 aa |
| 27 | QCVNLTTRTQLPPAYTNSFTRGVYYPDKVFRSSVLHSTQDLFLPFFSNVTWFHAIHVSGTNGTKRFDNP<br>VLPFNDGVYFASTEKSNIIRGWIFGTTLDSKTQSLLIVNNATNVVIKVCEFQFCNDPFLGVYYHKNNKS<br>WMESEFRVYSSANNCTFEYVSQPFLMDLEGKQGNFKNLREFVFKNIDGYFKIYSKHTPINLVRDLPQGF<br>SALEPLVDLPIGINITRFQTLLALHRSYLTPGDSSSGWTAGAAAYYVGYLQPRTFLLKYNENGTITDAV<br>DCALDPLSETKCTLKSFTVEKGIYQTSNFRVQPTESIVRFPNITNLCPFGEVFNATRFASVYAWNRKRI<br>SNCVADYSVLYNSASFSTFKCYGVSPTKLNDLCFTNVYADSFVIRGDEVRQIAPGQTGKIADYNYKLPD<br>DFTGCVIAWNSNNLDSKVGGNYNYLYRLFRKSNLKPFERDISTEIYQAGSTPCNGVEGFNCYFPLQSYG<br>FQPTNGVGYQPYRVVVLSFELLHAPATVCGPKKSTNLVKNKCVNFNFNGLTGTGVLTESNKKFLPFQQF<br>GRDIADTTDAVRDPQTLEILDITPCSFGGVSVITPGTNTSNQVAVLYQDVNCTEVPVAIHADQLTPTWR | Hu-1 SARS-CoV-2 spike protein extracellular domain without signal peptide |

EP 4 722 230 A1

81

| SEQ ID NO. | Sequence | Description |
|---|---|---|
|  | VYSTGSNVFQTRAGCLIGAEHVNNSYECDIPIGAGICASYQTQTNSPRRA**R**SVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLD**KV**EAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ |  |
| 28 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQDVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | Hu-1 SARS-CoV-2 spike protein extracellular domain without signal peptide, S1/S2 furin cleavage site 1 mutant (685R→685 A) |

| SEQ ID NO. | Sequence | Description |
|---|---|---|
| 29 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQDVNCTEVPVAIHADQLTPTWR VYSTGSNVFQTRAGCLIGAEHVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | Hu-1 SARS-CoV-2 spike protein extracellular domain without signal peptide, proline mutant (986K/987V →986P/987 P) |
| 30 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQDVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | Hu-1 SARS-CoV-2 spike protein extracellular domain without signal peptide, S1/S2 furin cleavage site 1 and proline mutant (685R→685 A, 986K/987V →986P/987 P) |

| SEQ ID NO. | Sequence | Description |
|---|---|---|
| 31 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKC | Hu-1 SARS-CoV-2 spike protein NTD/RBD fragment without signal peptide |
| 32 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQDVNCTEVPVAIHADQLTPTWR VYSTGSNVFQTRAGCLIGAEHVNNSYECDIPIGAGICASYQTQTNSP | Hu-1 SARS-CoV-2 spike protein S1 fragment without signal peptide |
| 33 | SVASQSIIAYTMSLGAENSVAYSNNSIAIPTNFTISVTTEILPVSMTKTSVDCTMYICGDSTECSNLLL QYGSFCTQLNRALTGIAVEQDKNTQEVFAQVKQIYKTPPIKDFGGFNFSQILPDPSKPSKRSFIEDLLF NKVTLADAGFIKQYGDCLGDIAARDLICAQKFNGLTVLPPLLTDEMIAQYTSALLAGTITSGWTFGAGA ALQIPFAMQMAYRFNGIGVTQNVLYENQKLIANQFNSAIGKIQDSLSSTASALGKLQDVVNQNAQALNT LVKQLSSNFGAISSVLNDILSRLDKVEAEVQIDRLITGRLQSLQTYVTQQLIRAAEIRASANLAATKMS ECVLGQSKRVDFCGKGYHLMSFPQSAPHGVVFLHVTYVPAQEKNFTTAPAICHDGKAHFPREGVFVSNG THWFVTQRNFYEPQIITTDNTFVSGNCDVVIGIVNNTVYDPLQPELDSFKEELDKYFKNHTSPDVDLGD ISGINASVVNIQKEIDRLNEVAKNLNESLIDLQELGKYEQ | Hu-1 SARS-CoV-2 spike protein S2 fragment (cleaved at site 1, S1/S2) |
| 34 | TMSLGAENSVAYSNNSIAIPTNFTISVTTEILPVSMTKTSVDCTMYICGDSTECSNLLLQYGSFCTQLN RALTGIAVEQDKNTQEVFAQVKQIYKTPPIKDFGGFNFSQILPDPSKPSKRSFIEDLLFNKVTLADAGF IKQYGDCLGDIAARDLICAQKFNGLTVLPPLLTDEMIAQYTSALLAGTITSGWTFGAGAALQIPFAMQM AYRFNGIGVTQNVLYENQKLIANQFNSAIGKIQDSLSSTASALGKLQDVVNQNAQALNTLVKQLSSNFG AISSVLNDILSRLDKVEAEVQIDRLITGRLQSLQTYVTQQLIRAAEIRASANLAATKMSECVLGQSKRV DFCGKGYHLMSFPQSAPHGVVFLHVTYVPAQEKNFTTAPAICHDGKAHFPREGVFVSNGTHWFVTQRNF YEPQIITTDNTFVSGNCDVVIGIVNNTVYDPLQPELDSFKEELDKYFKNHTSPDVDLGDISGINASVVN IQKEIDRLNEVAKNLNESLIDLQELGKYEQ | Hu-1 SARS-CoV-2 spike protein S2 fragment (cleaved at site 2, S1/S2) |

EP 4 722 230 A1

84

| SEQ ID NO. | Sequence | Description |
|---|---|---|
| 35 | SFIEDLLFNKVTLADAGFIKQYGDCLGDIAARDLICAQKFNGLTVLPPLLTDEMIAQYTSALLAGTITS GWTFGAGAALQIPFAMQMAYRFNGIGVTQNVLYENQKLIANQFNSAIGKIQDSLSSTASALGKLQDVVN QNAQALNTLVKQLSSNFGAISSVLNDILSRLDKVEAEVQIDRLITGRLQSLQTYVTQQLIRAAEIRASA NLAATKMSECVLGQSKRVDFCGKGYHLMSFPQSAPHGVVFLHVTYVPAQEKNFTTAPAICHDGKAHFPR EGVFVSNGTHWFVTQRNFYEPQIITTDNTFVSGNCDVVIGIVNNTVYDPLQPELDSFKEELDKYFKNHT SPDVDLGDISGINASVVNIQKEIDRLNEVAKNLNESLIDLQELGKYEQ | Hu-1 SARS-CoV-2 spike protein S2 fragment (cleaved at S2') |
| 36 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGNIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 South African variant B.1.351 spike protein extracellular domain without signal peptide |

(continued)

| SEQ ID NO. | Sequence | Description |
|---|---|---|
| 37 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPIVDLPIGINITRFQTLLALHRSYLTPGDSSSGWTAGAAAYYVGYLQPRTFLLKYNENGTITDAV DCAIDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGNIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 South African variant B.1.351 spike protein extracellular domain without signal peptide, S1/S2 furin cleavage site 1 mutant (685R→685 A) |
| 38 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPIVDLPIGINITRFQTLLALHRSYLTPGDSSSGWTAGAAAYYVGYLQPRTFLLKYNENGTITDAV DCAIDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGNIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 South African variant B.1.351 spike protein extracellular domain without signal peptide, proline mutant (986K/987V →986P/987 P) |

(continued)

| SEQ ID NO. | Sequence | Description |
|---|---|---|
| 39 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGNIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 South African variant B.1.351 spike protein extracellular domain without signal peptide, S1/S2 furin cleavage site 1 and proline mutant (685R→685 A, 986K/987V →986P/987 P) |
| 40 | QCVNFTNRTQLPSAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNYPFLGVYYHKNNKS WMESEFRVYSSANNCTFEYVSQPFLMDLEGKQGNFKNLSEFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGTIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEYVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAAIKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 Brazilian variant P.1 spike protein extracellular domain without signal peptide |

87

(continued)

| SEQ ID NO. | Sequence | Description |
|---|---|---|
| 41 | QCVNFTNRTQLPSAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNYPFLGVYYHKNNKS WMESEFRVYSSANNCTFEYVSQPFLMDLEGKQGNFKNLSEFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGTIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEYVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAAIKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 Brazilian variant P.1 spike protein extracellular domain without signal peptide, S1/S2 furin cleavage site 1 mutant (685R→685 A) |
| 42 | QCVNFTNRTQLPSAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNYPFLGVYYHKNNKS WMESEFRVYSSANNCTFEYVSQPFLMDLEGKQGNFKNLSEFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGTIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEYVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAAIKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 Brazilian variant P.1 spike protein extracellular domain without signal peptide, proline mutant (986K/987V →986P/987 P) |

| SEQ ID NO. | Sequence | Description |
|---|---|---|
| 43 | QCVNFTNRTQLPSAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNYPFLGVYYHKNNKS WMESEFRVYSSANNCTFEYVSQPFLMDLEGKQGNFKNLSEFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGTIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEYVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAAIKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 Brazilian variant P.1 spike protein extracellular domain without signal peptide, S1/S2 furin cleavage site 1 and proline mutant (685R→685 A, 986K/987V →986P/987 P) |
| 44 | QCVNLTTRTQLPPAYTNSFTRGVYYPDKVFRSSVLHSTQDLFLPFFSNVTWFHAISGTNGTKRFDNPVL PFNDGVYFASTEKSNIIRGWIFGTTLDSKTQSLLIVNNATNVVIKVCEFQFCNDPFLGVYYHKNNKSWM ESEFRVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYLYRLFRKSNLKPFERDISTEIYQAGSTPCNGVEGFNCYFPLQSYGFQ PTYGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSHRRARSVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQDVVNQNAQALNTLVKQLSSNFGAISSVLNDIL SRLDKVEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQ | SARS-CoV-2 British variant B.1.1.7 spike protein extracellular domain without signal peptide |

(continued)

| SEQ ID NO. | Sequence | Description |
|---|---|---|
| 45 | QCVNLTTRTQLPPAYTNSFTRGVYYPDKVFRSSVLHSTQDLFLPFFSNVTWFHAISGTNGTKRFDNPVL PFNDGVYFASTEKSNIIRGWIFGTTLDSKTQSLLIVNNATNVVIKVCEFQFCNDPFLGVYYHKNNKSWM ESEFRVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYLYRLFRKSNLKPFERDISTEIYQAGSTPCNGVEGFNCYFPLQSYGFQ PTYGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSHRRAASVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQDVVNQNAQALNTLVKQLSSNFGAISSVLNDIL SRLDKVEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQ | SARS-CoV-2 British variant B.1.1.7 spike protein extracellular domain without signal peptide, S1/S2 furin cleavage site 1 mutant (685R→685 A) |
| 46 | QCVNLTTRTQLPPAYTNSFTRGVYYPDKVFRSSVLHSTQDLFLPFFSNVTWFHAISGTNGTKRFDNPVL PFNDGVYFASTEKSNIIRGWIFGTTLDSKTQSLLIVNNATNVVIKVCEFQFCNDPFLGVYYHKNNKSWM ESEFRVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYLYRLFRKSNLKPFERDISTEIYQAGSTPCNGVEGFNCYFPLQSYGFQ PTYGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSHRRARSVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQDVVNQNAQALNTLVKQLSSNFGAISSVLNDIL SRLDPPEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQ | SARS-CoV-2 British variant B.1.1.7 spike protein extracellular domain without signal peptide, proline mutant (986K/987V →986P/987 P) |

90

(continued)

| SEQ ID NO. | Sequence | Description |
|---|---|---|
| 47 | QCVNLTTRTQLPPAYTNSFTRGVYYPDKVFRSSVLHSTQDLFLPFFSNVTWFHAISGTNGTKRFDNPVL PFNDGVYFASTEKSNIIRGWIFGTTLDSKTQSLLIVNNATNVVIKVCEFQFCNDPFLGVYYHKNNKSWM ESEFRVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYLYRLFRKSNLKPFERDISTEIYQAGSTPCNGVEGFNCYFPLQSYGFQ PTYGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSHRRAASVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQDVVNQNAQALNTLVKQLSSNFGAISSVLNDIL SRLDPPEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQ | SARS-CoV-2 British variant B.1.1.7 spike protein extracellular domain without signal peptide, S1/S2 furin cleavage site and proline mutant (685R→685 A, 986K/987V →986P/987 P) |
| 48 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 variant D614G spike protein extracellular domain without signal peptide |

91

EP 4 722 230 A1

| SEQ ID NO. | Sequence | Description |
|---|---|---|
| 49 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDKVEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 variant D614G spike protein extracellular domain without signal peptide, S1/S2 furin cleavage site 1 mutant (685R→685 A) |
| 50 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRARSVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 variant D614G spike protein extracellular domain without signal peptide, proline mutant (986K/987V →986P/987 P) |

| SEQ ID NO. | Sequence | Description |
|---|---|---|
| 51 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGKIADYNYKLPD DFTGCVIAWNSNNLDSKVGGNYNYLYRLFRKSNLKPFERDISTEIYQAGSTPCNGVEGFNCYFPLQSYG FQPTNGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQ | SARS-CoV-2 variant D614G spike protein extracellular domain without signal peptide, S1/S2 furin cleavage site 1 and proline mutant (685R→685 A, 986K/987V →986P/987 P) |
| 52 | SDLDRCTTFDDVQAPNYTQHTSSMRGVYYPDEIFRSDTLYLTQDLFLPFYSNVTGFHTINHTFDNPVIP FKDGIYFAATEKSNVVRGWVFGSTMNNKSQSVIIINNSTNVVIRACNFELCDNPFFAVSKPMGTQTHTM IFDNAFNCTFEYISDAFSLDVSEKSGNFKHLREFVFKNKDGFLYVYKGYQPIDVVRDLPSGFNTLKPIF KLPLGINITNFRAILTAFLPAQDTWGTSAAAYFVGYLKPTTFMLKYDENGTITDAVDCSQNPLAELKCS VKSFEIDKGIYQTSNFRVVPSRDVVRFPNITNLCPFGEVFNATKFPSVYAWERKRISNCVADYSVLYNS TFFSTFKCYGVSATKLNDLCFSNVYADSFVVKGDDVRQIAPGQTGVIADYNYKLPDDFMGCVLAWNTRN IDATSTGNYNYKYRYLRHGKLRPFERDISNVPFSPDGKPCTPPALNCYWPLNDYGFYTTTGIGYQPYRV VVLSFELLNAPATVCGPKLSTDLIKNQCVNFNFNGLTGTGVLTPSSKRFQPFQQFGRDVSDFTDSVRDP KTSEILDISPCSFGGVSVITPGTNASSEVAVLYQDVNCTDVSTAIHADQLTPAWRIYSTGNNVFQTQAG CLIGAEHVDTSYECDIPIGAGICASYHTVSLLRSTSQKSIVAYTMSLGADSSIAYSNNTIAIPTNFSIS ITTEVMPVSMAKTSVDCNMYICGDSTECANLLLQYGSFCTQLNRALSGIAAEQDRNTREVFAQVKQMYK TPTLKDFGGFNFSQILPDPLKPTKRSFIEDLLFNKVTLADAGFMKQYGECLGDINARDLICAQKFNGLT VLPPLLTDDMIAAYTAALVSGTATAGWTFGAGAALQIPFAMQMAYRFNGIGVTQNVLYENQKQIANQFN KAISQIQESLTTTSTALGKLQDVVNQNAQALNTLVKQLSSNFGAISSVLNDILSRLDKVEAEVQIDRLI TGRLQSLQTYVTQQLIRAAEIRASANLAATKMSECVLGQSKRVDFCGKGYHLMSFPQAAPHGVVFLHVT YVPSQERNFTTAPAICHEGKAYFPREGVFVFNGTSWFITQRNFFSPQIITTDNTFVSGNCDVVIGIINN TVYDPLQPELDSFKEELDKYFKNHTSPDVDLGDISGINASVVNIQEEIDRLNEVAKNLNESLIDLQELG KYEQ | SARS-CoV-1 spike protein extracellular domain without signal peptide |
| 53 | MFIFLLFLTLTSG | SARS-CoV-1 spike protein signal peptide |
| 54 | MFVFLVLLPLVSS | Hu-1 SARS-CoV-2 spike protein signal peptide |

| SEQ ID NO. | Sequence | Description |
|---|---|---|
| 55 | MFVFLVLLPLVSSQCVNLTTRTQLPPAYTNSFTRGVYYPDKVFRSSVLHSTQDLFLPFFSNVTWFHAIH VSGTNGTKRFDNPVLPFNDGVYFASTEKSNIIRGWIFGTTLDSKTQSLLIVNNATNVVIKVCEFQFCND PFLGVYYHKNNKSWMESEFRVYSSANNCTFEYVSQPFLMDLEGKQGNFKNLREFVFKNIDGYFKIYSKH TPINLVRDLPQGFSALEPLVDLPIGINITRFQTLLALHRSYLTPGDSSSGWTAGAAAYYVGYLQPRTFL LKYNENGTITDAVDCALDPLSETKCTLKSFTVEKGIYQTSNFRVQPTESIVRFPNI**TNLCPFGEVFNAT RFASVYAWNRKRISNCVADYSVLYNSASFSTFKCYGVSPTKLNDLCFTNVYADSFVIRGDEVRQIAPGQ TGKIADYNYKLPDDFTGCVIAW<u>NSNNLDSKVGGNYNYLYRLFRKSNLKPFERDISTEIYQAGSTPCNGV EGFNCYFPLQSYGFQPTNGVGYQPYRVVVLSFELLHAPATVCG</u>**PKKSTNLVKNKCVNFNFNGLTGTGVL TESNKKFLPFQQFGRDIADTTDAVRDPQTLEILDITPCSFGGVSVITPGTNTSNQVAVLYQDVNCTEVP VAIHADQLTPTWRVYSTGSNVFQTRAGCLIGAEHVNNSYECDIPIGAGICASYQTQTNSPRRARSVASQ SIIAYTMSLGAENSVAYSNNSIAIPTNFTISVTTEILPVSMTKTSVDCTMYICGDSTECSNLLLQYGSF CTQLNRALTGIAVEQDKNTQEVFAQVKQIYKTPPIKDFGGFNFSQILPDPSKPSKRSFIEDLLFNKVTL ADAGFIKQYGDCLGDIAARDLICAQKFNGLTVLPPLLTDEMIAQYTSALLAGTITSGWTFGAGAALQIP FAMQMAYRFNGIGVTQNVLYENQKLIANFNSAIGKIQDSLSSTASALGKLQDVVNQNAQALNTLVKQL SSNFGAISSVLNDILSRLDKVEAEVQIDRLITGRLQSLQTYVTQQLIRAAEIRASANLAATKMSECVLG QSKRVDFCGKGYHLMSFPQSAPHGVVFLHVTYVPAQEKNFTTAPAICHDGKAHFPREGVFVSNGTHWFV TQRNFYEPQIITTDNTFVSGNCDVVIGIVNNTVYDPLQPELDSFKEELDKYFKNHTSPDVDLGDISGIN ASVVNIQKEIDRLNEVAKNLNESLIDLQELGKYEQYIKWPWYIWLGFIAGLIAIVMVTIMLCCMTSCCS CLKGCCSCGSCCKFDEDDSEPVLKGVKLHYT | Hu-1 SARS-CoV-2 full-length spike protein, 1273 aa |
| 56 | MFVFLVLLPLVSSQCVNLTTRTQLPPAYTNSFTRGVYYPDKVFRSSVLHSTQDLFLPFFSNVTWFHAIH VSGTNGTKRFDNPVLPFNDGVYFASTEKSNIIRGWIFGTTLDSKTQSLLIVNNATNVVIKVCEFQFCND PFLGVYYHKNNKSWMESEFRVYSSANNCTFEYVSQPFLMDLEGKQGNFKNLREFVFKNIDGYFKIYSKH TPINLVRDLPQGFSALEPLVDLPIGINITRFQTLLALHRSYLTPGDSSSGWTAGAAAYYVGYLQPRTFL LKYNENGTITDAVDCALDPLSETKCTLKSFTVEKGIYQTSNFRVQPTESIVRFPNITNLCPFGEVFNAT RFASVYAWNRKRISNCVADYSVLYNSASFSTFKCYGVSPTKLNDLCFTNVYADSFVIRGDEVRQIAPGQ TGKIADYNYKLPDDFTGCVIAWNSNNLDSKVGGNYNYLYRLFRKSNLKPFERDISTEIYQAGSTPCNGV EGFNCYFPLQSYGFQPTNGVGYQPYRVVVLSFELLHAPATVCGPKKSTNLVKNKCVNFNFNGLTGTGVL TESNKKFLPFQQFGRDIADTTDAVRDPQTLEILDITPCSFGGVSVITPGTNTSNQVAVLYQDVNCTEVP VAIHADQLTPTWRVYSTGSNVFQTRAGCLIGAEHVNNSYECDIPIGAGICASYQTQTNSPRRARSVASQ SIIAYTMSLGAENSVAYSNNSIAIPTNFTISVTTEILPVSMTKTSVDCTMYICGDSTECSNLLLQYGSF CTQLNRALTGIAVEQDKNTQEVFAQVKQIYKTPPIKDFGGFNFSQILPDPSKPSKRSFIEDLLFNKVTL ADAGFIKQYGDCLGDIAARDLICAQKFNGLTVLPPLLTDEMIAQYTSALLAGTITSGWTFGAGAALQIP FAMQMAYRFNGIGVTQNVLYENQKLIANFNSAIGKIQDSLSSTASALGKLQDVVNQNAQALNTLVKQL SSNFGAISSVLNDILSRLDKVEAEVQIDRLITGRLQSLQTYVTQQLIRAAEIRASANLAATKMSECVLG QSKRVDFCGKGYHLMSFPQSAPHGVVFLHVTYVPAQEKNFTTAPAICHDGKAHFPREGVFVSNGTHWFV TQRNFYEPQIITTDNTFVSGNCDVVIGIVNNTVYDPLQPELDSFKEELDKYFKNHTSPDVDLGDISGIN ASVVNIQKEIDRLNEVAKNLNESLIDLQELGKYEQ | Hu-1 SARS-CoV-2 spike protein extracellular domain with signal peptide |

(continued)

| SEQ ID NO. | Description | Sequence |
|---|---|---|
| 57 | Hu-1 SARS-CoV-2 spike protein NTD without signal peptide, 290 aa | VNLTTRTQLPPAYTNSFTRGVYYPDKVFRSSVLHSTQDLFLPFFSNVTWFHAIHVSGTNGTKRFDNPVL PFNDGVYFASTEKSNIIRGWIFGTTLDSKTQSLLIVNNATNVVIKVCEFQFCNDPFLGVYYHKNNKSWM ESEFRVYSSANNCTFEYVSQPFLMDLEGKQGNFKNLREFVFKNIDGYFKIYSKHTPINLVRDLPQGFSA LEPIVDLPIGINITRFQTLLALHRSYLTPGDSSSGWTAGAAAYYVGYLQPRTFLLKYNENGTITDAVDC ALDPLSETKCTIKS |
| 58 | Hu-1 SARS-CoV-2 spike protein RBD, 192 aa | PNITNLCPFGEVFNATRFASVYAWNRKRISNCVADYSVLYNSASFSTFKCYGVSPTKLNDLCFTNVYAD SFVIRGDEVRQIAPGQTGKIADYNYKLPDDFTGCVIAWNSNNLDSKVGGNYNYLYRLFRKSNLKPFERD ISTEIYQAGSTPCNGVEGFNCYFPLQSYGFQPTNGVGYQPYRVVVLSFELLHAP |
| 59 | Hu-1 SARS-CoV-2 spike protein S1/S2 | RRAR |
| 60 | Hu-1 SARS-CoV-2 spike protein S1/S2 mutant | GSAG |
| 61 | Hu-1 SARS-CoV-2 spike protein fusion peptide (FP) sequence | SFIEDLLFNKVTLADAGF |
| 62 | Hu-1 SARS-CoV-2 spike protein heptapeptide repeat 1 (HR1) | GIGVTQNVLYENQKLIANQFNSAIGKIQDSLSSTASALGKLQDVVNQNAQALNTLVKQLSSNFGAISSV LNDILSRLD |
| 63 | Hu-1 SARS-CoV-2 spike protein central helix (CH) | KVEAEVQIDRLITGRLQSLQTYVTQQLIRAAEIRASANLAATKMSECVLG |
| 64 | Hu-1 SARS-CoV-2 spike protein connector domain (CD) | TTAPAICHDGKAHFPREGVFVSNGTHWFVTQRNFYEPQIITTDNTFVSGNCDVVIGIVNNTVYDPL |
| 65 | Hu-1 SARS-CoV-2 spike protein heptapeptide repeat 2 (HR2) | EELDKYFKNHTSPDVDLGDISGINASVVNIQKEIDRLNEVAKNLNESLIDLQELGKYEQ |
| 66 | Hu-1 SARS-CoV-2 spike protein transmembrane (TM) domain | WPWYIWLGFIAGLIAIVMVTIML |
| 67 | Trimeric peptide (type I), QT version | ANVVRDRDLEVDTTLKSLSQQIENIRSPEGSRKNPARTCRDLKMCHSDWKSGEYWIDPNQGCNLDAIKV FCNMETGETCVYPTQPSVAQKNWYISKNPKDKRHVWFGESMTDGFQFEYGGQGSDPADVAIQLTFLRLM STEASQNITYHCKNSVAYMDQQTGNLKKALLLQGSNEIEIRAEGNSRFTYSVTVDGCTSHTGAWGKTVI EYKTTKTSRLPIIDVAPLDVGAPDQEFGFDVGPVCFL |
| 68 | Trimerized peptide (type I) with glycine-X-Y repeat and D→N mutation at the BMP-1 site, QT version | NGLPGPIGPPGPRGTGDAGPVGPPGPPGPGPPPSAGFDFSFLPQPPQEKAHDGGRYYRANDANVV RDRDLEVDTTLKSLSQQIENIRSPEGSRKNPARTCRDLKMCHSDWKSGEYWIDPNQGCNLDAIKVFCNM ETGETCVYPTQPSVAQKNWYISKNPKDKRHVWFGESMTDGFQFEYGGQGSDPADVAIQLTFLRLMSTEA SQNITYHCKNSVAYMDQQTGNLKKALLLQGSNEIEIRAEGNSRFTYSVTVDGCTSHTGAWGKTVIEYKT TKTSRLPIIDVAPLDVGAPDQEFGFDVGPVCFL |

| SEQ ID NO. | Sequence | Description |
|---|---|---|
| 69 | NGLPGPIGPPGPRGRTGDAGPVGPPGPPGPPGPPGPPSAGFDFSFLPQPPQEKAHDGGRYYRNDDANVV RDRDLEVDTTLKSLSQQIENIRSPEGSRKNPARTCRDLKMCHSDWKSGEYWIDPNQGCNLDAIKVFCNM ETGETCVYPTQPSVAQKNWYISKNPKDKRHVWFGESMTDGFQFEYGGQGSDPADVAIQLTFLRLMSTEA SQNITYHCKNSVAYMDQQTGNLKKALLLQGSNEIEIRAEGNSRFTYSVTVDGCTSHTGAWGKTVIEYKT TKTSRLPIIDVAPLDVGAPDQEFGFDVGPVCFL | Trimerized peptide (type I) with glycine-X-Y repeat and A→N mutation at the BMP-1 site, QT version |
| 70 | RSNGLPGPIGPPGPRGRTGDAGPVGPPGPPGPPGPPGPPSAGFDFSFLPQPPQEKAHDGGRYYRANDAN VVRDRDLEVDTTLKSLSQQIENIRSPEGSRKNPARTCRDLKMCHSDWKSGEYWIDPNQGCNLDAIKVFC NMETGETCVYPTQPSVAQKNWYISKNPKDKRHVWFGESMTDGFQFEYGGQGSDPADVAIQLTFLRLMST EASQNITYHCKNSVAYMDQQTGNLKKALLLQGSNEIEIRAEGNSRFTYSVTVDGCTSHTGAWGKTVIEY KTTKTSRLPIIDVAPLDVGAPDQEFGFDVGPVCFL | Trimerized peptide (type I) with glycine-X-Y repeat and D→N mutation at the BMP-1 site, QT version |
| 71 | GSNGLPGPIGPPGPRGRTGDAGPVGPPGPPGPPGPPGPPSAGFDFSFLPQPPQEKAHDGGRYYRANDAN VVRDRDLEVDTTLKSLSQQIENIRSPEGSRKNPARTCRDLKMCHSDWKSGEYWIDPNQGCNLDAIKVFC NMETGETCVYPTQPSVAQKNWYISKNPKDKRHVWFGESMTDGFQFEYGGQGSDPADVAIQLTFLRLMST EASQNITYHCKNSVAYMDQQTGNLKKALLLQGSNEIEIRAEGNSRFTYSVTVDGCTSHTGAWGKTVIEY KTTKTSRLPIIDVAPLDVGAPDQEFGFDVGPVCFL | Trimerized peptide (type I) with glycine-X-Y repeat and D→N mutation at the BMP-1 site, QT version |
| 72 | ANVVRDRDLEVDTTLKSLSQQIENIRSPEGSRKNPARTCRDLKMCHSDWKSGEYWIDPNQGCNLDAIKV FCNMETGETCVYPTQPSVAQKNWYISKNPKDKRHVWFGESMTDGFQFEYGGQGSDPADVAIQLTFLRLM STEASQNITYHCKNSVAYMDQQTGNLKKALLLKGSNEIEIRAEGNSRFTYSVTVDGCTSHTGAWGKTVI EYKTTKSSRLPIIDVAPLDVGAPDQEFGFDVGPVCFL | Trimeric peptide (type I), KS version |
| 73 | NGLPGPIGPPGPRGRTGDAGPVGPPGPPGPPGPPGPPSAGFDFSFLPQPPQEKAHDGGRYYRANDANVV RDRDLEVDTTLKSLSQQIENIRSPEGSRKNPARTCRDLKMCHSDWKSGEYWIDPNQGCNLDAIKVFCNM ETGETCVYPTQPSVAQKNWYISKNPKDKRHVWFGESMTDGFQFEYGGQGSDPADVAIQLTFLRLMSTEA SQNITYHCKNSVAYMDQQTGNLKKALLLKGSNEIEIRAEGNSRFTYSVTVDGCTSHTGAWGKTVIEYKT TKSSRLPIIDVAPLDVGAPDQEFGFDVGPVCFL | Trimerized peptide (type I) with glycine-X-Y repeat and D→N mutation at the BMP-1 site, KS version |
| 74 | NGLPGPIGPPGPRGRTGDAGPVGPPGPPGPPGPPGPPSAGFDFSFLPQPPQEKAHDGGRYYRNDDANVV RDRDLEVDTTLKSLSQQIENIRSPEGSRKNPARTCRDLKMCHSDWKSGEYWIDPNQGCNLDAIKVFCNM ETGETCVYPTQPSVAQKNWYISKNPKDKRHVWFGESMTDGFQFEYGGQGSDPADVAIQLTFLRLMSTEA SQNITYHCKNSVAYMDQQTGNLKKALLLKGSNEIEIRAEGNSRFTYSVTVDGCTSHTGAWGKTVIEYKT TKSSRLPIIDVAPLDVGAPDQEFGFDVGPVCFL | Trimerized peptide (type I) with glycine-X-Y repeat and A→N mutation at the BMP-1 site, KS version |
| 75 | RSNGLPGPIGPPGPRGRTGDAGPVGPPGPPGPPGPPGPPSAGFDFSFLPQPPQEKAHDGGRYYRANDAN VVRDRDLEVDTTLKSLSQQIENIRSPEGSRKNPARTCRDLKMCHSDWKSGEYWIDPNQGCNLDAIKVFC NMETGETCVYPTQPSVAQKNWYISKNPKDKRHVWFGESMTDGFQFEYGGQGSDPADVAIQLTFLRLMST EASQNITYHCKNSVAYMDQQTGNLKKALLLKGSNEIEIRAEGNSRFTYSVTVDGCTSHTGAWGKTVIEY KTTKSSRLPIIDVAPLDVGAPDQEFGFDVGPVCFL | Trimerized peptide (type I) with glycine-X-Y repeat and D→N mutation at the BMP-1 site, KS version |

| SEQ ID NO. | Sequence | Description |
|---|---|---|
| 76 | GSNGLPGPIGPPGPRGRTGDAGPVGPPGPPGPPGPPGPPSAGFDFSFLPQPPQEKAHDGGRYYRANDAN VVRDRDLEVDTTLKSLSQQIENIRSPEGSRKNPARTCRDLKMCHSDWKSGEYWIDPNQGCNLDAIKVFC NMETGETCVYPTQPSVAQKNWYISKNPKDKRHVWFGESMTDGFQFEYGGQGSDPADVAIQLTFLRLMST EASQNITYHCKNSVAYMDQQTGNLKKALLLKGSNEIEIRAEGNSRFTYSVTVDGCTSHTGAWGKTVIEY KTTKSSRLPIIDVAPLDVGAPDQEFGFDVGPVCFL | Trimerized peptide (type I) with glycine-X-Y repeat and D→N mutation at the BMP-1 site, KS version |
| 77 | DEIMTSLKSVNGQIESLISPDGSRKNPARNCRDLKFCHPELKSGEYWVDPNQGCKLDAIKVFCNMETGE TCISANPLNVPRKHWWTDSSAEKKHVWFGESMDGGFQFSYGNPELPEDVLDVQLAFLRLLSSRASQNIT YHCKNSIAYMDQASGNVKKALKLMGSNEGEFKAEGNSKFTYTVLEDGCTKHTGEWSKTVFEYRTRKAVR LPIVDIAPYDIGGPDQEFGVDVGPVCF | Trimeric peptide (type III) |
| 78 | EPMDFKINTDEIMTSLKSVNGQIESLISPDGSRKNPARNCRDLKFCHPELKSGEYWVDPNQGCKLDAIK VFCNMETGETCISANPLNVPRKHWWTDSSAEKKHVWFGESMDGGFQFSYGNPELPEDVLDVQLAFLRLL SSRASQNITYHCKNSIAYMDQASGNVKKALKLMGSNEGEFKAEGNSKFTYTVLEDGCTKHTGEWSKTVF EYRTRKAVRLPIVDIAPYDIGGPDQEFGVDVGPVCFL | Trimeric peptide (type III) |
| 79 | SEPMDFKINTDEIMTSLKSVNGQIESLISPDGSRKNPARNCRDLKFCHPELKSGEYWVDPNQGCKLDAI KVFCNMETGETCISANPLNVPRKHWWTDSSAEKKHVWFGESMDGGFQFSYGNPELPEDVLDVQLAFLRL LSSRASQNITYHCKNSIAYMDQASGNVKKALKLMGSNEGEFKAEGNSKFTYTVLEDGCTKHTGEWSKTV FEYRTRKAVRLPIVDIAPYDIGGPDQEFGVDVGPVCFL | Trimeric peptide (type III) |
| 80 | RSEPMDFKINTDEIMTSLKSVNGQIESLISPDGSRKNPARNCRDLKFCHPELKSGEYWVDPNQGCKLDA IKVFCNMETGETCISANPLNVPRKHWWTDSSAEKKHVWFGESMDGGFQFSYGNPELPEDVLDVQLAFLR LLSSRASQNITYHCKNSIAYMDQASGNVKKALKLMGSNEGEFKAEGNSKFTYTVLEDGCTKHTGEWSKT VFEYRTRKAVRLPIVDIAPYDIGGPDQEFGVDVGPVCFL | Trimeric peptide (type III) |

| SEQ ID NO. | Sequence | Description |
|---|---|---|
| 81 | QCVNLTTRTQLPPAYTNSFTRGVYYPDKVFRSSVLHSTQDLFLPFFSNVTWFHVISGTNGTKRFDNPVL PFNDGVYFASIEKSNIIRGWIFGTTLDSKTQSLLIVNNATNVVIKVCEFQFCNDPFLDHKNNKSWMESE FRVYSSANNCTFEYVSQPFLMDLEGKQGNFKNLREFVFKNIDGYFKIYSKHTPIIVREPEDLPQGFSAL EPLVDLPIGINITRFQTLLALHRSYLTPGDSSSGWTAGAAAYYVGYLQPRTFLLKYNENGTITDAVDCA LDPLSETKCTLKSFTVEKGIYQTSNFRVQPTESIVRFPNITNLCPFDEVFNATKFASVYAWNRKRISNC VADYSVLYNLAPFFTFKCYGVSPTKLNDLCFTNVYADSFVIRGDEVRQIAPGQTGNIADYNYKLPDDFT GCVIAWNSNKLDSKVSGNYNYLYRLFRKSNLKPFERDISTEIYQAGNKPCNGVAGFNCYFPLRSYSFRP TYGVGHQPYRVVVLSFELLHAPATVCGPKKSTNLVKNKCVNFNFNGLKGTGVLTESNKKFLPFQQFGRD IADTTDAVRDPQTLEILDITPCSFGGVSVITPGTNTSNQVAVLYQGVNCTEVPVAIHADQLTPTWRVYS TGSNVFQTRAGCLIGAEYVNNSYECDIPIGAGICASYQTQTKSHRRARSVASQSIIAYTMSLGAENSVA YSNNSIAIPTNFTISVTTEILPVSMTKTSVDCTMYICGDSTECSNLLLQYGSFCTQLKRALTGIAVEQD KNTQEVFAQVKQIYKTPPIKYFGGFNFSQILPDPSKPSKRSFIEDLLFNKVTLADAGFIKQYGDCLGDI AARDLICAQKFKGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNHNAQALNTLVKQLSSKFGAISSVLNDIFS RLDKVEAEVQIDRLITGRLQSLQTYVTQQLIRAAEIRASANLAATKMSECVLGQSKRVDFCGKGYHLMS FPQSAPHGVVFLHVTYVPAQEKNFTTAPAICHDGKAHFPREGVFVSNGTHWFVTQRNFYEPQIITTDNT FVSGNCDVVIGIVNNTVYDPLQPELDSFKEELDKYFKNHTSPDVDLGDISGINASVVNIQKEIDRLNEV AKNLNESLIDLQELGKYEQYIK | SARS-CoV-2 Omicron spike protein extracellular domain without signal peptide |
| 82 | QCVNLTTRTQLPPAYTNSFTRGVYYPDKVFRSSVLHSTQDLFLPFFSNVTWFHVISGTNGTKRFDNPVL PFNDGVYFASIEKSNIIRGWIFGTTLDSKTQSLLIVNNATNVVIKVCEFQFCNDPFLDHKNNKSWMESE FRVYSSANNCTFEYVSQPFLMDLEGKQGNFKNLREFVFKNIDGYFKIYSKHTPIIVREPEDLPQGFSAL EPLVDLPIGINITRFQTLLALHRSYLTPGDSSSGWTAGAAAYYVGYLQPRTFLLKYNENGTITDAVDCA LDPLSETKCTLKSFTVEKGIYQTSNFRVQPTESIVRFPNITNLCPFDEVFNATKFASVYAWNRKRISNC VADYSVLYNLAPFFTFKCYGVSPTKLNDLCFTNVYADSFVIRGDEVRQIAPGQTGNIADYNYKLPDDFT GCVIAWNSNKLDSKVSGNYNYLYRLFRKSNLKPFERDISTEIYQAGNKPCNGVAGFNCYFPLRSYSFRP TYGVGHQPYRVVVLSFELLHAPATVCGPKKSTNLVKNKCVNFNFNGLKGTGVLTESNKKFLPFQQFGRD IADTTDAVRDPQTLEILDITPCSFGGVSVITPGTNTSNQVAVLYQGVNCTEVPVAIHADQLTPTWRVYS TGSNVFQTRAGCLIGAEYVNNSYECDIPIGAGICASYQTQTKSHRRAASVASQSIIAYTMSLGAENSVA YSNNSIAIPTNFTISVTTEILPVSMTKTSVDCTMYICGDSTECSNLLLQYGSFCTQLKRALTGIAVEQD KNTQEVFAQVKQIYKTPPIKYFGGFNFSQILPDPSKPSKRSFIEDLLFNKVTLADAGFIKQYGDCLGDI AARDLICAQKFKGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNHNAQALNTLVKQLSSKFGAISSVLNDIFS RLDKVEAEVQIDRLITGRLQSLQTYVTQQLIRAAEIRASANLAATKMSECVLGQSKRVDFCGKGYHLMS FPQSAPHGVVFLHVTYVPAQEKNFTTAPAICHDGKAHFPREGVFVSNGTHWFVTQRNFYEPQIITTDNT FVSGNCDVVIGIVNNTVYDPLQPELDSFKEELDKYFKNHTSPDVDLGDISGINASVVNIQKEIDRLNEV AKNLNESLIDLQELGKYEQYIK | SARS-CoV-2 Omicron spike protein extracellular domain without signal peptide, S1/S2 furin cleavage site 1 mutant (685R→685 A) |

(continued)

| SEQ ID NO. | Sequence | Description |
|---|---|---|
| 83 | QCVNLTTRTQLPPAYTNSFTRGVYYPDKVFRSSVLHSTQDLFLPFFSNVTWFHVISGTNGTKRFDNPVL PFNDGVYFASIEKSNIIRGWIFGTTLDSKTQSLLIVNNATNVVIKVCEFQFCNDPFLDHKNNKSWMESE FRVYSSANNCTFEYVSQPFLMDLEGKQGNFKNLREFVFKNIDGYFKIYSKHTPIIVREPEDLPQGFSAL EPLVDLPIGINITRFQTLLALHRSYLTPGDSSSGWTAGAAAYYVGYLQPRTFLLKYNENGTITDAVDCA LDPLSETKCTLKSFTVEKGIYQTSNFRVQPTESIVRFPNITNLCPFDEVFNATKFASVYAWNRKRISNC VADYSVLYNLAPFFTFKCYGVSPTKLNDLCFTNVYADSFVIRGDEVRQIAPGQTGNIADYNYKLPDDFT GCVIAWNSNKLDSKVSGNYNYLYRLFRKSNLKPFERDISTEIYQAGNKPCNGVAGFNCYFPLRSYSFRP TYGVGHQPYRVVVLSFELLHAPATVCGPKKSTNLVKNKCVNFNFNGLKGTGVLTESNKKFLPFQQFGRD IADTTDAVRDPQTLEILDITPCSFGGVSVITPGTNTSNQVAVLYQGVNCTEVPVAIHADQLTPTWRVYS TGSNVFQTRAGCLIGAEYVNNSYECDIPIGAGICASYQTQTKSHRRAASVASQSIIAYTMSLGAENSVA YSNNSIAIPTNFTISVTTEILPVSMTKTSVDCTMYICGDSTECSNLLLQYGSFCTQLKRALTGIAVEQD KNTQEVFAQVKQIYKTPPIKYFGGFNFSQILPDPSKPSKRSFIEDLLFNKVTLADAGFIKQYGDCLGDI AARDLICAQKFKGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNHNAQALNTLVKQLSSKFGAISSVLNDIFS RLDPPEAEVQIDRLITGRLQSLQTYVTQQLIRAAEIRASANLAATKMSECVLGQSKRVDFCGKGYHLMS FPQSAPHGVVFLHVTYVPAQEKNFTTAPAICHDGKAHFPREGVFVSNGTHWFVTQRNFYEPQIITTDNT FVSGNCDVVIGIVNNTVYDPLQPELDSFKEELDKYFKNHTSPDVDLGDISGINASVVNIQKEIDRLNEV AKNLNESLIDLQELGKYEQYIK | SARS-CoV-2 Omicron spike protein extracellular domain without signal peptide, S1/S2 furin cleavage site 1 and proline mutant (685R→685 A, 986K/987V →986P/987 P) |
| 84 | QCVNLTTRTQLPPAYTNSFTRGVYYPDKVFRSSVLHSTQDLFLPFFSNVTWFHVISGTNGTKRFDNPVL PFNDGVYFASIEKSNIIRGWIFGTTLDSKTQSLLIVNNATNVVIKVCEFQFCNDPFLDHKNNKSWMESE FRVYSSANNCTFEYVSQPFLMDLEGKQGNFKNLREFVFKNIDGYFKIYSKHTPIIVREPEDLPQGFSAL EPLVDLPIGINITRFQTLLALHRSYLTPGDSSSGWTAGAAAYYVGYLQPRTFLLKYNENGTITDAVDCA LDPLSETKCTLKSFTVEKGIYQTSNFRVQPTESIVRFPNITNLCPFDEVFNATKFASVYAWNRKRISNC VADYSVLYNLAPFFTFKCYGVSPTKLNDLCFTNVYADSFVIRGDEVRQIAPGQTGNIADYNYKLPDDFT GCVIAWNSNKLDSKVSGNYNYLYRLFRKSNLKPFERDISTEIYQAGNKPCNGVAGFNCYFPLRSYSFRP TYGVGHQPYRVVVLSFELLHAPATVCGPKKSTNLVKNKCVNFNFNGLKGTGVLTESNKKFLPFQQFGRD IADTTDAVRDPQTLEILDITPCSFGGVSVITPGTNTSNQVAVLYQGVNCTEVPVAIHADQLTPTWRVYS TGSNVFQTRAGCLIGAEYVNNSYECDIPIGAGICASYQTQTKSHRRARSVASQSIIAYTMSLGAENSVA YSNNSIAIPTNFTISVTTEILPVSMTKTSVDCTMYICGDSTECSNLLLQYGSFCTQLKRALTGIAVEQD KNTQEVFAQVKQIYKTPPIKYFGGFNFSQILPDPSKPSKRSFIEDLLFNKVTLADAGFIKQYGDCLGDI AARDLICAQKFKGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNHNAQALNTLVKQLSSKFGAISSVLNDIFS RLDPPEAEVQIDRLITGRLQSLQTYVTQQLIRAAEIRASANLAATKMSECVLGQSKRVDFCGKGYHLMS FPQSAPHGVVFLHVTYVPAQEKNFTTAPAICHDGKAHFPREGVFVSNGTHWFVTQRNFYEPQIITTDNT FVSGNCDVVIGIVNNTVYDPLQPELDSFKEELDKYFKNHTSPDVDLGDISGINASVVNIQKEIDRLNEV AKNLNESLIDLQELGKYEQYIK | SARS-CoV-2 Omicron spike protein extracellular domain without signal peptide, proline mutant (986K/987V →986P/987 P) |

(continued)

| SEQ ID NO. | Sequence | Description |
|---|---|---|
| 85 | QCVNLTTRTQLPPAYTNSFTRGVYYPDKVFRSSVLHSTQDLFLPFFSNVTWFHVISGTNGTKRFDNPVL PFNDGVYFASIEKSNIIRGWIFGTTLDSKTQSLLIVNNATNVVIKVCEFQFCNDPFLDHKNNKSWMESE FRVYSSANNCTFEYVSQPFLMDLEGKQGNFKNLREFVFKNIDGYFKIYSKHTPIIVREPEDLPQGFSAL EPLVDLPIGINITRFQTLLALHRSYLTPGDSSSGWTAGAAAYYVGYLQPRTFLLKYNENGTITDAVDCA LDPLSETKCTLKSFTVEKGIYQTSNFRVQPTESIVRFPNITNLCPFDEVFNATKFASVYAWNRKRISNC VADYSVLYNLAPFFTFKCYGVSPTKLNDLCFTNVYADSFVIRGDEVRQIAPGQTGNIADYNYKLPDDFT GCVIAWNSNKLDSKVSGNYNYLYRLFRKSNLKPFERDISTEIYQAGNKPCNGVAGFNCYFPLRSYSFRP TYGVGHQPYRVVVLSFELLHAPATVCGPKKSTNLVKNKCVNFNFNGLKGTGVLTESNKKFLPFQQFGRD IADTTDAVRDPQTLEILDITPCSFGGVSVITPGTNTSNQVAVLYQGVNCTEVPVAIHADQLTPTWRVYS TGSNVFQTRAGCLIGAEYVNNSYECDIPIGAGICASYQTQTKSHRRARSVASQSIIAYTMSLGAENSVA YSNNSIAIPTNFTISVTTEILPVSMTKTSVDCTMYICGDSTECSNLLLQYGSFCTQLKRALTGIAVEQD KNTQEVFAQVKQIYKTPPIKYFGGFNFSQILPDPSKPSKRSFIEDLLFNKVTLADAGFIKQYGDCLGDI AARDLICAQKFKGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNHNAQALNTIVKQLSSKFGAISSVLNDIFS RLDKVEAEVQIDRLITGRLQSLQTYVTQQLIRAAEIRASANLAATKMSECVLGQSKRVDFCGKGYHLMS FPQSAPHGVVFLHVTYVPAQEKNFTTAPAICHDGKAHFPREGVFVSNGTHWFVTQRNFYEPQIITTDNT FVSGNCDVVIGIVNNTVYDPLQPELDSFKEELDKYFKNHTSPDVDLGDISGINASVVNIQKEIDRLNEV AKNLNESLIDLQELGKYEQYIKRSNGLPGPIGPPGPRGRTGDAGPVGPVGPPGPPGPPSAGFDFSF LPQPPQEKAHDGGRYYRANDANVVRDRDLEVDTTLKSLSQQIENIRSPEGSRKNPARTCRDLKMCHSDW KSGEYWIDPNQGCNLDAIKVFCNMETGETCVYPTQPSVAQKNWYISKNPKDKRHVWFGESMTDGFQFEY GGQGSDPADVAIQLTFLRLMSTEASQNITYHCKNSVAYMDQQTGNLKKALLLQGSNEIEIRAEGNSRFT YSVTVDGCTSHTGAWGKTVIEYKTKTSRLPIIDVAPLDVGAPDQEFGFDVGPVCFL | SARS-CoV-2 Omicron S-trimer fusion polypeptide without signal peptide |

(continued)

| SEQ ID NO. | Sequence | Description |
|---|---|---|
| 86 | QCVNLTTRTQLPPAYTNSFTRGVYYPDKVFRSSVLHSTQDLFLPFFSNVTWFHVISGTNGTKRFDNPVL PFNDGVYFASIEKSNIIRGWIFGTTLDSKTQSLLIVNNATNVVIKVCEFQFCNDPFLDHKNNKSWMESE FRVYSSANNCTFEYVSQPFLMDLEGKQGNFKNLREFVFKNIDGYFKIYSKHTPIIVREPEDLPQGFSAL EPLVDLPIGINITRFQTLLALHRSYLTPGDSSSGWTAGAAAYYVGYLQPRTFLLKYNENGTITDAVDCA LDPLSETKCTLKSFTVEKGIYQTSNFRVQPTESIVRFPNITNLCPFDEVFNATKFASVYAWNRKRISNC VADYSVLYNLAPFFTFKCYGVSPTKLNDLCFTNVYADSFVIRGDEVRQIAPGQTGNIADYNYKLPDDFT GCVIAWNSNKLDSKVSGNYNYLYRLFRKSNLKPFERDISTEIYQAGNKPCNGVAGFNCYFPLRSYSFRP TYGVGHQPYRVVVLSFELLHAPATVCGPKKSTNLVKNKCVNFNFNGLKGTGVLTESNKKFLPFQQFGRD IADTTDAVRDPQTLEILDITPCSFGGVSVITPGTNTSNQVAVLYQGVNCTEVPVAIHADQLTPTWRVYS TGSNVFQTRAGCLIGAEYVNNSYECDIPIGAGICASYQTQTKSHRRAASVASQSIIAYTMSLGAENSVA YSNNSIAIPTNFTISVTTEILPVSMTKTSVDCTMYICGDSTECSNLLLQYGSFCTQLKRALTGIAVEQD KNTQEVFAQVKQIYKTPPIKYFGGFNFSQILPDPSKPSKRSFIEDLLFNKVTLADAGFIKQYGDCLGDI AARDLICAQKFKGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNHNAQALNTLVKQLSSKFGAISSVLNDIFS RLDKVEAEVQIDRLITGRLQSLQTYVTQQLIRAAEIRASANLAATKMSECVLGQSKRVDFCGKGYHLMS FPQSAPHGVVFLHVTYVPAQEKNFTTAPAICHDGKAHFPREGVFVSNGTHWFVTQRNFYEPQIITTDNT FVSGNCDVVIGIVNNTVYDPLQPELDSFKEELDKYFKNHTSPDVDLGDISGINASVVNIQKEIDRLNEV AKNLNESLIDLQELGKYEQYIKRSNGLPGPIGPPGPRGRTGDAGPVGPPGPPGPPGPPSAGFDFSF LPQPPQEKAHDGGRYYRANDANVVRDRDLEVDTTLKSLSQQIENIRSPEGSRKNPARTCRDLKMCHSDW KSGEYWIDPNQGCNLDAIKVFCNMETGETCVYPTQPSVAQKNWYISKNPKDKRHVWFGESMTDGFQFEY GGQGSDPADVAIQLTFLRLMSTEASQNITYHCKNSVAYMDQQTGNLKKALLLQGSNEIEIRAEGNSRFT YSVTVDGCTSHTGAWGKTVIEYKTTKTSRLPIIDVAPLDVGAPDQEFGFDVGPVCFL | SARS-CoV-2 Omicron S-trimer fusion polypeptide without signal peptide, S1/S2 furin cleavage site 1 mutant (685R→685 A) |

| SEQ ID NO. | Sequence | Description |
|---|---|---|
| 87 | QCVNLTTRTQLPPAYTNSFTRGVYYPDKVFRSSVLHSTQDLFLPFFSNVTWFHVISGTNGTKRFDNPVL<br>PFNDGVYFASIEKSNIIRGWIFGTTLDSKTQSLLIVNNATNVVIKVCEFQFCNDPFLDHKNNKSWMESE<br>FRVYSSANNCTFEYVSQPFLMDLEGKQGNFKNLREFVFKNIDGYFKIYSKHTPIIVREPEDLPQGFSAL<br>EPLVDLPIGINITRFQTLLALHRSYLTPGDSSSGWTAGAAAYYVGYLQPRTFLLKYNENGTITDAVDCA<br>LDPLSETKCTLKSFTVEKGIYQTSNFRVQPTESIVRFPNITNLCPFDEVFNATKFASVYAWNRKRISNC<br>VADYSVLYNLAPFFTFKCYGVSPTKLNDLCFTNVYADSFVIRGDEVRQIAPGQTGNIADYNYKLPDDFT<br>GCVIAWNSNKLDSKVSGNYNYLYRLFRKSNLKPFERDISTEIYQAGNKPCNGVAGFNCYFPLRSYSFRP<br>TYGVGHQPYRVVVLSFELLHAPATVCGPKKSTNLVKNKCVNFNFNGLKGTGVLTESNKKFLPFQQFGRD<br>IADTTDAVRDPQTLEILDITPCSFGGVSVITPGTNTSNQVAVLYQGVNCTEVPVAIHADQLTPWRVYS<br>TGSNVFQTRAGCLIGAEYVNNSYECDIPIGAGICASYQTQTKSHRRAASVASQSIIAYTMSLGAENSVA<br>YSNNSIAIPTNFTISVTTEILPVSMTKTSVDCTMYICGDSTECSNLLLQYGSFCTQLKRALTGIAVEQD<br>KNTQEVFAQVKQIYKTPPIKYFGGFNFSQILPDPSKPSKRSFIEDLLFNKVTLADAGFIKQYGDCLGDI<br>AARDLICAQKFKGLTVLPPLLTDEMIAQYTSALLAGTITSGWTFGAGAALQIPFAMQMAYRFNGIGVTQ<br>NVLYENQKLIANQFNSAIGKIQDSLSSTASALGKLQDVVNHNAQALNTLVKQLSSKFGAISSVLNDIFS<br>RLDPPEAEVQIDRLITGRLQSLQTYVTQQLIRAAEIRASANLAATKMSECVLGQSKRVDFCGKGYHLMS<br>FPQSAPHGVVFLHVTYVPAQEKNFTTAPAICHDGKAHFPREGVFVSNGTHWFVTQRNFYEPQIITTDNT<br>FVSGNCDVVIGIVNNTVYDPLQPELDSFKEELDKYFKNHTSPDVDLGDISGINASVVNIQKEIDRLNEV<br>AKNLNESLIDLQELGKYEQYIKRSNGLPGPIGPPGPRGRTGDAGPVGPPGPPGPPGPPGPPSAGFDFSF<br>LPQPPQEKAHDGGRYYRANDANVVRDRDLEVDTTLKSLSQQIENIRSPEGSRKNPARTCRDLKMCHSDW<br>KSGEYWIDPNQGCNLDAIKVFCNMETGETCVYPTQPSVAQKNWYISKNPKDKRHVWFGESMTDGFQFEY<br>GGQGSDPADVAIQLTFLRLMSTEASQNITYHCKNSVAYMDQQTGNLKKALLLQGSNEIEIRAEGNSRFT<br>YSVTVDGCTSHTGAWGKTVIEYKTTKTSRLPIIDVAPLDVGAPDQEFGFDVGPVCFL | SARS-CoV-2 Omicron S-trimer fusion polypeptide without signal peptide, S1/S2 furin cleavage site 1 and proline mutant (685R→685 A, 986K/987V →986P/987 P) |

EP 4 722 230 A1

| SEQ ID NO. | Sequence | Description |
|---|---|---|
| 88 | QCVNLTTRTQLPPAYTNSFTRGVYYPDKVFRSSVLHSTQDLFLPFFSNVTWFHVISGTNGTKRFDNPVL PFNDGVYFASIEKSNIIRGWIFGTTLDSKTQSLLIVNNATNVVIKVCEFQFCNDPFLDHKNNKSWMESE FRVYSSANNCTFEYVSQPFLMDLEGKQGNFKNLREFVFKNIDGYFKIYSKHTPIIVREPEDLPQGFSAL EPLVDLPIGINITRFQTLLALHRSYLTPGDSSSGWTAGAAAYYVGYLQPRTFLLKYNENGTITDAVDCA LDPLSETKCTLKSFTVEKGIYQTSNFRVQPTESIVRFPNITNLCPFDEVFNATKFASVYAWNRKRISNC VADYSVLYNLAPFFTFKCYGVSPTKLNDLCFTNVYADSFVIRGDEVRQIAPGQTGNIADYNYKLPDDFT GCVIAWNSNKLDSKVSGNYNYLYRLFRKSNLKPFERDISTEIYQAGNKPCNGVAGFNCYFPLRSYSFRP TYGVGHQPYRVVVLSFELLHAPATVCGPKKSTNLVKNKCVNFNFNGLKGTGVLTESNKKFLPFQQFGRD IADTTDAVRDPQTLEILDITPCSFGGVSVITPGTNTSNQVAVLYQGVNCTEVPVAIHADQLTPTWRVYS TGSNVFQTRAGCLIGAEYVNNSYECDIPIGAGICASYQTQTKSHRRARSVASQSIIAYTMSLGAENSVA YSNNSIAIPTNFTISVTTEILPVSMTKTSVDCTMYICGDSTECSNLLLQYGSFCTQLKRALTGIAVEQD KNTQEVFAQVKQIYKTPPIKYFGGFNFSQILPDPSKPSKRSFIEDLLFNKVTLADAGFIKQYGDCLGDI AARDLICAQKFKGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNHNAQALNTLVKQLSSKFGAISSVLNDIFS RLDPPEAEVQIDRLITGRLQSLQTYVTQQLIRAAEIRASANLAATKMSECVLGQSKRVDFCGKGYHLMS FPQSAPHGVVFLHVTYVPAQEKNFTTAPAICHDGKAHFPREGVFVSNGTHWFVTQRNFYEPQIITTDNT FVSGNCDVVIGIVNNTVYDPLQPELDSFKEELDKYFKNHTSPDVDLGDISGINASVVNIQKEIDRLNEV AKNLNESLIDLQELGKYEQYIKRSNGLPGPIGPPGPRGRTGDAGPVGPPGPPGPPGPPGPPSAGFDFSF LPQPPQEKAHDGGRYYRANDANVVRDRDLEVDTTLKSLSQQIENIRSPEGSRKNPARTCRDLKMCHSDW KSGEYWIDPNQGCNLDAIKVFCNMETGETCVYPTQPSVAQKNWYISKNPKDKRHVWFGESMTDGFQFEY GGQGSDPADVAIQLTFLRLMSTEASQNITYHCKNSVAYMDQQTGNLKKALLLQGSNEIEIRAEGNSRFT YSVTVDGCTSHTGAWGKTVIEYKTTKTSRLPIIDVAPLDVGAPDQEFGFDVGPVCFL | SARS-CoV-2 Omicron S-trimer fusion polypeptide without signal peptide, proline mutant (986K/987V →986P/987 P) |

EP 4 722 230 A1

| SEQ ID NO. | Sequence | Description |
|---|---|---|
| 89 | MFVFLVLLPLVSSQCVNLTTRTQLPPAYTNSFTRGVYYPDKVFRSSVLHSTQDLFLPFFSNVTWFHVIS GTNGTKRFDNPVLPFNDGVYFASIEKSNIIRGWIFGTTLDSKTQSLLIVNNATNVVIKVCEFQFCNDPF LDHKNNKSWMESEFRVYSSANNCTFEYVSQPFLMDLEGKQGNFKNLREFVFKNIDGYFKIYSKHTPIIV REPEDLPQGFSALEPLVDLPIGINITRFQTLLALHRSYLTPGDSSSGWTAGAAAYYVGYLQPRTFLLKY NENGTITDAVDCALDPLSETKCTLKSFTVEKGIYQTSNFRVQPTESIVRFPNITNLCPFDEVFNATKFA SVYAWNRKRISNCVADYSVLYNLAPFFTFKCYGVSPTKLNDLCFTNVYADSFVIRGDEVRQIAPGQTGN IADYNYKLPDDFTGCVIAWNSNKLDSKVSGNYNYLYRLFRKSNLKPFERDISTEIYQAGNKPCNGVAGF NCYFPLRSYSFRPTYGVGHQPYRVVVLSFELLHAPATVCGPKKSTNLVKNKCVNFNFNGLKGTGVLTES NKKFLPFQQFGRDIADTTDAVRDPQTLEILDITPCSFGGVSVITPGTNTSNQVAVLYQGVNCTEVPVAI HADQLTPTWRVYSTGSNVFQTRAGCLIGAEYVNNSYECDIPIGAGICASYQTQTKSHRRARSVASQSII AYTMSLGAENSVAYSNNSIAIPTNFTISVTTEILPVSMTKTSVDCTMYICGDSTECSNLLLQYGSFCTQ LKRALTGIAVEQDKNTQEVFAQVKQIYKTPPIKYFGGFNFSQILPDPSKPSKRSFIEDLLFNKVTLADA GFIKQYGDCLGDIAARDLICAQKFKGLTVLPPLLTDEMIAQYTSALLAGTITSGWTFGAGAALQIPFAM QMAYRFNGIGVTQNVLYENQKLIANQFNSAIGKIQDSLSSTASALGKLQDVVNHNAQALNTLVKQLSSK FGAISSVLNDIFSRLDKVEAEVQIDRLITGRLQSLQTYVTQQLIRAAEIRASANLAATKMSECVLGQSK RVDFCGKGYHLMSFPQSAPHGVVFLHVTYVPAQEKNFTTAPAICHDGKAHFPREGVFVSNGTHWFVTQR NFYEPQIITTDNTFVSGNCDVVIGIVNNTVYDPLQPELDSFKEELDKYFKNHTSPDVDLGDISGINASV VNIQKEIDRLNEVAKNLNESLIDLQELGKYEQYIKRSNGLPGPIGPPGPRGRTGDAGPVGPPGPPGPPG PPGPPSAGFDFSFLPQPPQEKAHDGGRYYRANDANVVRDRDLEVDTTLKSLSQQIENIRSPEGSRKNPA RTCRDLKMCHSDWKSGEYWIDPNQGCNLDAIKVFCNMETGETCVYPTQPSVAQKNWYISKNPKDKRHVW FGESMTDGFQFEYGGQGSDPADVAIQLTFLRLMSTEASQNITYHCKNSVAYMDQQTGNLKKALLLQGSN EIEIRAEGNSRFTYSVTVDGCTSHTGAWGKTVIEYKTTKTSRLPIIDVAPLDVGAPDQEFGFDVGPVCF L | SARS-CoV-2 Omicron S-trimer fusion polypeptide with signal peptide |

| SEQ ID NO. | Sequence | Description |
|---|---|---|
| 90 | MFVFLVLLPLVSSQCVNLTTRTQLPPAYTNSFTRGVYYPDKVFRSSVLHSTQDLFLPFFSNVTWFHVIS GTNGTKRFDNPVLPFNDGVYFASIEKSNIIRGWIFGTTLDSKTQSLLIVNNATNVVIKVCEFQFCNDPF LDHKNNKSWMESEFRVYSSANNCTFEYVSQPFLMDLEGKQGNFKNLREFVFKNIDGYFKIYSKHTPIIV REPEDLPQGFSALEPLVDLPIGINITRFQTLLALHRSYLTPGDSSSGWTAGAAAYYVGYLQPRTFLLKY NENGTITDAVDCALDPLSETKCTLKSFTVEKGIYQTSNFRVQPTESIVRFPNITNLCPFDEVFNATKFA SVYAWNRKRISNCVADYSVLYNLAPFFTFKCYGVSPTKLNDLCFTNVYADSFVIRGDEVRQIAPGQTGN IADYNYKLPDDFTGCVIAWNSNKLDSKVSGNYNYLYRLFRKSNLKPFERDISTEIYQAGNKPCNGVAGF NCYFPLRSYSFRPTYGVGHQPYRVVVLSFELLHAPATVCGPKKSTNLVKNKCVNFNFNGLKGTGVLTES NKKFLPFQQFGRDIADTTDAVRDPQTLEILDITPCSFGGVSVITPGTNTSNQVAVLYQGVNCTEVPVAI HADQLTPTWRVYSTGSNVFQTRAGCLIGAEYVNNSYECDIPIGAGICASYQTQTKSHRRAASVASQSII AYTMSLGAENSVAYSNNSIAIPTNFTISVTTEILPVSMTKTSVDCTMYICGDSTECSNLLLQYGSFCTQ LKRALTGIAVEQDKNTQEVFAQVKQIYKTPPIKYFGGFNFSQILPDPSKPSKRSFIEDLLFNKVTLADA GFIKQYGDCLGDIAARDLICAQKFKGLTVLPPLLTDEMIAQYTSALLAGTITSGWTFGAGAALQIPFAM QMAYRFNGIGVTQNVLYENQKLIANQFNSAIGKIQDSLSSTASALGKLQDVVNHNAQALNTLVKQLSSK FGAISSVLNDIFSRLDKVEAEVQIDRLITGRLQSLQTYVTQQLIRAAEIRASANLAATKMSECVLGQSK RVDFCGKGYHLMSFPQSAPHGVVFLHVTYVPAQEKNFTTAPAICHDGKAHFPREGVFVSNGTHWFVTQR NFYEPQIITTDNTFVSGNCDVVIGIVNNTVYDPLQPELDSFKEELDKYFKNHTSPDVDLGDISGINASV VNIQKEIDRLNEVAKNLNESLIDLQELGKYEQYIKRSNGLPGPIGPPGPRGRTGDAGPVGPPGPPGPPG PPGPPSAGFDFSFLPQPPQEKAHDGGRYYRANDANVVRDRDLEVDTTLKSLSQQIENIRSPEGSRKNPA RTCRDLKMCHSDWKSGEYWIDPNQGCNLDAIKVFCNMETGETCVYPTQPSVAQKNWYISKNPKDKRHVW FGESMTDGFQFEYGGQGSDPADVAIQLTFLRLMSTEASQNITYHCKNSVAYMDQQTGNLKKALLLQGSN EIEIRAEGNSRFTYSVTVDGCTSHTGAWGKTVIEYKTTKTSRLPIIDVAPLDVGAPDQEFGFDVGPVCF L | SARS-CoV-2 Omicron S-trimer fusion polypeptide with signal peptide, S1/S2 furin cleavage site 1 mutant (685R→685 A) |

| SEQ ID NO. | Sequence | Description |
|---|---|---|
| 91 | MFVFLVLLPLVSSQCVNLTTRTQLPPAYTNSFTRGVYYPDKVFRSSVLHSTQDLFLPFFSNVTWFHVIS GTNGTKRFDNPVLPFNDGVYFASIEKSNIIRGWIFGTTLDSKTQSLLIVNNATNVVIKVCEFQFCNDPF LDHKNNKSWMESEFRVYSSANNCTFEYVSQPFLMDLEGKQGNFKNLREFVFKNIDGYFKIYSKHTPIIV REPEDLPQGFSALEPLVDLPIGINITRFQTLLALHRSYLTPGDSSSGWTAGAAAYYVGYLQPRTFLLKY NENGTITDAVDCALDPLSETKCTLKSFTVEKGIYQTSNFRVQPTESIVRFPNITNLCPFDEVFNATKFA SVYAWNRKRISNCVADYSVLYNLAPFFTFKCYGVSPTKLNDLCFTNVYADSFVIRGDEVRQIAPGQTGN IADYNYKLPDDFTGCVIAWNSNKLDSKVSGNYNYLYRLFRKSNLKPFERDISTEIYQAGNKPCNGVAGF NCYFPLRSYSFRPTYGVGHQPYRVVVLSFELLHAPATVCGPKKSTNLVKNKCVNFNFNGLKGTGVLTES NKKFLPFQQFGRDIADTTDAVRDPQTLEILDITPCSFGGVSVITPGTNTSNQVAVLYQGVNCTEVPVAI HADQLTPTWRVYSTGSNVFQTRAGCLIGAEYVNNSYECDIPIGAGICASYQTQTKSHRRAASVASQSII AYTMSLGAENSVAYSNNSIAIPTNFTISVTTEILPVSMTKTSVDCTMYICGDSTECSNLLLQYGSFCTQ LKRALTGIAVEQDKNTQEVFAQVKQIYKTPPIKYFGGFNFSQILPDPSKPSKRSFIEDLLFNKVTLADA GFIKQYGDCLGDIAARDLICAQKFKGLTVLPPLLTDEMIAQYTSALLAGTITSGWTFGAGAALQIPFAM QMAYRFNGIGVTQNVLYENQKLIANQFNSAIGKIQDSLSSTASALGKLQDVVNHNAQALNTLVKQLSSK FGAISSVLNDIFSRLDPPEAEVQIDRLITGRLQSLQTYVTQQLIRAAEIRASANLAATKMSECVLGQSK RVDFCGKGYHLMSFPQSAPHGVVFLHVTYVPAQEKNFTTAPAICHDGKAHFPREGVFVSNGTHWFVTQR NFYEPQIITTDNTFVSGNCDVVIGIVNNTVYDPLQPELDSFKEELDKYFKNHTSPDVDLGDISGINASV VNIQKEIDRLNEVAKNLNESLIDLQELGKYEQYIKRSNLGPGIPGPIGPPGPRGRTGDAGPVGPPGPPG PPGPPSAGFDFSFLPQPPQEKAHDGGRYYRANDANVVRDRDLEVDTTLKSLSQQIENIRSPEGSRKNPA RTCRDLKMCHSDWKSGEYWIDPNQGCNLDAIKVFCNMETGETCVYPTQPSVAQKNWYISKNPKDKRHVW FGESMTDGFQFEYGGQGSDPADVAIQLTFLRLMSTEASQNITYHCKNSVAYMDQQTGNLKKALLLQGSN EIEIRAEGNSRFTYSVTVDGCTSHTGAWGKTVIEYKTTKTSRLPIIDVAPLDVGAPDQEFGFDVGPVCF L | SARS-CoV-2 Omicron S-trimer fusion polypeptide with signal peptide, S1/S2 furin cleavage site 1 and proline mutant (685R→685 A, 986K/987V →986P/987 P) |

| SEQ ID NO. | Sequence | Description |
|---|---|---|
| 92 | MFVFLVLLPLVSSQCVNLTTRTQLPPAYTNSFTRGVYYPDKVFRSSVLHSTQDLFLPFFSNVTWFHVIS GTNGTKRFDNPVLPFNDGVYFASIEKSNIIRGWIFGTTLDSKTQSLLIVNNATNVVIKVCEFQFCNDPF LDHKNNKSWMESEFRVYSSANNCTFEYVSQPFLMDLEGKQGNFKNLREFVFKNIDGYFKIYSKHTPIIV REPEDLPQGFSALEPLVDLPIGINITRFQTLLALHRSYLTPGDSSSGWTAGAAAYYVGYLQPRTFLLKY NENGTITDAVDCALDPLSETKCTLKSFTVEKGIYQTSNFRVQPTESIVRFPNITNLCPFDEVFNATKFA SVYAWNRKRISNCVADYSVLYNLAPFFTFKCYGVSPTKLNDLCFTNVYADSFVIRGDEVRQIAPGQTGN IADYNYKLPDDFTGCVIAWNSNKLDSKVSGNYNYLYRLFRKSNLKPFERDISTEIYQAGNKPCNGVAGF NCYFPLRSYSFRPTYGVGHQPYRVVVLSFELLHAPATVCGPKKSTNLVKNKCVNFNFNGLKGTGVLTES NKKFLPFQQFGRDIADTTDAVRDPQTLEILDITPCSFGGVSVITPGTNTSNQVAVLYQGVNCTEVPVAI HADQLTPTWRVYSTGSNVFQTRAGCLIGAEYVNNSYECDIPIGAGICASYQTQTKSHRRARSVASQSII AYTMSLGAENSVAYSNNSIAIPTNFTISVTTEILPVSMTKTSVDCTMYICGDSTECSNLLLQYGSFCTQ LKRALTGIAVEQDKNTQEVFAQVKQIYKTPPIKYFGGFNFSQILPDPSKPSKRSFIEDLLFNKVTLADA GFIKQYGDCLGDIAARDLICAQKFKGLTVLPPLLTDEMIAQYTSALLAGTITSGWTFGAGAALQIPFAM QMAYRFNGIGVTQNVLYENQKLIANQFNSAIGKIQDSLSSTASALGKLQDVVNHNAQALNTLVKQLSSK FGAISSVLNDIFSRLDPPEAEVQIDRLITGRLQSLQTYVTQQLIRAAEIRASANLAATKMSECVLGQSK RVDFCGKGYHLMSFPQSAPHGVVFLHVTYVPAQEKNFTTAPAICHDGKAHFPREGVFVSNGTHWFVTQR NFYEPQIITTDNTFVSGNCDVVIGIVNNTVYDPLQPELDSFKEELDKYFKNHTSPDVDLGDISGINASV VNIQKEIDRLNEVAKNLNESLIDLQELGKYEQYIKRSNGLPGPIGPPGPRGRTGDAGPVGPPGPPGPPG PPGPPSAGFDFSFLPQPPQEKAHDGGRYYRANDANVVRDRDLEVDTTLKSLSQQIENIRSPEGSRKNPA RTCRDLKMCHSDWKSGEYWIDPNQGCNLDAIKVFCNMETGETCVYPTQPSVAQKNWYISKNPKDKRHVW FGESMTDGFQFEYGGQGSDPADVAIQLTFLRLMSTEASQNITYHCKNSVAYMDQQTGNLKKALLLQGSN EIEIRAEGNSRFTYSVTVDGCTSHTGAWGKTVIEYKTTKTSRLPIIDVAPLDVGAPDQEFGFDVGPVCF L | SARS-CoV-2 Omicron S-trimer fusion polypeptide with signal peptide, proline mutant (986K/987V →986P/987 P) |

EP 4 722 230 A1

107

| SEQ ID NO. | Sequence | Description |
|---|---|---|
| 93 | QCVNFTTRTQLPPAYTNSFTRGVYYPDKVFRSSVLHSTQDLFLPFFSNVTWFHAIHVSGTNGTKRFANP<br>VLPFNDGVYFASTEKSNIIRGWIFGTTLDSKTQSLLIVNNATNVVIKVCEFQFCNDPFLGVYYHKNNKS<br>WMESEFRVYSSANNCTFEYVSQPFLMDLEGKQGNFKNLREFVFKNIDGYFKIYSKHTPINLVRGLPQGF<br>SALEPLVDLPIGINITRFQTLHISYLTPGDSSSGWTAGAAAYYVGYLQPRTFLLKYNENGTITDAVDCA<br>LDPLSETKCTLKSFTVEKGIYQTSNFRVQPTESIVRFPNITNLCPFGEVFNATRFASVYAWNRKRISNC<br>VADYSVLYNSASFSTFKCYGVSPTKLNDLCFTNVYADSFVIRGDEVRQIAPGQTGNIADYNYKLPDDFT<br>GCVIAWNSNNLDSKVGGNYNYLYRLFRKSNLKPFERDISTEIYQAGSTPCNGVKGFNCYFPLQSYGFQP<br>TYGVGYQPYRVVVLSFELLHAPATVCGPKKSTNLVKNKCVNFNFNGLTGTGVLTESNKKFLPFQQFGRD<br>IADTTDAVRDPQTLEILDITPCSFGGVSVITPGTNTSNQVAVLYQGVNCTEVPVAIHADQLTPTWRVYS<br>TGSNVFQTRAGCLIGAEHVNNSYECDIPIGAGICASYQTQTNSPRRARSVASQSIIAYTMSLGVENSVA<br>YSNNSIAIPTNFTISVTTEILPVSMTKTSVDCTMYICGDSTECSNLLLQYGSFCTQLNRALTGIAVEQD<br>KNTQEVFAQVKQIYKTPPIKDFGGFNFSQILPDPSKPSKRSFIEDLLFNKVTLADAGFIKQYGDCLGDI<br>AARDLICAQKFNGLTVLPPLLTDEMIAQYTSALLAGTITSGWTFGAGAALQIPFAMQMAYRFNGIGVTQ<br>NVLYENQKLIANQFNSAIGKIQDSLSSTASALGKLQDVVNQNAQALNTLVKQLSSNFGAISSVLNDILS<br>RLDKVEAEVQIDRLITGRLQSLQTYVTQQLIRAAEIRASANLAATKMSECVLGQSKRVDFCGKGYHLMS<br>FPQSAPHGVVFLHVTYVPAQEKNFTTAPAICHDGKAHFPREGVFVSNGTHWFVTQRNFYEPQIITTDNT<br>FVSGNCDVVIGIVNNTVYDPLQPELDSFKEELDKYFKNHTSPDVDLGDISGINASVVNIQKEIDRLNEV<br>AKNLNESLIDLQELGKYEQYIK | SARS-CoV-2 Beta spike protein extracellular domain without signal peptide |
| 94 | QCVNFTTRTQLPPAYTNSFTRGVYYPDKVFRSSVLHSTQDLFLPFFSNVTWFHAIHVSGTNGTKRFANP<br>VLPFNDGVYFASTEKSNIIRGWIFGTTLDSKTQSLLIVNNATNVVIKVCEFQFCNDPFLGVYYHKNNKS<br>WMESEFRVYSSANNCTFEYVSQPFLMDLEGKQGNFKNLREFVFKNIDGYFKIYSKHTPINLVRGLPQGF<br>SALEPLVDLPIGINITRFQTLHISYLTPGDSSSGWTAGAAAYYVGYLQPRTFLLKYNENGTITDAVDCA<br>LDPLSETKCTLKSFTVEKGIYQTSNFRVQPTESIVRFPNITNLCPFGEVFNATRFASVYAWNRKRISNC<br>VADYSVLYNSASFSTFKCYGVSPTKLNDLCFTNVYADSFVIRGDEVRQIAPGQTGNIADYNYKLPDDFT<br>GCVIAWNSNNLDSKVGGNYNYLYRLFRKSNLKPFERDISTEIYQAGSTPCNGVKGFNCYFPLQSYGFQP<br>TYGVGYQPYRVVVLSFELLHAPATVCGPKKSTNLVKNKCVNFNFNGLTGTGVLTESNKKFLPFQQFGRD<br>IADTTDAVRDPQTLEILDITPCSFGGVSVITPGTNTSNQVAVLYQGVNCTEVPVAIHADQLTPTWRVYS<br>TGSNVFQTRAGCLIGAEHVNNSYECDIPIGAGICASYQTQTNSPRRAASVASQSIIAYTMSLGVENSVA<br>YSNNSIAIPTNFTISVTTEILPVSMTKTSVDCTMYICGDSTECSNLLLQYGSFCTQLNRALTGIAVEQD<br>KNTQEVFAQVKQIYKTPPIKDFGGFNFSQILPDPSKPSKRSFIEDLLFNKVTLADAGFIKQYGDCLGDI<br>AARDLICAQKFNGLTVLPPLLTDEMIAQYTSALLAGTITSGWTFGAGAALQIPFAMQMAYRFNGIGVTQ<br>NVLYENQKLIANQFNSAIGKIQDSLSSTASALGKLQDVVNQNAQALNTLVKQLSSNFGAISSVLNDILS<br>RLDKVEAEVQIDRLITGRLQSLQTYVTQQLIRAAEIRASANLAATKMSECVLGQSKRVDFCGKGYHLMS<br>FPQSAPHGVVFLHVTYVPAQEKNFTTAPAICHDGKAHFPREGVFVSNGTHWFVTQRNFYEPQIITTDNT<br>FVSGNCDVVIGIVNNTVYDPLQPELDSFKEELDKYFKNHTSPDVDLGDISGINASVVNIQKEIDRLNEV<br>AKNLNESLIDLQELGKYEQYIK | SARS-CoV-2 Beta spike protein extracellular domain without signal peptide, S1/S2 furin cleavage site 1 mutant (685R→685 A) |

| SEQ ID NO. | Sequence | Description |
|---|---|---|
| 95 | QCVNFTTRTQLPPAYTNSFTRGVYYPDKVFRSSVLHSTQDLFLPFFSNVTWFHAIHVSGTNGTKRFANP<br>VLPFNDGVYFASTEKSNIIRGWIFGTTLDSKTQSLLIVNNATNVVIKVCEFQFCNDPFLGVYYHKNNKS<br>WMESEFRVYSSANNCTFEYVSQPFLMDLEGKQGNFKNLREFVFKNIDGYFKIYSKHTPINLVRGLPQGF<br>SALEPLVDLPIGINITRFQTLHISYLTPGDSSSGWTAGAAAYYVGYLQPRTFLLKYNENGTITDAVDCA<br>LDPLSETKCTLKSFTVEKGIYQTSNFRVQPTESIVRFPNITNLCPFGEVFNATRFASVYAWNRKRISNC<br>VADYSVLYNSASFSTFKCYGVSPTKLNDLCFTNVYADSFVIRGDEVRQIAPGQTGNIADYNYKLPDDFT<br>GCVIAWNSNNLDSKVGGNYNYLYRLFRKSNLKPFERDISTEIYQAGSTPCNGVKGFNCYFPLQSYGFQP<br>TYGVGYQPYRVVVLSFELLHAPATVCGPKKSTNLVKNKCVNFNFNGLTGTGVLTESNKKFLPFQQFGRD<br>IADTTDAVRDPQTLEILDITPCSFGGVSVITPGTNTSNQVAVLYQGVNCTEVPVAIHADQLTPTWRVYS<br>TGSNVFQTRAGCLIGAEHVNNSYECDIPIGAGICASYQTQTNSPRRAASVASQSIIAYTMSLGVENSVA<br>YSNNSIAIPTNFTISVTTEILPVSMTKTSVDCTMYICGDSTECSNLLLQYGSFCTQLNRALTGIAVEQD<br>KNTQEVFAQVKQIYKTPPIKDFGGFNFSQILPDPSKPSKRSFIEDLLFNKVTLADAGFIKQYGDCLGDI<br>AARDLICAQKFNGLTVLPPLLTDEMIAQYTSALLAGTITSGWTFGAGAALQIPFAMQMAYRFNGIGVTQ<br>NVLYENQKLIANQFNSAIGKIQDSLSSTASALGKLQDVVNQNAQALNTLVKQLSSNFGAISSVLNDILS<br>RLDPPEAEVQIDRLITGRLQSLQTYVTQQLIRAAEIRASANLAATKMSECVLGQSKRVDFCGKGYHLMS<br>FPQSAPHGVVFLHVTYVPAQEKNFTTAPAICHDGKAHFPREGVFVSNGTHWFVTQRNFYEPQIITTDNT<br>FVSGNCDVVIGIVNNTVYDPLQPELDSFKEELDKYFKNHTSPDVDLGDISGINASVVNIQKEIDRLNEV<br>AKNLNESLIDLQELGKYEQYIK | SARS-CoV-2 Beta spike protein extracellular domain without signal peptide, S1/S2 furin cleavage site 1 and proline mutant (685R→685 A, 986K/987V →986P/987 P) |
| 96 | QCVNFTTRTQLPPAYTNSFTRGVYYPDKVFRSSVLHSTQDLFLPFFSNVTWFHAIHVSGTNGTKRFANP<br>VLPFNDGVYFASTEKSNIIRGWIFGTTLDSKTQSLLIVNNATNVVIKVCEFQFCNDPFLGVYYHKNNKS<br>WMESEFRVYSSANNCTFEYVSQPFLMDLEGKQGNFKNLREFVFKNIDGYFKIYSKHTPINLVRGLPQGF<br>SALEPLVDLPIGINITRFQTLHISYLTPGDSSSGWTAGAAAYYVGYLQPRTFLLKYNENGTITDAVDCA<br>LDPLSETKCTLKSFTVEKGIYQTSNFRVQPTESIVRFPNITNLCPFGEVFNATRFASVYAWNRKRISNC<br>VADYSVLYNSASFSTFKCYGVSPTKLNDLCFTNVYADSFVIRGDEVRQIAPGQTGNIADYNYKLPDDFT<br>GCVIAWNSNNLDSKVGGNYNYLYRLFRKSNLKPFERDISTEIYQAGSTPCNGVKGFNCYFPLQSYGFQP<br>TYGVGYQPYRVVVLSFELLHAPATVCGPKKSTNLVKNKCVNFNFNGLTGTGVLTESNKKFLPFQQFGRD<br>IADTTDAVRDPQTLEILDITPCSFGGVSVITPGTNTSNQVAVLYQGVNCTEVPVAIHADQLTPTWRVYS<br>TGSNVFQTRAGCLIGAEHVNNSYECDIPIGAGICASYQTQTNSPRRARSVASQSIIAYTMSLGVENSVA<br>YSNNSIAIPTNFTISVTTEILPVSMTKTSVDCTMYICGDSTECSNLLLQYGSFCTQLNRALTGIAVEQD<br>KNTQEVFAQVKQIYKTPPIKDFGGFNFSQILPDPSKPSKRSFIEDLLFNKVTLADAGFIKQYGDCLGDI<br>AARDLICAQKFNGLTVLPPLLTDEMIAQYTSALLAGTITSGWTFGAGAALQIPFAMQMAYRFNGIGVTQ<br>NVLYENQKLIANQFNSAIGKIQDSLSSTASALGKLQDVVNQNAQALNTLVKQLSSNFGAISSVLNDILS<br>RLDPPEAEVQIDRLITGRLQSLQTYVTQQLIRAAEIRASANLAATKMSECVLGQSKRVDFCGKGYHLMS<br>FPQSAPHGVVFLHVTYVPAQEKNFTTAPAICHDGKAHFPREGVFVSNGTHWFVTQRNFYEPQIITTDNT<br>FVSGNCDVVIGIVNNTVYDPLQPELDSFKEELDKYFKNHTSPDVDLGDISGINASVVNIQKEIDRLNEV<br>AKNLNESLIDLQELGKYEQYIK | SARS-CoV-2 Beta spike protein extracellular domain without signal peptide, proline mutant (986K/987V→986P/987 P) |

EP 4 722 230 A1

109

| SEQ ID NO. | Sequence | Description |
|---|---|---|
| 97 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGNIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIK | Chimeric prototype (Hu-1) spike protein extracellular domain containing SARS-CoV-2 Beta (South African) variant RBD without signal peptide, S1/S2 furin cleavage site 1 and proline mutant (685R→685 A, 986K/987V →986P/987 P) |

| SEQ ID NO. | Sequence | Description |
|---|---|---|
| 98 | QCVNFTTRTQLPPAYTNSFTRGVYYPDKVFRSSVLHSTQDLFLPFFSNVTWFHAIHVSGTNGTKRFANP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRGLPQGF SALEPLVDLPIGINITRFQTLHISYLTPGDSSSGWTAGAAAYYVGYLQPRTFLLKYNENGTITDAVDCA LDPLSETKCTLKSFTVEKGIYQTSNFRVQPTESIVRFPNITNLCPFGEVFNATRFASVYAWNRKRISNC VADYSVLYNSASFSTFKCYGVSPTKLNDLCFTNVYADSFVIRGDEVRQIAPGQTGNIADYNYKLPDDFT GCVIAWNSNNLDSKVGGNYNYLYRLFRKSNLKPFERDISTEIYQAGSTPCNGVKGFNCYFPLQSYGFQP TYGVGYQPYRVVVLSFELLHAPATVCGPKKSTNLVKNKCVNFNFNGLTGTGVLTESNKKFLPFQQFGRD IADTTDAVRDPQTLEILDITPCSFGGVSVITPGTNTSNQVAVLYQGVNCTEVPVAIHADQLTPTWRVYS TGSNVFQTRAGCLIGAEHVNNSYECDIPIGAGICASYQTQTNSPRRARSVASQSIIAYTMSLGVENSVA YSNNSIAIPTNFTISVTTEILPVSMTKTSVDCTMYICGDSTECSNLLLQYGSFCTQLNRALTGIAVEQD KNTQEVFAQVKQIYKTPPIKDFGGFNFSQILPDPSKPSKRSFIEDLLFNKVTLADAGFIKQYGDCLGDI AARDLICAQKFNGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNQNAQALNTLVKQLSSNFGAISSVLNDILS RLDKVEAEVQIDRLITGRLQSLQTYVTQQLIRAAEIRASANLAATKMSECVLGQSKRVDFCGKGYHLMS FPQSAPHGVVFLHVTYVPAQEKNFTTAPAICHDGKAHFPREGVFVSNGTHWFVTQRNFYEPQIITTDNT FVSGNCDVVIGIVNNTVYDPLQPELDSFKEELDKYFKNHTSPDVDLGDISGINASVVNIQKEIDRLNEV AKNLNESLIDLQELGKYEQYIKRSNGLPGPIGPPGPRGRTGDAGPVGPPGPPGPPGPPGPPSAGFDFSF LPQPPQEKAHDGGRYYRANDANVVRDRDLEVDTTLKSLSQQIENIRSPEGSRKNPARTCRDLKMCHSDW KSGEYWIDPNQGCNLDAIKVFCNMETGETCVYPTQPSVAQKNWYISKNPKDKRHVWFGESMTDGFQFEY GGQGSDPADVAIQLTFLRLMSTEASQNITYHCKNSVAYMDQQTGNLKKALLLQGSNEIEIRAEGNSRFT YSVTVDGCTSHTGAWGKTVIEYKTTKTSRLPIIDVAPLDVGAPDQEFGFDVGPVCFL | SARS-CoV-2 Beta S-trimer fusion polypeptide without signal peptide |

| SEQ ID NO. | Sequence | Description |
|---|---|---|
| 99 | QCVNFTTRTQLPPAYTNSFTRGVYYPDKVFRSSVLHSTQDLFLPFFSNVTWFHAIHVSGTNGTKRFANP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRGLPQGF SALEPLVDLPIGINITRFQTLHISYLTPGDSSSGWTAGAAAYYVGYLQPRTFLLKYNENGTITDAVDCA LDPLSETKCTLKSFTVEKGIYQTSNFRVQPTESIVRFPNITNLCPFGEVFNATRFASVYAWNRKRISNC VADYSVLYNSASFSTFKCYGVSPTKLNDLCFTNVYADSFVIRGDEVRQIAPGQTGNIADYNYKLPDDFT GCVIAWNSNNLDSKVGGNYNYLYRLFRKSNLKPFERDISTEIYQAGSTPCNGVKGFNCYFPLQSYGFQP TYGVGYQPYRVVVLSFELLHAPATVCGPKKSTNLVKNKCVNFNFNGLTGTGVLTESNKKFLPFQQFGRD IADTTDAVRDPQTLEILDITPCSFGGVSVITPGTNTSNQVAVLYQGVNCTEVPVAIHADQLTPTWRVYS TGSNVFQTRAGCLIGAEHVNNSYECDIPIGAGICASYQTQTNSPRRAASVASQSIIAYTMSLGVENSVA YSNNSIAIPTNFTISVTTEILPVSMTKTSVDCTMYICGDSTECSNLLLQYGSFCTQLNRALTGIAVEQD KNTQEVFAQVKQIYKTPPIKDFGGFNFSQILPDPSKPSKRSFIEDLLFNKVTLADAGFIKQYGDCLGDI AARDLICAQKFNGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNQNAQALNTLVKQLSSNFGAISSVLNDILS RLDKVEAEVQIDRLITGRLQSLQTYVTQQLIRAAEIRASANLAATKMSECVLGQSKRVDFCGKGYHLMS FPQSAPHGVVFLHVTYVPAQEKNFTTAPAICHDGKAHFPREGVFVSNGTHWFVTQRNFYEPQIITTDNT FVSGNCDVVIGIVNNTVYDPLQPELDSFKEELDKYFKNHTSPDVDLGDISGINASVVNIQKEIDRLNEV AKNLNESLIDLQELGKYEQYIKRSNGLPGPIGPPGPRGRTGDAGPVGPPGPPGPPGPPGPPSAGFDFSF LPQPPQEKAHDGGRYYRANDANVVRDRDLEVDTTLKSLSQQIENIRSPEGSRKNPARTCRDLKMCHSDW KSGEYWIDPNQGCNLDAIKVFCNMETGETCVYPTQPSVAQKNWYISKNPKDKRHVWFGESMTDGFQFEY GGQGSDPADVAIQLTFLRLMSTEASQNITYHCKNSVAYMDQQTGNLKKALLLQGSNEIEIRAEGNSRFT YSVTVDGCTSHTGAWGKTVIEYKTTKTSRLPIIDVAPLDVGAPDQEFGFDVGPVCFL | SARS-CoV-2 Beta S-trimer fusion polypeptide without signal peptide, S1/S2 furin cleavage site 1 mutant (685R→685 A) |

| SEQ ID NO. | Sequence | Description |
|---|---|---|
| 100 | QCVNFTTRTQLPPAYTNSFTRGVYYPDKVFRSSVLHSTQDLFLPFFSNVTWFHAIHVSGTNGTKRFANP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRGLPQGF SALEPLVDLPIGINITRFQTLHISYLTPGDSSSGWTAGAAAYYVGYLQPRTFLLKYNENGTITDAVDCA LDPLSETKCTLKSFTVEKGIYQTSNFRVQPTESIVRFPNITNLCPFGEVFNATRFASVYAWNRKRISNC VADYSVLYNSASFSTFKCYGVSPTKLNDLCFTNVYADSFVIRGDEVRQIAPGQTGNIADYNYKLPDDFT GCVIAWNSNNLDSKVGGNYNYLYRLFRKSNLKPFERDISTEIYQAGSTPCNGVKGFNCYFPLQSYGFQP TYGVGYQPYRVVVLSFELLHAPATVCGPKKSTNLVKNKCVNFNFNGLTGTGVLTESNKKFLPFQQFGRD IADTTDAVRDPQTLEILDITPCSFGGVSVITPGTNTSNQVAVLYQGVNCTEVPVAIHADQLTPTWRVYS TGSNVFQTRAGCLIGAEHVNNSYECDIPIGAGICASYQTQTNSPRRAASVASQSIIAYTMSLGVENSVA YSNNSIAIPTNFTISVTTEILPVSMTKTSVDCTMYICGDSTECSNLLLQYGSFCTQLNRALTGIAVEQD KNTQEVFAQVKQIYKTPPIKDFGGFNFSQILPDPSKPSKRSFIEDLLFNKVTLADAGFIKQYGDCLGDI AARDLICAQKFNGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNQNAQALNTLVKQLSSNFGAISSVLNDILS RLDPPEAEVQIDRLITGRLQSLQTYVTQQLIRAAEIRASANLAATKMSECVLGQSKRVDFCGKGYHLMS FPQSAPHGVVFLHVTYVPAQEKNFTTAPAICHDGKAHFPREGVFVSNGTHWFVTQRNFYEPQIITTDNT FVSGNCDVVIGIVNNTVYDPLQPELDSFKEELDKYFKNHTSPDVDLGDISGINASVVNIQKEIDRLNEV AKNLNESLIDLQELGKYEQYIKRSNGLPGPIGPPGPRGRTGDAGPVGPPGPPGPPGPPGPPSAGFDFSF LPQPPQEKAHDGGRYYRANDANVVRDRDLEVDTTLKSLSQQIENIRSPEGSRKNPARTCRDLKMCHSDW KSGEYWIDPNQGCNLDAIKVFCNMETGETCVYPTQPSVAQKNWYISKNPKDKRHVWFGESMTDGFQFEY GGQGSDPADVAIQLTFLRLMSTEASQNITYHCKNSVAYMDQQTGNLKKALLLQGSNEIEIRAEGNSRFT YSVTVDGCTSHTGAWGKTVIEYKTTKTSRLPIIDVAPLDVGAPDQEFGFDVGPVCFL | SARS-CoV-2 Beta S-trimer fusion polypeptide without signal peptide, S1/S2 furin cleavage site 1 and proline mutant (685R→685 A, 986K/987V →986P/987 P) |

EP 4 722 230 A1

| SEQ ID NO. | Sequence | Description |
|---|---|---|
| 101 | QCVNFTTRTQLPPAYTNSFTRGVYYPDKVFRSSVLHSTQDLFLPFFSNVTWFHAIHVSGTNGTKRFANP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRGLPQGF SALEPLVDLPIGINITRFQTLHISYLTPGDSSSGWTAGAAAYYVGYLQPRTFLLKYNENGTITDAVDCA LDPLSETKCTLKSFTVEKGIYQTSNFRVQPTESIVRFPNITNLCPFGEVFNATRFASVYAWNRKRISNC VADYSVLYNSASFSTFKCYGVSPTKLNDLCFTNVYADSFVIRGDEVRQIAPGQTGNIADYNYKLPDDFT GCVIAWNSNNLDSKVGGNYNYLYRLFRKSNLKPFERDISTEIYQAGSTPCNGVKGFNCYFPLQSYGFQP TYGVGYQPYRVVVLSFELLHAPATVCGPKKSTNLVKNKCVNFNFNGLTGTGVLTESNKKFLPFQQFGRD IADTTDAVRDPQTLEILDITPCSFGGVSVITPGTNTSNQVAVLYQGVNCTEVPVAIHADQLTPTWRVYS TGSNVFQTRAGCLIGAEHVNNSYECDIPIGAGICASYQTQTNSPRRARSVASQSIIAYTMSLGVENSVA YSNNSIAIPTNFTISVTTEILPVSMTKTSVDCTMYICGDSTECSNLLLQYGSFCTQLNRALTGIAVEQD KNTQEVFAQVKQIYKTPPIKDFGGFNFSQILPDPSKPSKRSFIEDLLFNKVTLADAGFIKQYGDCLGDI AARDLICAQKFNGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNQNAQALNTLVKQLSSNFGAISSVLNDILS RLDPPEAEVQIDRLITGRLQSLQTYVTQQLIRAAEIRASANLAATKMSECVLGQSKRVDFCGKGYHLMS FPQSAPHGVVFLHVTYVPAQEKNFTTAPAICHDGKAHFPREGVFVSNGTHWFVTQRNFYEPQIITTDNT FVSGNCDVVIGIVNNTVYDPLQPELDSFKEELDKYFKNHTSPDVDLGDISGINASVVNIQKEIDRLNEV AKNLNESLIDLQELGKYEQYIKRSNGLPGPIGPPGPRGRTGDAGPVGPPGPPGPPGPPGPPSAGFDFSF LPQPPQEKAHDGGRYYRANDANVVRDRDLEVDTTLKSLSQQIENIRSPEGSRKNPARTCRDLKMCHSDW KSGEYWIDPNQGCNLDAIKVFCNMETGETCVYPTQPSVAQKNWYISKNPKDKRHVWFGESMTDGFQFEY GGQGSDPADVAIQLTFLRLMSTEASQNITYHCKNSVAYMDQQTGNLKKALLLQGSNEIEIRAEGNSRFT YSVTVDGCTSHTGAWGKTVIEYKTTKTSRLPIIDVAPLDVGAPDQEFGFDVGPVCFL | SARS-CoV-2 Beta S-trimer fusion polypeptide without signal peptide, proline mutant (986K/987V →986P/987 P) |

| SEQ ID NO. | Sequence | Description |
|---|---|---|
| 102 | QCVNLTTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLGVYYHKNNKS WMESEFRVYSSANNCTFEYVSQPFLMDLEGKQGNFKNLREFVFKNIDGYFKIYSKHTPINLVRDLPQGF SALEPLVDLPIGINITRFQTLLALHRSYLTPGDSSSGWTAGAAAYYVGYLQPRTFLLKYNENGTITDAV DCALDPLSETKCTLKSFTVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQIAPGQTGNIADYNYKLPD DFTGCVIAWNSNNLDSKVGGNYNYLYRLFRKSNLKPFERDISTEIYQAGSTPCNGVKGFNCYFPLQSYG FQPTYGVGYQPYRVVVLSFELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGVNCTEVPVAIHADQLTPTWR VYSTGSNVFQTRAGCLIGAEHVNNSYECDIPIGAGICASYQTQTNSPRRAASVASQSIIAYTMSLGAEN SVAYSNNSIAIPTNFTISVTTEILPVSMTKTSVDCTMYICGDSTECSNLLLQYGSFCTQLNRALTGIAV EQDKNTQEVFAQVKQIYKTPPIKDFGGFNFSQILPDPSKPSKRSFIEDLLFNKVTLADAGFIKQYGDCL GDIAARDLICAQKFNGLTVLPPLLTDEMIAQYTSALLAGTITSGWTFGAGAALQIPFAMQMAYRFNGIG VTQNVLYENQKLIANQFNSAIGKIQDSLSSTASALGKLQDVVNQNAQALNTLVKQLSSNFGAISSVLND ILSRLDPPEAEVQIDRLITGRLQSLQTYVTQQLIRAAEIRASANLAATKMSECVLGQSKRVDFCGKGYH LMSFPQSAPHGVVFLHVTYVPAQEKNFTTAPAICHDGKAHFPREGVFVSNGTHWFVTQRNFYEPQIITT DNTFVSGNCDVVIGIVNNTVYDPLQPELDSFKEELDKYFKNHTSPDVDLGDISGINASVVNIQKEIDRL NEVAKNLNESLIDLQELGKYEQYIKRSNGLPGPIGPPGPRGRTGDAGPVGPPGPPGPPGPPGPPSAGFD FSFLPQPPQEKAHDGGRYYRANDANVVRDRDLEVDTTLKSLSQQIENIRSPEGSRKNPARTCRDLKMCH SDWKSGEYWIDPNQGCNLDAIKVFCNMETGETCVYPTQPSVAQKNWYISKNPKDKRHVWFGESMTDGFQ FEYGGQGSDPADVAIQLTFLRLMSTEASQNITYHCKNSVAYMDQQTGNLKKALLLQGSNEIEIRAEGNS RFTYSVTVDGCTSHTGAWGKTVIEYKTTKTSRLPIIDVAPLDVGAPDQEFGFDVGPVCFL | Chimeric prototype (Hu-1) S-trimer fusion polypeptide containing SARS-CoV-2 Beta (South African) variant RBD without signal peptide, S1/S2 furin cleavage site 1 and proline mutants (685R→685 A, 986K/987V →986P/987 P) |

115

| SEQ ID NO. | Sequence | Description |
|---|---|---|
| 103 | MFVFLVLLPLVSSQCVNFTTRTQLPPAYTNSFTRGVYYPDKVFRSSVLHSTQDLFLPFFSNVTWFHAIH VSGTNGTKRFANPVLPFNDGVYFASTEKSNIIRGWIFGTTLDSKTQSLLIVNNATNVVIKVCEFQFCND PFLGVYYHKNNKSWMESEFRVYSSANNCTFEYVSQPFLMDLEGKQGNFKNLREFVFKNIDGYFKIYSKH TPINLVRGLPQGFSALEPLVDLPIGINITRFQTLHISYLTPGDSSSGWTAGAAAYYVGYLQPRTFLLKY NENGTITDAVDCALDPLSETKCTLKSFTVEKGIYQTSNFRVQPTESIVRFPNITNLCPFGEVFNATRFA SVYAWNRKRISNCVADYSVLYNSASFSTFKCYGVSPTKLNDLCFTNVYADSFVIRGDEVRQIAPGQTGN IADYNYKLPDDFTGCVIAWNSNNLDSKVGGNYNYLYRLFRKSNLKPFERDISTEIYQAGSTPCNGVKGF NCYFPLQSYGFQPTYGVGYQPYRVVVLSFELLHAPATVCGPKKSTNLVKNKCVNFNFNGLTGTGVLTES NKKFLPFQQFGRDIADTTDAVRDPQTLEILDITPCSFGGVSVITPGTNTSNQVAVLYQGVNCTEVPVAI HADQLTPTWRVYSTGSNVFQTRAGCLIGAEHVNNSYECDIPIGAGICASYQTQTNSPRRARSVASQSII AYTMSLGVENSVAYSNNSIAIPTNFTISVTTEILPVSMTKTSVDCTMYICGDSTECSNLLLQYGSFCTQ LNRALTGIAVEQDKNTQEVFAQVKQIYKTPPIKDFGGFNFSQILPDPSKPSKRSFIEDLLFNKVTLADA GFIKQYGDCLGDIAARDLICAQKFNGLTVLPPLLTDEMIAQYTSALLAGTITSGWTFGAGAALQIPFAM QMAYRFNGIGVTQNVLYENQKLIANQFNSAIGKIQDSLSSTASALGKLQDVVNQNAQALNTLVKQLSSN FGAISSVLNDILSRLDKVEAEVQIDRLITGRLQSLQTYVTQQLIRAAEIRASANLAATKMSECVLGQSK RVDFCGKGYHLMSFPQSAPHGVVFLHVTYVPAQEKNFTTAPAICHDGKAHFPREGVFVSNGTHWFVTQR NFYEPQIITTDNTFVSGNCDVVIGIVNNTVYDPLQPELDSFKEELDKYFKNHTSPDVDLGDISGINASV VNIQKEIDRLNEVAKNLNESLIDLQELGKYEQYIKRSNGLPGPIGPPGPRGRTGDAGPVGPPGPPGPPG PPGPPSAGFDFSFLPQPPQEKAHDGGRYYRANDANVVRDRDLEVDTTLKSLSQQIENIRSPEGSRKNPA RTCRDLKMCHSDWKSGEYWIDPNQGCNLDAIKVFCNMETGETCVYPTQPSVAQKNWYISKNPKDKRHVW FGESMTDGFQFEYGGQGSDPADVAIQLTFLRLMSTEASQNITYHCKNSVAYMDQQTGNLKKALLLQGSN EIEIRAEGNSRFTYSVTVDGCTSHTGAWGKTVIEYKTTKTSRLPIIDVAPLDVGAPDQEFGFDVGPVCF L | SARS-CoV-2 Beta S-trimer fusion polypeptide with signal peptide |

(continued)

| SEQ ID NO. | Sequence | Description |
|---|---|---|
| 104 | MFVFLVLLPLVSSQCVNFTTRTQLPPAYTNSFTRGVYYPDKVFRSSVLHSTQDLFLPFFSNVTWFHAIH VSGTNGTKRFANPVLPFNDGVYFASTEKSNIIRGWIFGTTLDSKTQSLLIVNNATNVVIKVCEFQFCND PFLGVYYHKNNKSWMESEFRVYSSANNCTFEYVSQPFLMDLEGKQGNFKNLREFVFKNIDGYFKIYSKH TPINLVRGLPQGFSALEPLVDLPIGINITRFQTLHISYLTPGDSSSGWTAGAAAYYVGYLQPRTFLLKY NENGTITDAVDCALDPLSETKCTLKSFTVEKGIYQTSNFRVQPTESIVRFPNITNLCPFGEVFNATRFA SVYAWNRKRISNCVADYSVLYNSASFSTFKCYGVSPTKLNDLCFTNVYADSFVIRGDEVRQIAPGQTGN IADYNYKLPDDFTGCVIAWNSNNLDSKVGGNYNYLYRLFRKSNLKPFERDISTEIYQAGSTPCNGVKGF NCYFPLQSYGFQPTYGVGYQPYRVVVLSFELLHAPATVCGPKKSTNLVKNKCVNFNFNGLTGTGVLTES NKKFLPFQQFGRDIADTTDAVRDPQTLEILDITPCSFGGVSVITPGTNTSNQVAVLYQGVNCTEVPVAI HADQLTPTWRVYSTGSNVFQTRAGCLIGAEHVNNSYECDIPIGAGICASYQTQTNSPRRAASVASQSII AYTMSLGVENSVAYSNNSIAIPTNFTISVTTEILPVSMTKTSVDCTMYICGDSTECSNLLLQYGSFCTQ LNRALTGIAVEQDKNTQEVFAQVKQIYKTPPIKDFGGFNFSQILPDPSKPSKRSFIEDLLFNKVTLADA GFIKQYGDCLGDIAARDLICAQKFNGLTVLPPLLTDEMIAQYTSALLAGTITSGWTFGAGAALQIPFAM QMAYRFNGIGVTQNVLYENQKLIANQFNSAIGKIQDSLSSTASALGKLQDVVNQNAQALNTLVKQLSSN FGAISSVLNDILSRLDKVEAEVQIDRLITGRLQSLQTYVTQQLIRAAEIRASANLAATKMSECVLGQSK RVDFCGKGYHLMSFPQSAPHGVVFLHVTYVPAQEKNFTTAPAICHDGKAHFPREGVFVSNGTHWFVTQR NFYEPQIITTDNTFVSGNCDVVIGIVNNTVYDPLQPELDSFKEELDKYFKNHTSPDVLGDISGINASV VNIQKEIDRLNEVAKNLNESLIDLQELGKYEQYIKRSNGLPGPIGPPGPRGRTGDAGPVGPPGPGPPG PPGPPSAGFDFSFLPQPPQEKAHDGGRYYRANDANVVRDRDLEVDTTLKSLSQQIENIRSPEGSRKNPA RTCRDLKMCHSDWKSGEYWIDENQGCNLDAIKVFCNMETGETCVYPTQPSVAQKNWYISKNPKDKRHVM FGESMTDGFQFEYGGQGSDPADVAIQLTFLRLMSTEASQNITYHCKNSVAYMDQQTGNLKKALLLQGSN EIEIRAEGNSRFTYSVTVDGCTSHTGAWGKTVIEYKTTKTSRLPIIDVAPLDVGAPDQEFGFDVGPVCF L | SARS-CoV-2 Beta S-trimer fusion polypeptide with signal peptide, S1/S2 furin cleavage site 1 mutant (685R→685 A) |

117

| SEQ ID NO. | Sequence | Description |
|---|---|---|
| 105 | MFVFLVLLPLVSSQCVNFTTRTQLPPAYTNSFTRGVYYPDKVFRSSVLHSTQDLFLPFFSNVTWFHAIH VSGTNGTKRFANPVLPFNDGVYFASTEKSNIIRGWIFGTTLDSKTQSLLIVNNATNVVIKVCEFQFCND PFLGVYYHKNNKSWMESEFRVYSSANNCTFEYVSQPFLMDLEGKQGNFKNLREFVFKNIDGYFKIYSKH TPINLVRGLPQGFSALEPLVDLPIGINITRFQTLHISYLTPGDSSSGWTAGAAAYYVGYLQPRTFLLKY NENGTITDAVDCALDPLSETKCTLKSFTVEKGIYQTSNFRVQPTESIVRFPNITNLCPFGEVFNATRFA SVYAWNRKRISNCVADYSVLYNSASFSTFKCYGVSPTKLNDLCFTNVYADSFVIRGDEVRQIAPGQTGN IADYNYKLPDDFTGCVIAWNSNNLDSKVGGNYNYLYRLFRKSNLKPFERDISTEIYQAGSTPCNGVKGF NCYFPLQSYGFQPTYGVGYQPYRVVVLSFELLHAPATVCGPKKSTNLVKNKCVNFNFNGLTGTGVLTES NKKFLPFQQFGRDIADTTDAVRDPQTLEILDITPCSFGGVSVITPGTNTSNQVAVLYQGVNCTEVPVAI HADQLTPTWRVYSTGSNVFQTRAGCLIGAEHVNNSYECDIPIGAGICASYQTQTNSPRRAASVASQSII AYTMSLGVENSVAYSNNSIAIPTNFTISVTTEILPVSMTKTSVDCTMYICGDSTECSNLLLQYGSFCTQ LNRALTGIAVEQDKNTQEVFAQVKQIYKTPPIKDFGGFNFSQILPDPSKPSKRSFIEDLLFNKVTLADA GFIKQYGDCLGDIAARDLICAQKFNGLTVLPPLLTDEMIAQYTSALLAGTITSGWTFGAGAALQIPFAM QMAYRFNGIGVTQNVLYENQKLIANQFNSAIGKIQDSLSSTASALGKLQDVVNQNAQALNTLVKQLSSN FGAISSVLNDILSRLDPPEAEVQIDRLITGRLQSLQTYVTQQLIRAAEIRASANLAATKMSECVLGQSK RVDFCGKGYHLMSFPQSAPHGVVFLHVTYVPAQEKNFTTAPAICHDGKAHFPREGVFVSNGTHWFVTQR NFYEPQIITTDNTFVSGNCDVVIGIVNNTVYDPLQPELDSFKEELDKYFKNHTSPDVDLGDISGINASV VNIQKEIDRLNEVAKNLNESLIDLQELGKYEQYIKRSNGLPGPIGPPGPRGRTGDAGPVGPPGPPGPPG PPGPPSAGFDFSFLPQPPQEKAHDGGRYYRANDANVVRDRDLEVDTTLKSLSQQIENIRSPEGSRKNPA RTCRDLKMCHSDWKSGEYWIDPNQGCNLDAIKVFCNMETGETCVYPTQPSVAQKNWYISKNPKDKRHVW FGESMTDGFQFEYGGQGSDPADVAIQLTFLRLMSTEASQNITYHCKNSVAYMDQQTGNLKKALLQGSN EIEIRAEGNSRFTYSVTVDGCTSHTGAWGKTVIEYKTTKTSRLPIIDVAPLDVGAPDQEFGFDVGPVCF L | SARS-CoV-2 Beta S-trimer fusion polypeptide with signal peptide, S1/S2 furin cleavage site 1 and proline mutant (685R→685 A, 986K/987V→986P/987 P) |

(continued)

| SEQ ID NO. | Sequence | Description |
|---|---|---|
| 106 | MFVFLVLLPLVSSQCVNFTTRTQLPPAYTNSFTRGVYYPDKVFRSSVLHSTQDLFLPFFSNVTWFHAIH VSGTNGTKRFANPVLPFNDGVYFASTEKSNIIRGWIFGTTLDSKTQSLLIVNNATNVVIKVCEFQFCND PFLGVYYHKNNKSWMESEFRVYSSANNCTFEYVSQPFLMDLEGKQGNFKNLREFVFKNIDGYFKIYSKH TPINLVRGLPQGFSALEPLVDLPIGINITRFQTLHISYLTPGDSSSGWTAGAAAYYVGYLQPRTFLLKY NENGTITDAVDCALDPLSETKCTLKSFTVEKGIYQTSNFRVQPTESIVRFPNITNLCPFGEVFNATRFA SVYAWNRKRISNCVADYSVLYNSASFSTFKCYGVSPTKLNDLCFTNVYADSFVIRGDEVRQIAPGQTGN IADYNYKLPDDFTGCVIAWNSNNLDSKVGGNYNYLYRLFRKSNLKPFERDISTEIYQAGSTPCNGVKGF NCYFPLQSYGFQPTYGVGYQPYRVVVLSFELLHAPATVCGPKKSTNLVKNKCVNFNFNGLTGTGVLTES NKKFLPFQQFGRDIADTTDAVRDPQTLEILDITPCSFGGVSVITPGTNTSNQVAVLYQGVNCTEVPVAI HADQLTPTWRVYSTGSNVFQTRAGCLIGAEHVNNSYECDIPIGAGICASYQTQTNSPRRARSVASQSII AYTMSLGVENSVAYSNNSIAIPTNFTISVTTEILPVSMTKTSVDCTMYICGDSTECSNLLLQYGSFCTQ LNRALTGIAVEQDKNTQEVFAQVKQIYKTPPIKDFGGFNFSQILPDPSKPSKRSFIEDLLFNKVTLADA GFIKQYGDCLGDIAARDLICAQKFNGLTVLPPLLTDEMIAQYTSALLAGTITSGWTFGAGAALQIPFAM QMAYRFNGIGVTQNVLYENQKLIANQFNSAIGKIQDSLSSTASALGKLQDVVNQNAQALNTLVKQLSSN FGAISSVLNDILSRLDPPEAEVQIDRLITGRLQSLQTYVTQQLIRAAEIRASANLAATKMSECVLGQSK RVDFCGKGYHLMSFPQSAPHGVVFLHVTYVPAQEKNFTTAPAICHDGKAHFPREGVFVSNGTHWFVTQR NFYEPQIITTDNTFVSGNCDVVIGIVNNTVYDPLQPELDSFKEELDKYFKNHTSPDVDLGDISGINASV VNIQKEIDRLNEVAKNLNESLIDLQELGKYEQYIKRSNGLPGPIGPPGPRGRTGDAGPVGPPGPPGPPG PPGPPSAGFDFSFLPQPPQEKAHDGGRYYRANDANVVRDRDLEVDTTLKSLSQQIENIRSPEGSRKNPA RTCRDLKMCHSDWKSGEYWIDPNQGCNLDAIKVFCNMETGETCVYPTQPSVAQKNWYISKNPKDKRHVW FGESMTDGFQFEYGGQGSDPADVAIQLTFLRLMSTEASQNITYHCKNSVAYMDQQTGNLKKALLLQGSN EIEIRAEGNSRFTYSVTVDGCTSHTGAWGKTVIEYKTTKTSRLPIIDVAPLDVGAPDQEFGFDVGPVCF L | SARS-CoV-2 Beta S-trimer fusion polypeptide with signal peptide, proline mutant (986K/987V →986P/987 P) |

EP 4 722 230 A1

119

| SEQ ID NO. | Sequence | Description |
|---|---|---|
| 107 | MFVFLVLLPLVSSQCVNLTTRTQLPPAYTNSFTRGVYYPDKVFRSSVLHSTQDLFLPFFSNVTWFHAIH<br>VSGTNGTKRFDNPVLPFNDGVYFASTEKSNIIRGWIFGTTLDSKTQSLLIVNNATNVVIKVCEFQFCND<br>PFLGVYYHKNNKSWMESEFRVYSSANNCTFEYVSQPFLMDLEGKQGNFKNLREFVFKNIDGYFKIYSKH<br>TPINLVRDLPQGFSALEPLVDLPIGINITRFQTLLALHRSYLTPGDSSSGWTAGAAAYYVGYLQPRTFL<br>LKYNENGTITDAVDCALDPLSETKCTLKSFTVEKGIYQTSNFRVQPTESIVRFPNITNLCPFGEVFNAT<br>RFASVYAWNRKRISNCVADYSVLYNSASFSTFKCYGVSPTKLNDLCFTNVYADSFVIRGDEVRQIAPGQ<br>TGNIADYNYKLPDDFTGCVIAWNSNNLDSKVGGNYNYLYRLFRKSNLKPFERDISTEIYQAGSTPCNGV<br>KGFNCYFPLQSYGFQPTYGVGYQPYRVVVLSFELLHAPATVCGPKKSTNLVKNKCVNFNFNGLTGTGVL<br>TESNKKFLPFQQFGRDIADTTDAVRDPQTLEILDITPCSFGGVSVITPGTNTSNQVAVLYQGVNCTEVP<br>VAIHADQLTPTWRVYSTGSNVFQTRAGCLIGAEHVNNSYECDIPIGAGICASYQTQTNSPRRAASVASQ<br>SIIAYTMSLGAENSVAYSNNSIAIPTNFTISVTTEILPVSMTKTSVDCTMYICGDSTECSNLLLQYGSF<br>CTQLNRALTGIAVEQDKNTQEVFAQVKQIYKTPPIKDFGGFNFSQILPDPSKPSKRSFIEDLLFNKVTL<br>ADAGFIKQYGDCLGDIAARDLICAQKFNGLTVLPPLLTDEMIAQYTSALLAGTITSGWTFGAGAALQIP<br>FAMQMAYRFNGIGVTQNVLYENQKLIANQFNSAIGKIQDSLSSTASALGKLQDVVNQNAQALNTLVKQL<br>SSNFGAISSVLNDILSRLDPPEAEVQIDRLITGRLQSLQTYVTQQLIRAAEIRASANLAATKMSECVLG<br>QSKRVDFCGKGYHLMSFPQSAPHGVVFLHVTYVPAQEKNFTTAPAICHDGKAHFPREGVFVSNGTHWFV<br>TQRNFYEPQIITTDNTFVSGNCDVVIGIVNNTVYDPLQPELDSFKEELDKYFKNHTSPDVDLGDISGIN<br>ASVVNIQKEIDRLNEVAKNLNESLIDLQELGKYEQYIKRSNGLPGPIGPPGPRGRTGDAGPVGPPGPPG<br>PPGPPGPPSAGFDFSFLPQPPQEKAHDGGRYYRANDANVVRDRDLEVDTTLKSLSQQIENIRSPEGSRK<br>NPARTCRDLKMCHSDWKSGEYWIDPNQGCNLDAIKVFCNMETGETCVYPTQPSVAQKNWYISKNPKDKR<br>HVWFGESMTDGFQFEYGGQGSDPADVAIQLTFLRLMSTEASQNITYHCKNSVAYMDQQTGNLKKALLLQ<br>GSNEIEIRAEGNSRFTYSVTVDGCTSHTGAWGKTVIEYKTTKTSRLPIIDVAPLDVGAPDQEFGFDVGP<br>VCFL | Chimeric prototype (Hu-1)S-trimer fusion polypeptide containing SARS-CoV-2 Beta (South African) variant RBD with signal peptide, S1/S2 furin cleavage site 1 and proline mutants (685R→685 A, 986K/987V →986P/987 P) |
| | | |

120

EP 4 722 230 A1

| SEQ ID NO. | Sequence | Description |
|---|---|---|
| 108 | QCVNLRTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLDVYYHKNNKS WMESGVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYRYRLFRKSNLKPFERDISTEIYQAGSKPCNGVEGFNCYFPLQSYGFQ PTNGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSRRRARSVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQNVVVNQNAQALNTLVKQLSSNFGAISSVLNDIL SRLDKVEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQYIK | SARS-CoV-2 Delta spike protein extracellular domain without signal peptide |
| 109 | QCVNLRTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLDVYYHKNNKS WMESGVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYRYRLFRKSNLKPFERDISTEIYQAGSKPCNGVEGFNCYFPLQSYGFQ PTNGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSRRRAASVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQNVVVNQNAQALNTLVKQLSSNFGAISSVLNDIL SRLDKVEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQYIK | SARS-CoV-2 Delta spike protein extracellular domain without signal peptide, S1/S2 furin cleavage site 1 mutant (685R→685 A) |

EP 4 722 230 A1

| SEQ ID NO. | Sequence | Description |
|---|---|---|
| 110 | QCVNLRTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLDVYYHKNNKS WMESGVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYRYRLFRKSNLKPFERDISTEIYQAGSKPCNGVEGFNCYFPLQSYGFQ PTNGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSRRRAASVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQNVVNQNAQALNTLVKQLSSNFGAISSVLNDIL SRLDPPEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQYIK | SARS-CoV-2 Delta spike protein extracellular domain without signal peptide, S1/S2 furin cleavage site 1 and proline mutant (685R→685 A, 986K/987V →986P/987 P) |
| 111 | QCVNLRTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLDVYYHKNNKS WMESGVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYRYRLFRKSNLKPFERDISTEIYQAGSKPCNGVEGFNCYFPLQSYGFQ PTNGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSRRRARSVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQNVVNQNAQALNTLVKQLSSNFGAISSVLNDIL SRLDPPEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQYIK | SARS-CoV-2 Delta spike protein extracellular domain without signal peptide, proline mutant (986K/987V →986P/987 P) |

| SEQ ID NO. | Sequence | Description |
|---|---|---|
| 112 | QCVNLRTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLDVYYHKNNKS WMESGVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYRYRLFRKSNLKPFERDISTEIYQAGSKPCNGVEGFNCYFPLQSYGFQ PTNGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSRRRARSVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQNVVNQNAQALNTLVKQLSSNFGAISSVLNDIL SRLDKVEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQYIKRSNGLPGPIGPPGPRGRTGDAGPVGPPGPPGPPGPPGPPSAGFDFS FLPQPPQEKAHDGGRYYRANDANVVRDRDLEVDTTLKSLSQQIENIRSPEGSRKNPARTCRDLKMCHSD WKSGEYWIDPNQGCNLDAIKVFCNMETGETCVYPTQPSVAQKNWYISKNPKDKRHVWFGESMTDGFQFE YGGQGSDPADVAIQLTFLRLMSTEASQNITYHCKNSVAYMDQQTGNLKKALLLQGSNEIEIRAEGNSRF TYSVTVDGCTSHTGAWGKTVIEYKTTKTSRLPIIDVAPLDVGAPDQEFGFDVGPVCFL | SARS-CoV-2 Delta S-trimer fusion polypeptide without signal peptide |

EP 4 722 230 A1

123

(continued)

| SEQ ID NO. | Sequence | Description |
|---|---|---|
| 113 | QCVNLRTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLDVYYHKNNKS WMESGVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYRYRLFRKSNLKPFERDISTEIYQAGSKPCNGVEGFNCYFPLQSYGFQ PTNGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSRRRAASVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQNVVNQNAQALNTLVKQLSSNFGAISSVLNDIL SRLDKVEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQYIKRSNGLPGPIGPPGPRGRTGDAGPVGPPGPPGPPGPPGPPSAGFDFS FLPQPPQEKAHDGGRYYRANDANVVRDRDLEVDTTLKSLSQQIENIRSPEGSRKNPARTCRDLKMCHSD WKSGEYWIDPNQGCNLDAIKVFCNMETGETCVYPTQPSVAQKNWYISKNPKDKRHVWFGESMTDGFQFE YGGQGSDPADVAIQLTFLRLMSTEASQNITYHCKNSVAYMDQQTGNLKKALLLQGSNEIEIRAEGNSRF TYSVTVDGCTSHTGAWGKTVIEYKTTKTSRLPIIDVAPLDVGAPDQEFGFDVGPVCFL | SARS-CoV-2 Delta S-trimer fusion polypeptide without signal peptide, S1/S2 furin cleavage site 1 mutant (685R→685 A) |

124

EP 4 722 230 A1

| SEQ ID NO. | Sequence | Description |
|---|---|---|
| 114 | QCVNLRTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLDVYYHKNNKS WMESGVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYRYRLFRKSNLKPFERDISTEIYQAGSKPCNGVEGFNCYFPLQSYGFQ PTNGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSRRRAASVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQNVVNQNAQALNTLVKQLSSNFGAISSVLNDIL SRLDPPEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQYIKRSNGLPGPIGPPGPRGRTGDAGPVGPPGPPGPPGPPGPPSAGFDFS FLPQPPQEKAHDGGRYYRANDANVVRDRDLEVDTTLKSLSQQIENIRSPEGSRKNPARTCRDLKMCHSD WKSGEYWIDPNQGCNLDAIKVFCNMETGETCVYPTQPSVAQKNWYISKNPKDKRHVWFGESMTDGFQFE YGGQGSDPADVAIQLTFLRLMSTEASQNITYHCKNSVAYMDQQTGNLKKALLLQGSNEIEIRAEGNSRF TYSVTVDGCTSHTGAWGKTVIEYKTTKTSRLPIIDVAPLDVGAPDQEFGFDVGPVCFL | SARS-CoV-2 Delta S-trimer fusion polypeptide without signal peptide, S1/S2 furin cleavage site 1 and proline mutant (685R→685 A, 986K/987V →986P/987 P) |

| SEQ ID NO. | Sequence | Description |
|---|---|---|
| 115 | QCVNLRTRTQLPPAYTNSFTRGVYYPDKVFRSSVLHSTQDLFLPFFSNVTWFHAIHVSGTNGTKRFDNP VLPFNDGVYFASTEKSNIIRGWIFGTTLDSKTQSLLIVNNATNVVIKVCEFQFCNDPFLDVYYHKNNKS WMESGVYSSANNCTFEYVSQPFLMDLEGKQGNFKNLREFVFKNIDGYFKIYSKHTPINLVRDLPQGFSA LEPLVDLPIGINITRFQTLLALHRSYLTPGDSSSGWTAGAAAYYVGYLQPRTFLLKYNENGTITDAVDC ALDPLSETKCTLKSFTVEKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIAPGQTGKIADYNYKLPDDF TGCVIAWNSNNLDSKVGGNYNYRYRLFRKSNLKPFERDISTEIYQAGSKPCNGVEGFNCYFPLQSYGFQ PTNGVGYQPYRVVVLSFELLHAPATVCGPKKSTNLVKNKCVNFNFNGLTGTGVLTESNKKFLPFQQFGR DIADTTDAVRDPQTLEILDITPCSFGGVSVITPGTNTSNQVAVLYQGVNCTEVPVAIHADQLTPTWRVY STGSNVFQTRAGCLIGAEHVNNSYECDIPIGAGICASYQTQTNSRRRARSVASQSIIAYTMSLGAENSV AYSNNSIAIPTNFTISVTTEILPVSMTKTSVDCTMYICGDSTECSNLLLQYGSFCTQLNRALTGIAVEQ DKNTQEVFAQVKQIYKTPPIKDFGGFNFSQILPDPSKPSKRSFIEDLLFNKVTLADAGFIKQYGDCLGD IAARDLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQNVVNQNAQALNTLVKQLSSNFGAISSVLNDIL SRLDPPEAEVQIDRLITGRLQSLQTYVTQQLIRAAEIRASANLAATKMSECVLGQSKRVDFCGKGYHLM SFPQSAPHGVVFLHVTYVPAQEKNFTTAPAICHDGKAHFPREGVFVSNGTHWFVTQRNFYEPQIITTDN TFVSGNCDVVIGIVNNTVYDPLQPELDSFKEELDKYFKNHTSPDVDLGDISGINASVVNIQKEIDRLNE VAKNLNESLIDLQELGKYEQYIKRSNGLPGPIGPPGPRGRTGDAGPVGPPGPPGPPGPPGPPSAGFDFS FLPQPPQEKAHDGGRYYRANDANVVRDRDLEVDTTLKSLSQQIENIRSPEGSRKNPARTCRDLKMCHSD WKSGEYWIDPNQGCNLDAIKVFCNMETGETCVYPTQPSVAQKNWYISKNPKDKRHVWFGESMTDGFQFE YGGQGSDPADVAIQLTFLRLMSTEASQNITYHCKNSVAYMDQQTGNLKKALLLQGSNEIEIRAEGNSRF TYSVTVDGCTSHTGAWGKTVIEYKTTKTSRLPIIDVAPLDVGAPDQEFGFDVGPVCFL | SARS-CoV-2 Delta S-trimer fusion polypeptide without signal peptide, proline mutant (986K/987V →986P/987 P) |

126

EP 4 722 230 A1

| SEQ ID NO. | Sequence | Description |
|---|---|---|
| 116 | MFVFLVLLPLVSSQCVNLRTRTQLPPAYTNSFTRGVYYPDKVFRSSVLHSTQDLFLPFFSNVTWFHAIH VSGTNGTKRFDNPVLPFNDGVYFASTEKSNIIRGWIFGTTLDSKTQSLLIVNNATNVVIKVCEFQFCND PFLDVYYHKNNKSWMESGVYSSANNCTFEYVSQPFLMDLEGKQGNFKNLREFVFKNIDGYFKIYSKHTP INLVRDLPQGFSALEPLVDLPIGINITRFQTLLALHRSYLTPGDSSSGWTAGAAAYYVGYLQPRTFLLK YNENGTITDAVDCALDPLSETKCTLKSFTVEKGIYQTSNFRVQPTESIVRFPNITNLCPFGEVFNATRF ASVYAWNRKRISNCVADYSVLYNSASFSTFKCYGVSPTKLNDLCFTNVYADSFVIRGDEVRQIAPGQTG KIADYNYKLPDDFTGCVIAWNSNNLDSKVGGNYNYRYLFRKSNLKPFERDISTEIYQAGSKPCNGVEG FNCYFPLQSYGFQPTNGVGYQPYRVVVLSFELLHAPATVCGPKKSTNLVKNKCVNFNFNGLTGTGVLTE SNKKFLPFQQFGRDIADTTDAVRDPQTLEILDITPCSFGGVSVITPGTNTSNQVAVLYQGVNCTEVPVA IHADQLTPTWRVYSTGSNVFQTRAGCLIGAEHVNNSYECDIPIGAGICASYQTQTNSRRRARSVASQSI IAYTMSLGAENSVAYSNNSIAIPTNFTISVTTEILPVSMTKTSVDCTMYICGDSTECSNLLLQYGSFCT QLNRALTGIAVEQDKNTQEVFAQVKQIYKTPPIKDFGGFNFSQILPDPSKPSKRSFIEDLLFNKVTLAD AGFIKQYGDCLGDIAARDLICAQKFNGLTVLPPLLTDEMIAQYTSALLAGTITSGWTFGAGAALQIPFA MQMAYRFNGIGVTQNVLYENQKLIANQFNSAIGKIQDSLSSTASALGKLQNVVNQNAQALNTLVKQLSS NFGAISSVLNDILSRLDKVEAEVQIDRLITGRLQSLQTYVTQQLIRAAEIRASANLAATKMSECVLGQS KRVDFCGKGYHLMSFPQSAPHGVVFLHVTYVPAQEKNFTTAPAICHDGKAHFPREGVFVSNGTHWFVTQ RNFYEPQIITTDNTFVSGNCDVVIGIVNNTVYDPLQPELDSFKEELDKYFKNHTSPDVDLGDISGINAS VVNIQKEIDRLNEVAKNLNESLIDLQELGKYEQYIKRSNGLPGPIGPPGPRGRTGDAGPVGPPGPPGPP GPPGPPSAGFDFSFLPQPPQEKAHDGGRYYRANDANVVRDRDLEVDTTLKSLSQQIENIRSPEGSRKNP ARTCRDLKMCHSDWKSGEYWIDPNQGCNLDAIKVFCNMETGETCVYPTQPSVAQKNWYISKNPKDKRHV WFGESMTDGFQFEYGGQGSDPADVAIQLTFLRLMSTEASQNITYHCKNSVAYMDQQTGNLKKALLLQGS NEIEIRAEGNSRFTYSVTVDGCTSHTGAWGKTVIEYKTTKTSRLPIIDVAPLDVGAPDQEFGFDVGPVC FL | SARS-CoV-2 Delta S-trimer fusion polypeptide with signal peptide |

EP 4 722 230 A1

127

| SEQ ID NO. | Sequence | Description |
|---|---|---|
| 117 | MFVFLVLLPLVSSQCVNLRTRTQLPPAYTNSFTRGVYYPDKVFRSSVLHSTQDLFLPFFSNVTWFHAIH VSGTNGTKRFDNPVLPFNDGVYFASTEKSNIIRGWIFGTTLDSKTQSLLIVNNATNVVIKVCEFQFCND PFLDVYYHKNNKSWMESGVYSSANNCTFEYVSQPFLMDLEGKQGNFKNLREFVFKNIDGYFKIYSKHTP INLVRDLPQGFSALEPLVDLPIGINITRFQTLLALHRSYLTPGDSSSGWTAGAAAYYVGYLQPRTFLLK YNENGTITDAVDCALDPLSETKCTLKSFTVEKGIYQTSNFRVQPTESIVRFPNITNLCPFGEVFNATRF ASVYAWNRKRISNCVADYSVLYNSASFSTFKCYGVSPTKLNDLCFTNVYADSFVIRGDEVRQIAPGQTG KIADYNYKLPDDFTGCVIAWNSNNLDSKVGGNYNYRYRLFRKSNLKPFERDISTEIYQAGSKPCNGVEG FNCYFPLQSYGFQPTNGVGYQPYRVVVLSFELLHAPATVCGPKKSTNLVKNKCVNFNFNGLTGTGVLTE SNKKFLPFQQFGRDIADTTDAVRDPQTLEILDITPCSFGGVSVITPGTNTSNQVAVLYQGVNCTEVPVA IHADQLTPTWRVYSTGSNVFQTRAGCLIGAEHVNNSYECDIPIGAGICASYQTQTNSRRRAASVASQSI IAYTMSLGAENSVAYSNNSIAIPTNFTISVTTEILPVSMTKTSVDCTMYICGDSTECSNLLLQYGSFCT QLNRALTGIAVEQDKNTQEVFAQVKQIYKTPPIKDFGGFNFSQILPDPSKPSKRSFIEDLLFNKVTLAD AGFIKQYGDCLGDIAARDLICAQKFNGLTVLPPLLTDEMIAQYTSALLAGTITSGWTFGAGAALQIPFA MQMAYRFNGIGVTQNVLYENQKLIANQFNSAIGKIQDSLSSTASALGKLQNVVNQNAQALNTLVKQLSS NFGAISSVLNDILSRLDKVEAEVQIDRLITGRLQSLQTYVTQQLIRAAEIRASANLAATKMSECVLGQS KRVDFCGKGYHLMSFPQSAPHGVVFLHVTYVPAQEKNFTTAPAICHDGKAHFPREGVFVSNGTHWFVTQ RNFYEPQIITTDNTFVSGNCDVVIGIVNNTVYDPLQPELDSFKEELDKYFKNHTSPDVDLGDISGINAS VVNIQKEIDRLNEVAKNLNESLIDLQELGKYEQYIKRSNGLPGPIGPPGPRGRTGDAGPVGPPGPPGPP GPPGPPSAGFDFSFLPQPPQEKAHDGGRYYRANDANVVRDRDLEVDTTLKSLSQQIENIRSPEGSRKNP ARTCRDLKMCHSDWKSGEYWIDPNQGCNLDAIKVFCNMETGETCVYPTQPSVAQKNWYISKNPKDKRHV WFGESMTDGFQFEYGGQGSDPADVAIQLTFLRLMSTEASQNITYHCKNSVAYMDQQTGNLKKALLLQGS NEIEIRAEGNSRFTYSVTVDGCTSHTGAWGKTVIEYKTTKTSRLPIIDVAPLDVGAPDQEFGFDVGPVC FL | SARS-CoV-2 Delta S-trimer fusion polypeptide with signal peptide, S1/S2 furin cleavage site 1 mutant (685R→685 A) |
| 118 | MFVFLVLLPLVSSQCVNLRTRTQLPPAYTNSFTRGVYYPDKVFRSSVLHSTQDLFLPFFSNVTWFHAIH VSGTNGTKRFDNPVLPFNDGVYFASTEKSNIIRGWIFGTTLDSKTQSLLIVNNATNVVIKVCEFQFCND PFLDVYYHKNNKSWMESGVYSSANNCTFEYVSQPFLMDLEGKQGNFKNLREFVFKNIDGYFKIYSKHTP INLVRDLPQGFSALEPLVDLPIGINITRFQTLLALHRSYLTPGDSSSGWTAGAAAYYVGYLQPRTFLLK YNENGTITDAVDCALDPLSETKCTLKSFTVEKGIYQTSNFRVQPTESIVRFPNITNLCPFGEVFNATRF ASVYAWNRKRISNCVADYSVLYNSASFSTFKCYGVSPTKLNDLCFTNVYADSFVIRGDEVRQIAPGQTG | SARS-CoV-2 Delta S-trimer fusion polypeptide with signal peptide, |

EP 4 722 230 A1

128

| SEQ ID NO. | Sequence | Description |
|---|---|---|
|  | KIADYNYKLPDDFTGCVIAWNSNNLDSKVGGNYNYRYRLFRKSNLKPFERDISTEIYQAGSKPCNGVEG FNCYFPLQSYGFQPTNGVGYQPYRVVVLSFELLHAPATVCGPKKSTNLVKNKCVNFNFNGLTGTGVLTE SNKKFLPFQQFGRDIADTTDAVRDPQTLEILDITPCSFGGVSVITPGTNTSNQVAVLYQGVNCTEVPVA IHADQLTPTWRVYSTGSNVFQTRAGCLIGAEHVNNSYECDIPIGAGICASYQTQTNSRRRAASVASQSI IAYTMSLGAENSVAYSNNSIAIPTNFTISVTTEILPVSMTKTSVDCTMYICGDSTECSNLLLQYGSFCT QLNRALTGIAVEQDKNTQEVFAQVKQIYKTPPIKDFGGFNFSQILPDPSKPSKRSFIEDLLFNKVTLAD AGFIKQYGDCLGDIAARDLICAQKFNGLTVLPPLLTDEMIAQYTSALLAGTITSGWTFGAGAALQIPFA MQMAYRFNGIGVTQNVLYENQKLIANQFNSAIGKIQDSLSSTASALGKLQNVVNQNAQALNTLVKQLSS NFGAISSVLNDILSRLDPPEAEVQIDRLITGRLQSLQTYVTQQLIRAAEIRASANLAATKMSECVLGQS KRVDFCGKGYHLMSFPQSAPHGVVFLHVTYVPAQEKNFTTAPAICHDGKAHFPREGVFVSNGTHWFVTQ RNFYEPQIITTDNTFVSGNCDVVIGIVNNTVYDPLQPELDSFKEELDKYFKNHTSPDVDLGDISGINAS VVNIQKEIDRLNEVAKNLNESLIDLQELGKYEQYIKRSNGLPGPIGPPGPRGRTGDAGPVGPPGPPGPP GPPGPPSAGFDFSFLPQPPQEKAHDGGRYYRANDANVVRDRDLEVDTTLKSLSQQIENIRSPEGSRKNP ARTCRDLKMCHSDWKSGEYWIDPNQGCNLDAIKVFCNMETGETCVYPTQPSVAQKNWYISKNPKDKRHV WFGESMTDGFQFEYGGQGSDPADVAIQLTFLRLMSTEASQNITYHCKNSVAYMDQQTGNLKKALLLQGS NEIEIRAEGNSRFTYSVTVDGCTSHTGAWGKTVIEYKTTKTSRLPIIDVAPLDVGAPDQEFGFDVGPVC FL | S1/S2 furin cleavage site 1 and proline mutant (685R→685 A, 986K/987V →986P/987 P) |

| SEQ ID NO. | Sequence | Description |
|---|---|---|
| 119 | MFVFLVLLPLVSSQCVNLRTRTQLPPAYTNSFTRGVYYPDKVFRSSVLHSTQDLFLPFFSNVTWFHAIH VSGTNGTKRFDNPVLPFNDGVYFASTEKSNIIRGWIFGTTLDSKTQSLLIVNNATNVVIKVCEFQFCND PFLDVYYHKNNKSWMESGVYSSANNCTFEYVSQPFLMDLEGKQGNFKNLREFVFKNIDGYFKIYSKHTP INLVRDLPQGFSALEPLVDLPIGINITRFQTLLALHRSYLTPGDSSSGWTAGAAAYYVGYLQPRTFLLK YNENGTITDAVDCALDPLSETKCTLKSFTVEKGIYQTSNFRVQPTESIVRFPNITNLCPFGEVFNATRF ASVYAWNRKRISNCVADYSVLYNSASFSTFKCYGVSPTKLNDLCFTNVYADSFVIRGDEVRQIAPGQTG KIADYNYKLPDDFTGCVIAWNSNNLDSKVGGNYNYRYRLFRKSNLKPFERDISTEIYQAGSKPCNGVEG FNCYFPLQSYGFQPTNGVGYQPYRVVVLSFELLHAPATVCGPKKSTNLVKNKCVNFNFNGLTGTGVLTE SNKKFLPFQQFGRDIADTTDAVRDPQTLEILDITPCSFGGVSVITPGTNSNQVAVLYQGVNCTEVPVA IHADQLTPTWRVYSTGSNVFQTRAGCLIGAEHVNNSYECDIPIGAGICASYQTQTNSRRRARSVASQSI IAYTMSLGAENSVAYSNNSIAIPTNFTISVTTEILPVSMTKTSVDCTMYICGDSTECSNLLLQYGSFCT QLNRALTGIAVEQDKNTQEVFAQVKQIYKTPPIKDFGGFNFSQILPDPSKPSKRSFIEDLLFNKVTLAD AGFIKQYGDCLGDIAARDLICAQKFNGLTVLPPLLTDEMIAQYTSALLAGTITSGWTFGAGAALQIPFA MQMAYRFNGIGVTQNVLYENQKLIANQFNSAIGKIQDSLSSTASALGKLQNVVNQNAQALNTLVKQLSS NFGAISSVLNDILSRLDPPEAEVQIDRLITGRLQSLQTYVTQQLIRAAEIRASANLAATKMSECVLGQS KRVDFCGKGYHLMSFPQSAPHGVVFLHVTYVPAQEKNFTTAPAICHDGKAHFPREGVFVSNGTHWFVTQ RNFYEPQIITTDNTFVSGNCDVVIGIVNNTVYDPLQPELDSFKEELDKYFKNHTSPDVDLGDISGINAS VVNIQKEIDRLNEVAKNLNESLIDLQELGKYEQYIKRSNGLPGPIGPPGPRGRTGDAGPVGPPGPPGPP GPPGPPSAGFDFSFLPQPPQEKAHDGGRYYRANDANVVRDRDLEVDTTLKSLSQQIENIRSPEGSRKNP ARTCRDLKMCHSDWKSGEYWIDPNQGCNLDAIKVFCNMETGETCVYPTQPSVAQKNWYISKNPKDKRHV WFGESMTDGFQFEYGGQGSDPADVAIQLTFLRLMSTEASQNITYHCKNSVAYMDQQTGNLKKALLLQGS NEIEIRAEGNSRFTYSVTVDGCTSHTGAWGKTVIEYKTTKTSRLPIIDVAPLDVGAPDQEFGFDVGPVC FL | SARS-CoV-2 Delta S-trimer fusion polypeptide with signal peptide, proline mutant (986K/987V →986P/987 P) |

130

EP 4 722 230 A1

| SEQ ID NO. | Sequence | Description |
|---|---|---|
| 120 | QCVNLITRTQSYTNSFTRGVYYPDKVFRSSVLHSTQDLFLPFFSNVTWFHAISGTNGTKRFDNPVLPFN DGVYFASTEKSNIIRGWIFGTTLDSKTQSLLIVNNATNVVIKVCEFQFCNDPFLDVYYHKNNKSWMESE FRVYSSANNCTFEYVSQPFLMDLEGKQGNFKNLREFVFKNIDGYFKIYSKHTPINLGRDLPQGFSALEP LVDLPIGINITRFQTLLALHRSYLTPGDSSSGWTAGAAAYYVGYLQPRTFLLKYNENGTITDAVDCALD PLSETKCTLKSFTVEKGIYQTSNFRVQPTESIVRFPNITNLCPFDEVFNATRFASVYAWNRKRISNCVA DYSVLYNFAPFFAFKCYGVSPTKLNDLCFTNVYADSFVIRGNEVSQIAPGQTGNIADYNYKLPDDFTGC VIAWNSNKLDSKVGGNYNYRYRLFRKSNLKPFERDISTEIYQAGNKPCNGVAGVNCYFPLQSYGFRPTY GVGHQPYRVVVLSFELLHAPATVCGPKKSTNLVKNKCVNFNFNGLTGTGVLTESNKKFLPFQQFGRDIA DTTDAVRDPQTLEILDITPCSFGGVSVITPGTNTSNQVAVLYQGVNCTEVPVAIHADQLTPTWRVYSTG SNVFQTRAGCLIGAEYVNNSYECDIPIGAGICASYQTQTKSHRRAASVASQSIIAYTMSLGAENSVAYS NNSIAIPTNFTISVTTEILPVSMTKTSVDCTMYICGDSTECSNLLLQYGSFCTQLKRALTGIAVEQDKN TQEVFAQVKQIYKTPPIKYFGGFNFSQILPDPSKPSKRSFIEDLLFNKVTLADAGFIKQYGDCLGDIAA RDLICAQKFNGLTVLPPLLTDEMIAQYTSALLAGTITSGWTFGAGAALQIPFAMQMAYRFNGIGVTQNV LYENQKLIANQFNSAIGKIQDSLSSTASALGKLQDVVNHNAQALNTLVKQLSSKFGAISSVLNDILSRL DPPEAEVQIDRLITGRLQSLQTYVTQQLIRAAEIRASANLAATKMSECVLGQSKRVDFCGKGYHLMSFP QSAPHGVVFLHVTYVPAQEKNFTTAPAICHDGKAHFPREGVFVSNGTHWFVTQRNFYEPQIITTDNTFV SGNCDVVIGIVNNTVYDPLQPELDSFKEELDKYFKNHTSPDVDLGDISGINASVVNIQKEIDRLNEVAK NLNESLIDLQELGKYEQYIKRSNGLPGPIGPPGPRGRTGDAGPVGPPGPPGPPGPPGPPSAGFDFSFLP QPPQEKAHDGGRYYRANDANVVRDRDLEVDTTLKSLSQQIENIRSPEGSRKNPARTCRDLKMCHSDWKS GEYWIDPNQGCNLDAIKVFCNMETGETCVYPTQPSVAQKNWYISKNPKDKRHVWFGESMTDGFQFEYGG QGSDPADVAIQLTFLRLMSTEASQNITYHCKNSVAYMDQQTGNLKKALLLQGSNEIEIRAEGNSRFTYS VTVDGCTSHTGAWGKTVIEYKTTKTSRLPIIDVAPLDVGAPDQEFGFDVGPVCFL | SARS-CoV-2 Omicron (BA.5) S-trimer fusion polypeptide without signal peptide, S1/S2 furin cleavage site 1 and proline mutant (685R→685 A, 986K/987V →986P/987 P) |
| 121 | MFVFLVLLPLVSSQCVNLITRTQSYTNSFTRGVYYPDKVFRSSVLHSTQDLFLPFFSNVTWFHAISGTN GTKRFDNPVLPFNDGVYFASTEKSNIIRGWIFGTTLDSKTQSLLIVNNATNVVIKVCEFQFCNDPFLDV YYHKNNKSWMESEFRVYSSANNCTFEYVSQPFLMDLEGKQGNFKNLREFVFKNIDGYFKIYSKHTPINL GRDLPQGFSALEPLVDLPIGINITRFQTLLALHRSYLTPGDSSSGWTAGAAAYYVGYLQPRTFLLKYNE NGTITDAVDCALDPLSETKCTLKSFTVEKGIYQTSNFRVQPTESIVRFPNITNLCPFDEVFNATRFASV | SARS-CoV-2 Omicron (BA.5) S-trimer fusion polypeptide |

131

EP 4 722 230 A1

（continued）

| SEQ ID NO. | Sequence | Description |
|---|---|---|
|  | YAWNRKRISNCVADYSVLYNFAPFFAFKCYGVSPTKLNDLCFTNVYADSFVIRGNEVSQIAPGQTGNIA DYNYKLPDDFTGCVIAWNSNKLDSKVGGNYNYRYRLFRKSNLKPFERDISTEIYQAGNKPCNGVAGVNC YFPLQSYGFRPTYGVGHQPYRVVVLSFELLHAPATVCGPKKSTNLVKNKCVNFNFNGLTGTGVLTESNK KFLPFQQFGRDIADTTDAVRDPQTLEILDITPCSFGGVSVITPGTNTSNQVAVLYQGVNCTEVPVAIHA DQLTPTWRVYSTGSNVFQTRAGCLIGAEYVNNSYECDIPIGAGICASYQTQTKSHRRAASVASQSIIAY TMSLGAENSVAYSNNSIAIPTNFTISVTTEILPVSMTKTSVDCTMYICGDSTECSNLLLQYGSFCTQLK RALTGIAVEQDKNTQEVFAQVKQIYKTPPIKYFGGFNFSQILPDPSKPSKRSFIEDLLFNKVTLADAGF IKQYGDCLGDIAARDLICAQKFNGLTVLPPLLTDEMIAQYTSALLAGTITSGWTFGAGAALQIPFAMQM AYRFNGIGVTQNVLYENQKLIANQFNSAIGKIQDSLSSTASALGKLQDVVNHNAQALNTLVKQLSSKFG AISSVLNDILSRLDPPEAEVQIDRLITGRLQSLQTYVTQQLIRAAEIRASANLAATKMSECVLGQSKRV DFCGKGYHLMSFPQSAPHGVVFLHVTYVPAQEKNFTTAPAICHDGKAHFPREGVFVSNGTHWFVTQRNF YEPQIITTDNTFVSGNCDVVIGIVNNTVYDPLQPELDSFKEELDKYFKNHTSPDVDLGDISGINASVVN IQKEIDRLNEVAKNLNESLIDLQELGKYEQYIKRSNGLPGPIGPPGPRGRTGDAGPVGPPGPPGPPGPP GPPSAGFDFSFLPQPPQEKAHDGGRYYRANDANVVRDRDLEVDTTLKSLSQQIENIRSPEGSRKNPART CRDLKMCHSDWKSGEYWIDPNQGCNLDAIKVFCNMETGETCVYPTQPSVAQKNWYISKNPKDKRHVWFG ESMTDGFQFEYGGQGSDPADVAIQLTFLRLMSTEASQNITYHCKNSVAYMDQQTGNLKKALLLQGSNEI EIRAEGNSRFTYSVTVDGCTSHTGAWGKTVIEYKTTKTSRLPIIDVAPLDVGAPDQEFGFDVGPVCFL | with signal peptide, S1/S2 furin cleavage site 1 and proline mutant (685R→685 A, 986K/987V →986P/987 P) |
| 122 | NIDGYFKIYSKHTPINLGRDLPQGFSALEPLVDLPIGINITRFQTLLALHRSYLTPGDSSSGWTAGAAA YYVGYLQPRTFLLKYNENGTITDAVDCALDPLSETKCTLKSFTVEKGIYQTSNFRVQPTESIVRFPNIT NLCPFDEVFNATRFASVYAWNRKRISNCVADYSVLYNFAPFFAFKCYGVSPTKLNDLCFTNVYADSFVI RGNEVSQIAPGQTGNIADYNYKLPDDFTGCVIAWNSNKLDSKVGGNYNYRYRLFRKSNLKPFERDISTE IYQAGNKPCNGVAGVNCYFPLQSYGFRPTYGVGHQPYRVVVLSFELLHAPATVCGPKKSTNLVKNKCVN FNFNGLTGTGVLTESNKKFLPFQQFGRDIADTTDAVRDPQTLEILDITPCSFGGVSVITPGTNTSNQVA VLYQGVNCTEVPVAIHADQLTPTWRVYSTGSNVFQTRAGCLIGAEYVNNSYECDIPIGAGICASYQTQT KSHRRAASVASQSIIAYTMSLGAENSVAYSNNSIAIPTNFTISVTTEILPVSMTKTSVDCTMYICGDST ECSNLLLQYGSFCTQLKRALTGIAVEQDKNTQEVFAQVKQIYKTPPIKYFGGFNFSQILPDPSKPSKRS FIEDLLFNKVTLADAGFIKQYGDCLGDIAARDLICAQKFNGLTVLPPLLTDEMIAQYTSALLAGTITSG WTFGAGAALQIPFAMQMAYRFNGIGVTQNVLYENQKLIANQFNSAIGKIQDSLSSTASALGKLQDVVNH NAQALNTLVKQLSSKFGAISSVLNDILSRLDPPEAEVQIDRLITGRLQSLQTYVTQQLIRAAEIRASAN LAATKMSECVLGQSKRVDFCGKGYHLMSFPQSAPHGVVFLHVTYVPAQEKNFTTAPAICHDGKAHFPRE GVFVSNGTHWFVTQRNFYEPQIITTDNTFVSGNCDVVIGIVNNTVYDPLQPELDSFKEELDKYFKNHTS PDVDLGDISGINASVVNIQKEIDRLNEVAKNLNESLIDLQELGKYEQYIK | SARS-CoV-2 Omicron (BA.5) spike protein extracellular domain without signal peptide, S1/S2 furin cleavage site 1 and proline mutant (685R→685 A, 986K/987V →986P/987 P) |

| SEQ ID NO. | Sequence | Description |
|---|---|---|
| 123 | MFVFLVLLPLVSSNIDGYFKIYSKHTPINLGRDLPQGFSALEPLVDLPIGINITRFQTLLALHRSYLTP GDSSSGWTAGAAAYYVGYLQPRTFLLKYNENGTITDAVDCALDPLSETKCTLKSFTVEKGIYQTSNFRV QPTESIVRFPNITNLCPFDEVFNATRFASVYAWNRKRISNCVADYSVLYNFAPFFAFKCYGVSPTKLND LCFTNVYADSFVIRGNEVSQIAPGQTGNIADYNYKLPDDFTGCVIAWNSNKLDSKVGGNYNYRYRLFRK SNLKPFERDISTEIYQAGNKPCNGVAGVNCYFPLQSYGFRPTYGVGHQPYRVVVLSFELLHAPATVCGP KKSTNLVKNKCVNFNFNGLTGTGVLTESNKKFLPFQQFGRDIADTTDAVRDPQTLEILDITPCSFGGVS VITPGTNTSNQVAVLYQGVNCTEVPVAIHADQLTPTWRVYSTGSNVFQTRAGCLIGAEYVNNSYECDIP IGAGICASYQTQTKSHRRAASVASQSIIAYTMSLGAENSVAYSNNSIAIPTNFTISVTTEILPVSMTKT SVDCTMYICGDSTECSNLLLQYGSFCTQLKRALTGIAVEQDKNTQEVFAQVKQIYKTPPIKYFGGFNFS QILPDPSKPSKRSFIEDLLFNKVTLADAGFIKQYGDCLGDIAARDLICAQKFNGLTVLPPLLTDEMIAQ YTSALLAGTITSGWTFGAGAALQIPFAMQMAYRFNGIGVTQNVLYENQKLIANQFNSAIGKIQDSLSST ASALGKLQDVVNHNAQALNTLVKQLSSKFGAISSVLNDILSRLDPPEAEVQIDRLITGRLQSLQTYVTQ QLIRAAEIRASANLAATKMSECVLGQSKRVDFCGKGYHLMSFPQSAPHGVVFLHVTYVPAQEKNFTTAP AICHDGKAHFPREGVFVSNGTHWFVTQRNFYEPQIITTDNTFVSGNCDVVIGIVNNTVYDPLQPELDSF KEELDKYFKNHTSPDVDLGDISGINASVVNIQKEIDRLNEVAKNLNESLIDLQELGKYEQYIK | SARS-CoV-2 Omicron (BA.5) spike protein extracellular domain with signal peptide, S1/S2 furin cleavage site 1 and proline mutants (→685R685 A, 986K/→987 V986P/987P) |
| 124 | QCVNLITRTQSYTNSFTRGVYYPDKVFRSSVLHSTQDLFLPFFSNVTWFHAIHVSGTNGTKRFDNPALP FNDGVYFASTEKSNIIRGWIFGTTLDSKTQSLLIVNNATNVVIKVCEFQFCNDPFLDVYQKNNKSWMES EFRVYSSANNCTFEYVSQPFLMDLEGKEGNFKNLREFVFKNIDGYFKIYSKHTPINLERDLPQGFSALE PLVDLPIGINITRFQTLLALHRSYLTPVDSSSGWTAGAAAYYVGYLQPRTFLLKYNENGTITDAVDCAL DPLSETKCTLKSFTVEKGIYQTSNFRVQPTESIVRFPNITNLCPFHEVFNATTFASVYAWNRKRISNCV ADYSVIYNFAPFFAFKCYGVSPTKLNDLCFTNVYADSFVIRGNEVSQIAPGQTGNIADYNYKLPDDFTG CVIAWNSNKLDSKPSGNYNYLYRLFRKSKLKPFERDISTEIYQAGNKPCNGVAGPNCYSPLQSYGFRPT YGVGHQPYRVVVLSFELLHAPATVCGPKKSTNLVKNKCVNFNFNGLTGTGVLTESNKKFLPFQQFGRDI ADTTDAVRDPQTLEILDITPCSFGGVSVITPGTNTSNQVAVLYQGVNCTEVPVAIHADQLTPTWRVYST GSNVFQTRAGCLIGAEYVNNSYECDIPIGAGICASYQTQTKSHRRARSVASQSIIAYTMSLGAENSVAY SNNSIAIPTNFTISVTTEILPVSMTKTSVDCTMYICGDSTECSNLLLQYGSFCTQLKRALTGIAVEQDK NTQEVFAQVKQIYKTPPIKYFGGFNFSQILPDPSKPSKRSFIEDLLFNKVTLADAGFIKQYGDCLGDIA ARDLICAQKFNGLTVLPPLLTDEMIAQYTSALLAGTITSGWTFGAGAALQIPFAMQMAYRFNGIGVTQN VLYENQKLIANQFNSAIGKIQDSLSSTASALGKLQDVVNHNAQALNTLVKQLSSKFGAISSVLNDILSR LDKVEAEVQIDRLITGRLQSLQTYVTQQLIRAAEIRASANLAATKMSECVLGQSKRVDFCGKGYHLMSF PQSAPHGVVFLHVTYVPAQEKNFTTAPAICHDGKAHFPREGVFVSNGTHWFVTQRNFYEPQIITTDNTF VSGNCDVVIGIVNNTVYDPLQPELDSFKEELDKYFKNHTSPDVDLGDISGINASVVNIQKEIDRLNEVA KNLNESLIDLQELGKYEQYIKRSNGLPGPIGPPGPRGRTGDAGPVGPPGPPGPPGPPGPPSAGFDFSFL PQPPQEKAHDGGRYYRANDANVVRDRDLEVDTTLKSLSQQIENIRSPEGSRKNPARTCRDLKMCHSDWK SGEYWIDPNQGCNLDAIKVFCNMETGETCVYPTQPSVAQKNWYISKNPKDKRHVWFGESMTDGFQFEYG GQGSDPADVAIQLTFLRLMSTEASQNITYHCKNSVAYMDQQTGNLKKALLLQGSNEIEIRAEGNSRFTY SVTVDGCTSHTGAWGKTVIEYKTTKTSRLPIIDVAPLDVGAPDQEFGFDVGPVCFL | SARS-CoV-2 Omicron (XBB.1.5) S-trimer fusion polypeptide without signal peptide, S1/S2 furin cleavage site 1 and proline mutant (685R→685 A, 986K/987V →986P/987 P) |

133

EP 4 722 230 A1

(continued)

| SEQ ID NO. | Sequence | Description |
|---|---|---|
| 125 | MFVFLVLLPLVSSQCVNLITRTQSYTNSFTRGVYYPDKVFRSSVLHSTQDLFLPFFSNVTWFHAIHVSG<br>TNGTKRFDNPALPFNDGVYFASTEKSNIIRGWIFGTTLDSKTQSLLIVNNATNVVIKVCEFQFCNDPFL<br>DVYQKNNKSWMESEFRVYSSANNCTFEYVSQPFLMDLEGKEGNFKNLREFVFKNIDGYFKIYSKHTPIN<br>LERDLPQGFSALEPLVDLPIGINITRFQTLLALHRSYLTPVDSSSGWTAGAAAYYVGYLQPRTFLLKYN<br>ENGTITDAVDCALDPLSETKCTLKSFTVEKGIYQTSNFRVQPTESIVRFPNITNLCPFHEVFNATTFAS<br>VYAWNRKRISNCVADYSVIYNFAPFFAFKCYGVSPTKLNDLCFTNVYADSFVIRGNEVSQIAPGQTGNI<br>ADYNYKLPDDFTGCVIAWNSNKLDSKPSGNYNYLYRLFRKSKLKPFERDISTEIYQAGNKPCNGVAGPN<br>CYSPLQSYGFRPTYGVGHQPYRVVVLSFELLHAPATVCGPKKSTNLVKNKCVNFNFNGLTGTGVLTESN<br>KKFLPFQQFGRDIADTTDAVRDPQTLEILDITPCSFGGVSVITPGTNTSNQVAVLYQGVNCTEVPVAIH<br>ADQLTPTWRVYSTGSNVFQTRAGCLIGAEYVNNSYECDIPIGAGICASYQTQTKSHRRARSVASQSIIA<br>YTMSLGAENSVAYSNNSIAIPTNFTISVTTEILPVSMTKTSVDCTMYICGDSTECSNLLLQYGSFCTQL<br>KRALTGIAVEQDKNTQEVFAQVKQIYKTPPIKYFGGFNFSQILPDPSKPSKRSFIEDLLFNKVTLADAG<br>FIKQYGDCLGDIAARDLICAQKFNGLTVLPPLLTDEMIAQYTSALLAGTITSGWTFGAGAALQIPFAMQ<br>MAYRFNGIGVTQNVLYENQKLIANQFNSAIGKIQDSLSSTASALGKLQDVVNHNAQALNTLVKQLSSKF<br>GAISSVLNDILSRLDKVEAEVQIDRLITGRLQSLQTYVTQQLIRAAEIRASANLAATKMSECVLGQSKR<br>VDFCGKGYHLMSFPQSAPHGVVFLHVTYVPAQEKNFTTAPAICHDGKAHFPREGVFVSNGTHWFVTQRN<br>FYEPQIITTDNTFVSGNCDVVIGIVNNTVYDPLQPELDSFKEELDKYFKNHTSPDVDLGDISGINASVV<br>NIQKEIDRLNEVAKNLNESLIDLQELGKYEQYIKRSNGLPGPIGPPGPRGRTGDAGPVGPPGPPGPPGP<br>PGPPSAGFDFSFLPQPPQEKAHDGGRYYRANDANVVRDRDLEVDTTLKSLSQQIENIRSPEGSRKNPAR<br>TCRDLKMCHSDWKSGEYWIDPNQGCNLDAIKVFCNMETGETCVYPTQPSVAQKNWYISKNPKDKRHVWF<br>GESMTDGFQFEYGGQGSDPADVAIQLTFLRLMSTEASQNITYHCKNSVAYMDQQTGNLKKALLLQGSNE<br>IEIRAEGNSRFTYSVTVDGCTSHTGAWGKTVIEYKTTKTSRLPIIDVAPLDVGAPDQEFGFDVGPVCFL | SARS-CoV-2 Omicron (XBB.1.5) S-trimer fusion polypeptide with signal peptide, S1/S2 furin cleavage site 1 and proline mutant (685R→685 A, 986K/987V →986P/987 P) |

134

(continued)

| SEQ ID NO. | Sequence | Description |
|---|---|---|
| 126 | QCVNLITRTQSYTNSFTRGVYYPDKVFRSSVLHSTQDLFLPFFSNVTWFHAIHVSGTNGTKRFDNPALP FNDGVYFASTEKSNIIRGWIFGTTLDSKTQSLLIVNNATNVVIKVCEFQFCNDPFLDVYQKNNKSWMES EFRVYSSANNCTFEYVSQPFLMDLEGKEGNFKNLREFVFKNIDGYFKIYSKHTPINLERDLPQGFSALE PLVDLPIGINITRFQTLLALHRSYLTPVDSSSGWTAGAAAYYVGYLQPRTFLLKYNENGTITDAVDCAL DPLSETKCTLKSFTVEKGIYQTSNFRVQPTESIVRFPNITNLCPFHEVFNATTFASVYAWNRKRISNCV ADYSVIYNFAPFFAFKCYGVSPTKLNDLCFTNVYADSFVIRGNEVSQIAPGQTGNIADYNYKLPDDFTG CVIAWNSNKLDSKPSGNYNYLYRLFRKSKLKPFERDISTEIYQAGNKPCNGVAGPNCYSPLQSYGFRPT YGVGHQPYRVVVLSFELLHAPATVCGPKKSTNLVKNKCVNFNFNGLTGTGVLTESNKKFLPFQQFGRDI ADTTDAVRDPQTLEILDITPCSFGGVSVITPGTNTSNQVAVLYQGVNCTEVPVAIHADQLTPTWRVYST GSNVFQTRAGCLIGAEYVNNSYECDIPIGAGICASYQTQTKSHRRARSVASQSIIAYTMSLGAENSVAY SNNSIAIPTNFTISVTTEILPVSMTKTSVDCTMYICGDSTECSNLLLQYGSFCTQLKRALTGIAVEQDK NTQEVFAQVKQIYKTPPIKYFGGFNFSQILPDPSKPSKRSFIEDLLFNKVTLADAGFIKQYGDCLGDIA ARDLICAQKFNGLTVLPPLLTDEMIAQYTSALLAGTITSGWTFGAGAALQIPFAMQMAYRFNGIGVTQN VLYENQKLIANQFNSAIGKIQDSLSSTASALGKLQDVVNHNAQALNTLVKQLSSKFGAISSVLNDILSR LDKVEAEVQIDRLITGRLQSLQTYVTQQLIRAAEIRASANLAATKMSECVLGQSKRVDFCGKGYHLMSF PQSAPHGVVFLHVTYVPAQEKNFTTAPAICHDGKAHFPREGVFVSNGTHWFVTQRNFYEPQIITTDNTF VSGNCDVVIGIVNNTVYDPLQPELDSFKEELDKYFKNHTSPDVDLGDISGINASVVNIQKEIDRLNEVA KNLNESLIDLQELGKYEQYIK | SARS-CoV-2 Omicron (XBB.1.5) spike protein extracellular domain without signal peptide, S1/S2 furin cleavage site 1 and proline mutant (685R→685 A, 986K/987V →986P/987 P) |
| 127 | MFVFLVLLPLVSSQCVNLITRTQSYTNSFTRGVYYPDKVFRSSVLHSTQDLFLPFFSNVTWFHAIHVSG TNGTKRFDNPALPFNDGVYFASTEKSNIIRGWIFGTTLDSKTQSLLIVNNATNVVIKVCEFQFCNDPFL DVYQKNNKSWMESEFRVYSSANNCTFEYVSQPFLMDLEGKEGNFKNLREFVFKNIDGYFKIYSKHTPIN LERDLPQGFSALEPLVDLPIGINITRFQTLLALHRSYLTPVDSSSGWTAGAAAYYVGYLQPRTFLLKYN ENGTITDAVDCALDPLSETKCTLKSFTVEKGIYQTSNFRVQPTESIVRFPNITNLCPFHEVFNATTFAS VYAWNRKRISNCVADYSVIYNFAPFFAFKCYGVSPTKLNDLCFTNVYADSFVIRGNEVSQIAPGQTGNI ADYNYKLPDDFTGCVIAWNSNKLDSKPSGNYNYLYRLFRKSKLKPFERDISTEIYQAGNKPCNGVAGPN CYSPLQSYGFRPTYGVGHQPYRVVVLSFELLHAPATVCGPKKSTNLVKNKCVNFNFNGLTGTGVLTESN KKFLPFQQFGRDIADTTDAVRDPQTLEILDITPCSFGGVSVITPGTNTSNQVAVLYQGVNCTEVPVAIH ADQLTPTWRVYSTGSNVFQTRAGCLIGAEYVNNSYECDIPIGAGICASYQTQTKSHRRARSVASQSIIA YTMSLGAENSVAYSNNSIAIPTNFTISVTTEILPVSMTKTSVDCTMYICGDSTECSNLLLQYGSFCTQL KRALTGIAVEQDKNTQEVFAQVKQIYKTPPIKYFGGFNFSQILPDPSKPSKRSFIEDLLFNKVTLADAG FIKQYGDCLGDIAARDLICAQKFNGLTVLPPLLTDEMIAQYTSALLAGTITSGWTFGAGAALQIPFAMQ MAYRFNGIGVTQNVLYENQKLIANQFNSAIGKIQDSLSSTASALGKLQDVVNHNAQALNTLVKQLSSKF GAISSVLNDILSRLDKVEAEVQIDRLITGRLQSLQTYVTQQLIRAAEIRASANLAATKMSECVLGQSKR VDFCGKGYHLMSFPQSAPHGVVFLHVTYVPAQEKNFTTAPAICHDGKAHFPREGVFVSNGTHWFVTQRN FYEPQIITTDNTFVSGNCDVVIGIVNNTVYDPLQPELDSFKEELDKYFKNHTSPDVDLGDISGINASVV NIQKEIDRLNEVAKNLNESLIDLQELGKYEQYIK | SARS-CoV-2 Omicron (XBB.1.5) spike protein extracellular domain with signal peptide, S1/S2 furin cleavage site 1 and proline mutant (685R→685 A, 986K/987V →986P/987 P) |

**Claims**

1. A multivalent vaccine comprising a coronavirus (S) protein surface antigen or a fragment, variant or mutant thereof, wherein the coronavirus (S) protein surface antigen or the fragment, variant or mutant thereof is selected from soluble SARS-CoV-2 Hu-1, SARS-CoV-2 Omicron (B.1.1.529) and subtypes thereof, Alpha, Beta, Gamma, Delta, and Mu spike (S) protein surface antigens or fragments, variants or mutants thereof, wherein the coronavirus (S) protein surface antigen or the fragment, variant or mutant thereof is linked to a C-terminal propeptide portion of collagen to form a disulfide bond-connected trimeric fusion protein.

2. The multivalent vaccine according to claim 1, wherein the SARS-CoV-2 Omicron subtype spike (S) protein surface antigen or the fragment, variant, or mutant thereof comprises BA.1, BA.2, BA.2.75, BA.5, BQ.1.1, BF.7, XBB, XBB.1.5, and BQ.1.1 spike (S) protein surface antigens or fragments, variants or mutants thereof.

3. The multivalent vaccine according to claim 1 or 2, comprising the soluble SARS-CoV-2 Hu-1 (S) protein surface antigen or the fragment, variant or mutant thereof.

4. The multivalent vaccine according to any one of claims 1 to 3, comprising the soluble SARS-CoV-2 Omicron BA.5 (S) protein surface antigen or the fragment, variant or mutant thereof.

5. The multivalent vaccine according to any one of claims 1 to 4, comprising the soluble SARS-CoV-2 Omicron XBB.1.5 (S) protein surface antigen or the fragment, variant or mutant thereof.

6. The multivalent vaccine according to claim 5, wherein the multivalent vaccine is a trivalent vaccine and comprises the soluble SARS-CoV-2 Hu-1, the Omicron strains BA.5 and XBB.1.5 spike (S) protein surface antigens or the fragments, variants or mutants thereof in a weight ratio of 0.5-4:0.5-4:0.5-4.

7. The multivalent vaccine according to claim 6, wherein a weight ratio of the soluble SARS-CoV-2 Hu-1, the Omicron strains BA.5 and XBB.1.5 spike (S) protein surface antigens or the fragments, variants or mutants is 1:1:1 or 1:2:2.

8. The multivalent vaccine according to any one of claims 1 to 5, wherein the coronavirus (S) protein surface antigen or the fragment, variant or mutant thereof comprises a sequence set forth in any one of SEQ ID NOs: 27-66, or an amino acid sequence having at least or about 80%, 85%, 90%, 92%, 95%, 97%, or 99% sequence identity with the sequence set forth in any one of SEQ ID NOs: 27-66.

9. The multivalent vaccine according to any one of claims 1 to 8, wherein the coronavirus (S) protein surface antigen or the fragment, variant or mutant thereof comprises a sequence set forth in any one of SEQ ID NOs: 27-34, or an amino acid sequence having at least or about 80%, 85%, 90%, 92%, 95%, 97%, or 99% sequence identity with the sequence set forth in any one of SEQ ID NOs: 27-34.

10. The multivalent vaccine according to any one of claims 1 to 9, wherein the coronavirus (S) protein surface antigen or the fragment, variant or mutant thereof comprises a sequence set forth in any one of SEQ ID NOs: 81-84, or an amino acid sequence having at least or about 80%, 85%, 90%, 92%, 95%, 97%, or 99% sequence identity with the sequence set forth in any one of SEQ ID NOs: 81-84.

11. The multivalent vaccine according to any one of claims 1 to 10, wherein the coronavirus (S) protein surface antigen or the fragment, variant or mutant thereof comprises a sequence set forth in SEQ ID NO: 126, or an amino acid sequence having at least or about 80%, 85%, 90%, 92%, 95%, 97%, or 99% sequence identity with the sequence set forth in SEQ ID NO: 126.

12. The multivalent vaccine according to any one of claims 1 to 11, wherein the coronavirus (S) protein surface antigen or the fragment, variant or mutant thereof comprises a sequence set forth in SEQ ID NO: 122, or an amino acid sequence having at least or about 80%, 85%, 90%, 92%, 95%, 97%, or 99% sequence identity with the sequence set forth in SEQ ID NO: 122.

13. The multivalent vaccine according to any one of claims 2 or 3, wherein the multivalent vaccine is a bivalent vaccine and may be selected from the SARS-CoV-2 Hu-1, Alpha, Beta, Gamma, Delta, Mu spike, Omicron (B.1.1.529), BA.1, BA.2, BA.2.75, BA.5, BQ.1.1, BF.7, XBB, XBB. 1.5, BQ.1.1, XBB.1.16.1, EG.5.1 spike (S) protein surface antigens or the fragments, variants, or mutants thereof, wherein the bivalent vaccine comprises two coronavirus (S) protein surface antigens or the fragments, variants or mutants thereof in a weight ratio of 0.5-4:0.5-4.

14. The multivalent vaccine according to claim 13, wherein the bivalent vaccine comprises the BA.5 and the XBB.1.5 in a weight ratio of 1:1 or 1:2; or the bivalent vaccine comprises the Hu-1 and the BA.5 in a weight ratio of 1:1 or 1:2; or the bivalent vaccine comprises the Hu-1 and the XBB.1.5 in a weight ratio of 1:1 or 1:2.

15. The multivalent vaccine according to any one of claims 1 to 14, wherein the multivalent vaccine is administered without an adjuvant and optionally is used as a primary series, an additional dose, and/or a homologous or heterologous booster dose, such as a first dose, a second dose, a third dose, a fourth dose, and/or more doses, and optionally the primary series, the additional dose, or the heterologous booster dose is used in combination with any one or more of other recombinant subunit vaccines, nanoparticle vaccines, mRNA vaccines, DNA vaccines, adenovirus vector vaccines, and inactivated virus vaccines.

16. The multivalent vaccine according to any one of claims 1 to 14, wherein the multivalent vaccine is administered together with one or more adjuvants, and optionally is used as a primary series, an additional dose and/or a homologous or heterologous booster dose, such as a first dose, a second dose, a third dose, a fourth dose and/or more doses, and optionally the primary series, the additional dose or the heterologous booster dose is used in combination with any one or more of other recombinant subunit vaccines, nanoparticle vaccines, mRNA vaccines, DNA vaccines, adenovirus vector vaccines, and inactivated virus vaccines.

17. The multivalent vaccine according to any one of claims 1 to 14 and 16, wherein the adjuvant in the primary series, the additional dose, and/or the homologous or heterologous booster dose may independently comprise: an aluminum-containing adjuvant, such as the adjuvant containing alum and/or aluminum hydroxide; an oligonucleotide-containing adjuvant, such as the adjuvant containing CpG oligodeoxynucleotide (CpG-ODN); a TLR9 agonist-containing adjuvant; an adjuvant containing metabolizable oil, $\alpha$-tocopherol, and/or polyoxyethylene sorbitan monooleate (Tween-80), such as the adjuvant in the form of an oil-in-water emulsion containing squalene, $\alpha$-tocopherol, and Tween-80 and/or Span 85; or any combination of the adjuvants.

18. The multivalent vaccine according to any one of claims 1 to 14 and 16 to 17, wherein the adjuvant in the primary series, the additional dose, and/or the homologous or heterologous booster dose may comprise the adjuvant containing CpG oligodeoxynucleotide (CpG-ODN) and/or the adjuvant containing alum and/or aluminum hydroxide.

19. The multivalent vaccine according to any one of claims 1 to 18, wherein the multivalent vaccine is administered by intramuscular injection.

20. The multivalent vaccine according to any one of claims 1 to 18, wherein the multivalent vaccine is administered by intranasal spray.

21. The multivalent vaccine according to any one of claims 1 to 20, wherein the multivalent vaccine is administered in a single dose or in a series of doses spaced weekly or monthly.

22. The multivalent vaccine according to any one of claims 1 to 21, wherein the multivalent vaccine can prevent or treat coronavirus infection, such as severe acute respiratory syndrome (SARS)-coronavirus 2 (SARS-CoV-2) infection.

23. The multivalent vaccine according to any one of claims 1 to 21, wherein the C-terminal propeptide comprises any one of SEQ ID NOs: 67-80 or an amino acid sequence having at least 80%, 85%, 90%, 92%, 95%, 97%, or 99% identity thereto.

FIG. 1

A.

C.

FIG. 2

FIG. 2 (continued)

FIG. 2 (continued)

EP 4 722 230 A1

FIG. 3

FIG. 4

Design (n=8-9/group):

Dose 1    Dose 2      Dose 3              Dose 4
                  Bloodletting   Bloodletting    Bloodletting   Bloodletting

D0     D21     ~5M pd2     D14 pd3    D28 pd3    D14 pd4
                  D156       D170      D184      D198

| Group # n= | Primary (D0 and D21) | Booster at about 5 months post dose 2 (D156) (+ CpG 1018: 150ug, Alum: 75ug) | Booster at about 28 days post dose 3 (+ CpG 1018: 150ug, Alum: 75ug) |
|---|---|---|---|
| 1 n=8 | | Hu-1 3ug | Hu-1 3ug |
| 2 n=9 | 2 x Hu-1 | Hu-1 + XBB.1.5 1:1 3ug | Hu-1 + XBB.1.5 1:1 3ug |
| 3 n=9 | | BA.5 + XBB.1.5 1:1 3ug | BA.5 + XBB.1.5 1:1 3ug |
| 4 n=9 | | Hu-1 + BA. 5+ XBB.1.5 1:1:1 3ug | Hu-1 + BA. 5+ XBB.1.5 1:1:1 3ug |

FIG. 5

FIG. 6

E.

BA.2

F.

BA.2.75

G.

BA.5

H.

BF.7

FIG. 6 (continued)

FIG. 6 (continued)

FIG. 7

| Sample Name | Batch No. | Strength |
|---|---|---|
| Changxing DP monovalent PPQ1 | 230712002 | 0.5 ml/vial |
| Changxing DP trivalent | 230611002 | 0.5 ml/vial |
| SCB-2019 DP1 | 220906004 | 8.75 ml/vial |
| DP2 | 220907003 | 1.25 ml/vial |

FIG. 8

FIG. 9

FIG. 9 (continued)

FIG. 9 (continued)

FIG. 10

FIG. 11

# EP 4 722 230 A1

<table>
<tr><td colspan="2">**INTERNATIONAL SEARCH REPORT**</td><td>International application No.<br>**PCT/CN2024/096427**</td></tr>
</table>

**A. CLASSIFICATION OF SUBJECT MATTER**

C07K14/00(2006.01)i; C07K19/00(2006.01)i; A61P31/00(2006.01)i; A61K38/00(2006.01)i; A61P31/14(2006.01)i; A61K39/39(2006.01)i; A61K39/215(2006.01)i

According to International Patent Classification (IPC) or to both national classification and IPC

**B. FIELDS SEARCHED**

Minimum documentation searched (classification system followed by classification symbols)

IPC:C07K A61P A61K

Documentation searched other than minimum documentation to the extent that such documents are included in the fields searched

Electronic data base consulted during the international search (name of data base and, where practicable, search terms used)

CNTXT: WPABS; ENTXT; USTXT; JPTXT; KRTXT; DWPI; VEN; CJFD; ISI web of Science: PUBMED; 百度学术, BAIDU SCHOLAR: 百度, BAIDU; 读秀, DUXIU; 万方, WANFANG; CNKI; Genbank; EBI; ENA: 中国专利生物序列检索, China Patents Biological Sequence Search System; STN: 冠状病毒, 新冠, 疫苗, 多价, 三价, 二价, S蛋白, 刺突蛋白, 胶原, 三聚体, 二硫键, 奥密克戎, 阿尔法, 贝塔, 伽马, 德尔塔, SARS-CoV-2, COVID-19, vaccine, bivalent, spike protein, omicron, Hu-1, BA.1, BA.2, BA.2.75, BA.5, BQ.1.1, BF.7, XBB, XBB.1.5, BQ.1.1, SEQ ID NO: 1-127

**C. DOCUMENTS CONSIDERED TO BE RELEVANT**

| Category* | Citation of document, with indication, where appropriate, of the relevant passages | Relevant to claim No. |
|---|---|---|
| PX | WO 2023179513 A1 (SICHUAN CLOVER BIOPHARMACEUTICALS LIMITED COMPANY) 28 September 2023 (2023-09-28)<br>claims 1-89, description, paragraphs 010-053, 063-065, 418-488, and 509-510, and sequence listing | 1-5, 8-10, 12-13, 15-23 |
| PY | WO 2023179513 A1 (SICHUAN CLOVER BIOPHARMACEUTICALS LIMITED COMPANY) 28 September 2023 (2023-09-28)<br>claims 1-89, description, paragraphs 010-053, 063-065, 418-488, and 509-510, and sequence listing | 6-7, 11, 14 |
| PX | CN 116836294 A (SICHUAN CLOVER BIOPHARMACEUTICALS LIMITED COMPANY) 03 October 2023 (2023-10-03)<br>claims 1-10, description, paragraphs 0059-0072, 0340-0480, and 0501, and sequence listing | 1-5, 8-10, 12-13, 15-23 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **04 September 2024** | **09 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/096427** |

**C.    DOCUMENTS CONSIDERED TO BE RELEVANT**

| Category* | Citation of document, with indication, where appropriate, of the relevant passages | Relevant to claim No. |
|---|---|---|
| PY | CN 116836294 A (SICHUAN CLOVER BIOPHARMACEUTICALS LIMITED COMPANY) 03 October 2023 (2023-10-03) claims 1-10, description, paragraphs 0059-0072, 0340-0480, and 0501, and sequence listing | 6-7, 11, 14 |
| X | CN 114206946 A (SICHUAN CLOVER BIOPHARMACEUTICALS LIMITED COMPANY) 18 March 2022 (2022-03-18) claims 1-62, description, paragraphs 0006-0044, 0067, 0081-0082, 0150, and 0448, sequence listing | 1-5, 8-10, 12-13, 15-23 |
| Y | CN 114206946 A (SICHUAN CLOVER BIOPHARMACEUTICALS LIMITED COMPANY) 18 March 2022 (2022-03-18) claims 1-62, description, paragraphs 0006-0044, 0067, 0081-0082, 0150, and 0448, and sequence listing | 6-7, 11, 14 |
| Y | US 2021379181 A1 (CUREVAC AG) 09 December 2021 (2021-12-09) abstract, and sequence listing | 11 |
| Y | HE, Cai et al. "A recombinant spike-XBB.1.5 protein vaccine induces broad-spectrum immune responses against XBB.1.5-included Omicron variants of SARS-CoV-2" *MedComm*, Vol. (4), No. 3, 26 April 2023 (2023-04-26), e263 abstract, page 2, right-hand column, paragraph 2, and page 3, left-hand column, last paragraph | 6-7, 14 |
| A | ISLAM, M. A. et al. "XBB.1.5: A new threatening SARS-CoV-2 Omicron subvariant" *Frontiers in Microbiology*, 17 April 2023 (2023-04-17), pages 1-4 | 1-23 |

Form PCT/ISA/210 (second sheet) (July 2022)

## INTERNATIONAL SEARCH REPORT

| International application No. |
| --- |
| **PCT/CN2024/096427** |

**Box No. I    Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

   a.  ☑ forming part of the international application as filed.

   b.  ☐ furnished subsequent to the international filing date for the purposes of international search (Rule 13*ter*.1(a)),

       ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT
### Information on patent family members

International application No.

**PCT/CN2024/096427**

| Patent document cited in search report | | | Publication date (day/month/year) | Patent family member(s) | | | Publication date (day/month/year) |
|---|---|---|---|---|---|---|---|
| WO | 2023179513 | A1 | 28 September 2023 | None | | | |
| CN | 116836294 | A | 03 October 2023 | None | | | |
| CN | 114206946 | A | 18 March 2022 | JP | 2023530434 | A | 18 July 2023 |
| | | | | WO | 2021249116 | A1 | 16 December 2021 |
| | | | | WO | 2021249116 | A9 | 10 February 2022 |
| | | | | WO | 2021249451 | A1 | 16 December 2021 |
| | | | | US | 2023096093 | A1 | 30 March 2023 |
| | | | | US | 2022016235 | A1 | 20 January 2022 |
| | | | | US | 11389528 | B2 | 19 July 2022 |
| | | | | EP | 3947475 | A1 | 09 February 2022 |
| | | | | EP | 3947475 | A4 | 05 October 2022 |
| US | 2021379181 | A1 | 09 December 2021 | US | 11241493 | B2 | 08 February 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310663932
- CN 202311196430
- US 7268116 B
- US 7666837 B
- US 7691815 B
- US 10618949 B
- US 10906944 B
- US 10960070 B
- US 20200009244 A
- US 6060273 A
- US 20070116690 A
- US 6589940 B
- US 7255868 B
- US 7491706 B
- US 7479285 B
- US 7745598 B
- US 7785610 B
- US 8003115 B
- US 8133874 B
- US 8114418 B
- US 8222398 B
- US 8333980 B
- US 8597665 B
- US 8669237 B
- US 9028845 B
- US 10052378 B
- US 5643578 A
- US 5593972 A
- US 5817637 A
- US 5880103 A
- US 4722848 A
- US 5589466 A
- CN 2021087066 W
- CN 2021093895 W
- CN 2021099285 W

**Non-patent literature cited in the description**

- **KIRCHDOERFER et al.** Pre-fusion structure of a human coronavirus spike protein. *Nature*, 2016, vol. 531, 118-121
- **WU et al.** *Nature*, 2020, vol. 579, 265-269
- **HARBURY et al.** *Science*, 1993, vol. 262, 1401-1407
- **HOPPE et al.** *FEBS Lett*, 1994, vol. 344, 191-195
- **MCALINDEN et al.** *J Biol Chem*, 2003, vol. 278, 42200-42207
- **MIROSHNIKOV et al.** *Protein Eng*, 1998, vol. 11, 329-414
- **DE FELIPE**. *Genetic Vaccines and Ther.*, 2004, vol. 2, 13
- **DEFELIPE et al.** *Traffic*, 2004, vol. 5, 616-626
- **JIANG et al.** *J. Biol. Stand.*, 1986, vol. 14, 103-9
- **BRADLEY et al.** *J. Med. Virol.*, 1984, vol. 14, 373-86
- **JAMES et al.** *N. Engl. J. Med.*, 1995, vol. 332, 1262-6
- Remingtons Pharmaceutical Sciences. Mack Publishing Company, 1995
- **NEWMAN et al.** *Critical Reviews in Therapeutic Drug Carrier Systems*, 1998, vol. 15, 89-142
- **O'HAGAN et al.** The history of MF59® adjuvant: a phoenix that arose from the ashes. *Expert Review of Vaccines*, 2013
- **MOREL et al.** Adjuvant System AS03 containing α-tocopherol modulates innate immune response and leads to improved adaptive immunity. *Vaccine*, 2011
- **COFFMAN et al.** *Immunity*, 2010, vol. 33, 492-503
- **BRAUN et al.** *J Immunol*, 1988, vol. 141, 2084-2089
- **LATIMER et al.** *Mol Immunol*, 1995, vol. 32, 1057-1064
- **BODE et al.** CpG DNA as a vaccine adjuvant. *Expert Rev Vaccines*, 2011, vol. 10 (4), 499-511
- **SHAH et al.** *Methods Mol Biol*, 2017, vol. 1494, 1-14
- **PRAMANICK et al.** *Pharma Times*, 2013, vol. 45, 65-77
- **BAROUCH et al.** *J. Virol*, 2005, vol. 79, 8828-8834
- **STOVER**. *Nature*, 1991, vol. 351, 456-460
- **PETSCH et al.** Protective efficacy of in vitro synthesized, specific mRNA vaccines against influenza A virus infection. *Nature biotechnology*, 2012, vol. 30 (12), 1210-6

- **GEALL et al.** Nonviral delivery of self-amplifying RNA vaccines. *PNAS*, 2012, vol. 109 (36), 14604-14609
- **MAGINI et al.** Self-Amplifying mRNA Vaccines Expressing Multiple Conserved Influenza Antigens Confer Protection against Homologous and Heterosubtypic Viral Challenge. *PLoS One*, 2016, vol. 11 (8), e0161193
- **BRITO et al.** Self-amplifying mRNA vaccines. *Adv Genet.*, 2015, vol. 89, 179-233
- **BAKER et al.** *Biophys. J.*, 1991, vol. 60, 1445-1456
- **HAGENSEE et al.** *J. Virol.*, 1994, vol. 68, 4503-4505
- **LIANG et al.** *Nat. Comms.*, 2021, vol. 12, 1346
- **RICHMOND et al.** *Lancet*, 2021, vol. 397, 682-694
- **NIE J. et al.** *Quantification of SARS-CoV-2 neutralizing antibody by a pseudotyped virus based assay*